# EUROPEAN PATENT APPLICATION

(11) **EP 3 875 452 A1**
(43) Date of publication of application: **08.09.2021**
(21) Application number: 20161029.2
(22) Date of filing: 04.03.2020
(51) Int. Cl.: C07D 241/04, C07D 295/108, C07D 401/04, C07D 401/12, C07D 403/12, C07D 405/12, C07D 413/12, C07D 417/12, A61P 35/00, A61K 31/495

(54) **MONOACYLGLYCEROL LIPASE INHIBITORS**

(71) Applicant: Stichting Het Nederlands Kanker Instituut- Antoni van Leeuwenhoek Ziekenhuis, 1066 CX Amsterdam (NL); Universiteit Leiden, 2311 EZ Leiden (NL); Academisch Ziekenhuis Leiden h.o.d.n. LUMC, 2333 ZA Leiden (NL); Pivot Park Screening Centre B.V., 5349 AB Oss (NL)
(72) Inventor: VAN DER STELT, Marcelis, 2333 AB Leiden (NL); JIANG, Ming, 2333 BV Leiden (NL); MOHR, Florian David, 70839 Gerlingen (DE); VAN BOECKEL, Constant Adriaan Anton, 5345 LX Oss (NL); HUIZENGA, Mirjam Cornelia Willemina, 3021 GP Rotterdam (NL); AMEDI, Avand, 2547 KR Den Haag (NL)
(74) Representative: HGF

(57) **Abstract**

Provided are compounds of formula (I), or a pharmaceutically acceptable salt or solvate thereof: 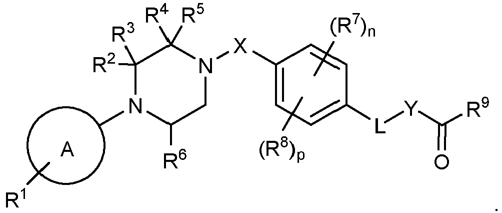 Also provided are compositions comprising compounds of formula (I). The compounds and compositions are also provided for use as medicaments, for example as medicaments useful in the treatment of a condition modulated by monoacylglycerol lipase (MAGL). Also provided are the use of compounds and compositions for the inhibition of monoacylglycerol lipase (MAGL).

## Description

This invention relates to compounds that are useful as inhibitors, in particular as inhibitors of monoacylglycerol lipase (MAGL).

### BACKGROUND

Cancers represent the second leading cause of death internationally. The rewiring of energy metabolism is of crucial importance to cancer cells. Deregulated metabolic pathways, resulting in a lipogenic phenotype, enable cancer cells to promote their survival, migration and invasion. Not only the *de novo* fatty acid synthesis, but also the hydrolytic generation of free fatty acids from stored (phospho)lipid precursors constitutes an important pathway of lipogenesis. Next to a metabolic role, these liberated fatty acids and their secondary metabolites (e.g. prostaglandins) serve also as oncogenic signalling purposes.

Monoacylglycerol lipase (MAGL) represents a key lipolytic enzyme in cancer cells that produces free fatty acids from lipid precursors. It has been suggested that inhibition of MAGL constitutes a novel therapeutic avenue to treat cancer via interfering with a fatty acid network that promotes cancer pathogenesis (DK Nomura et al. Monoacylglycerol Lipase Regulates a Fatty Acid Network that Promotes Cancer Pathogenesis. Cell. 2010, 140(1): 49 - 61; Pagano et al., Pharmacological inhibition of MAGL attenuates experimental colon carcinogenesis. Pharmacol Res., 2017, 5(119): 227-236). MAGL, a serine hydrolase, converts monoacylglycerols to free fatty acids, including arachidonic acid. MAGL is highly expressed in aggressive cancer cells and primary human tumors and its overexpression in nonaggressive tumor cells increases their pathogenicity. MAGL activity is co-opted by aggressive cancer cells and its elevated activity is associated with the translation of the lipogenic state of cancer cells into an array of protumorigenic signals, including LPA, LPC and PGE₂, that promote cancer cell migration, invasion and *in vivo* tumor growth (Nomura *et al.,* 2010, *supra*). Inhibition of MAGL activity, by RNA interference technology or pharmacological inhibition, resulted in a reduction of free fatty acid levels and impaired cancer pathogenicity in a multitude of *in vitro* and *in vivo* models (Nomura *et al.,* 2010, *supra*). MAGL may also be implicated in other conditions.

There is thus a general need to develop further treatments for cancer. There is also a need to identify (further) MAGL inhibitors. An object of the invention is therefore to provide compounds that are useful in the inhibition of MAGL. Another object of the invention is to provide compounds that are useful in the treatment of cancer. A further object of the invention is to provide compounds that are useful in the treatment of pathologies that are amenable to treatment with MAGL inhibitors.

### BRIEF SUMMARY OF THE DISCLOSURE

The invention provides compounds that are useful as inhibitors of monoacylglycerol lipase (MAGL).

A first aspect of the present invention provides a compound of formula (I), or a pharmaceutically acceptable salt or solvate thereof:

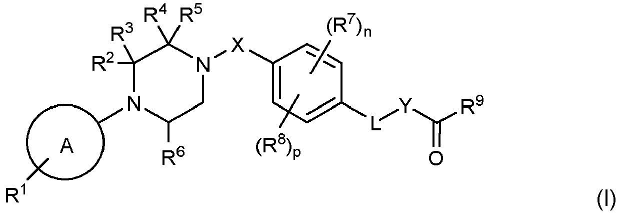

A is a 6-membered aromatic ring, or a 5- or 6-membered heteroaromatic ring optionally comprising at least one N atom. X is selected from -C(O)- and -C(R^{a}R^{b})-. L is selected from S(=O)-, -SO₂-, -S-, -O-, -C(O)-, -CH₂-, -C(R^{c}R^{d})-. Y is -CH₂-, -CF₂-, - C(R^{c}R^{d})-; R¹ is selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl. Each R² and R⁴ is independently selected from H and C₁₋₄ alkyl. each R³, R⁵ and R⁶ is independently selected from H and C₁₋₄ alkyl; or R³ and R⁵ together form a 5- or 6-membered ring and R⁶ is selected from H and C₁₋₄ alkyl; or R³ and R⁶ together form an alkyl or heteroalkyl bridge and R⁵ is selected from H and C₁₋₄ alkyl. Each R⁷ and R⁸ is independently selected from H, halo, C₁₋₄ alkyl, C₁₋₄ haloalkyl, C₁₋₄ alkoxy, CN and NO₂. R⁹ is selected from H, C₁₋₆ haloalkyl, OR¹⁰, C₁₋₆ alkyl, C₂₋₈ alkyl interrupted by 1, 2 or 3 ether linkages, C₃₋₈ cycloalkyl, C₆ aryl, -(C₂₋₄ haloalkyl)-OR¹¹, -(C₁₋₆ haloalkyl)-R¹¹, ,or heterocyclyl. R¹¹ is selected from C₁₋₆ alkyl, C₂₋₈ alkyl interrupted by 1, 2 or 3 ether linkages, C₃₋₈ cycloalkyl, C₆ aryl, or heterocyclyl. Each R⁹, R¹⁰ and R¹¹ is optionally substituted where chemically possible with one, two or three groups independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R^{a} and R^{b} are independently selected from H, and C₁₋₄ alkyl; or R^{a} and R^{b} together form a 3- to 6-membered cycloalkyl or heterocycloalkyl ring system. R^{c} and R^{d} are independently selected at each occurrence from H, F, C₁₋₄ alkyl, and C₁₋₄ haloalkyl; or R^{c} and R^{d} together form a 3- to 6-membered cycloalkyl or heterocycloalkyl ring system. a, b, d and e are independently selected from 0, 1 and 2. p is selected from 0, 1, or 2.

A second aspect of the invention provides a pharmaceutical composition or formulation comprising a compound of the invention. The pharmaceutical composition or formulation may comprise a pharmaceutically acceptable excipient.

A third aspect may comprise a compound of the invention, or a pharmaceutical composition or formulation of the invention for use as a medicament.

A fourth aspect provides a compound of the invention, or a pharmaceutical composition or formulation of the invention for use in the treatment of a condition modulated by MAGL.

A fifth aspect provides a compound of the invention, or a pharmaceutical composition or formulation of the invention for use in the treatment of a condition selected from acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

A sixth aspect provides use of a compound of the invention, or a pharmaceutical composition or formulation of the invention for the inhibition of monoacylglycerol lipase (MAGL).

A seventh aspect provides use of a compound of the invention in the manufacture of a medicament for use in the treatment of a condition selected from: acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

### DETAILED DESCRIPTION

The abbreviations used herein have their conventional meaning within the chemical and biological arts.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

For the avoidance of doubt, it is hereby stated that the information disclosed earlier in this specification under the heading "Background" is relevant to the invention and is to be read as part of the disclosure of the invention.

All publications, patent applications, patents, and other references mentioned herein are incorporated by reference in their entirety. In case of conflict, the present specification, including definitions, will control.

### DEFINITIONS

The following explanations of terms and methods are provided to better describe the present disclosure and to guide those of ordinary skill in the art in the practice of the present disclosure.

The invention concerns amongst other things the treatment of a disease. The term "treatment", and the therapies encompassed by this invention, include the following and combinations thereof: (1) hindering, e.g. delaying initiation and/or progression of, an event, state, disorder or condition, for example arresting, reducing or delaying the development of the event, state, disorder or condition, or a relapse thereof in case of maintenance treatment or secondary prophylaxis, or of at least one clinical or subclinical symptom thereof; (2) preventing or delaying the appearance of clinical symptoms of an event, state, disorder or condition developing in an animal (e.g. human) that may be afflicted with or predisposed to the state, disorder or condition but does not yet experience or display clinical or subclinical symptoms of the state, disorder or condition; and/or (3) relieving and/or curing an event, state, disorder or condition (*e.g*., causing regression of the event, state, disorder or condition or at least one of its clinical or subclinical symptoms, curing a patient or putting a patient into remission). The benefit to a patient to be treated may be either statistically significant or at least perceptible to the patient or to the physician. It will be understood that a medicament will not necessarily produce a clinical effect in each patient to whom it is administered; thus, in any individual patient or even in a particular patient population, a treatment may fail or be successful only in part, and the meanings of the terms "treatment", "prophylaxis" and "inhibitor" and of cognate terms are to be understood accordingly. The compositions and methods described herein are of use for therapy and/or prophylaxis of the mentioned conditions.

The term "prophylaxis" includes reference to treatment therapies for the purpose of preserving health or inhibiting or delaying the initiation and/or progression of an event, state, disorder or condition, for example for the purpose of reducing the chance of an event, state, disorder or condition occurring. The outcome of the prophylaxis may be, for example, preservation of health or delaying the initiation and/or progression of an event, state, disorder or condition. It will be recalled that, in any individual patient or even in a particular patient population, a treatment may fail, and this paragraph is to be understood accordingly.

The term "inhibit" (and "inhibiting") includes reference to delaying, stopping, reducing the incidence of, reducing the risk of and/or reducing the severity of an event, state, disorder or condition. Inhibiting an event, state, disorder or condition may therefore include delaying or stopping initiation and/or progression of such, and reducing the risk of such occurring. The products of the disclosure may be used to inhibit one or more proteases and thereby inhibit osteoporosis and/or other events, disorders and/or conditions which are disclosed herein. For example, the compounds of the invention may inhibit MAGL.

An "inhibitor" is a molecule that binds to an enzyme and decreases its activity. An "irreversible inhibitor" is an inhibitor where the binding involves a chemical reaction, e.g. formation of a covalent bond between the molecule and enzyme. The compounds of the invention may act as inhibitors of MAGL.

The term "alkyl" as used herein include reference to a straight or branched chain alkyl moiety having 1, 2, 3, 4, 5, 6, 7, 8, 9 or 10 carbon atoms. The term includes reference to, for example, methyl, ethyl, propyl (n-propyl or isopropyl), butyl (n-butyl, sec-butyl or tert-butyl), pentyl, hexyl and the like. In particular, alkyl may be a "C₁-C₄ alkyl", i.e. an alkyl having 1, 2, 3 or 4 carbon atoms; or a "C₁-C₆ alkyl", i.e. an alkyl having 1, 2, 3, 4, 5 or 6 carbon atoms; or a "C₁-C₃ alkyl", i.e. an alkyl having 1, 2 or 3 carbon atoms. The term "lower alkyl" includes reference to alkyl groups having 1, 2, 3 or 4 carbon atoms.

The term "cycloalkyl" as used herein includes reference to an alicyclic moiety having 3, 4, 5 or 6 carbon atoms. The group may be a bridged or polycyclic ring system. More often cycloalkyl groups are monocyclic. This term includes reference to groups such as cyclopropyl, cyclobutyl, cyclopentyl, cyclohexyl and the like.

The term "heterocycloalkyl" as used herein includes reference to a saturated heterocyclic moiety having 3, 4, 5, 6 or 7 ring carbon atoms and 1, 2, 3, 4 or 5 ring heteroatoms selected from nitrogen, oxygen, phosphorus and sulphur. For example, a heterocycolalkyl may comprise 3, 4, or 5 ring carbon atoms and 1 or 2 ring heteroatoms selected from nitrogen and oxygen. The group may be a polycyclic ring system but more often is monocyclic. This term includes reference to groups such as azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolizidinyl and the like.

The terms "halo" or "halogen" as used herein includes reference to F, Cl, Br or I, for example F, Cl or Br. In a particular class of embodiments, halogen is F or Cl, of which F is more common.

The term "haloalkyl" refers to an alkyl group where one or more hydrogen atoms are substituted by a corresponding number of halogens. An exemplary haloalkyl group is trifluoromethyl. Another exemplary haloalkyl group is 2-fluoro-2-methylpropyl.

The term "alkoxy" as used herein include reference to -O-alkyl, wherein alkyl is straight or branched chain and comprises 1, 2, 3, 4, 5 or 6 carbon atoms. In one class of embodiments, alkoxy has 1, 2, 3 or 4 carbon atoms, e.g. 1, 2 or 3 carbon atoms. This term includes reference to, for example, methoxy, ethoxy, propoxy, isopropoxy, butoxy, tert-butoxy, pentoxy, hexoxy and the like. The term "lower alkoxy" includes reference to alkoxy groups having 1, 2, 3 or 4 carbon atoms.

The term "haloalkoxy" as used herein refers to an alkoxy group where one or more hydrogen atoms are substituted by a corresponding number of halogens.

The term "substituted" as used herein in reference to a moiety means that one or more, especially up to 5, more especially 1, 2 or 3, of the hydrogen atoms in said moiety are replaced independently of each other by the corresponding number of the described substituents. Unless otherwise specified, exemplary substituents include -OH, -CN, -NH₂, =O, -halo, -C₁-C₆ alkyl, -C₂-C₆ alkenyl, -C₁-C₆ haloalkyl, -C₁-C₆ haloalkoxy and-C₂-C₆ haloalkenyl, -C₁-C₆ alkylcarboxylic acid (e.g. -CH₃COOH or -COOH). Where the substituent is a -C₁-C₆ alkyl or -C₁-C₆ haloalkyl, the C₁-C₆ chain is optionally interrupted by an ether linkage (-O-) or an ester linkage (-C(O)O-). Exemplary substituents for a substituted alkyl may include -OH, -CN, -NH₂, =O, -halo, -CO₂H, -C₁-C₆ haloalkyl, -C₁-C₆ haloalkoxy and-C₂-C₆haloalkenyl, -C₁-C₆ alkylcarboxylic acid (e.g. -CH₃COOH or -COOH). For example, exemplary substituents for an alkyl may include -OH, -CN, -NH₂, =O, -halo.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

Where steric issues determine placement of substituents on a group, the isomer having the lowest conformational energy may be preferred.

Where a compound, moiety, process or product is described as "optionally" having a feature, the disclosure includes such a compound, moiety, process or product having that feature and also such a compound, moiety, process or product not having that feature. Thus, when a moiety is described as "optionally substituted", the disclosure comprises the unsubstituted moiety and the substituted moiety.

Where two or more moieties are described as being "independently" or "each independently" selected from a list of atoms or groups, this means that the moieties may be the same or different. The identity of each moiety is therefore independent of the identities of the one or more other moieties.

The term "pharmaceutically acceptable" as used herein includes reference to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio. This term includes acceptability for both human and veterinary purposes.

The term "pharmaceutical formulation" as used herein includes reference to a formulation comprising at least one active compound and optionally one or more additional pharmaceutically acceptable ingredients, for example a pharmaceutically acceptable carrier. Where a pharmaceutical formulation comprises two or more active compounds, or comprises at least one active compound and one or more additional pharmaceutically acceptable ingredients, the pharmaceutical formulation is also a pharmaceutical composition. Unless the context indicates otherwise, all references to a "formulation" herein are references to a pharmaceutical formulation.

The term "product" or "product of the invention" as used herein includes reference to any product containing a compound of the present invention. In particular, the term product relates to compositions and formulations containing a compound of the present invention, such as a pharmaceutical composition, for example.

The term "therapeutically effective amount" as used herein refers to an amount of a drug, or pharmaceutical agent that, within the scope of sound pharmacological judgment, is calculated to (or will) provide a desired therapeutic response in a mammal (animal or human). The therapeutic response may for example serve to cure, delay the progression of or prevent a disease, disorder or condition.

The term "prodrug" as used herein represents compounds which are transformed *in vivo* to the parent compound, for example, by hydrolysis in blood. An example of such a prodrug is a pharmaceutically acceptable ester of a carboxylic acid. A thorough discussion is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987; H Bundgaard, ed, Design of Prodrugs, Elsevier, 1985; and Judkins, et al. Synthetic Communications, 26(23), 4351-4367 (1996); and The organic chemistry of drug design and drug action by Richard B Silverman in particular pages 497 to 546; each of which is incorporated herein by reference.

The term "pharmaceutically acceptable salts" is meant to include salts of the active compounds which are prepared with relatively nontoxic acids or bases, depending on the particular substituents found on the compounds described herein. When compounds contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge etal., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

Thus, the compounds of the present disclosure may exist as salts with pharmaceutically acceptable acids. The present invention includes such salts. Examples of such salts include hydrochlorides, hydrobromides, sulfates, methanesulfonates, nitrates, maleates, acetates, citrates, fumarates, tartrates (e.g. (+)-tartrates, (-)-tartrates or mixtures thereof including racemic mixtures, succinates, benzoates and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

In addition to salt forms, the present invention provides compounds, which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centres) or double bonds; the racemates, diastereomers, tautomers, geometric isomers and individual isomers are encompassed within the scope of the present invention. The compounds of the present invention do not include those which are known in the art to be too unstable to synthesize and/or isolate.

The compounds of the present invention may also contain unnatural proportions of atomic isotopes at one or more of the atoms that constitute such compounds. For example, the compounds may be radiolabelled with radioactive isotopes, such as for example tritium (³H), iodine-125 (¹²⁵I) or carbon-14 (¹⁴C). All isotopic variations of the compounds of the present invention, whether radioactive or not, are encompassed within the scope of the present invention.

### COMPOUNDS

In one aspect, the invention provides compounds of formula (I) as previously described or a pharmaceutically acceptable salt, stereoisomer, or prodrug thereof.

In embodiments, the compound is a compound of formula (II) or a pharmaceutically acceptable salt or solvate thereof: 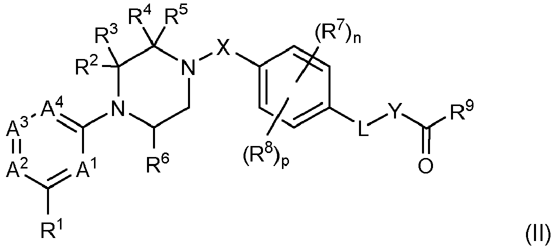 L, X, Y, R¹, R², R³, R⁴, R⁵, R⁶, R⁷, R⁸, R⁹, n and p are as defined herein. A¹, A³ and A⁴ are each independently selected from CH, CR¹² or N. A² is selected from CH or CR¹². Each R¹² is independently selected from H, halo, CN, OCF₃, C₁₋₄ alkyl, C₁₋₄ haloalkyl, and C₁₋₄ alkoxy.

In embodiments, the compound is a compound of formula (III) or a pharmaceutically acceptable salt or solvate thereof: 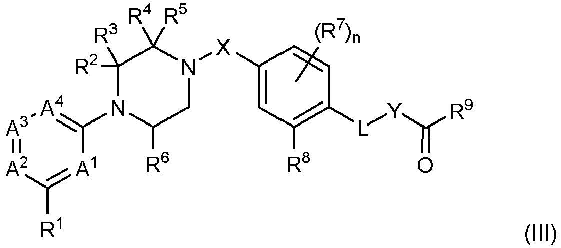 A¹, A², A³, A⁴, L, X, Y, R¹, R², R³, R⁴, R⁵, R⁶, R⁷, R⁸, R⁹, n and p are as defined herein.

In embodiments, the compound is a compound of formula (IVa) or a pharmaceutically acceptable salt or solvate thereof: 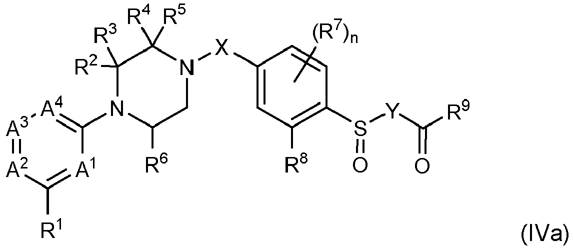 A¹, A², A³, A⁴, X, Y, R¹, R², R³, R⁴, R⁵, R⁶, R⁷, R⁸, R⁹ and n are as defined herein.

In embodiments, the compound is a compound of formula (IVb) or a pharmaceutically acceptable salt or solvate thereof: 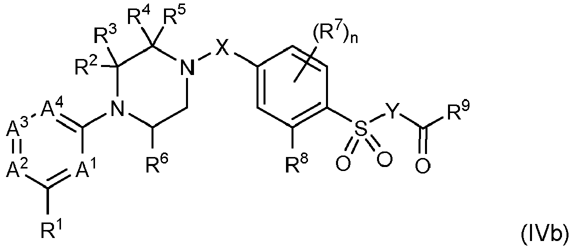 A¹, A², A³, A⁴, X, Y, R¹, R², R³, R⁴, R⁵, R⁶, R⁷, R⁸, R⁹ and n are as defined herein.

In embodiments, the compound is a compound of formula (IVc) or a pharmaceutically acceptable salt or solvate thereof: 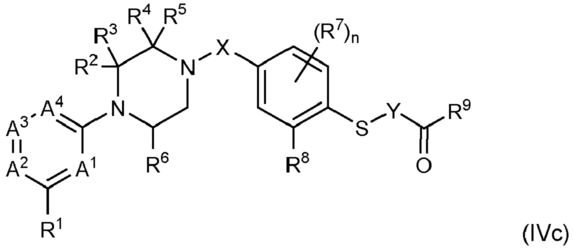 A¹, A², A³, A⁴, X, Y, R¹, R², R³, R⁴, R⁵, R⁶, R⁷, R⁸, R⁹ and n are as defined herein.

In embodiments, the compound is a compound of formula (V) or a pharmaceutically acceptable salt or solvate thereof: 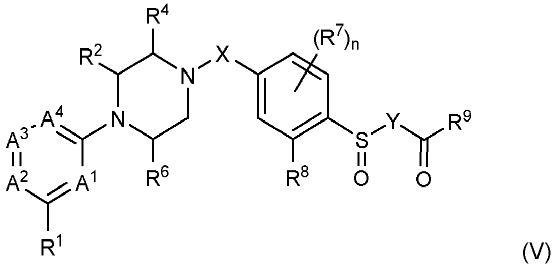 A¹, A², A³, A⁴, X, Y, R¹, R², R⁴, R⁶, R⁷, R⁸, R⁹ and n are as defined herein. R² and R⁴ may be arranged *trans* to one another.

In embodiments, one or more of A, A¹, A², A³ and A⁴, L, X, Y, R¹, R², R³, R⁴, R⁵, R⁶, R⁷, R⁸, R⁹, R¹⁰, R¹¹, R¹², R^{a}, R^{b}, R^{c}, R^{d}, a, b, d, e, n and p are as described in the following paragraphs:

A may be a 6-membered aromatic ring, or a 5- or 6-membered heteroaromatic ring comprising at least one N atom A may be a 6-membered aromatic ring. A may be a 5- or 6-membered heteroaromatic ring. A may be a 5- or 6-membered heteroaromatic ring comprising at least one N atom. A may be a 5-membered heteroaromatic ring comprising at least one N atom. A may be a 6-membered heteroaromatic ring comprising at least one N atom. A may be selected from an optionally substituted phenyl, pyridyl or pyrimidyl. A may be an optionally substituted phenyl. A may be an optionally substituted pyridyl. A may be an optionally substituted pyrimidyl.

A¹, A³ and A⁴ may each be independently selected from CH, CR¹² or N. A² may be selected from CH or CR¹². Any one, two or three of A¹, A³ and A⁴ may be N. Any one of A¹, A³ and A⁴ may be N. Any two of A¹, A³ and A⁴ may be N. In an embodiment, when one of A¹ and A⁴ is N, the other of A¹ and A⁴ is CH or CR¹², and A³ is CH or CR¹². In an embodiment, each of A¹ and A⁴ is N, and A³ is CH or CR¹². Each of each of A¹, A³ and A⁴ may be independently selected from CH or CR¹².

A¹, A³ and A⁴ may be selected such that the 6 membered ring to which they belong is selected from a substituted or unsubstituted phenyl, substituted or unsubstituted pyridyl, or substituted or unsubstituted pyrimidyl. A¹, A³ and A⁴ may be selected such that the 6 membered ring to which they belong is a substituted or unsubstituted phenyl. A¹, A³ and A⁴ may be selected such that the 6 membered ring to which they belong is a substituted or unsubstituted pyridyl. A¹, A³ and A⁴ may be selected such that the 6 membered ring to which they belong is a substituted or unsubstituted pyrimidyl.

A² may be H. A² may be CR¹².

X may be -C(O)-, -C(R^{a}R^{b})-, or -CH₂-. X may be -C(O)- or -CH₂-. X may be -C(O)-X may be -C(R^{a}R^{b})-. X may be -CH₂-.

R^{a} and R^{b} may be independently selected from H, and C₁₋₄ alkyl. R^{a} may be H and R^{b} may be C₁₋₄ alkyl. R^{a} and R^{b} may be H. R^{a} and R^{b} may together form a 3- to 6-membered (e.g. a 3 to 4-membered) cycloalkyl or heterocycloalkyl ring system. R^{a} and R^{b} may together form a 3- to 6-membered (e.g. a 3 to 4-membered) cycloalkyl ring system. R^{a} and R^{b} may together form a 3- to 6-membered heterocycloalkyl ring system.

Y may be -CH₂-, -CF₂-, -C(R^{c}R^{d})-. Y may be -CH₂-, -CF₂-, -CH(CH₃)-, 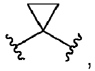 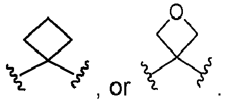 Y may be -CH₂-, -CF₂-, -CH(CH₃)-, 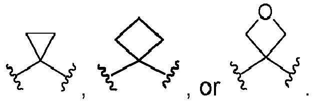 Y may be -CH₂-. Y may be -CF₂-. Y may be -CH(CH₃)-,

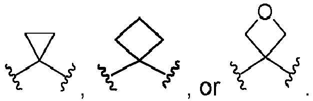

L may be selected from -S(=O)-, -SO₂-, -S-, -O-, -C(O)-, -CH₂-, -C(R^{c}R^{d})-. L may be selected from -S(=O)-, -SO₂- and -S-. L may be -S(=O)-. L may be -SO₂-. L may be -S-. L may be -C(R^{c}R^{d})-.

R^{c} and R^{d} may be independently selected at each occurrence from H, F, C₁₋₄ alkyl, and C₁₋₄ haloalkyl. R^{c} and R^{d} may be independently selected at each occurrence from H, F and C₁₋₄ alkyl. R^{c} and R^{d} may be independently selected at each occurrence from H and F. R^{c} and R^{d} may together form a 3- to 6-membered (e.g. 3- to 4-membered) cycloalkyl or heterocycloalkyl ring system. R^{c} and R^{d} may together form a 3- to 6-membered (e.g. 3- to 4-membered) cycloalkyl ring system. R^{c} and R^{d} may together form a 3- to 6-membered (e.g. 3- to 4-membered) heterocycloalkyl ring system.

R¹ may be selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl. R¹ may be selected from H, halo, C₁₋₄ alkyl, C₁₋₄ haloalkyl, or O-(C₁₋₄ alkyl)ₐ-aryl. R¹ may be selected from halo, C₁₋₄ alkyl, C₁₋₄ haloalkyl, or O-(C₁₋₄ alkyl)ₐ-aryl. R¹ may be halo (for example F, Cl or Br; e.g. Cl or Br). R¹ may be C₁₋₄ alkyl (e.g. methyl). R¹ may be , C₁₋₄ haloalkyl (e.g. CF₃). R¹ may be O-(C₁₋₄ alkyl)ₐ-aryl (e.g. O-Bn). R¹ may be O-(C₁₋₄ alkyl)ₐ-aryl. R¹ may be H. a may be 1 or 2. a may be 1. b may be 1 or 2. b may be 1.

Each R² and R⁴ may be independently selected from H and C₁₋₄ alkyl (e.g. CH₃). R² may be H. R² may be C₁₋₄ alkyl (e.g. CH₃). R⁴ may be H. R⁴ may be C₁₋₄ alkyl (e.g. CH₃). R² may be H and R⁴ may be C₁₋₄ alkyl (e.g. CH₃). R⁴ may be H and R² may be C₁₋₄ alkyl (e.g. CH₃). R² and R⁴ may both be C₁₋₄ alkyl (e.g. CH₃). R² and R⁴ may be arranged *trans* to one another.

Each R³, R⁵ and R⁶ may be independently selected from H and C₁₋₄ alkyl. Each R³ and R⁵ may together form a 5- or 6-membered ring and R⁶ may be selected from H and C₁₋₄ alkyl. R³ and R⁶ may together form an alkyl or heteroalkyl bridge and R⁵ may be selected from H and C₁₋₄ alkyl. Each R³, R⁵ and R⁶ may be independently selected from H and CH₃. Each R³ and R⁵ may together form a 5- or 6-membered ring and R⁶ may be selected from H and CH₃. R³ and R⁶ may together form an alkyl or heteroalkyl bridge and R⁵ may be selected from H and CH₃.

Each R⁷ and R⁸ may be independently selected from H, halo, C₁₋₄ alkyl, C₁₋₄ haloalkyl, C₁₋₄ alkoxy, CN and NO₂. Each R⁷ and R⁸ may be independently selected from H, halo, C₁₋₄ haloalkyl, and NO₂. Each R⁷ and R⁸ may be independently selected from halo, C₁₋₄ haloalkyl, and NO₂. Each R⁷ and R⁸ may be independently selected from halo (e.g. F, Cl, or Br), C₁₋₄ haloalkyl (e.g. CF₃), and NO₂. n may be 0 or 1. n may be 0. n may be 1. p may be 0 or 1. p may be 1 or 2. p may be 1. p may be 0. p may be 2. n may be 0 or 1 and p may be 1.

R⁹ may be selected from H, C₁₋₆ haloalkyl, OR¹⁰, C₁₋₆ alkyl, C₂₋₈ alkyl interrupted by 1, 2 or 3 ether linkages, C₃₋₈ cycloalkyl, C₆ aryl, -(C₂₋₄ haloalkyl)-OR¹¹, -(C₁₋₆ haloalkyl)-R¹¹, ,or heterocyclyl. R⁹ may be selected from C₁₋₄ haloalkyl, C₂₋₆ alkyl interrupted by 1, 2 or 3 ether linkages, OR¹⁰, -(C₂₋₄ haloalkyl)-OR¹¹, (C₁₋₆ haloalkyl)-R¹¹, substituted or unsubstituted 6-membered aryl, and substituted or unsubstituted 5- or 6-membered heterocyclyl. R⁹ may be optionally substituted where chemically possible with one, two or three groups (e.g. one group) independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R⁹ may be substituted where chemically possible with one, two or three groups (e.g. one group) each independently selected from H, OH, =O, CN, halo, C₁₋₄ haloalkyl, C₁₋₄ alkoxy, OCF₃, and phenyl. R⁹ may be unsubstituted.

R⁹ may be selected from -CF₂CH₃, -CF₂CH₂CH₃, -CF₃, -CHF₂, -CF₂-phenyl, - OCH₂CH₃, -OCH(CH₃)₂, -OCH(CH₃)CH₂CH₃, -CF₂CH₂O-fluorophenyl, -CF₂CH₂O-oxetane, - CF₂CH₂OCH₂CH₂OCH₃, -CF₂CH₂-pyrimidyl, -CF₂-oxetane, -OCH₂CH(OH)CH₂OH, - OCH₂CH₂OCH₃, -OCH(CH₃)CH₂OCH₃, -OCH₂CH₂CH₂CH₃, -OCH₂CH₂CH₂OH, - OCH₂CH₂OCH₂CH₂OCH₂CH₃, -OCH₂CF₃, -OCH₃, -OC(CH₃)₂CH₂CH₃, -OC(CH₃)₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, oxazol-2-yl, thiazol-2-yl, 5-methyloxazol-2-yl, pyridazin-3-yl, 6-(trifluoromethyl)pyridin-3-yl, pyridazin-4-yl, pyridin-4-yl, pyrazin-2-yl, pyridin-3-yl, phenyl, 4-cyanophenyl, 2,3-difluorophenyl, 3,4-difluorophenyl, 2,4-difluorophenyl, 2-fluorophenyl, 4-fluorophenyl, 3,5-difluorophenyl, or 4-(trifluoromethyl)phenyl.

R⁹ may be selected from -CF₂CH₃, -CF₂CH₂CH₃, -CF₃, -CHF₂, -CF₂-phenyl, - OCH₂CH₃, -OCH(CH₃)₂, -OCH(CH₃)CH₂CH₃, , -OCH₂CH(OH)CH₂OH, -OCH₂CH₂OCH₃, - OCH(CH₃)CH₂OCH₃, -OCH₂CH₂CH₂CH₃, -OCH₂CH₂CH₂OH, - OCH₂CH₂OCH₂CH₂OCH₂CH₃, -OCH₂CF₃, -OCH₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, 4-cyanophenyl,

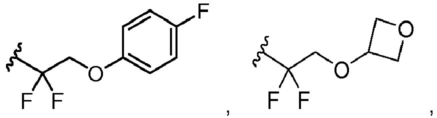

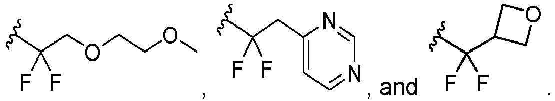

R⁹ may be selected from -CF₂CH₃, -CF₂CH₂CH₃, -CF₃, -CHF₂, -CF₂-phenyl, - OCH₂CH₃, -OCH(CH₃)₂, -OCH(CH₃)CH₂CH₃, , -OCH₂CH(OH)CH₂OH, -OCH₂CH₂OCH₃, - OCH(CH₃)CH₂OCH₃, -OCH₂CH₂CH₂CH₃, -OCH₂CH₂CH₂OH, - OCH₂CH₂OCH₂CH₂OCH₂CH₃, -OCH₂CF₃, -OCH₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, and 4-cyanophenyl.

R¹⁰ may be selected from H, C₁₋₆ alkyl, C₃₋₈ cycloalkyl, -(C₁₋₄ alkyl)_{d}-aryl, -(C₁₋₄ alkyl)ₑ-heterocyclyl. R¹⁰ may be selected from C₁₋₄ alkyl, C₃₋₈ cycloalkyl or -(C₁₋₄ alkyl)-aryl. d may be 1 or 2. d may be 1. e may be 1 or 2. e may be 1. R¹⁰ may be optionally substituted where chemically possible with one, two or three groups (e.g. one group) independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R¹⁰ may be substituted where chemically possible with one, two or three groups (e.g. one group) each independently selected from H, OH, =O, CN, halo, C₁₋₄ haloalkyl, C₁₋₄ alkoxy, OCF₃, and phenyl. R¹⁰ may be unsubstituted.

R¹¹ may be selected from C₁₋₆ alkyl, C₂₋₈ alkyl interrupted by 1, 2 or 3 ether linkages, C₃₋₈ cycloalkyl, C₆ aryl, or heterocyclyl. R¹¹ may be selected from C₁₋₄ alkyl, C₂₋₆ alkyl interrupted by 1, 2 or 3 ether linkages, C₃₋₆ cycloalkyl, or C₃₋₆ heterocycloalkyl. R¹¹ may be optionally substituted where chemically possible with one, two or three groups (e.g. one group) independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R¹¹ may be substituted where chemically possible with one, two or three groups (e.g. one group) each independently selected from H, OH, =O, CN, halo, C₁₋₄ haloalkyl, C₁₋₄ alkoxy, OCF₃, and phenyl. R¹⁰ may be unsubstituted.

Each R¹² may be independently selected from H, halo, CN, OCF₃, C₁₋₄ alkyl, C₁₋₄ haloalkyl, and C₁₋₄ alkoxy. Each R¹² may be H. One R¹² may be selected from halo, CN, OCF₃, C₁₋₄ alkyl, C₁₋₄ haloalkyl, and C₁₋₄ alkoxy; and each remaining R¹² (if any) may be H.

In an embodiment, the compound may be selected from the following compounds, or a pharmaceutically acceptable salt, stereoisomer, or prodrug thereof: Ethyl 2-((4-((S)-3-methyl-4-(m-tolyl)piperazine-1-carbonyl)-2-nitrophenyl)sulfinyl)acetate, Ethyl 2-((4-((R)-3-methyl-4-(m-tolyl)piperazine-1-carbonyl)-2-nitrophenyl)sulfinyl)acetate, Ethyl 2-((2-nitro-4-(4-(m-tolyl)piperazine-1-carbonyl)phenyl)sulfinyl)acetate, Ethyl (R)-2-((4-(3-methyl-4-(m-tolyl)piperazine-1-carbonyl)-2-nitrophenyl)thio)acetate, Ethyl 2-((4-((R)-3-methyl-4-(m-tolyl)piperazine-1-carbonyl)-2-nitrophenyl)sulfonyl)acetate, Ethyl 2-((4-((S)-3-methyl-4-(m-tolyl)piperazine-1-carbonyl)phenyl)sulfinyl)acetate, Ethyl 2-((2-nitro-4-(4-phenylpiperazine-1-carbonyl)phenyl)sulfinyl)acetate, Ethyl 2-((4-(4-(3-fluorophenyl)piperazine-1-carbonyl)-2-nitrophenyl)sulfinyl)acetate, Ethyl 2-((4-(4-(3-chlorophenyl)piperazine-1-carbonyl)-2-nitrophenyl)sulfinyl)acetate, Ethyl 2-((4-(4-(4-chlorophenyl)piperazine-1-carbonyl)-2-nitrophenyl)sulfinyl)acetate, Ethyl 2-((4-(4-(2-chlorophenyl)piperazine-1-carbonyl)-2-nitrophenyl)sulfinyl)acetate, Ethyl 2-((4-(4-(3-bromophenyl)piperazine-1-carbonyl)-2-nitrophenyl)sulfinyl)acetate, Ethyl 2-((4-(4-(4-bromophenyl)piperazine-1-carbonyl)-2-nitrophenyl)sulfinyl)acetate, Ethyl 2-((2-nitro-4-(4-(3-(trifluoromethyl)phenyl)piperazine-1-carbonyl)phenyl)sulfinyl)acetate, Ethyl 2-((2-nitro-4-(4-(4-(trifluoromethyl)phenyl)piperazine-1-carbonyl)phenyl)sulfinyl)acetate, Ethyl 2-((4-(4-(3-methoxyphenyl)piperazine-1-carbonyl)-2-nitrophenyl)sulfinyl)acetate, Ethyl 2-((4-(4-(4-methoxyphenyl)piperazine-1-carbonyl)-2-nitrophenyl)sulfinyl)acetate, Ethyl 2-((2-nitro-4-(4-(3-nitrophenyl)piperazine-1-carbonyl)phenyl)sulfinyl)acetate, Ethyl 2-((4-(4-([1,1'-biphenyl]-3-yl)piperazine-1-carbonyl)-2-nitrophenyl)sulfinyl)acetate, Ethyl 2-((4-(4-(3,5-dichlorophenyl)piperazine-1-carbonyl)-2-nitrophenyl)sulfinyl)acetate, Ethyl 2-((4-(4-(3,4-dichlorophenyl)piperazine-1-carbonyl)-2-nitrophenyl)sulfinyl)acetate, Ethyl 2-((4-(4-(2,4-dichlorophenyl)piperazine-1-carbonyl)-2-nitrophenyl)sulfinyl)acetate, Ethyl 2-((4-(4-(2,6-dichlorophenyl)piperazine-1-carbonyl)-2-nitrophenyl)sulfinyl)acetate, Ethyl 2-((4-((S)-4-(3-fluorophenyl)-3-methylpiperazine-1-carbonyl)-2-nitrophenyl)sulfinyl)acetate, Ethyl 2-((4-((R)-4-(3-chlorophenyl)-3-methylpiperazine-1-carbonyl)-2-nitrophenyl)sulfinyl)acetate, Ethyl 2-((4-((S)-4-(3-chlorophenyl)-3-methylpiperazine-1-carbonyl)-2-nitrophenyl)sulfinyl)acetate, Ethyl 2-((4-((R)-4-(3-bromophenyl)-3-methylpiperazine-1-carbonyl)-2-nitrophenyl)sulfinyl)acetate, Ethyl 2-((4-((S)-4-(3-bromophenyl)-3-methylpiperazine-1-carbonyl)-2-nitrophenyl)sulfinyl)acetate, Ethyl 2-((4-((R)-3-methyl-4-(3-(trifluoromethyl)phenyl)piperazine-1-carbonyl)-2-nitrophenyl)sulfinyl)acetate, Ethyl 2-((4-((S)-3-methyl-4-(3-(trifluoromethyl)phenyl)piperazine-1-carbonyl)-2-nitrophenyl)sulfinyl)acetate, Methyl 2-((4-((R)-4-(3-chlorophenyl)-3-methylpiperazine-1-carbonyl)-2-nitrophenyl)sulfinyl)acetate, 2-((4-((R)-4-(3-Chlorophenyl)-3-methylpiperazine-1-carbonyl)-2-nitrophenyl)sulfinyl)-N-ethyl-N-methylacetamide, Isopropyl 2-((4-((R)-4-(3-chlorophenyl)-3-methylpiperazine-1-carbonyl)-2-nitrophenyl)sulfinyl)acetate, 2,2,2-Trifluoroethyl 2-((4-((R)-4-(3-chlorophenyl)-3-methylpiperazine-1-carbonyl)-2-nitrophenyl)sulfinyl)acetate, Propyl 2-((4-((R)-4-(3-chlorophenyl)-3-methylpiperazine-1-carbonyl)-2-nitrophenyl)sulfinyl)acetate, Butyl 2-((4-((R)-4-(3-chlorophenyl)-3-methylpiperazine-1-carbonyl)-2-nitrophenyl)sulfinyl)acetate, sec-Butyl 2-((4-((R)-4-(3-chlorophenyl)-3-methylpiperazine-1-carbonyl)-2-nitrophenyl)sulfinyl)acetate, tert-Pentyl 2-((4-((R)-4-(3-chlorophenyl)-3-methylpiperazine-1-carbonyl)-2-nitrophenyl)sulfinyl)acetate, tert-Butyl 2-((4-((R)-4-(3-chlorophenyl)-3-methylpiperazine-1-carbonyl)-2-nitrophenyl)sulfinyl)acetate, 3-Hydroxypropyl 2-((4-((R)-4-(3-chlorophenyl)-3-methylpiperazine-1-carbonyl)-2-nitrophenyl)sulfinyl)acetate, Ethyl 2-((4-(4-(3-chlorophenyl)-3,5-dimethylpiperazine-1-carbonyl)-2-nitrophenyl)sulfinyl)acetate, Ethyl 2-((4-((R)-4-(3-chlorophenyl)-3-methylpiperazine-1-carbonyl)-2-fluorophenyl)sulfinyl)acetate, Ethyl 2-((4-(4-(3-chlorophenyl)-2-methylpiperazine-1-carbonyl)-2-fluorophenyl)sulfinyl)acetate, (±) Ethyl 2-((4-(4-(3-chlorophenyl)-cis-2,3-dimethylpiperazine-1-carbonyl)-2-fluorophenyl)sulfinyl)acetate, (±) Ethyl 2-((4-(4-(3-chlorophenyl)-trans-2,3-dimethylpiperazine-1-carbonyl)-2-fluorophenyl)sulfinyl)acetate, (±) Ethyl 2-((4-(4-(3-chlorophenyl)-3,3-dimethylpiperazine-1-carbonyl)-2-fluorophenyl)sulfinyl)acetate, (±) Ethyl 2-((4-(4-(3-chlorophenyl)-2,2-dimethylpiperazine-1-carbonyl)-2-fluorophenyl)sulfinyl)acetate, (±) Ethyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(3-chlorophenyl)-trans-2,3-dimethylpiperazine-1-carbonyl)phenyl)sulfinyl)acetate, (±) 2-((2-Chloro-4-(4-(3-chlorophenyl)-trans-2,3-dimethylpiperazine-1-carbonyl)phenyl)sulfinyl)acetic acid, (±) Ethyl 2-((2-chloro-4-(4-(3-chlorophenyl)-2,3-dimethylpiperazine-1-carbonyl)phenyl)thio)acetate, (±) Ethyl 2-((2-chloro-4-(4-(3-chlorophenyl)-trans-2,3-dimethylpiperazine-1-carbonyl)phenyl)sulfonyl)acetate, (±) Ethyl 2-((2-chloro-4-((4-(3-chlorophenyl)-trans-2,3-dimethylpiperazin-1-yl)methyl)phenyl)sulfinyl)acetate, (±) Ethyl 3-(2-chloro-4-(4-(3-chlorophenyl)-trans-2,3-dimethylpiperazine-1-carbonyl)phenyl)-3-oxopropanoate, (±) Isopropyl 2-((2-chloro-4-(4-(3-chlorophenyl)-trans-2,3-dimethylpiperazine-1-carbonyl)phenyl)sulfinyl)acetate, (±) sec-Butyl 2-((2-chloro-4-(4-(3-chlorophenyl)-trans-2,3-dimethylpiperazine-1-carbonyl)phenyl)sulfinyl)acetate, (±) Cyclobutyl 2-((2-chloro-4-(4-(3-chlorophenyl)-trans-2,3-dimethylpiperazine-1-carbonyl)phenyl)sulfinyl)acetate, (±) Cyclopentyl 2-((2-chloro-4-(4-(3-chlorophenyl)-trans-2,3-dimethylpiperazine-1-carbonyl)phenyl)sulfinyl)acetate, (±) Cyclohexyl 2-((2-chloro-4-(4-(3-chlorophenyl)-trans-2,3-dimethylpiperazine-1-carbonyl)phenyl)sulfinyl)acetate, (±) 2,3-Dihydroxypropyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(3-chlorophenyl)-trans-2,3-dimethylpiperazine-1-carbonyl)phenyl)sulfinyl)propanoate, (±) sec-Butyl 2-((2-chloro-4-(4-(3-chlorophenyl)-trans-2,3-dimethylpiperazine-1-carbonyl)phenyl)sulfinyl)propanoate, (±) Benzo[d][1,3]dioxol-5-ylmethyl 2-((2-chloro-4-(4-(3-chlorophenyl)-trans-2,3-dimethylpiperazine-1-carbonyl)phenyl)sulfinyl)acetate, (±) 2-Methoxyethyl 2-((2-chloro-4-(4-(3-chlorophenyl)-trans2,3-dimethylpiperazine-1-carbonyl)phenyl)sulfinyl)acetate, (±) 1-Methoxypropan-2-yl 2-((2-chloro-4-(4-(3-chlorophenyl)-trans-2,3-dimethylpiperazine-1-carbonyl)phenyl)sulfinyl)acetate, (±) 2-(2-Ethoxyethoxy)ethyl 2-((2-chloro-4-(4-(3-chlorophenyl)-trans-2,3-dimethylpiperazine-1-carbonyl)phenyl)sulfinyl)acetate, (±) Tetrahydro-2H-pyran-4-yl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(5-chloropyridin-3-yl)-trans-2,3-dimethylpiperazine-1-carbonyl)phenyl)sulfinyl)acetate, (±) Ethyl 2-((2-chloro-4-(4-(2-chloropyridin-4-yl)-trans-2,3-dimethylpiperazine-1-carbonyl)phenyl)sulfinyl)acetate, (±) Ethyl 2-((2-chloro-4-(4-(6-chloropyridin-2-yl)-trans-2,3-dimethylpiperazine-1-carbonyl)phenyl)sulfinyl)acetate, (±) Ethyl 2-((4-chloro-4-(4-(6-chloropyridin-2-yl)-trans-2,3-dimethylpiperazine-1-carbonyl)phenyl)sulfinyl)acetate, (±) 3-((2-Chloro-4-(4-(3-chlorophenyl)-trans-2,3-dimethylpiperazine-1-carbonyl)phenyl)thio)-1,1,1-trifluoropropan-2-one, (±) 3-((2-Chloro-4-(4-(3-chlorophenyl)-trans-2,3-dimethylpiperazine-1-carbonyl)phenyl)sulfinyl)-1,1,1-trifluoropropan-2-one, (±) 2-((2-Chloro-4-(4-(3-chlorophenyl)-trans-2,3-dimethylpiperazine-1-carbonyl)phenyl)sulfinyl)-1-(oxazol-2-yl)ethan-1-one, (±) 2-((2-Chloro-4-(4-(3-chlorophenyl)-trans-2,3-dimethylpiperazine-1-carbonyl)phenyl)sulfinyl)-1-(thiazol-2-yl)ethan-1-one, (±) 2-((2-Chloro-4-(4-(3-chlorophenyl)-trans-2,3-dimethylpiperazine-1-carbonyl)phenyl)sulfinyl)-1-(5-methyloxazol-2-yl)ethan-1-one, (±) 3-((2-Chloro-4-(4-(3-chlorophenyl)-trans-2,3-dimethylpiperazine-1-carbonyl)phenyl)sulfinyl)-1,1-difluoropropan-2-one, (±) 1-((2-Chloro-4-(4-(3-chlorophenyl)-trans-2,3-dimethylpiperazine-1-carbonyl)phenyl)sulfinyl)-3,3-difluorobutan-2-one, (±) 1-((2-Chloro-4-(4-(3-chlorophenyl)-trans-2,3-dimethylpiperazine-1-carbonyl)phenyl)sulfinyl)-3,3-difluoropentan-2-one, (±) 3-((2-Chloro-4-(4-(3-chlorophenyl)-trans-2,3-dimethylpiperazine-1-carbonyl)phenyl)sulfinyl)-1,1-difluoro-1-phenylpropan-2-one, (±) 1-((2-Chloro-4-(4-(4-chloropyridin-2-yl)-trans-2,3-dimethylpiperazine-1-carbonyl)phenyl)sulfinyl)-3,3-difluorobutan-2-one, (±) 1-((2-Chloro-4-(4-(4-chloropyridin-2-yl)-trans-2,3-dimethylpiperazine-1-carbonyl)phenyl)sulfinyl)-3,3-difluoropentan-2-one, (±) 1-((2-Chloro-4-(4-(6-chloropyridin-2-yl)-trans-2,3-dimethylpiperazine-1-carbonyl)phenyl)sulfinyl)-3,3-difluorobutan-2-one, (±) 1-((2-Chloro-4-(4-(6-chloropyridin-2-yl)-trans-2,3-dimethylpiperazine-1-carbonyl)phenyl)sulfinyl)-3,3-difluoropentan-2-one, (±) 2-((2-Chloro-4-(4-(3-chlorophenyl)-trans-2,3-dimethylpiperazine-1-carbonyl)phenyl)sulfinyl)-1-(pyridin-3-yl)ethan-1-one, (±) 2-((2-Chloro-4-(4-(3-chlorophenyl)-trans-2,3-dimethylpiperazine-1-carbonyl)phenyl)sulfinyl)-1-(4-fluorophenyl)ethan-1-one, (±) 2-((2-Chloro-4-(4-(3-chlorophenyl)-trans-2,3-dimethylpiperazine-1-carbonyl)phenyl)sulfinyl)-1-(3-fluorophenyl)ethan-1-one, (±) 2-((2-Chloro-4-(4-(3-chlorophenyl)-trans-2,3-dimethylpiperazine-1-carbonyl)phenyl)sulfinyl)-1-(pyridazin-3-yl)ethan-1-one, (±) 2-((2-Chloro-4-(trans-4-(3-chlorophenyl)-2,3-dimethylpiperazine-1-carbonyl)phenyl)sulfinyl)-1-(3,5-difluorophenyl)ethan-1-one, (±) 2-((2-Chloro-4-(4-(3-chlorophenyl)-trans-2,3-dimethylpiperazine-1-carbonyl)phenyl)sulfinyl)-1-(6-(trifluoromethyl)pyridin-3-yl)ethan-1-one, (±) 1-(4-Chloro-3-(trifluoromethyl)phenyl)-2-((2-chloro-4-(4-(3-chlorophenyl)-trans-2,3-dimethylpiperazine-1-carbonyl)phenyl)sulfinyl)ethan-1-one, (±) 2-((2-Chloro-4-(4-(3-chlorophenyl)-trans-2,3-dimethylpiperazine-1-carbonyl)phenyl)sulfinyl)-1-(4-fluoro-3-(trifluoromethyl)phenyl)ethan-1-one, (±) 2-((2-Chloro-4-(4-(3-chlorophenyl)-trans-2,3-dimethylpiperazine-1-carbonyl)phenyl)sulfinyl)-1-(2,3-difluorophenyl)ethan-1-one, (±) 2-((2-Chloro-4-(4-(3-chlorophenyl)-trans-2,3-dimethylpiperazine-1-carbonyl)phenyl)sulfinyl)-1-(3,4-difluorophenyl)ethan-1-one, (±) 2-((2-Chloro-4-(4-(3-chlorophenyl)-trans-2,3-dimethylpiperazine-1-carbonyl)phenyl)sulfinyl)-1-(2-fluorophenyl)ethan-1-one, (±) 2-((2-Chloro-4-(4-(3-chlorophenyl)-trans-2,3-dimethylpiperazine-1-carbonyl)phenyl)sulfinyl)-1-(pyridazin-4-yl)ethan-1-one, (±) 2-((2-Chloro-4-(4-(3-chlorophenyl)-trans-2,3-dimethylpiperazine-1-carbonyl)phenyl)sulfinyl)-1-(2,4-difluorophenyl)ethan-1-one, (±) 2-((2-Chloro-4-(4-(3-chlorophenyl)-trans-2,3-dimethylpiperazine-1-carbonyl)phenyl)sulfinyl)-1-(pyridin-4-yl)ethan-1-one, (±) 4-(2-((2-Chloro-4-(4-(3-chlorophenyl)-trans-2,3-dimethylpiperazine-1-carbonyl)phenyl)sulfinyl)acetyl)benzonitrile, (±) 2-((2-Chloro-4-(4-(3-chlorophenyl)-trans-2,3-dimethylpiperazine-1-carbonyl)phenyl)sulfinyl)-1-(pyrimidin-4-yl)ethan-1-one, (±) 2-((2-Chloro-4-(4-(3-chlorophenyl)-trans-2,3-dimethylpiperazine-1-carbonyl)phenyl)sulfinyl)-1-(pyrazin-2-yl)ethan-1-one, (±) 2-((2-Chloro-4-(4-(3-chlorophenyl)-trans-2,3-dimethylpiperazine-1-carbonyl)phenyl)sulfinyl)-1-(pyrimidin-2-yl)ethan-1-one, (±) 2-((2-Chloro-4-(4-(3-chlorophenyl)-trans-2,3-dimethylpiperazine-1-carbonyl)phenyl)sulfinyl)-1-(4-(trifluoromethyl)phenyl)ethan-1-one, (±) 2-((2-Chloro-4-(4-(3-chlorophenyl)-trans-2,3-dimethylpiperazine-1-carbonyl)phenyl)sulfinyl)-1-phenylethan-1-one, and (±) 6-(2-((2-Chloro-4-(4-(3-chlorophenyl)-trans-2,3-dimethylpiperazine-1-carbonyl)phenyl)sulfinyl)acetyl)nicotinonitrile.

In an embodiment, the compound may be selected from the following compounds, or a pharmaceutically acceptable salt, stereoisomer, or prodrug thereof: Ethyl 2-((4-((S)-3-methyl-4-(m-tolyl)piperazine-1-carbonyl)-2-nitrophenyl)sulfinyl)acetate, Ethyl 2-((4-((R)-3-methyl-4-(m-tolyl)piperazine-1-carbonyl)-2-nitrophenyl)sulfinyl)acetate, Ethyl 2-((2-nitro-4-(4-(m-tolyl)piperazine-1-carbonyl)phenyl)sulfinyl)acetate, Ethyl 2-((4-((S)-3-methyl-4-(m-tolyl)piperazine-1-carbonyl)phenyl)sulfinyl)acetate, Ethyl 2-((2-nitro-4-(4-phenylpiperazine-1-carbonyl)phenyl)sulfinyl)acetate, Ethyl 2-((4-(4-(3-fluorophenyl)piperazine-1-carbonyl)-2-nitrophenyl)sulfinyl)acetate, Ethyl 2-((4-(4-(3-chlorophenyl)piperazine-1-carbonyl)-2-nitrophenyl)sulfinyl)acetate, Ethyl 2-((4-(4-(4-chlorophenyl)piperazine-1-carbonyl)-2-nitrophenyl)sulfinyl)acetate, Ethyl 2-((4-(4-(2-chlorophenyl)piperazine-1-carbonyl)-2-nitrophenyl)sulfinyl)acetate, Ethyl 2-((4-(4-(3-bromophenyl)piperazine-1-carbonyl)-2-nitrophenyl)sulfinyl)acetate, Ethyl 2-((4-(4-(4-bromophenyl)piperazine-1-carbonyl)-2-nitrophenyl)sulfinyl)acetate, Ethyl 2-((2-nitro-4-(4-(3-(trifluoromethyl)phenyl)piperazine-1-carbonyl)phenyl)sulfinyl)acetate, Ethyl 2-((2-nitro-4-(4-(4-(trifluoromethyl)phenyl)piperazine-1-carbonyl)phenyl)sulfinyl)acetate, Ethyl 2-((4-(4-(3-methoxyphenyl)piperazine-1-carbonyl)-2-nitrophenyl)sulfinyl)acetate, Ethyl 2-((4-(4-(4-methoxyphenyl)piperazine-1-carbonyl)-2-nitrophenyl)sulfinyl)acetate, Ethyl 2-((2-nitro-4-(4-(3-nitrophenyl)piperazine-1-carbonyl)phenyl)sulfinyl)acetate, Ethyl 2-((4-(4-([1,1'-biphenyl]-3-yl)piperazine-1-carbonyl)-2-nitrophenyl)sulfinyl)acetate, Ethyl 2-((4-(4-(3,5-dichlorophenyl)piperazine-1-carbonyl)-2-nitrophenyl)sulfinyl)acetate, Ethyl 2-((4-(4-(3,4-dichlorophenyl)piperazine-1-carbonyl)-2-nitrophenyl)sulfinyl)acetate, Ethyl 2-((4-(4-(2,4-dichlorophenyl)piperazine-1-carbonyl)-2-nitrophenyl)sulfinyl)acetate, Ethyl 2-((4-(4-(2,6-dichlorophenyl)piperazine-1-carbonyl)-2-nitrophenyl)sulfinyl)acetate, Ethyl 2-((4-((S)-4-(3-fluorophenyl)-3-methylpiperazine-1-carbonyl)-2-nitrophenyl)sulfinyl)acetate, Ethyl 2-((4-((R)-4-(3-chlorophenyl)-3-methylpiperazine-1-carbonyl)-2-nitrophenyl)sulfinyl)acetate, Ethyl 2-((4-((S)-4-(3-chlorophenyl)-3-methylpiperazine-1-carbonyl)-2-nitrophenyl)sulfinyl)acetate, Ethyl 2-((4-((R)-4-(3-bromophenyl)-3-methylpiperazine-1-carbonyl)-2-nitrophenyl)sulfinyl)acetate, Ethyl 2-((4-((S)-4-(3-bromophenyl)-3-methylpiperazine-1-carbonyl)-2-nitrophenyl)sulfinyl)acetate, Ethyl 2-((4-((R)-3-methyl-4-(3-(trifluoromethyl)phenyl)piperazine-1-carbonyl)-2-nitrophenyl)sulfinyl)acetate, Ethyl 2-((4-((S)-3-methyl-4-(3-(trifluoromethyl)phenyl)piperazine-1-carbonyl)-2-nitrophenyl)sulfinyl)acetate, Methyl 2-((4-((R)-4-(3-chlorophenyl)-3-methylpiperazine-1-carbonyl)-2-nitrophenyl)sulfinyl)acetate, Isopropyl 2-((4-((R)-4-(3-chlorophenyl)-3-methylpiperazine-1-carbonyl)-2-nitrophenyl)sulfinyl)acetate, 2,2,2-Trifluoroethyl 2-((4-((R)-4-(3-chlorophenyl)-3-methylpiperazine-1-carbonyl)-2-nitrophenyl)sulfinyl)acetate, Propyl 2-((4-((R)-4-(3-chlorophenyl)-3-methylpiperazine-1-carbonyl)-2-nitrophenyl)sulfinyl)acetate, Butyl 2-((4-((R)-4-(3-chlorophenyl)-3-methylpiperazine-1-carbonyl)-2-nitrophenyl)sulfinyl)acetate, sec-Butyl 2-((4-((R)-4-(3-chlorophenyl)-3-methylpiperazine-1-carbonyl)-2-nitrophenyl)sulfinyl)acetate, tert-Pentyl 2-((4-((R)-4-(3-chlorophenyl)-3-methylpiperazine-1-carbonyl)-2-nitrophenyl)sulfinyl)acetate, tert-Butyl 2-((4-((R)-4-(3-chlorophenyl)-3-methylpiperazine-1-carbonyl)-2-nitrophenyl)sulfinyl)acetate, 3-Hydroxypropyl 2-((4-((R)-4-(3-chlorophenyl)-3-methylpiperazine-1-carbonyl)-2-nitrophenyl)sulfinyl)acetate, Ethyl 2-((4-(4-(3-chlorophenyl)-3,5-dimethylpiperazine-1-carbonyl)-2-nitrophenyl)sulfinyl)acetate, Ethyl 2-((4-((R)-4-(3-chlorophenyl)-3-methylpiperazine-1-carbonyl)-2-fluorophenyl)sulfinyl)acetate, Ethyl 2-((4-(4-(3-chlorophenyl)-2-methylpiperazine-1-carbonyl)-2-fluorophenyl)sulfinyl)acetate, (±) Ethyl 2-((4-(4-(3-chlorophenyl)-cis-2,3-dimethylpiperazine-1-carbonyl)-2-fluorophenyl)sulfinyl)acetate, (±) Ethyl 2-((4-(4-(3-chlorophenyl)-trans-2,3-dimethylpiperazine-1-carbonyl)-2-fluorophenyl)sulfinyl)acetate, (±) Ethyl 2-((4-(4-(3-chlorophenyl)-3,3-dimethylpiperazine-1-carbonyl)-2-fluorophenyl)sulfinyl)acetate, (±) Ethyl 2-((4-(4-(3-chlorophenyl)-2,2-dimethylpiperazine-1-carbonyl)-2-fluorophenyl)sulfinyl)acetate, (±) Ethyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(3-chlorophenyl)-trans-2,3-dimethylpiperazine-1-carbonyl)phenyl)sulfinyl)acetate, (±) 2-((2-Chloro-4-(4-(3-chlorophenyl)-trans-2,3-dimethylpiperazine-1-carbonyl)phenyl)sulfinyl)acetic acid, (±) Ethyl 2-((2-chloro-4-(4-(3-chlorophenyl)-trans-2,3-dimethylpiperazine-1-carbonyl)phenyl)sulfonyl)acetate, (±) Ethyl 2-((2-chloro-4-((4-(3-chlorophenyl)-trans-2,3-dimethylpiperazin-1-yl)methyl)phenyl)sulfinyl)acetate, (±) Ethyl 3-(2-chloro-4-(4-(3-chlorophenyl)-trans-2,3-dimethylpiperazine-1-carbonyl)phenyl)-3-oxopropanoate, (±) Isopropyl 2-((2-chloro-4-(4-(3-chlorophenyl)-trans-2,3-dimethylpiperazine-1-carbonyl)phenyl)sulfinyl)acetate, (±) sec-Butyl 2-((2-chloro-4-(4-(3-chlorophenyl)-trans-2,3-dimethylpiperazine-1-carbonyl)phenyl)sulfinyl)acetate, (±) Cyclobutyl 2-((2-chloro-4-(4-(3-chlorophenyl)-trans-2,3-dimethylpiperazine-1-carbonyl)phenyl)sulfinyl)acetate, (±) Cyclopentyl 2-((2-chloro-4-(4-(3-chlorophenyl)-trans-2,3-dimethylpiperazine-1-carbonyl)phenyl)sulfinyl)acetate, (±) Cyclohexyl 2-((2-chloro-4-(4-(3-chlorophenyl)-trans-2,3-dimethylpiperazine-1-carbonyl)phenyl)sulfinyl)acetate, (±) 2,3-Dihydroxypropyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(3-chlorophenyl)-trans-2,3-dimethylpiperazine-1-carbonyl)phenyl)sulfinyl)propanoate, (±) sec-Butyl 2-((2-chloro-4-(4-(3-chlorophenyl)-trans-2,3-dimethylpiperazine-1-carbonyl)phenyl)sulfinyl)propanoate, (±) Benzo[d][1,3]dioxol-5-ylmethyl 2-((2-chloro-4-(4-(3-chlorophenyl)-trans-2,3-dimethylpiperazine-1-carbonyl)phenyl)sulfinyl)acetate, (±) 2-Methoxyethyl 2-((2-chloro-4-(4-(3-chlorophenyl)-trans2,3-dimethylpiperazine-1-carbonyl)phenyl)sulfinyl)acetate, (±) 1-Methoxypropan-2-yl 2-((2-chloro-4-(4-(3-chlorophenyl)-trans-2,3-dimethylpiperazine-1-carbonyl)phenyl)sulfinyl)acetate, (±) 2-(2-Ethoxyethoxy)ethyl 2-((2-chloro-4-(4-(3-chlorophenyl)-trans-2,3-dimethylpiperazine-1-carbonyl)phenyl)sulfinyl)acetate, (±) Tetrahydro-2H-pyran-4-yl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(5-chloropyridin-3-yl)-trans-2,3-dimethylpiperazine-1-carbonyl)phenyl)sulfinyl)acetate, (±) Ethyl 2-((2-chloro-4-(4-(2-chloropyridin-4-yl)-trans-2,3-dimethylpiperazine-1-carbonyl)phenyl)sulfinyl)acetate, (±) Ethyl 2-((2-chloro-4-(4-(6-chloropyridin-2-yl)-trans-2,3-dimethylpiperazine-1-carbonyl)phenyl)sulfinyl)acetate, (±) Ethyl 2-((4-chloro-4-(4-(6-chloropyridin-2-yl)-trans-2,3-dimethylpiperazine-1-carbonyl)phenyl)sulfinyl)acetate, (±) 3-((2-Chloro-4-(4-(3-chlorophenyl)-trans-2,3-dimethylpiperazine-1-carbonyl)phenyl)thio)-1,1,1-trifluoropropan-2-one, (±) 3-((2-Chloro-4-(4-(3-chlorophenyl)-trans-2,3-dimethylpiperazine-1-carbonyl)phenyl)sulfinyl)-1,1,1-trifluoropropan-2-one, (±) 2-((2-Chloro-4-(4-(3-chlorophenyl)-trans-2,3-dimethylpiperazine-1-carbonyl)phenyl)sulfinyl)-1-(oxazol-2-yl)ethan-1-one, (±) 2-((2-Chloro-4-(4-(3-chlorophenyl)-trans-2,3-dimethylpiperazine-1-carbonyl)phenyl)sulfinyl)-1-(thiazol-2-yl)ethan-1-one, (±) 2-((2-Chloro-4-(4-(3-chlorophenyl)-trans-2,3-dimethylpiperazine-1-carbonyl)phenyl)sulfinyl)-1-(5-methyloxazol-2-yl)ethan-1-one, (±) 3-((2-Chloro-4-(4-(3-chlorophenyl)-trans-2,3-dimethylpiperazine-1-carbonyl)phenyl)sulfinyl)-1,1-difluoropropan-2-one, (±) 1-((2-Chloro-4-(4-(3-chlorophenyl)-trans-2,3-dimethylpiperazine-1-carbonyl)phenyl)sulfinyl)-3,3-difluorobutan-2-one, (±) 1-((2-Chloro-4-(4-(3-chlorophenyl)-trans-2,3-dimethylpiperazine-1-carbonyl)phenyl)sulfinyl)-3,3-difluoropentan-2-one, (±) 3-((2-Chloro-4-(4-(3-chlorophenyl)-trans-2,3-dimethylpiperazine-1-carbonyl)phenyl)sulfinyl)-1,1-difluoro-1-phenylpropan-2-one, (±) 1-((2-Chloro-4-(4-(4-chloropyridin-2-yl)-trans-2,3-dimethylpiperazine-1-carbonyl)phenyl)sulfinyl)-3,3-difluorobutan-2-one, (±) 1-((2-Chloro-4-(4-(4-chloropyridin-2-yl)-trans-2,3-dimethylpiperazine-1-carbonyl)phenyl)sulfinyl)-3,3-difluoropentan-2-one, (±) 1-((2-Chloro-4-(4-(6-chloropyridin-2-yl)-trans-2,3-dimethylpiperazine-1-carbonyl)phenyl)sulfinyl)-3,3-difluorobutan-2-one, (±) 1-((2-Chloro-4-(4-(6-chloropyridin-2-yl)-trans-2,3-dimethylpiperazine-1-carbonyl)phenyl)sulfinyl)-3,3-difluoropentan-2-one, (±) 2-((2-Chloro-4-(4-(3-chlorophenyl)-trans-2,3-dimethylpiperazine-1-carbonyl)phenyl)sulfinyl)-1-(pyridin-3-yl)ethan-1-one, (±) 2-((2-Chloro-4-(4-(3-chlorophenyl)-trans-2,3-dimethylpiperazine-1-carbonyl)phenyl)sulfinyl)-1-(4-fluorophenyl)ethan-1-one, (±) 2-((2-Chloro-4-(4-(3-chlorophenyl)-trans-2,3-dimethylpiperazine-1-carbonyl)phenyl)sulfinyl)-1-(pyridazin-3-yl)ethan-1-one, (±) 2-((2-Chloro-4-(trans-4-(3-chlorophenyl)-2,3-dimethylpiperazine-1-carbonyl)phenyl)sulfinyl)-1-(3,5-difluorophenyl)ethan-1-one, (±) 2-((2-Chloro-4-(4-(3-chlorophenyl)-trans-2,3-dimethylpiperazine-1-carbonyl)phenyl)sulfinyl)-1-(6-(trifluoromethyl)pyridin-3-yl)ethan-1-one, (±) 2-((2-Chloro-4-(4-(3-chlorophenyl)-trans-2,3-dimethylpiperazine-1-carbonyl)phenyl)sulfinyl)-1-(2,3-difluorophenyl)ethan-1-one, (±) 2-((2-Chloro-4-(4-(3-chlorophenyl)-trans-2,3-dimethylpiperazine-1-carbonyl)phenyl)sulfinyl)-1-(3,4-difluorophenyl)ethan-1-one, (±) 2-((2-Chloro-4-(4-(3-chlorophenyl)-trans-2,3-dimethylpiperazine-1-carbonyl)phenyl)sulfinyl)-1-(2-fluorophenyl)ethan-1-one, (±) 2-((2-Chloro-4-(4-(3-chlorophenyl)-trans-2,3-dimethylpiperazine-1-carbonyl)phenyl)sulfinyl)-1-(pyridazin-4-yl)ethan-1-one, (±) 2-((2-Chloro-4-(4-(3-chlorophenyl)-trans-2,3-dimethylpiperazine-1-carbonyl)phenyl)sulfinyl)-1-(2,4-difluorophenyl)ethan-1-one, (±) 2-((2-Chloro-4-(4-(3-chlorophenyl)-trans-2,3-dimethylpiperazine-1-carbonyl)phenyl)sulfinyl)-1-(pyridin-4-yl)ethan-1-one, (±) 4-(2-((2-Chloro-4-(4-(3-chlorophenyl)-trans-2,3-dimethylpiperazine-1-carbonyl)phenyl)sulfinyl)acetyl)benzonitrile, (±) 2-((2-Chloro-4-(4-(3-chlorophenyl)-trans-2,3-dimethylpiperazine-1-carbonyl)phenyl)sulfinyl)-1-(pyrimidin-4-yl)ethan-1-one, (±) 2-((2-Chloro-4-(4-(3-chlorophenyl)-trans-2,3-dimethylpiperazine-1-carbonyl)phenyl)sulfinyl)-1-(pyrazin-2-yl)ethan-1-one, (±) 2-((2-Chloro-4-(4-(3-chlorophenyl)-trans-2,3-dimethylpiperazine-1-carbonyl)phenyl)sulfinyl)-1-(pyrimidin-2-yl)ethan-1-one, (±) 2-((2-Chloro-4-(4-(3-chlorophenyl)-trans-2,3-dimethylpiperazine-1-carbonyl)phenyl)sulfinyl)-1-(4-(trifluoromethyl)phenyl)ethan-1-one, (±) 2-((2-Chloro-4-(4-(3-chlorophenyl)-trans-2,3-dimethylpiperazine-1-carbonyl)phenyl)sulfinyl)-1-phenylethan-1-one, and (±) 6-(2-((2-Chloro-4-(4-(3-chlorophenyl)-trans-2,3-dimethylpiperazine-1-carbonyl)phenyl)sulfinyl)acetyl)nicotinonitrile.

In an embodiment, the compound may be selected from the following compounds, or a pharmaceutically acceptable salt, stereoisomer, or prodrug thereof: Ethyl 2-((4-((R)-4-(3-chlorophenyl)-3-methylpiperazine-1-carbonyl)-2-nitrophenyl)sulfinyl)acetate, Ethyl 2-((4-((S)-4-(3-chlorophenyl)-3-methylpiperazine-1-carbonyl)-2-nitrophenyl)sulfinyl)acetate, Ethyl 2-((4-((R)-4-(3-bromophenyl)-3-methylpiperazine-1-carbonyl)-2-nitrophenyl)sulfinyl)acetate, Ethyl 2-((4-((S)-4-(3-bromophenyl)-3-methylpiperazine-1-carbonyl)-2-nitrophenyl)sulfinyl)acetate, Methyl 2-((4-((R)-4-(3-chlorophenyl)-3-methylpiperazine-1-carbonyl)-2-nitrophenyl)sulfinyl)acetate, 2,2,2-Trifluoroethyl 2-((4-((R)-4-(3-chlorophenyl)-3-methylpiperazine-1-carbonyl)-2-nitrophenyl)sulfinyl)acetate, Propyl 2-((4-((R)-4-(3-chlorophenyl)-3-methylpiperazine-1-carbonyl)-2-nitrophenyl)sulfinyl)acetate, Butyl 2-((4-((R)-4-(3-chlorophenyl)-3-methylpiperazine-1-carbonyl)-2-nitrophenyl)sulfinyl)acetate, sec-Butyl 2-((4-((R)-4-(3-chlorophenyl)-3-methylpiperazine-1-carbonyl)-2-nitrophenyl)sulfinyl)acetate, 3-Hydroxypropyl 2-((4-((R)-4-(3-chlorophenyl)-3-methylpiperazine-1-carbonyl)-2-nitrophenyl)sulfinyl)acetate, Ethyl 2-((4-((R)-4-(3-chlorophenyl)-3-methylpiperazine-1-carbonyl)-2-fluorophenyl)sulfinyl)acetate, Ethyl 2-((4-(4-(3-chlorophenyl)-2-methylpiperazine-1-carbonyl)-2-fluorophenyl)sulfinyl)acetate, (±) Ethyl 2-((4-(4-(3-chlorophenyl)-cis-2,3-dimethylpiperazine-1-carbonyl)-2-fluorophenyl)sulfinyl)acetate, (±) Ethyl 2-((4-(4-(3-chlorophenyl)-trans-2,3-dimethylpiperazine-1-carbonyl)-2-fluorophenyl)sulfinyl)acetate, (±) Ethyl 2-((4-(4-(3-chlorophenyl)-3,3-dimethylpiperazine-1-carbonyl)-2-fluorophenyl)sulfinyl)acetate, (±) Ethyl 2-((4-(4-(3-chlorophenyl)-2,2-dimethylpiperazine-1-carbonyl)-2-fluorophenyl)sulfinyl)acetate, (±) Ethyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-((4-(3-chlorophenyl)-trans-2,3-dimethylpiperazin-1-yl)methyl)phenyl)sulfinyl)acetate, (±) Isopropyl 2-((2-chloro-4-(4-(3-chlorophenyl)-trans-2,3-dimethylpiperazine-1-carbonyl)phenyl)sulfinyl)acetate, (±) sec-Butyl 2-((2-chloro-4-(4-(3-chlorophenyl)-trans-2,3-dimethylpiperazine-1-carbonyl)phenyl)sulfinyl)acetate, (±) Cyclobutyl 2-((2-chloro-4-(4-(3-chlorophenyl)-trans-2,3-dimethylpiperazine-1-carbonyl)phenyl)sulfinyl)acetate, (±) Cyclopentyl 2-((2-chloro-4-(4-(3-chlorophenyl)-trans-2,3-dimethylpiperazine-1-carbonyl)phenyl)sulfinyl)acetate, (±) Cyclohexyl 2-((2-chloro-4-(4-(3-chlorophenyl)-trans-2,3-dimethylpiperazine-1-carbonyl)phenyl)sulfinyl)acetate, (±) 2,3-Dihydroxypropyl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(3-chlorophenyl)-trans-2,3-dimethylpiperazine-1-carbonyl)phenyl)sulfinyl)propanoate, (±) sec-Butyl 2-((2-chloro-4-(4-(3-chlorophenyl)-trans-2,3-dimethylpiperazine-1-carbonyl)phenyl)sulfinyl)propanoate, (±) Benzo[d][1,3]dioxol-5-ylmethyl 2-((2-chloro-4-(4-(3-chlorophenyl)-trans-2,3-dimethylpiperazine-1-carbonyl)phenyl)sulfinyl)acetate, (±) 2-Methoxyethyl 2-((2-chloro-4-(4-(3-chlorophenyl)-trans2,3-dimethylpiperazine-1-carbonyl)phenyl)sulfinyl)acetate, (±) 1-Methoxypropan-2-yl 2-((2-chloro-4-(4-(3-chlorophenyl)-trans-2,3-dimethylpiperazine-1-carbonyl)phenyl)sulfinyl)acetate, (±) 2-(2-Ethoxyethoxy)ethyl 2-((2-chloro-4-(4-(3-chlorophenyl)-trans-2,3-dimethylpiperazine-1-carbonyl)phenyl)sulfinyl)acetate, (±) Tetrahydro-2H-pyran-4-yl 2-((2-chloro-4-(4-(3-chlorophenyl)-trans-2,3-dimethylpiperazine-1-carbonyl)phenyl)sulfinyl)acetate, (±) Ethyl 2-((2-chloro-4-(4-(5-chloropyridin-3-yl)-trans-2,3-dimethylpiperazine-1-carbonyl)phenyl)sulfinyl)acetate, (±) Ethyl 2-((2-chloro-4-(4-(2-chloropyridin-4-yl)-trans-2,3-dimethylpiperazine-1-carbonyl)phenyl)sulfinyl)acetate, (±) Ethyl 2-((2-chloro-4-(4-(6-chloropyridin-2-yl)-trans-2,3-dimethylpiperazine-1-carbonyl)phenyl)sulfinyl)acetate, (±) Ethyl 2-((4-chloro-4-(4-(6-chloropyridin-2-yl)-trans-2,3-dimethylpiperazine-1-carbonyl)phenyl)sulfinyl)acetate, (±) 3-((2-Chloro-4-(4-(3-chlorophenyl)-trans-2,3-dimethylpiperazine-1-carbonyl)phenyl)thio)-1,1,1-trifluoropropan-2-one, (±) 3-((2-Chloro-4-(4-(3-chlorophenyl)-trans-2,3-dimethylpiperazine-1-carbonyl)phenyl)sulfinyl)-1,1,1-trifluoropropan-2-one, (±) 3-((2-Chloro-4-(4-(3-chlorophenyl)-trans-2,3-dimethylpiperazine-1-carbonyl)phenyl)sulfinyl)-1,1-difluoropropan-2-one, (±) 1-((2-Chloro-4-(4-(3-chlorophenyl)-trans-2,3-dimethylpiperazine-1-carbonyl)phenyl)sulfinyl)-3,3-difluorobutan-2-one, (±) 1-((2-Chloro-4-(4-(3-chlorophenyl)-trans-2,3-dimethylpiperazine-1-carbonyl)phenyl)sulfinyl)-3,3-difluoropentan-2-one, (±) 3-((2-Chloro-4-(4-(3-chlorophenyl)-trans-2,3-dimethylpiperazine-1-carbonyl)phenyl)sulfinyl)-1,1-difluoro-1-phenylpropan-2-one, (±) 1-((2-Chloro-4-(4-(4-chloropyridin-2-yl)-trans-2,3-dimethylpiperazine-1-carbonyl)phenyl)sulfinyl)-3,3-difluorobutan-2-one, (±) 1-((2-Chloro-4-(4-(4-chloropyridin-2-yl)-trans-2,3-dimethylpiperazine-1-carbonyl)phenyl)sulfinyl)-3,3-difluoropentan-2-one, (±) 1-((2-Chloro-4-(4-(6-chloropyridin-2-yl)-trans-2,3-dimethylpiperazine-1-carbonyl)phenyl)sulfinyl)-3,3-difluorobutan-2-one, (±) 1-((2-Chloro-4-(4-(6-chloropyridin-2-yl)-trans-2,3-dimethylpiperazine-1-carbonyl)phenyl)sulfinyl)-3,3-difluoropentan-2-one, (±) 4-(2-((2-Chloro-4-(4-(3-chlorophenyl)-trans-2,3-dimethylpiperazine-1-carbonyl)phenyl)sulfinyl)acetyl)benzonitrile, (±) 2-((2-Chloro-4-(4-(3-chlorophenyl)-trans-2,3-dimethylpiperazine-1-carbonyl)phenyl)sulfinyl)-1-(pyrimidin-4-yl)ethan-1-one, (±) 2-((2-Chloro-4-(4-(3-chlorophenyl)-trans-2,3-dimethylpiperazine-1-carbonyl)phenyl)sulfinyl)-1-(pyrimidin-2-yl)ethan-1-one, and (±) 6-(2-((2-Chloro-4-(4-(3-chlorophenyl)-trans-2,3-dimethylpiperazine-1-carbonyl)phenyl)sulfinyl)acetyl)nicotinonitrile.

### USES

The Compounds of the invention are inhibitors of monoacylglycerol lipase (MAGL). MAGL is a serine hydrolase that plays a crucial role catalysing the hydrolysis of monoglycerides into glycerol and fatty acids. It links the endocannabinoid and eicosanoid systems together by degradation of the abundant endocannabinoid 2-arachidaoylglycerol into arachidonic acid, the precursor of prostaglandins and other inflammatory mediators.

MAGL is highly expressed in many cancer cells and is involved in metabolism more generally. In view of this, MAGL inhibitors are useful for treatments in many therapeutic fields, including as anti-nociceptive, anxiolytic, anti-inflammatory, and anti-cancer agents. ABX-1431, a first-in-class inhibitor of MAGL, is undergoing clinical studies for neurological disorders and other diseases (Cisar, J.S., Identification of ABX-1431, a Selective Inhibitor of Monoacylglycerol Lipase and Clinical Candidate for Treatment of Neurological Disorder, J. Med. Chem., 2018, Oct 25, 61(20), p062-9084).

MAGL is considered a therapeutic target in a number of disease areas, such as inflammation and neurological disorders, metabolic disorders, and cancer (Deng, H. and Li, W., Monoacylglycerol lipase inhibitors: Modulators for lipid metabolism in cancer malignancy, neurological and metabolic disorders, Acta Pharmaceutica Sinica B, https://doi.org/10.1016/j.apsb.2019.10.006). MAGL inhibition is applied in the area of pain and inflammation, metabolic disorders (such as obesity and diabetes), neurodegenerative pathologies (such as Alzheimer's disease), anxiety and epilepsy (Granchi, C. et al., Expert Opinion on Therapeutic Patents, 2017, 27(12), 1341-1351). MAGL inhibition is also considered a useful therapeutic approach in the treatment of many cancers and cancer associated symptoms, in particular in cancers such as breast cancer, ovarian cancer, melanoma, hepatocellular carcinoma, colorectal cancer, prostate cancer, or nasopharyngeal carcinoma. See, for example: Nomura D.K. et al., "Monoacylglycerol Lipase Regulates a Fatty Acid Network that Promotes Cancer Pathogenesis", Cell 140, 49-61, January 8, 2010; Zhu W. et al., "Monoacylglycerol lipase promotes progression of hepatocellular carcinoma via NF-κB-mediated epithelial-mesenchymal transition", Journal of Hematology & Oncology, 2016, 9, Article No. 127; Pagano E. et al., "Pharmacological inhibition of MAGL attenuates experimental colon carcinogenesis", Pharmacological Research, 2017, 119, 227-236; Nomura D.K. et al., "Monoacylglycerol Lipase Exerts Dual Control over Endocannabinoid and Fatty Acid Pathways to Support Prostate Cancer", Chemistry and Biology, 2011, 18(7), 846-856; Hu W.R et al., Monoacylglycerol lipase promotes metastases in nasopharyngeal carcinoma", Int. J. Clin. Exp. Pathol., 2014, 7(7), 3704-3713; Sticht M.A. et al., "Inhibition of monoacylglycerol lipase attenuates vomiting in Suncus murinus and 2-arachidonoyl glycerol attenuates nausea in rats", British Journal of Pharmacology, 2011, 165(8), https://doi.org/10.1111/j.1476-5381.2011.01407.x).

Accordingly, conditions that may be treated by the compounds of the invention include acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression

The condition may be cancer. The cancer may be selected from Acute Lymphoblastic Leukemia (ALL), Acute Myeloid Leukemia (AML), Adrenocortical Carcinoma, Anal Cancer, Appendix Cancer, Atypical Teratoid/Rhabdoid Tumor, Basal Cell Carcinoma, Bile Duct Cancer, Biliary Cancer, Bladder Cancer, Bone Cancer, Brain Tumor, Astrocytoma, Brain and Spinal Cord Tumor, Brain Stem Glioma, Central Nervous System Atypical Teratoid/Rhabdoid Tumor, Central Nervous System Embryonal Tumor, Breast Cancer, Bronchial Tumor, Burkitt Lymphoma, Carcinoid Tumor, Cervical Cancer, Childhood Cancer, Chordoma, Chronic Lymphocytic Leukemia (CLL), Chronic Myelogenous Leukemia (CML), Chronic Myeloproliferative Disorder, Colon Cancer, Colorectal Cancer, Craniopharyngioma, Cutaneous T-Cell Lymphoma, Ductal Carcinoma In Situ (DCIS), Embryonal Tumors, Endometrial Cancer, Ependymoblastoma, Ependymoma, Esophageal Cancer, Esthesioneuroblastoma, Ewing Sarcoma, Extracranial Germ Cell Tumor, Extragonadal Germ Cell Tumor, Extrahepatic Bile Duct Cancer, Eye Cancer, Fibrous Histiocytoma of Bone, Gallbladder Cancer, Gastric Cancer, Gastrointestinal Carcinoid Tumor, Gastrointestinal Stromal Tumors (GIST), Extracranial Germ Cell Tumor, Extragonadal Germ Cell Tumor, Ovarian Germ Cell Tumor, Gestational Trophoblastic Tumor, Glioma, Hairy Cell Leukemia, Head and Neck Cancer, Heart Cancer, Hepatocellular Cancer, Hodgkin Lymphoma, Hypopharyngeal Cancer, Intraocular Melanoma, Islet Cell Tumors, Kaposi Sarcoma, Kidney Cancer, Langerhans Cell Histiocytosis, Laryngeal Cancer, Liver Cancer, Lobular Carcinoma In Situ (LCIS), Lung Cancer, Lymphoma, AIDS-Related Lymphoma, Male Breast Cancer, Medulloblastoma, Medulloepithelioma, Melanoma, Merkel Cell Carcinoma, Malignant Mesothelioma, Metastatic Squamous Neck Cancer with Occult Primary, Midline Tract Carcinoma Involving *NUT* Gene, Mouth Cancer, Multiple Endocrine Neoplasia Syndrome, Multiple Myeloma/Plasma Cell Neoplasm, Mycosis Fungoides, Myelodysplastic Syndrome, Myelodysplastic/Myeloproliferative Neoplasm, Multiple Myeloma, Nasal Cavity Cancer, Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip Cancer, Oropharyngeal Cancer, Osteosarcoma, Ovarian Cancer, Pancreatic Cancer, Papillomatosis, Paraganglioma, Parathyroid Cancer, Penile Cancer, Pharyngeal Cancer, Pheochromocytoma, Pineal Parenchymal Tumors of Intermediate Differentiation, Pineoblastoma, Pituitary Tumor, Plasma Cell Neoplasm, Pleuropulmonary Blastoma, Breast Cancer, Primary Central Nervous System (CNS) Lymphoma, Prostate Cancer, Rectal Cancer, Renal Cell Cancer, Renal Pelvis Cancer, Ureter Cancer, Retinoblastoma, Rhabdomyosarcoma, Salivary Gland Cancer, Sezary Syndrome, Skin Cancer, Small Cell Lung Cancer, Small Intestine Cancer, Soft Tissue Sarcoma, Squamous Cell Carcinom, Supratentorial Primitive Neuroectodermal Tumors, T-Cell Lymphoma, Testicular Cancer, Throat Cancer, Thymoma, Thymic Carcinoma, Thyroid Cancer, Transitional Cell Cancer of the Renal Pelvis and Ureter, Gestational Trophoblastic Tumor, Urethral Cancer, Uterine Cancer, Uterine Sarcoma, Waldenstrom Macroglobulinemia, or Wilms Tumor.

An embodiment provides a compound of the invention, or a pharmaceutical composition or formulation of the invention for the inhibition of monoacylglycerol lipase (MAGL).

Another embodiment provides a compound of the invention, or a pharmaceutical composition or formulation of the invention for use in the treatment of a condition modulated by MAGL.

Another embodiment provides a compound of the invention, or a pharmaceutical composition or formulation of the invention for use in the treatment of a condition selected from acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

Another embodiment provides a method for the treatment, in a mammal in need of said treatment, of a condition selected from: acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression. The method comprises administering to the mammal a therapeutically effective amount of any of the compounds or any of the pharmaceutical compositions or formulations described herein.

Another embodiment provides use of a compound of the invention in the manufacture of a medicament for use in the treatment of a condition selected from: acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

In embodiments the condition is cancer. In embodiments where the condition is cancer, the cancer may be selected from Acute Lymphoblastic Leukemia (ALL), Acute Myeloid Leukemia (AML), Adrenocortical Carcinoma, Anal Cancer, Appendix Cancer, Atypical Teratoid/Rhabdoid Tumor, Basal Cell Carcinoma, Bile Duct Cancer, Biliary Cancer, Bladder Cancer, Bone Cancer, Brain Tumor, Astrocytoma, Brain and Spinal Cord Tumor, Brain Stem Glioma, Central Nervous System Atypical Teratoid/Rhabdoid Tumor, Central Nervous System Embryonal Tumor, Breast Cancer, Bronchial Tumor, Burkitt Lymphoma, Carcinoid Tumor, Cervical Cancer, Childhood Cancer, Chordoma, Chronic Lymphocytic Leukemia (CLL), Chronic Myelogenous Leukemia (CML), Chronic Myeloproliferative Disorder, Colon Cancer, Colorectal Cancer, Craniopharyngioma, Cutaneous T-Cell Lymphoma, Ductal Carcinoma In Situ (DCIS), Embryonal Tumors, Endometrial Cancer, Ependymoblastoma, Ependymoma, Esophageal Cancer, Esthesioneuroblastoma, Ewing Sarcoma, Extracranial Germ Cell Tumor, Extragonadal Germ Cell Tumor, Extrahepatic Bile Duct Cancer, Eye Cancer, Fibrous Histiocytoma of Bone, Gallbladder Cancer, Gastric Cancer, Gastrointestinal Carcinoid Tumor, Gastrointestinal Stromal Tumors (GIST), Extracranial Germ Cell Tumor, Extragonadal Germ Cell Tumor, Ovarian Germ Cell Tumor, Gestational Trophoblastic Tumor, Glioma, Hairy Cell Leukemia, Head and Neck Cancer, Heart Cancer, Hepatocellular Cancer, Hodgkin Lymphoma, Hypopharyngeal Cancer, Intraocular Melanoma, Islet Cell Tumors, Kaposi Sarcoma, Kidney Cancer, Langerhans Cell Histiocytosis, Laryngeal Cancer, Liver Cancer, Lobular Carcinoma In Situ (LCIS), Lung Cancer, Lymphoma, AIDS-Related Lymphoma, Male Breast Cancer, Medulloblastoma, Medulloepithelioma, Melanoma, Merkel Cell Carcinoma, Malignant Mesothelioma, Metastatic Squamous Neck Cancer with Occult Primary, Midline Tract Carcinoma Involving *NUT* Gene, Mouth Cancer, Multiple Endocrine Neoplasia Syndrome, Multiple Myeloma/Plasma Cell Neoplasm, Mycosis Fungoides, Myelodysplastic Syndrome, Myelodysplastic/Myeloproliferative Neoplasm, Multiple Myeloma, Nasal Cavity Cancer, Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip Cancer, Oropharyngeal Cancer, Osteosarcoma, Ovarian Cancer, Pancreatic Cancer, Papillomatosis, Paraganglioma, Parathyroid Cancer, Penile Cancer, Pharyngeal Cancer, Pheochromocytoma, Pineal Parenchymal Tumors of Intermediate Differentiation, Pineoblastoma, Pituitary Tumor, Plasma Cell Neoplasm, Pleuropulmonary Blastoma, Breast Cancer, Primary Central Nervous System (CNS) Lymphoma, Prostate Cancer, Rectal Cancer, Renal Cell Cancer, Renal Pelvis Cancer, Ureter Cancer, Retinoblastoma, Rhabdomyosarcoma, Salivary Gland Cancer, Sezary Syndrome, Skin Cancer, Small Cell Lung Cancer, Small Intestine Cancer, Soft Tissue Sarcoma, Squamous Cell Carcinom, Supratentorial Primitive Neuroectodermal Tumors, T-Cell Lymphoma, Testicular Cancer, Throat Cancer, Thymoma, Thymic Carcinoma, Thyroid Cancer, Transitional Cell Cancer of the Renal Pelvis and Ureter, Gestational Trophoblastic Tumor, Urethral Cancer, Uterine Cancer, Uterine Sarcoma, Waldenstrom Macroglobulinemia, or Wilms Tumor. In embodiments where the condition is cancer, the cancer may be selected from breast cancer, ovarian cancer, melanoma, hepatocellular carcinoma, colorectal cancer, prostate cancer, or nasopharyngeal carcinoma.

MAGL inhibition is also useful in other applications. For example, MAGL inhibitors may be used *in vitro,* in research and other settings where inhibition of one or more lipases and MAGL more specifically is desirable. Embodiments therefore provide use of a compound of the invention, or a pharmaceutical composition or formulation of the invention for the inhibition of MAGL.

### FORMULATIONS AND ADMINISTRATION

Compounds of the invention may be administered orally, topically, intravenously, subcutaneously, buccally, rectally, dermally, nasally, tracheally, bronchially, by any other parenteral route, as an oral or nasal spray or via inhalation. The compounds may be administered in the form of pharmaceutical preparations comprising prodrug or active compound either as a free compound or, for example, a pharmaceutically acceptable nontoxic organic or inorganic acid or base addition salt, in a pharmaceutically acceptable dosage form. Depending upon the disorder and patient to be treated and the route of administration, the compositions may be administered at varying doses.

Typically, therefore, the pharmaceutical compounds of the invention may be administered orally, topically, or parenterally ("parenterally" as used herein, refers to modes of administration which include intravenous, intramuscular, intraperitoneal, intrasternal, subcutaneous and intraarticular injection and infusion) to a host to obtain a protease-inhibitory effect. In the case of larger animals, such as humans, the compounds may be administered alone or as compositions in combination with pharmaceutically acceptable diluents, excipients or carriers.

Actual dosage levels of active ingredients in the pharmaceutical formulations and pharmaceutical compositions of this invention may be varied so as to obtain an amount of the active compound(s) that is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

In the treatment, prevention, control, amelioration, or reduction of risk of conditions which require inhibition of MAGL activity, an appropriate dosage level may generally be about 0.01 to 500 mg per kg patient body weight per day which can be administered in single or multiple doses. The dosage level will be about 0.1 to about 250 mg/kg per day; more preferably about 0.5 to about 100 mg/kg per day. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0 and 1000.0 milligrams of the active ingredient for the symptomatic adjustment of the dosage to the patient to be treated. The compounds may be administered on a regimen of 1 to 4 times per day, e.g. once or twice per day. The dosage regimen may be adjusted to provide the optimal therapeutic response.

According to a further aspect of the invention there is thus provided a pharmaceutical composition or formulation including a compound of the invention, optionally in admixture with a pharmaceutically acceptable adjuvant, diluents or carrier.

Pharmaceutical formulations or compositions of this invention for parenteral injection may comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters, such as ethyl oleate. Proper fluidity can be maintained, for example, by the use of coating materials, such as lecithin, by the maintenance of the required particle size in the case of dispersions and by the use of surfactants.

These compositions may also contain adjuvants such as preservative, wetting agents, emulsifying agents and dispersing agents. Inhibition of the action of microorganisms may be ensured by the inclusion of various antibacterial and antifungal agents, for example, paraben, chlorobutanol or phenol sorbic acid. It may also be desirable to include isotonic agents, such as sugars or sodium chloride, for example. Prolonged absorption of the injectable pharmaceutical form may be brought about by the inclusion of agents (for example, aluminium monostearate and gelatine) which delay absorption.

Solid dosage forms for oral administration include capsules, tablets, pills, powders and granules. In such solid dosage forms, the active compound is typically mixed with at least one inert, pharmaceutically acceptable excipient or carrier, such as sodium citrate or dicalcium phosphate and/or one or more: a) fillers or extenders, such as starches, lactose, sucrose, glucose, mannitol and silicic acid; b) binders, such as carboxymethylcellulose, alginates, gelatine, polyvinylpyrrolidone, sucrose and acacia; c) humectants, such as glycerol; d) disintegrating agents, such as agar-agar, calcium carbonate, potato or tapioca starch, alginic acid, certain silicates and sodium carbonate; e) solution retarding agents, such as paraffin; f) absorption accelerators, such as quaternary ammonium compounds; g) wetting agents, such as acetyl alcohol and glycerol monostearate; h) absorbents, such as kaolin and bentonite clay and i) lubricants, such as talc, calcium stearate, magnesium stearate, solid polyethylene glycols, sodium lauryl sulfate and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Oral formulations may contain a dissolution aid. Examples of dissolution aids include non-ionic surface active agents, such as sucrose fatty acid esters, glycerol fatty acid esters, sorbitan fatty acid esters (e.g. sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkyamine oxides; bile acid and salts thereof (eg chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsufonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract, and/or in delayed fashion. Examples of embedding compositions include polymeric substances and waxes.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavoring and perfuming agents. Suspensions, in addition to the active compounds, may contain suspending agents such as ethoxylated isostearyl alcohols, polyoxyethylene sorbitol and sorbitan esters, microcrystalline cellulose, aluminium metahydroxide, bentonite, agar-agar, and traganacanth and mixtures thereof.

Compositions for rectal or vaginal administration may be in the form of suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, sprays, creams, foams, gels, ointments and inhalants. The active compound is mixed under sterile conditions with a pharmaceutically acceptable carrier and any needed preservatives, buffers or propellants which may be required. Ophthalmic formulations, eye ointments, powders and solutions are also contemplated as being within the scope of this invention.

Insofar as they do not interfere with the activity of the compounds, the compositions and formulations according to the present subject matter may contain other active agents intended, in particular, for use in treating a condition selected from acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression. The other active ingredients may be intended for use in treating cancer, for example breast cancer, ovarian cancer, melanoma, hepatocellular carcinoma, colorectal cancer, prostate cancer, or nasopharyngeal carcinoma.

The formulations according to the present subject matter may also contain inactive components. Suitable inactive components are well known in the art and are described in standard textbooks, such as Goodman and Gillman's: The Pharmacological Bases of Therapeutics, 8thEd., Gilman et al, Eds. Pergamon Press (1990), and Remington's Pharmaceutical Sciences, 17th Ed., Mack Publishing Co., Easton, Pa. (1990), both of which are incorporated by reference herein in their entirety.

The formulations and compositions may be used in combination with an additional pharmaceutical dosage form to enhance their effectiveness in treating any of the disorders described herein. In this regard, the present formulations and compositions may be administered as part of a regimen additionally including any other pharmaceutical and/or pharmaceutical dosage form known in the art as effective for the treatment of any of these disorders.

### Synthesis of Compounds

Compounds of the invention may be synthesised according the synthetic schemes disclosed herein.

### General procedure A

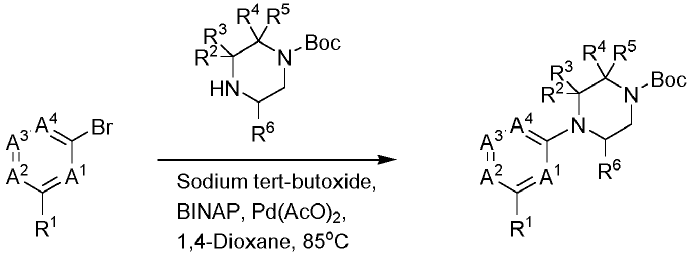

A mixture of appropriate bromobenzene (1eq) and appropriate mono Boc-protected piperazine (1eq) in the presence of palladium diacetate (0.04eq), BINAP (0.06eq ) and sodium tert-butoxide (1.5eq) in degassed 1,4-dioxane was heated to 85 °C under nitrogen atmosphere overnight. The reaction progress was monitored by TLC. Once completed, the reaction mixture was diluted with DCM, washed with water (1x), dried over anhydrous MgSO₄ and concentrated under reduced pressure. The residue was purified by silica gel column chromatography to give the product.

### General procedure B

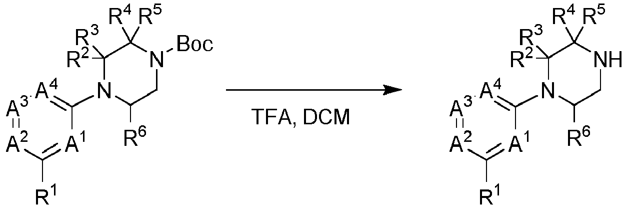

To a solution of appropriate tert-butyl phenylpiperazine-1-carboxylate in DCM was added TFA (20%, v/v) and the mixture was stirred at room temperature for 2h. The reaction progress was monitored by TLC. Once completed, then the mixture was diluted with DCM and washed with saturated aqueous NaHCO₃. The organic layers were collected and dried over anhydrous MgSO₄ and concentrated under reduced pressure. The residue was purified silica gel column chromatography to give the product.

### General procedure C

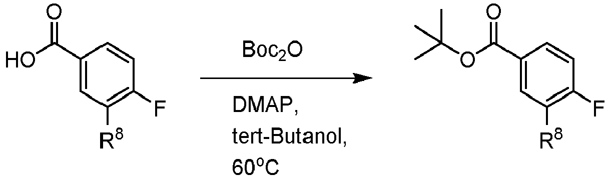

A mixture of benzoic acid (1 eq), di-*tert*-butyl dicarbonate (3 eq) and DMAP (0.3 eq) were dissolved in *tert*-butanol and the mixture was stirred at 60 °C overnight. The reaction progress was monitored by TLC. Once completed, the solvent was evaporated, and the residue was purified by silica gel column chromatography to give the product.

### General procedure D

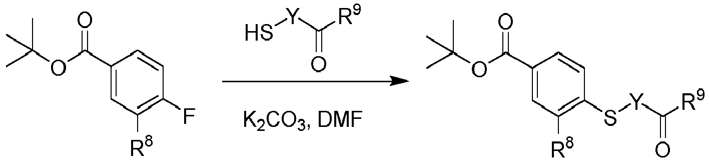

To a solution of appropriate tert-butyl 4-fluorobenzoate (1.2 eq) in ACN were added K₂CO₃ (3 eq) and appropriate thiol (1eq). The reaction mixture was stirred at RT overnight. The reaction progress was monitored by TLC. Once completed, the mixture was diluted with Et₂O, washed with water, dried over anhydrous MgSO₄. After filtration, the filtrate was concentrated under reduced pressure. The residue was purified by silica gel column chromatography.

### General procedure E

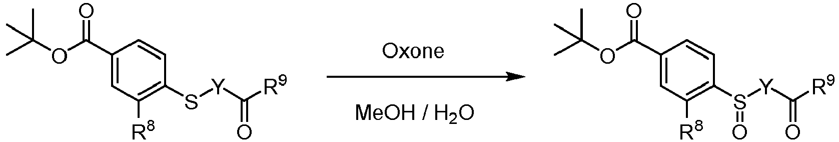

To a solution of appropriate sulfur (1 eq.) in MeOH was added oxone / water solution dropwise at 0 °C and the mixture was stirred at RT for 2h. The reaction progress was monitored by TLC. Once completed, the mixture was diluted with EtOAc and washed with water. The organic layer was dried over anhydrous MgSO₄ and after filtration, the filtrated was concentrated under reduced pressure. The residue was purified by silica column chromatography.

### General procedure F

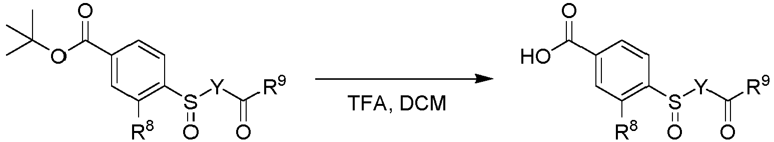

To a solution of appropriate *tert*-butyl protected carboxylic acid in DCM was added TFA (20%) and the mixture was stirred at RT for 6 h. The reaction progress was monitored by TLC. Once completed, the solvent was removed under reduced pressure and the residue was purified silica gel column chromatography.

### General procedure G

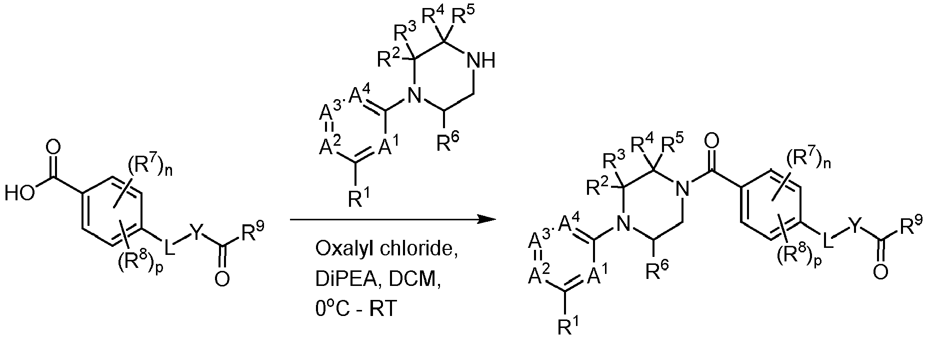

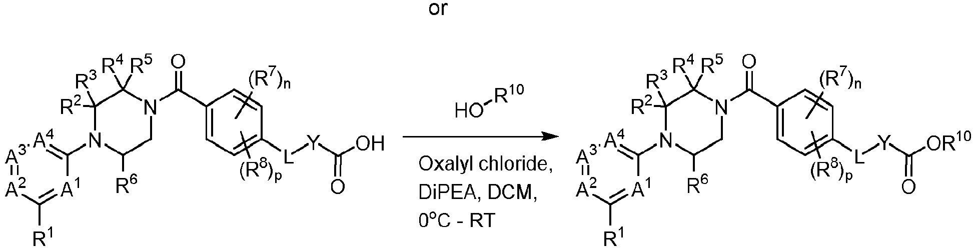

To a solution of appropriate carboxylic acid (1eq) in dried DCM was cooled down to 0 °C with ice bath. Then 2 drops of DMF and oxalyl chloride (1.2 eq) were added and the mixture was allowed to warm to room temperature and continuously stirred for 2h. The reaction progress was monitored by TLC. Once completed, then the mixture was added to a solution of appropriate alcohol or amine (3eq) and DIPEA (3eq) dropwise at 0 °C. Then the reaction mixture was stirred at room temperature overnight and diluted with DCM, washed with water (1x), dried over anhydrous MgSO₄ and concentrated under reduced pressure. The residue was purified by HPLC-MS to give the product.

### General procedure H

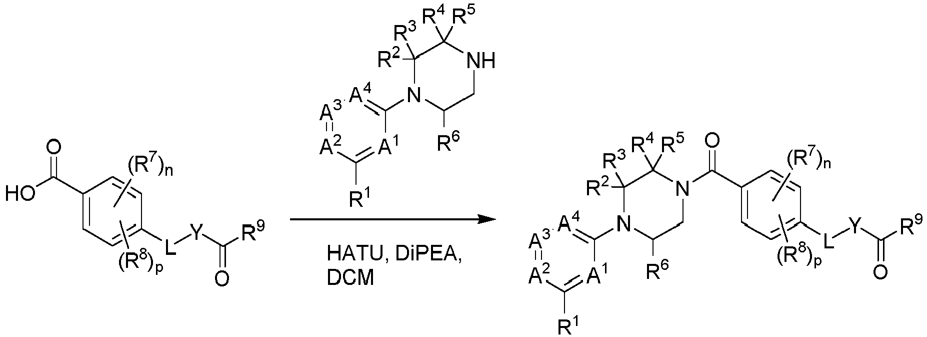

To a suspension or solution of appropriate benzoic acid (1eq) in DCM was added HATU (1.5eq) and DIPEA (3eq) and then the mixture was stirred at room temperature for 1h. The appropriate phenylpiperazine(1eq) was added and the mixture was stirred overnight. The reaction progress was monitored by TLC. Once completed, the mixture was diluted with DCM, washed with water (1x), dried over anhydrous MgSO₄ and concentrated under reduce pressure. The residue was purified by silica gel column chromatography to give the product.

### General procedure I

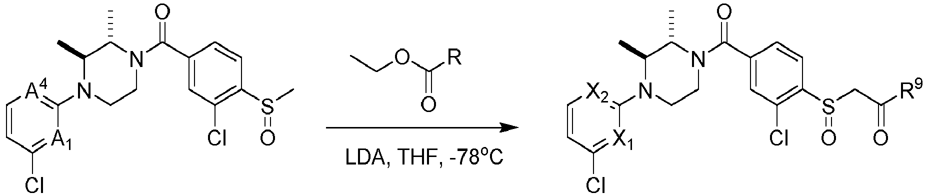

To a solution of appropriate methyl sulfoxide (1 eq) in anhydrous THF was added LDA (2 eq) at -78 °C and the reaction mixture was stirred for 30 min before the appropriate ester (10 eq) was added. The reaction progress was monitored by TLC. Once completed, the mixture was quenched with NH₄Cl solution, extracted with DCM and dried over anhydrous MgSO₄. After filtration, the filtrate was concentrated under reduced pressure. The residue was purified by silica gel column chromatography.

### General procedure J

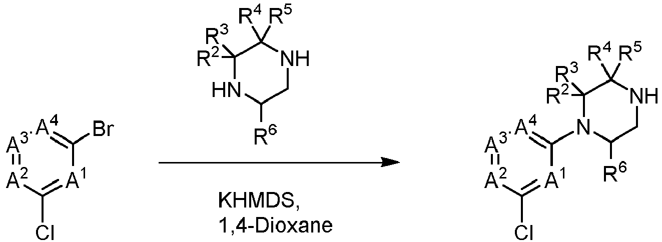

To a solution of appropriate piperazine (1 eq) and bromochlorobenzene or bromochloropyridine (1 eq) in 1,4-dioxane was added KHMDS (1.5 eq) and the mixture was stirred overnight at 100 °C or RT. The reaction progress was monitored by TLC. Once completed, the reaction mixture was diluted with DCM, washed with water and dried over anhydrous MgSO₄. After filtration, the filtrate was concentrated under reduced pressure. The residue was purified by silica gel column chromatography.

### General Scheme A - Examples 2-4, 8-31

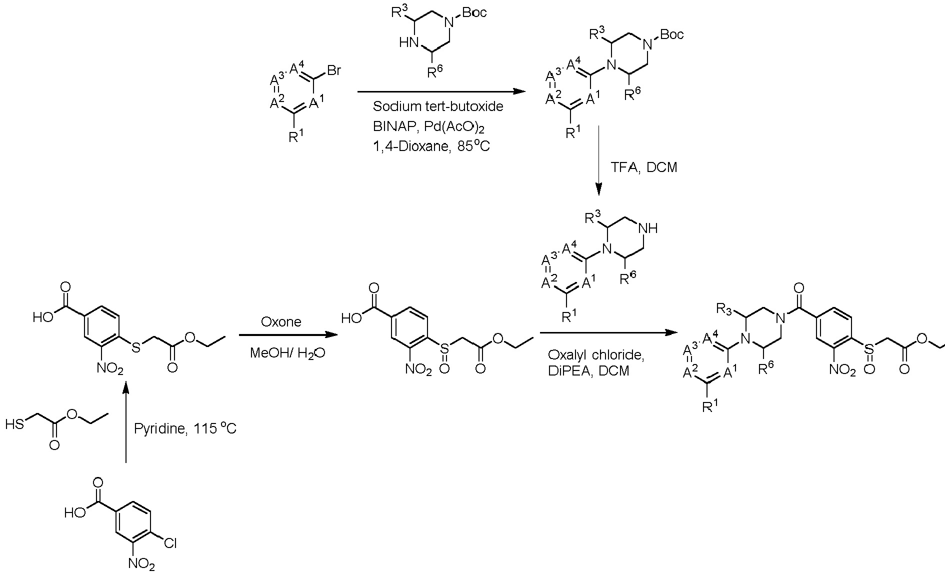

### General Scheme B - Examples 5-6

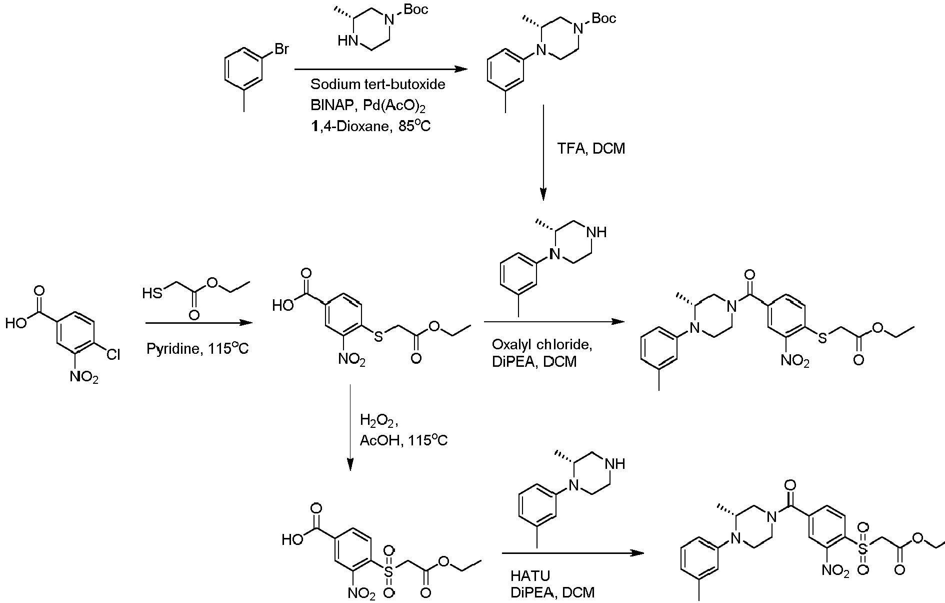

### General Scheme C - Example 7

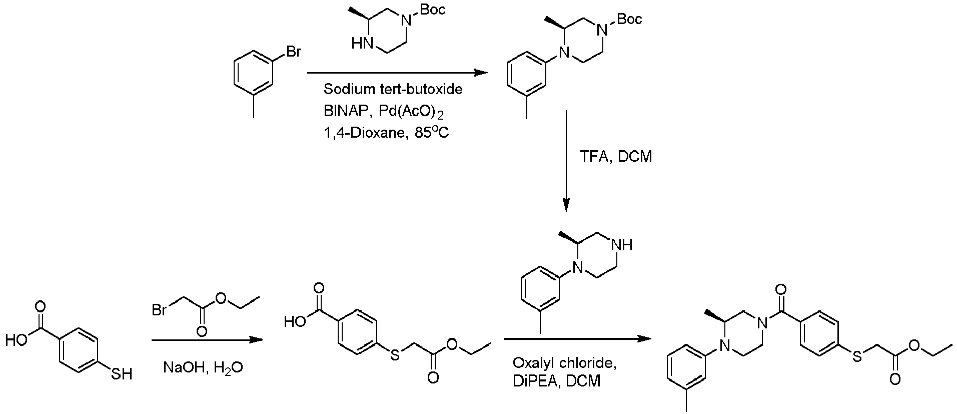

### General Scheme D - Examples 32-41

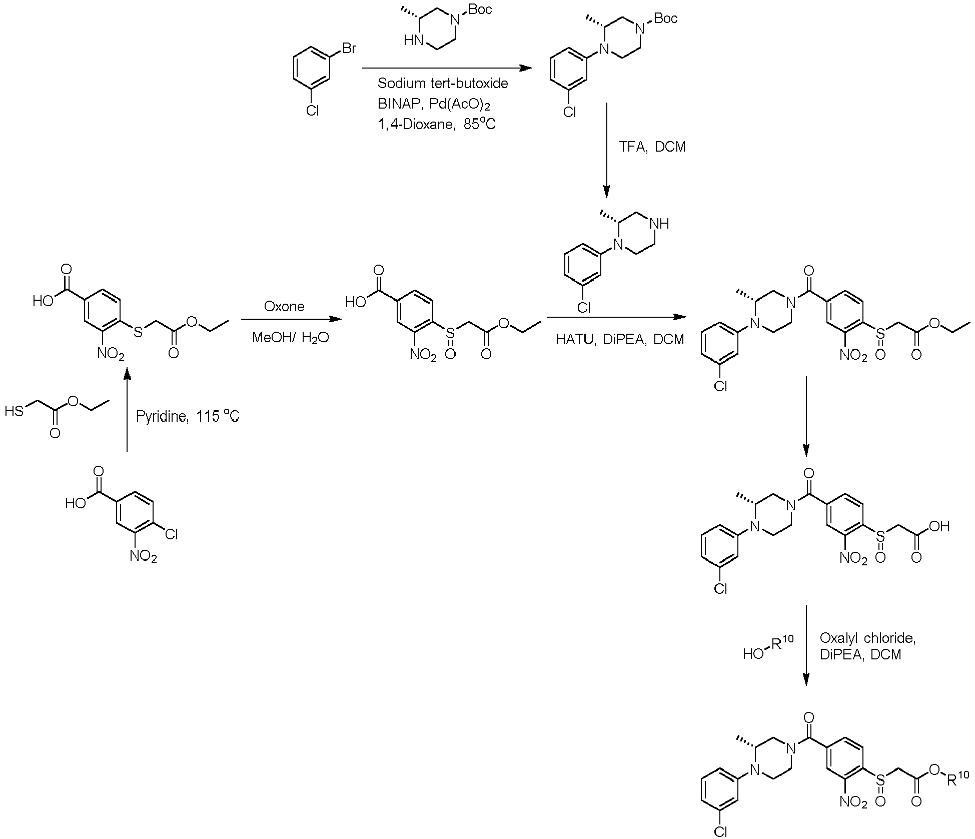

### General Scheme E - Examples 42-50

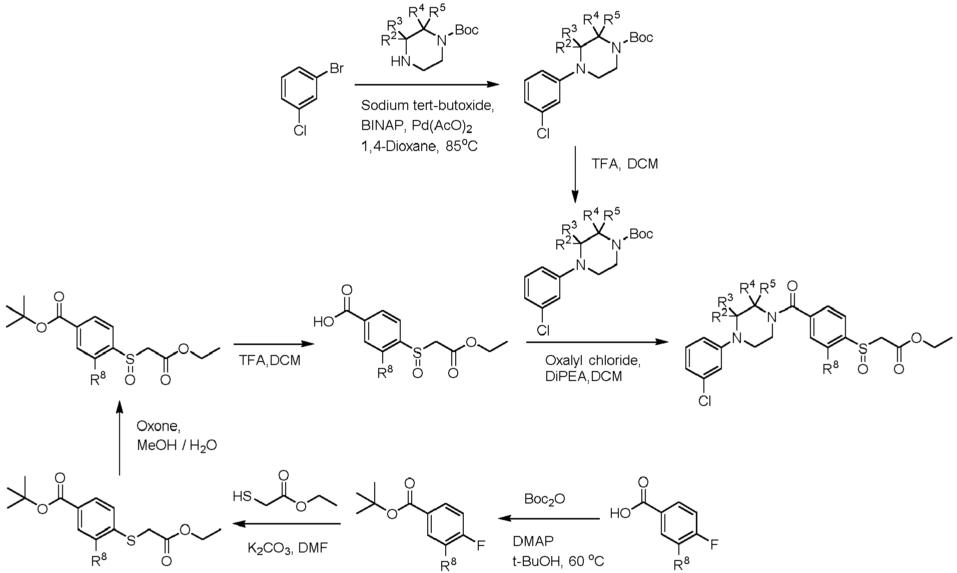

### General Scheme F - Examples 51-53, 56-67

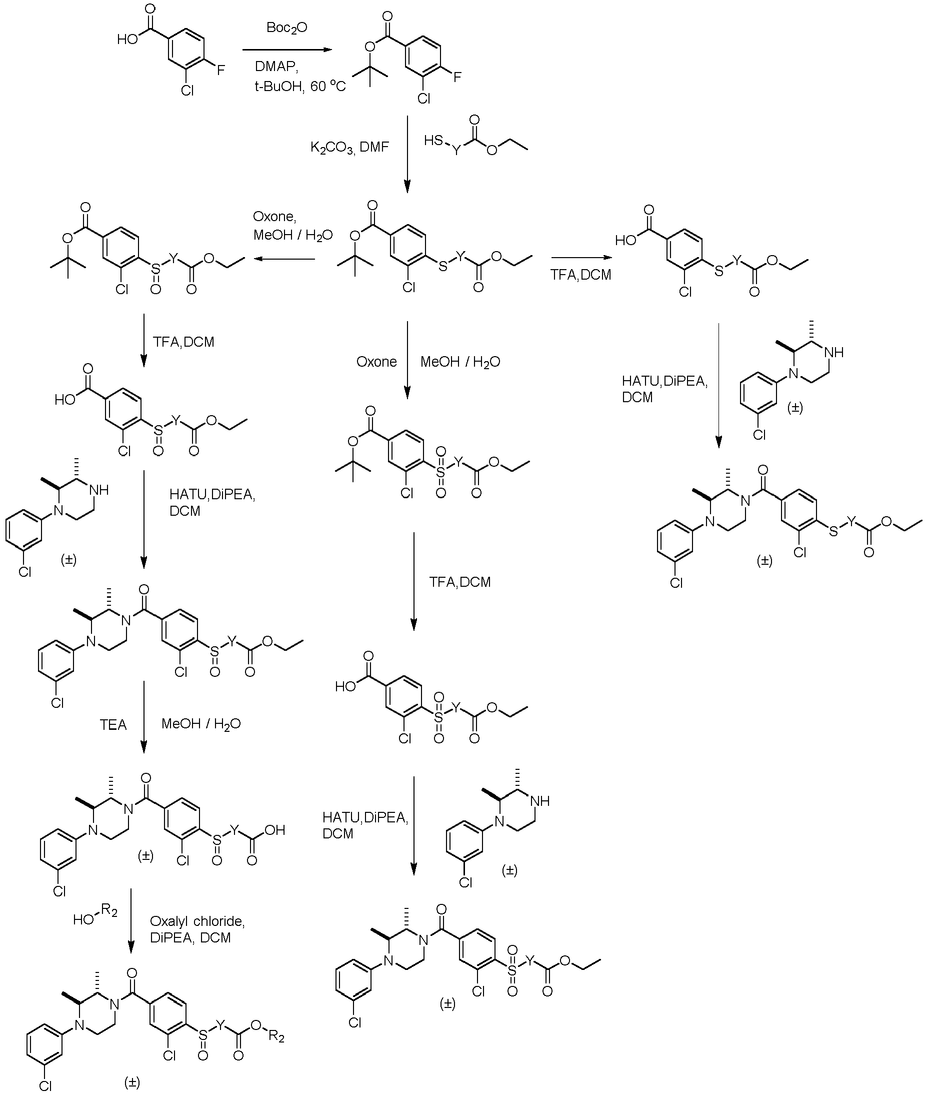

### General Scheme G - Example 54

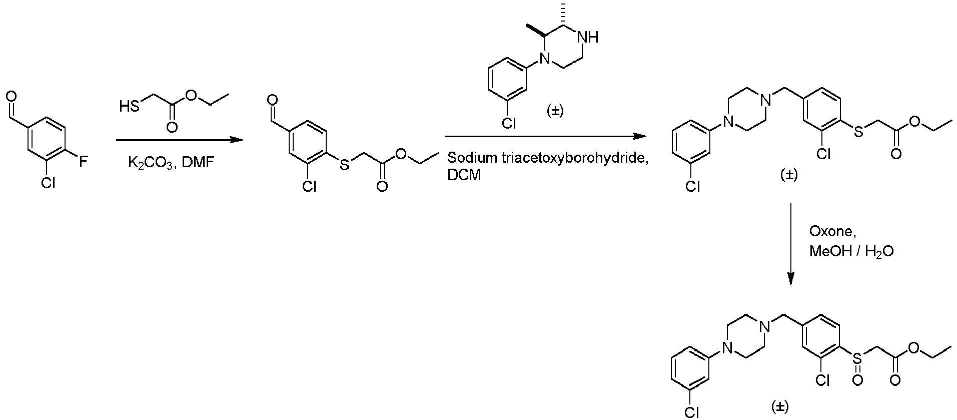

### General Scheme H - Example 55

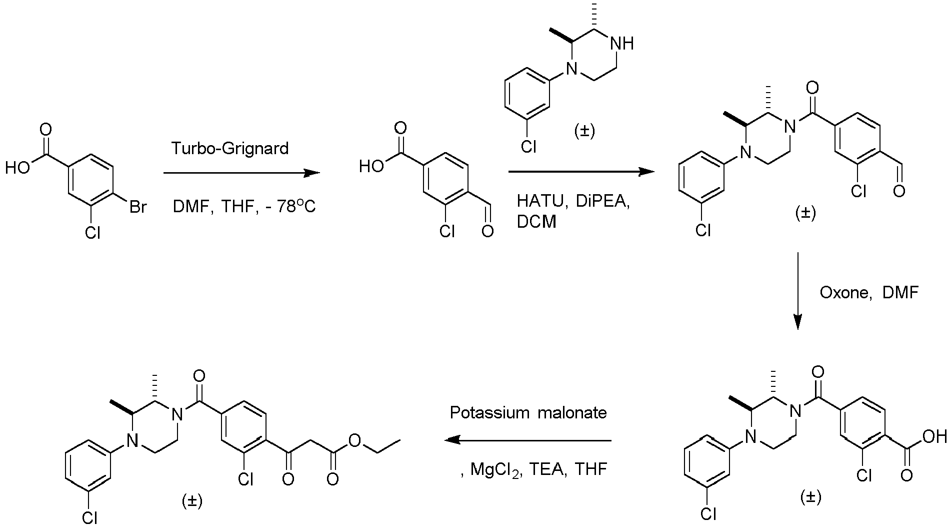

### General Scheme I - Examples 68-71

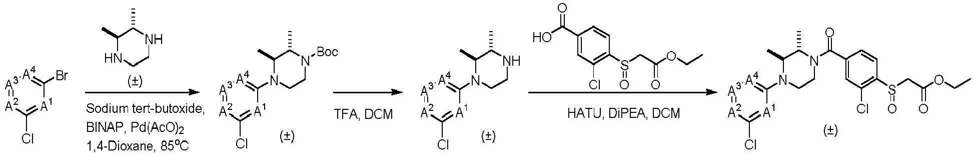

### General Scheme J - Example 72

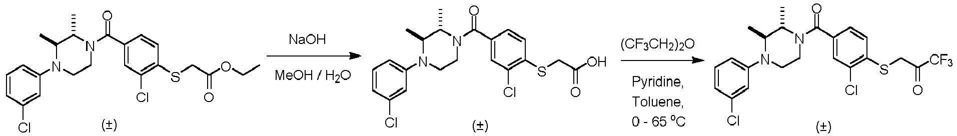

### General Scheme K - Examples 73-105

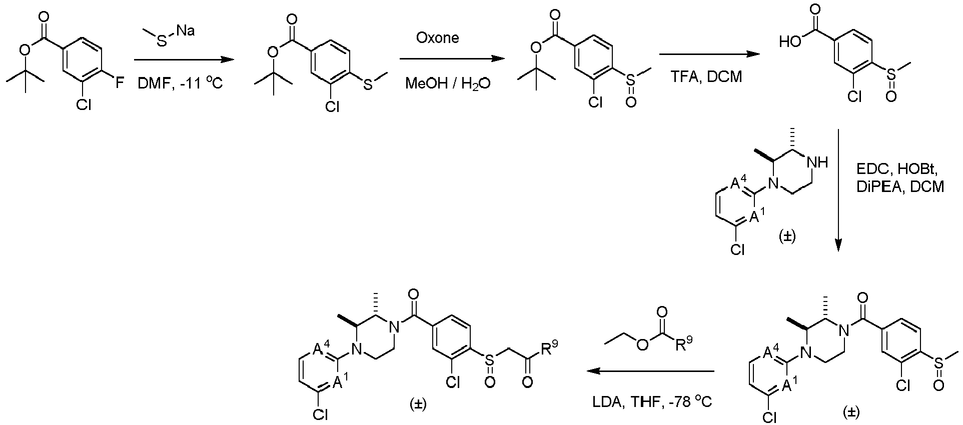

### FURTHER EMBODIMENTS

The invention and disclosure further include the subject matter of the following numbered clauses:
1. A compound of formula (I), or pharmaceutically acceptable salts or solvates thereof: 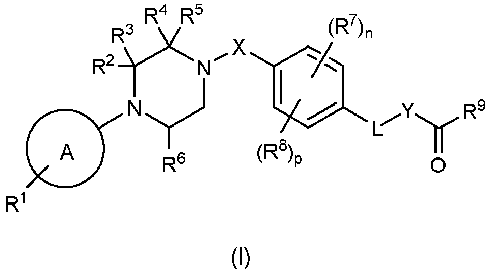 wherein
   A is a 6-membered aromatic ring, or a 5- or 6-membered heteroaromatic ring optionally comprising at least one N atom;
   X is selected from -C(O)- and -C(R^{a}R^{b})-;
   L is selected from S(=O)-, -SO₂-, -S-, -O-, -C(O)-, -CH₂-, -C(R^{c}R^{d})-;
   Y is -CH₂-, -CF₂-, -C(R^{c}R^{d})-;
   R¹ is selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl;
   each R² and R⁴ is independently selected from H and C₁₋₄ alkyl;
   each R³, R⁵ and R⁶ is independently selected from H and C₁₋₄ alkyl; or R³ and R⁵ together form a 5- or 6-membered ring and R⁶ is selected from H and C₁₋₄ alkyl; or R³ and R⁶ together form an alkyl or heteroalkyl bridge and R⁵ is selected from H and C₁₋₄ alkyl;
   each R⁷ and R⁸ is independently selected from H, halo, C₁₋₄ alkyl, C₁₋₄ haloalkyl, C₁₋₄ alkoxy, CN and NO₂;
   R⁹ is selected from H, C₁₋₆ haloalkyl, OR¹⁰, C₁₋₆ alkyl, C₂₋₈ alkyl interrupted by 1, 2 or 3 ether linkages, C₃₋₈ cycloalkyl, C₆ aryl, -(C₂₋₄ haloalkyl)-OR¹¹, -(C₁₋₆ haloalkyl)-R¹¹, ,or heterocyclyl;
   R¹⁰ is selected from H, C₁₋₆ alkyl, C₃₋₈ cycloalkyl, -(C₁₋₄ alkyl)_{d}-aryl, -(C₁₋₄ alkyl)ₑ-heterocyclyl;
   R¹¹ is selected from C₁₋₆ alkyl, C₂₋₈ alkyl interrupted by 1, 2 or 3 ether linkages, C₃₋₈ cycloalkyl, C₆ aryl, or heterocyclyl;
   wherein each R⁹, R¹⁰ and R¹¹ is optionally substituted where chemically possible with one, two or three groups independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl;
   R^{a} and R^{b} are independently selected from H, and C₁₋₄ alkyl; or wherein R^{a} and R^{b} together form a 3- to 6-membered cycloalkyl or heterocycloalkyl ring system;
   R^{c} and R^{d} are independently selected at each occurrence from H, F, C₁₋₄ alkyl, and C₁₋₄ haloalkyl; or R^{c} and R^{d} together form a 3- to 6-membered cycloalkyl or heterocycloalkyl ring system;
   a, b, d and e are independently selected from 0, 1 and 2;
   n is selected from 0, 1, or 2; and
   p is selected from 0, 1, or 2.
2. The compound of clause 1, wherein A is selected from phenyl, pyridyl or pyrimidyl.
3. The compound of clause 1, wherein the compound is a compound of formula (II) or a pharmaceutically acceptable salt or solvate thereof: 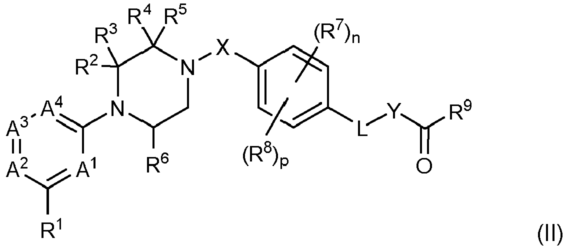
   wherein A¹, A³ and A⁴ are each independently selected from CH, CR¹² or N, and A² is selected from CH or CR¹²; and
   R¹² is selected from H, halo, CN, OCF₃, C₁₋₄ alkyl, C₁₋₄ haloalkyl, and C₁₋₄ alkoxy.
4. The compound of clause 3, wherein any one, two or three of A¹, A³ and A⁴ are N.
5. The compound of clause 3 or clause 4, wherein one of A¹ and A⁴ is N, one of A¹ and A⁴ is CH or CR¹², and A³ is CH or CR¹².
6. The compound of clause 3, wherein each of A¹, A³ and A⁴ is independently selected from CH or CR¹².
7. The compound of any preceding clause, wherein the compound is a compound of formula (III) or pharmaceutically acceptable salt or solvate thereof: 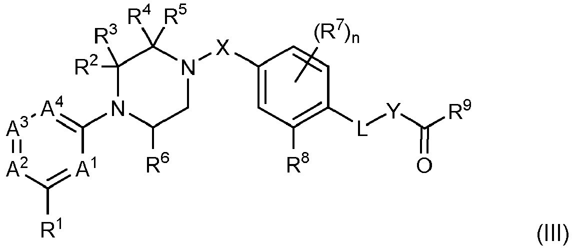
8. The compound of any preceding clause, wherein the compound is a compound of any of formulae (IVa), (IVb), or (IVc), or pharmaceutically acceptable salt or solvate thereof: 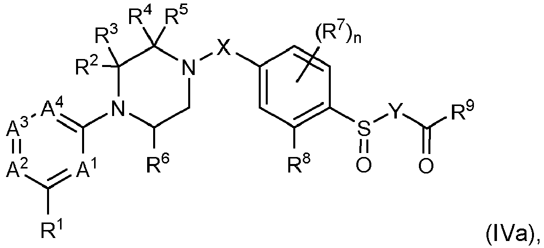 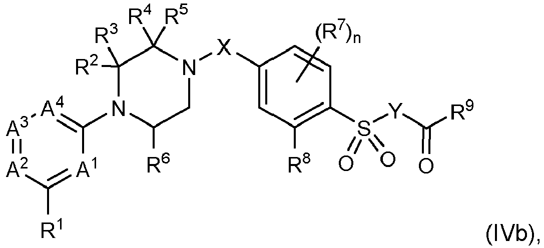 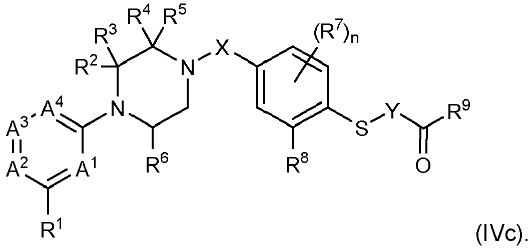
9. The compound of any preceding clause, wherein the compound is a compound of formula (V) or pharmaceutically acceptable salt thereof: 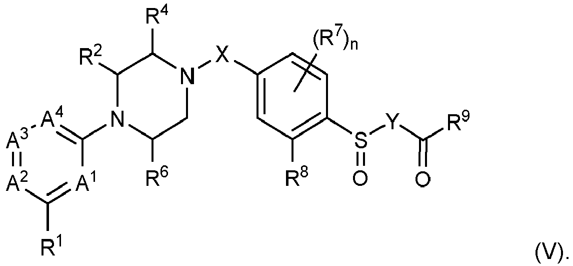
10. The compound of clause 9, wherein R² and R⁴ are arranged *trans* to one another.
11. The compound any preceding clause, wherein X is -C(O)- or -CH₂-.
12. The compound of clause 10, wherein X is -C(O)-.
13. The compound of any preceding clause, wherein Y is -CH₂-, -CH(CH₃)-, 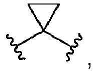 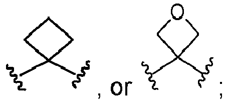 optionally wherein Y is -CH₂-.
14. The compound of any preceding clause, wherein R⁹ is selected from C₁₋₄ haloalkyl, C₂₋₆ alkyl interrupted by 1, 2 or 3 ether linkages, OR¹⁰, -(C₂₋₄ haloalkyl)-OR¹¹, (C₁₋₆ haloalkyl)-R¹¹, substituted or unsubstituted 6-membered aryl, and substituted or unsubstituted 5- or 6-membered heterocyclyl,
   wherein R¹⁰ is selected from C₁₋₄ alkyl, C₃₋₈ cycloalkyl or -(C₁₋₄ alkyl)-aryl,
   wherein R¹¹ is selected from C₁₋₄ alkyl, C₂₋₆ alkyl interrupted by 1, 2 or 3 ether linkages, C₃₋₆ cycloalkyl, or C₃₋₆ heterocycloalkyl,
   wherein each R⁹, R¹⁰ and R¹¹ is optionally substituted where chemically possible with one, two or three groups each independently selected from H, OH, =O, CN, halo, C₁₋₄ haloalkyl, C₁₋₄ alkoxy, OCF₃, and phenyl.
15. The compound of clause 14, wherein R⁹ is selected from -CF₂CH₃, -CF₂CH₂CH₃, - CF₃, -CHF₂, -CF₂-phenyl, -OCH₂CH₃, -OCH(CH₃)₂, -OCH(CH₃)CH₂CH₃, -CF₂CH₂O-fluorophenyl, -CF₂CH₂O-oxetane, -CF₂CH₂OCH₂CH₂OCH₃, -CF₂CH₂-pyrimidyl, -CF₂-oxetane, -OCH₂CH(OH)CH₂OH, -OCH₂CH₂OCH₃, -OCH(CH₃)CH₂OCH₃, - OCH₂CH₂CH₂CH₃, -OCH₂CH₂CH₂OH, -OCH₂CH₂OCH₂CH₂OCH₂CH₃, -OCH₂CF₃, -OCH₃, - OC(CH₃)₂CH₂CH₃, -OC(CH₃)₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, oxazol-2-yl, thiazol-2-yl, 5-methyloxazol-2-yl, pyridazin-3-yl, 6-(trifluoromethyl)pyridin-3-yl, pyridazin-4-yl, pyridin-4-yl, pyrazin-2-yl, pyridin-3-yl, phenyl, 4-cyanophenyl, 2,3-difluorophenyl, 3,4-difluorophenyl, 2,4-difluorophenyl, 2-fluorophenyl, 4-fluorophenyl, 3,5-difluorophenyl, or 4-(trifluoromethyl)phenyl.
16. The compound of clause 14 or clause 15, wherein R⁹ is selected from -CF₂CH₃, - CF₂CH₂CH₃, -CF₃, -CHF₂, -CF₂-phenyl, -OCH₂CH₃, -OCH(CH₃)₂, -OCH(CH₃)CH₂CH₃, , - OCH₂CH(OH)CH₂OH, -OCH₂CH₂OCH₃, -OCH(CH₃)CH₂OCH₃, -OCH₂CH₂CH₂CH₃, - OCH₂CH₂CH₂OH, -OCH₂CH₂OCH₂CH₂OCH₂CH₃, -OCH₂CF₃, -OCH₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, 4-cyanophenyl,. 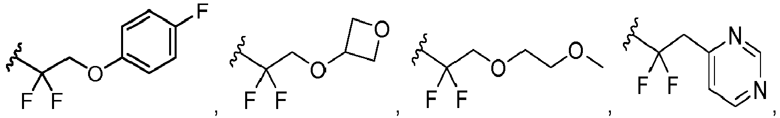 and 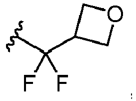 optionally wherein R⁹ is selected from -CF₂CH₃, -CF₂CH₂CH₃, -CF₃, -CHF₂,-CF₂-phenyl, -OCH₂CH₃, -OCH(CH₃)₂, -OCH(CH₃)CH₂CH₃, , -OCH₂CH(OH)CH₂OH,-OCH₂CH₂OCH₃, -OCH(CH₃)CH₂OCH₃, -OCH₂CH₂CH₂CH₃, -OCH₂CH₂CH₂OH,-OCH₂CH₂OCH₂CH₂OCH₂CH₃, -OCH₂CF₃, -OCH₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, and 4-cyanophenyl.
17. The compound of any preceding clause, wherein L is selected from -S(=O)-, -SO₂- and -S-; optionally wherein L is selected from -S(=O)-, and -S-; further optionally wherein L is -S(=O)-.
18. The compound of any preceding clause, wherein R¹ is selected from H, halo, C₁₋₄ alkyl, C₁₋₄ haloalkyl, or O-(C₁₋₄ alkyl)ₐ-aryl; optionally wherein R¹ is selected from H, halo, C₁₋₄ alkyl, C₁₋₄ haloalkyl, or O-(C₁₋₄ alkyl)ₐ-aryl; further optionally wherein R¹ is halo.
19. The compound of any preceding clause, wherein a is selected from 1 and 2; optionally wherein a is 1.
20. The compound of any preceding clause, wherein b is selected from 1 and 2; optionally wherein b is 1.
21. The compound of any preceding clause, wherein at least one of R² and R⁴ is C₁₋₄ alkyl (e.g. CH₃); optionally wherein both of R² and R⁴ are C₁₋₄ alkyl (e.g. CH₃).
22. The compound of clause 21, wherein R² and R⁴ are arranged *trans* to one another.
23. The compound of any preceding clause, wherein each R³, R⁵ and R⁶ may be independently selected from H and C₁₋₄ alkyl; optionally wherein each R³, R⁵ and R⁶ may be independently selected from H and CH₃.
24. The compound of any preceding clause, wherein each R⁷ and R⁸ may be independently selected from H, halo, C₁₋₄ haloalkyl, and NO₂; optionally wherein each R⁷ and R⁸ may be independently selected from halo, C₁₋₄ haloalkyl, and NO₂.
25. The compound of any preceding claim, wherein p is 0 or 1.
26. The compound of any preceding claim, wherein n is 0 or 1, optionally wherein p is 1.
27. A pharmaceutical composition comprising a compound of any preceding claim.
28. The composition of clause 27, further comprising a pharmaceutically acceptable excipient.
29. The compound of any of clauses 1 to 26 or the pharmaceutical composition of clause 27 or clause 28 for use as a medicament.
30. The compound of any of clauses 1 to 26 or the pharmaceutical composition of clause 27 or clause 28 for use in the treatment of a condition which is modulated by monoacylglycerol lipase (MAGL).
31. The compound of any of clauses 1 to 26 or the pharmaceutical composition of clause 27 or clause 28 for use in the treatment of a condition selected from acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.
32. The compound for use according to clause 31, wherein the condition is cancer.
33. The compound for use according to clause 32, wherein the condition is breast cancer, ovarian cancer, melanoma, hepatocellular carcinoma, colorectal cancer, prostate cancer, or nasopharyngeal carcinoma.
34. Use of the compound of any of clauses 1 to 26 or the pharmaceutical composition of clause 27 or clause 28 for the inhibition of monoacylglycerol lipase (MAGL).
35. Use of a compound of any one of clauses 1 to 26 in the manufacture of a medicament for use in the treatment of a condition selected from: acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

### EXAMPLES

### Example 1: Synthesis of Intermediates

### Intermediate 1: 4-((2-Ethoxy-2-oxoethyl)thio)-3-nitrobenzoic acid.

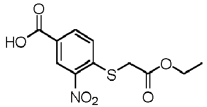

To a solution of 4-chloro-3-nitrobenzoic acid (1258 mg, 6.24.00 mmol, 1.5 eq) in pyridine (5 mL) was added ethyl mercaptoacetate (500 mg, 4.16 mmol, 1.00 eq) and the mixture was heated to 115 °C overnight in an oil bath. The reaction progress was monitored by TLC. Once completed, the resulted mixture was allowed to cool to rt, and the pH was adjusted to 1 with 1M HCI solution . The precipitate was filtered and the solid were washed with water to provide the product (1010mg, 3.54mmol, 85%). ¹H NMR (400 MHz, Methanol-d4) δ 8.73 (d, *J* = 1.9 Hz, 1H), 8.16 (dd, *J* = 8.5, 1.9 Hz, 1H), 7.66 (d, *J* = 8.5 Hz, 1H), 4.20 (q, *J* = 7.1 Hz, 2H), 4.00 (s, 2H), 1.25 (t, *J* = 7.1 Hz, 3H). ¹³C NMR (101 MHz, MeOD) δ 170.38, 167.34, 143.24, 135.02, 129.44, 128.46, 128.08, 63.20, 35.65, 14.53.

### Intermediate 2: 4-((2-Ethoxy-2-oxoethyl)sulfinyl)-3-nitrobenzoic acid.

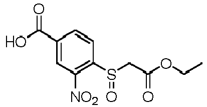

To a solution of 4-((2-ethoxy-2-oxoethyl)thio)-3-nitrobenzoic acid (285 mg, 1.00 mmol, 1.00 eq) in 13ml methanol was added Oxone (62 mg, 1.00 mmol, 1.00 eq)/ water (4 mL) solution dropwise at 0 °C, and the reaction mixture was stirred at rt for 2.5 h. The reaction progress was monitored by TLC. Once completed, the mixture was diluted with water and extracted with DCM. The organic layers were washed with brine and dried over anhydrous magnesium sulfate. After filtration, the filtrate was concentrated under reduced pressure. The residue was purified by silica gel column chromatography (MeOH/DCM, 1%→2%) to afford the product (210 mg, 0.70 mmol, 70%). ¹H NMR (400 MHz, Methanol-d4) δ 8.88 (d, *J* = 1.6 Hz, 1H), 8.61 (dd, *J* = 8.2, 1.6 Hz, 1H), 8.34 (d, *J* = 8.2 Hz, 1H), 4.36 (d, *J* = 14.4 Hz, 1H), 4.29 - 4.13 (m, 2H), 3.82 (d, *J* = 14.5 Hz, 1H), 1.26 (t, *J* = 7.1 Hz, 3H). ¹³C NMR (101 MHz, MeOD) δ 166.82, 166.60, 147.30, 136.89, 136.55, 128.43, 127.26, 63.37, 61.32, 14.54.

### Intermediate 3: 4-((2-Ethoxy-2-oxoethyl)sulfonyl)-3-nitrobenzoic acid

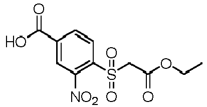

To a solution of 4-((2-ethoxy-2-oxoethyl)thio)-3-nitrobenzoic acid (1100 mg, 3.86 mmol, 1 eq) in 20ml AcOH was added 35% H₂O₂, tert-butylammonium hydrogen sulfate (65 mg, 0.19 mmol, 0.05 eq) and sodium tungstate dihydrate (127 mg, 0.39 mmol, 0.1 eq). Then the reaction mixture was refluxed for 3h. The reaction progress was monitored by TLC. Once completed, the mixture was cooled down to room temperature and diluted with water, extracted with EtOAc and dried over anhydrous magnesium sulfate. After filtration, the filtrate was concentrated under reduced pressure. The residue was purified by silica gel column chromatography (MeOH/DCM, 1%→2%) to afford the product (618mg, 1.95 mmol, 51%). ¹H NMR (400 MHz, DMSO-d) δ 8.51 (d, J = 1.6 Hz, 1H), 8.43 (dd, J = 8.2, 1.7 Hz, 1H), 8.26 (d, J = 8.2 Hz, 1H), 4.93 (s, 2H), 4.10 (q, J = 7.1 Hz, 2H), 1.10 (t, J = 7.1 Hz, 3H). ¹³C NMR (101 MHz, DMSO-d) δ 164.46, 162.06, 148.41, 137.81, 134.35, 133.34, 133.28, 125.63, 62.09, 60.66, 13.71.

### Intermediate 4: 4-((2-Ethoxy-2-oxoethyl)thio)benzoic acid

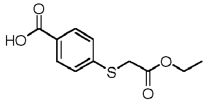

To a suspension of 4-mercaptobenzoic acid (462mg, 2.99 mmol, 1 eq) in 5ml water was added NaOH (180mg, 4.49 mmol, 1.5 eq) and the resulting solution was stirred for 30min at room temperature. Then ethyl 2-bromoacetate (500mg, 2.99 mmol, 1 eq) was slowly added to the solution and the reaction mixture was stirred for another 2h at room temperature. The reaction progress was monitored by TLC. Once completed, the mixture was acidified with 1M HCI and the obtained precipitate was filtrated, washed with water and dried to give the product (252mg, 1.05 mmol, 35 %). ¹H NMR (CDCl3, 400 MHz) δ 7.88 (d, J = 8.3 Hz, 2H), 7.40 - 7.26 (d, J = 8.3 Hz, 2H), 4.10 (q, J = 7.3 Hz, 2H), 3.79 (s, 2H), 1.15 (t, J = 7.2 Hz, 3H). ¹³C NMR (MeOD, 101 MHz) δ 170.94, 169.28, 143.74, 131.16, 129.19, 127.98, 62.72, 35.35, 14.37.

### Intermediate 5: 4-((2-Ethoxy-2-oxoethyl)sulfinyl)benzoic acid

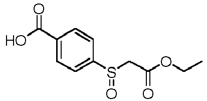

To a solution of 4-((2-Ethoxy-2-oxoethyl)thio)benzoic acid (100 mg, 0.42 mmol, 1.00 eq) in 5ml methanol was added Oxone (26 mg, 1.00 mmol, 1.00 eq)/water (5 mL) solution dropwise at 0 °C, and the reaction mixture was stirred at rt for 2.5 h. The reaction progress was monitored by TLC. Once completed, the mixture was diluted with water and extracted with DCM. The organic layers were washed with brine and dried over anhydrous magnesium sulfate. After filtration, the filtrate was concentrated under reduced pressure. The residue was purified by silica gel column chromatography (MeOH/DCM, 1%→2%) to afford the product (83mg, 0.33 mmol, 78%). ¹H NMR (400 MHz, Methanol-d4) δ 8.22 (d, J = 8.5 Hz, 2H), 7.84 (d, J = 8.5 Hz, 2H), 4.16 (q, J = 7.1 Hz, 2H), 4.06 (d, J = 14.4 Hz, 1H), 3.94 (d, J = 14.3 Hz, 1H), 1.20 (t, J = 7.1 Hz, 3H). ¹³C NMR (101 MHz, Methanol-d4) δ 168.37, 166.14, 148.49, 135.28, 131.64, 125.61, 63.07, 61.45, 14.33.

### Intermediate 6: tert-Butyl 4-phenylpiperazine-1-carboxylate

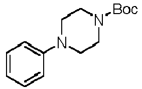

The title compound was synthesized using bromobenzene (160 mg, 1.00 mmol, 1 eq), *tert*-butyl piperazine-1-carboxylate (190 mg, 1.00 mmol, 1 eq), sodium *tert*-butoxide (240 mg, 2.50 mmol, 2.5 eq), rac-BINAP (60 mg, 0.10 mmol, 0.10 eq) and palladium diacetate ( 3 mg, 0.015 mmol, 0.015 eq) according to general procedure A. This yielded the product ( 252 mg, 0.10 mmol, 97%). 1H NMR (400 MHz, CDCl3) δ 7.31 - 7.25 (m, 2H), 6.97 - 6.91 (m, 2H), 6.91 - 6.86 (m, 1H), 3.58 (t, J = 6.0, 4.4 Hz, 4H), 3.13 (t, 4H), 1.48 (s, 9H). 13C NMR (101 MHz, CDCl3) δ 154.82, 151.37, 129.28, 120.37, 116.72, 79.95, 49.51, 28.53.

### Intermediate 7: 1-Phenylpiperazine

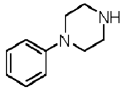

The title compound was synthesized using *tert*-butyl 4-phenylpiperazine-1-carboxylate ( 100 mg, 0.38 mmol, 1 eq.) according to general procedure B. This yield the product (54 mg, 0.34 mmol, 89%). 1H NMR (400 MHz, CDCl3) δ 7.31 - 7.21 (m, 2H), 6.96 - 6.88 (m, 2H), 6.90 - 6.81 (m, 1H), 3.13 (t, 4H), 3.02 (t, J = 6.2, 2.6 Hz, 4H), 2.37 (s, 1H). 13C NMR (101 MHz, CDCl3) δ 151.57, 129.20, 120.46, 116.63, 83.53.

### Intermediate 8: tert-Butyl 4-(m-tolyl)piperazine-1-carboxylate

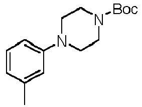

The title compound was synthesized using 1-bromo-3-methylbenzene (500 mg, 2.92 mmol, 1 eq), *tert*-butyl piperazine-1-carboxylate (817 mg, 4.39 mmol, 1.5 eq), caesium carbonate (1429 mg, 4.39 mmol, 1.5 eq), rac-BINAP (116 mg, 0.175 mmol, 0.06 eq) and palladium diacetate (26.3 mg, 0.117 mmol, 0.04 eq) according to general procedure A. This yielded the product (746 mg, 2.70 mmol, 92%). 1H NMR (CDCl3, 400 MHz) δ 7.13 (t, J = 7.8 Hz, 1H), 6.75 - 6.64 (m, 3H), 3.54 (t, J = 5.2 Hz, 4H), 3.06 (t, J = 5.2 Hz, 4H), 2.30 (s, 3H), 1.48 (s, 9H). 13C NMR (CDCl3, 101 MHz) δ 154.51, 151.22, 138.59, 128.86, 121.01, 117.33, 113.63, 79.57, 49.34, 28.32, 21.63.

### Intermediate 9: 1-(m-Tolyl)piperazine

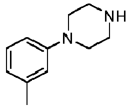

The title compound was synthesized using tert-butyl 4-(m-tolyl)piperazine-1-carboxylate ( 746 mg, 2.70 mmol, 1 eq) according to general procedure B. This yield the product (450 mg, 2.55 mmol, 95%). 1H NMR (400 MHz, Chloroform-d) δ 7.19 (dd, J = 8.2, 7.2 Hz, 1H), 6.85 - 6.68 (m, 3H), 5.93 (s, 1H), 3.37 - 3.27 (m, 4H), 3.26 - 3.19 (m, 4H), 2.35 (s, 3H). 13C NMR (101 MHz, Chloroform-d) δ 150.88, 138.88, 129.02, 121.48, 117.42, 113.72, 48.52, 44.66, 21.70.

### Intermediate 10: tert-Butyl 4-(3-fluorophenyl)piperazine-1-carboxylate

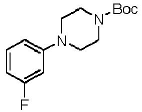

The title compound was synthesized using 1-bromo-3-fluorobenzene (100 mg, 0.57 mmol, 1 eq), *tert*-butyl piperazine-1-carboxylate (128 mg, 0.69 mmol, 1.2 eq), sodium tert-butoxide (82 mg, 0.86 mmol, 1.5 eq), rac-BINAP (21 mg, 0.034 mmol, 0.06 eq) and palladium diacetate (5 mg, 0.023 mmol, 0.04 eq) according to general procedure A. This yielded the product (145 mg, 0.52 mmol, 91%). LC/MS: calculated for [C₁₅H₂₁FN₂O₂ + H] = 281.17, found = 280.98.

### Intermediate 11: 1-(3-Fluorophenyl)piperazine

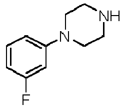

The title compound was synthesized using *tert*-butyl 4-(3-fluorophenyl)piperazine-1-carboxylate (100 mg, 0.36 mmol, 1 eq) according to general procedure B. This yield the product (62 mg, 0.34 mmol, 96 %). ¹H NMR (400 MHz, Chloroform-d) δ 7.19 (td, J = 8.2, 7.0 Hz, 1H), 6.67 (ddd, J = 8.4, 2.4, 0.8 Hz, 1H), 6.62 - 6.46 (m, 2H),3.37 (br, 1H), 3.25 - 3.12 (m, 4H), 3.08 - 2.95 (m, 4H). ¹³C NMR (101 MHz, Chloroform-d) δ 163.89 (d, J = 243.2 Hz), 153.32 (d, J = 9.7 Hz), 130.19 (d, J = 10.0 Hz), 111.30, 106.05 (d, J = 21.5 Hz), 102.80 (d, J = 25.0 Hz), 49.54, 45.76.

### Intermediate 12: tert-Butyl 4-(3-chlorophenyl)piperazine-1-carboxylate

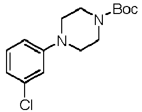

The title compound was synthesized using 1-bromo-3-chlorobenzene (191 mg, 1.00 mmol, 1 eq), *tert*-butyl piperazine-1-carboxylate (186 mg, 1 mmol, 1.00 eq), sodium tert-butoxide (144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (190 mg, 0.64 mmol, 64 %). ¹H NMR (400 MHz, Chloroform-d) δ 7.16 (t, J = 8.1 Hz, 1H), 6.87 (t, J = 2.2 Hz, 1H), 6.80 (dddd, J = 20.9, 8.4, 2.2, 0.9 Hz, 2H), 3.64 - 3.42 (t, J = 5.2 Hz, 4H), 3.12 (t, J = 5.2 Hz, 4H), 1.48 (s, 9H). ¹³C NMR (101 MHz, Chloroform-d) δ 154.67, 152.31, 134.99, 130.15, 119.83, 116.30, 114.44, 80.01, 48.89, 28.45.

### Intermediate 13: 1-(3-Chlorophenyl)piperazine

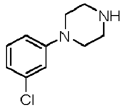

The title compound was synthesized using *tert*-butyl 4-(3-chlorophenyl)piperazine-1-carboxylate (100 mg, 0.34 mmol, 1 eq) according to general procedure B. This yield the product (62 mg, 0.31 mmol, 93 %). ¹H NMR (400 MHz, Chloroform-d) δ 7.18 (t, J = 8.0 Hz, 1H), 6.94 - 6.67 (m, 3H), 6.47 (br, 1H), 3.24 (t, J = 5.1 Hz, 3H), 3.14 (t, J = 5.2 Hz, 4H). ¹³C NMR (101 MHz, Chloroform-d) δ 152.09, 135.03, 130.23, 120.18, 116.31, 114.43, 48.41, 44.78.

### Intermediate 14: tert-Butyl 4-(4-chlorophenyl)piperazine-1-carboxylate

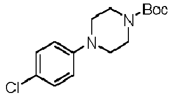

The title compound was synthesized using 1-bromo-4-chlorobenzene (191 mg, 1.00 mmol, 1 eq), *tert*-butyl piperazine-1-carboxylate (186 mg, 1 mmol, 1.00 eq), sodium tert-butoxide (144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (264 mg, 0.89 mmol, 89 %). ¹H NMR (400 MHz, Chloroform-d) δ 7.24 - 7.16 (m, 2H), 6.86 - 6.76 (m, 2H), 3.62 - 3.49 (m, 4H), 3.08 (t, J = 5.2 Hz, 4H), 1.48 (s, 9H). ¹³C NMR (101 MHz, Chloroform-d) δ 154.73, 149.97, 129.11, 125.18, 117.89, 80.05, 49.48, 28.50.

### Intermediate 15: 1-(4-Chlorophenyl)piperazine

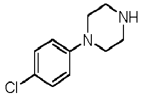

The title compound was synthesized using *tert*-butyl 4-(4-chlorophenyl)piperazine-1-carboxylate (100 mg, 0.34 mmol, 1 eq) according to general procedure B. This yield the product (66 mg, 0.33 mmol, 99 %). ¹H NMR (400 MHz, Chloroform-d) δ 7.23 - 7.17 (m, 2H), 6.86 - 6.79 (m, 2H), 3.12 (dt, J = 6.1, 3.7 Hz, 4H), 3.05 (dd, J = 6.4, 3.3 Hz, 4H). ¹³C NMR (101 MHz, Chloroform-d) δ 150.32, 129.06, 124.84, 117.50, 50.10, 45.81.

### Intermediate 16: tert-Butyl 4-(2-chlorophenyl)piperazine-1-carboxylate

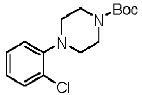

The title compound was synthesized using 1-bromo-2-chlorobenzene (191 mg, 1.00 mmol, 1 eq), *tert*-butyl piperazine-1-carboxylate (186 mg, 1 mmol, 1.00 eq), sodium tert-butoxide (144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (241 mg, 0.81 mmol, 81 %). ¹H NMR (400 MHz, Chloroform-d) δ 7.35 (dt, J = 7.9, 1.6 Hz, 1H), 7.28 - 7.11 (m, 1H), 7.05 - 6.89 (m, 2H), 3.68 - 3.49 (m, 4H), 3.09 - 2.83 (m, 4H), 1.49 (s, 9H). ¹³C NMR (101 MHz, CDCl3) δ 154.74, 149.05, 130.62, 128.87, 127.58, 123.95, 120.45, 79.66, 51.18, 44.08, 28.42.

### Intermediate 17: 1-(2-Chlorophenyl)piperazine

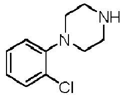

The title compound was synthesized using *tert*-butyl 4-(2-chlorophenyl)piperazine-1-carboxylate (100 mg, 0.34 mmol, 1 eq) according to general procedure B. This yield the product (63 mg, 0.32 mmol, 95 %). ¹H NMR (400 MHz, Chloroform-d) δ 7.35 (d, J = 8.0 Hz, 1H), 7.22 (t, J = 7.7 Hz, 1H), 7.08 - 6.90 (m, 2H), 4.27 (br, 1H), 3.21 - 2.86 (m, 8H). ¹³C NMR (101 MHz, Chloroform-d) δ 149.21, 131.61, 130.64, 127.65, 123.94, 120.50, 51.62, 45.63.

### Intermediate 18: tert-Butyl 4-(3-bromophenyl)piperazine-1-carboxylate

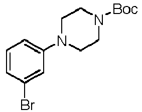

The title compound was synthesized using 1,3-dibromobenzene (236 mg, 1.00 mmol, 1 eq ), *tert*-butyl piperazine-1-carboxylate (186 mg, 1 mmol, 1.00 eq), sodium tert-butoxide ( 144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (259 mg, 0.76 mmol, 76 %). 1H NMR (400 MHz, Chloroform-d) δ 7.10 (t, J = 8.1 Hz, 1H), 7.02 (t, J = 2.1 Hz, 1H), 6.97 (ddd, J = 7.9, 1.8, 0.9 Hz, 1H), 6.81 (ddd, J = 8.3, 2.5, 0.9 Hz, 1H), 3.62 - 3.46 (m, 4H), 3.11 (t, J = 5.1 Hz, 4H), 1.48 (s, 9H). 13C NMR (101 MHz, Chloroform-d) δ 154.66, 152.45, 130.44, 123.26, 122.79, 119.24, 114.94, 80.02, 48.91, 43.69, 28.46.

### Intermediate 19: 1-(3-Bromophenyl)piperazine

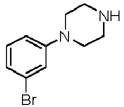

The title compound was synthesized using tert-butyl 4-(3-bromophenyl)piperazine-1-carboxylate (100 mg, 0.29 mmol, 1 eq) according to general procedure B. This yield the product (66 mg, 0.28 mmol, 94 %). 1H NMR (400 MHz, Chloroform-d) δ 7.14 - 7.05 (m, 1H), 7.02 (t, J = 2.1 Hz, 1H), 6.98 - 6.89 (m, 1H), 6.82 (ddd, J = 8.4, 2.5, 0.9 Hz, 1H), 3.12 (m, 4H), 3.03 - 2.94 (m, 4H), 1.84 (bs, 1H). 13C NMR (101 MHz, Chloroform-d) δ 153.02, 130.35, 123.27, 122.23, 118.71, 114.44, 49.89, 46.02.

### Intermediate 20: tert-Butyl 4-(4-bromophenyl)piperazine-1-carboxylate

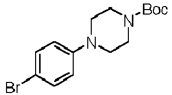

The title compound was synthesized using 1,4-dibromobenzene (236 mg, 1.00 mmol, 1 eq ), tert-butyl piperazine-1-carboxylate (186 mg, 1 mmol, 1.00 eq), sodium tert-butoxide ( 144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (256 mg, 0.75 mmol, 75 %). 1H NMR (400 MHz, Chloroform-d) δ 7.38 - 7.31 (m, 2H), 6.82 - 6.73 (m, 2H), 3.68 - 3.41 (m, 4H), 3.09 (t, J = 5.2 Hz, 4H), 1.48 (s, 9H). 13C NMR (101 MHz, Chloroform-d) δ 154.71, 150.33, 132.02, 118.25, 112.49, 80.06, 49.29, 43.74, 28.50.

### Intermediate 21: 1-(4-Bromophenyl)piperazine

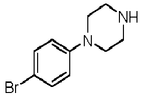

The title compound was synthesized using tert-butyl 4-(4-bromophenyl)piperazine-1-carboxylate (100 mg, 0.29 mmol, 1 eq) according to general procedure B. This yield the product (66 mg, 0.28 mmol, 93 %). ¹H NMR (400 MHz, Chloroform-d) δ 7.37 - 7.31 (m, 2H), 6.82 - 6.75 (m, 2H), 3.16 - 3.09 (m, 4H), 3.07 - 2.97 (m, 4H). ¹³C NMR (101 MHz, Chloroform-d) δ 150.80, 131.98, 117.85, 112.07, 50.07, 45.91.

### Intermediate 22: tert-Butyl 4-(3-(trifluoromethyl)phenyl)piperazine-1-carboxylate

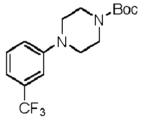

The title compound was synthesized using 1-bromo-3-(trifluoromethyl)benzene (225 mg, 1.00 mmol, 1 eq), *tert*-butyl piperazine-1-carboxylate (186 mg, 1 mmol, 1.00 eq), sodium tert-butoxide (144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (289 mg, 0.88 mmol, 88 %). 1H NMR (400 MHz, Chloroform-d) δ 8.36 (ddt, J = 8.2, 7.2, 0.9 Hz, 1H), 8.14 - 8.05 (m, 3H), 4.65 - 4.55 (m, 4H), 4.19 (t, J = 5.2 Hz, 4H), 2.49 (s, 9H). 13C NMR (101 MHz, Chloroform-d) δ 154.78, 151.41, 131.65 (q, J = 31.7 Hz), 129.79, 124.35 (q, J = 272.70 Hz), 119.51, 116.65 (q, J = 3.6 Hz), 112.89 (q, J = 3.8 Hz), 80.22, 49.10, 28.54.

### Intermediate 23: 1-(3-(Trifluoromethyl)phenyl)piperazine

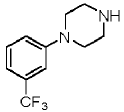

The title compound was synthesized using tert-butyl 4-(3-(trifluoromethyl)phenyl)piperazine-1-carboxylate (100 mg, 0.30 mmol, 1 eq) according to general procedure B. This yield the product (68 mg, 0.29 mmol, 97 %). 1H NMR (400 MHz, Chloroform-d) δ 7.34 (t, J = 7.9 Hz, 1H), 7.17 - 6.99 (m, 3H), 3.87 (s, 2H), 3.27 - 3.19 (m, 4H), 3.14 - 3.01 (m, 4H). 13C NMR (101 MHz, Chloroform-d) δ 151.62, 131.43 (q, J = 31.7 Hz), 129.65, 124.35 (q, J = 272.4 Hz), 119.03, 116.19 (q, J = 3.8 Hz), 112.39 (q, J = 3.9 Hz), 49.24, 45.50.

### Intermediate 24: tert-Butyl 4-(4-(trifluoromethyl)phenyl)piperazine-1-carboxylate

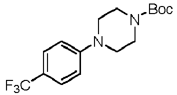

The title compound was synthesized using 1-bromo-4-(trifluoromethyl)benzene (225 mg, 1.00 mmol, 1 eq), *tert*-butyl piperazine-1-carboxylate (186 mg, 1 mmol, 1.00 eq), sodium tert-butoxide (144 mg, 1.50 mmol, 1.5 eq), rac-BINAP (37.4 mg, 0.06 mmol, 0.06 eq) and palladium diacetate (9 mg, 0.04 mmol, 0.04 eq) according to general procedure A. This yielded the product (218 mg, 0.66 mmol, 66 %). 1H NMR (400 MHz, Chloroform-d) δ 7.55 - 7.44 (m, 2H), 6.95 (d, J = 8.7 Hz, 2H), 3.65 - 3.53 (m, 4H), 3.25 (t, J = 5.2 Hz, 4H). 1.49 (s, 9 H).

### Intermediate 25: 1-(4-(Trifluoromethyl)phenyl)piperazine

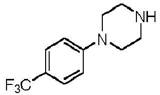

The title compound was synthesized using tert-butyl 4-(4-(trifluoromethyl)phenyl)piperazine-1-carboxylate (100 mg, 0.30 mmol, 1 eq) according to general procedure B. This yield the product (60 mg, 0.26 mmol, 86 %). 1H NMR (400 MHz, Chloroform-d) δ 7.48 (d, J = 8.7 Hz, 2H), 6.92 (d, J = 8.6 Hz, 2H), 4.26 (br, 1H), 3.36 - 3.24 (m, 4H), 3.13 - 3.04 (m, 4H). 13C NMR (101 MHz, Chloroform-d) δ 153.48, 126.47 (q, J = 3.7 Hz), 124.75 (q, J = 271.69 Hz), 120.86 (q, J = 32.6 Hz), 114.82, 48.49, 45.36.

### Intermediate 26: tert-Butyl 4-(3-methoxyphenyl)piperazine-1-carboxylate

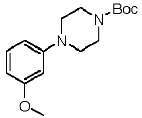

The title compound was synthesized using 1-bromo-3-methoxybenzene (374 mg, 2.00 mmol, 1 eq), *tert*-butyl piperazine-1-carboxylate (373 mg, 2 mmol, 1.00 eq), sodium tert-butoxide (288 mg, 3.00 mmol, 1.5 eq), rac-BINAP (75 mg, 0.12 mmol, 0.06 eq) and palladium diacetate (18 mg, 0.08 mmol, 0.04 eq) according to general procedure A. This yielded the product (433 mg, 1.48 mmol, 74 %). 1H NMR (400 MHz, Chloroform-d) δ 7.16 (t, J = 8.1 Hz, 1H), 6.52 (dd, J = 8.3, 2.5 Hz, 1H), 6.44 (dp, J = 7.6, 2.3 Hz, 2H), 3.77 (s, 3H), 3.56 (q, J = 5.0, 4.6 Hz, 4H), 3.21 - 2.99 (m, 4H), 1.48 (s, 9H). 13C NMR (101 MHz, CDCl3) δ 160.57, 154.63, 152.61, 129.81, 109.25, 104.93, 102.95, 79.77, 55.10, 49.25, 28.41.

### Intermediate 27: 1-(3-Methoxyphenyl)piperazine

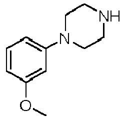

The title compound was synthesized using tert-butyl 4-(4-(trifluoromethyl)phenyl)piperazine-1-carboxylate (200 mg, 0.68 mmol, 1 eq) according to general procedure B. This yield the product (130 mg, 0.68 mmol, 99 %). 1H NMR (400 MHz, Chloroform-d) δ 9.73 (br, 1H), 7.21 (t, J = 8.2 Hz, 1H), 6.55 - 6.49 (m, 2H), 6.45 (t, J = 2.3 Hz, 1H), 3.79 (s, 3H), 3.41 (m Hz, 4H), 3.33 (m, 4H). 13C NMR (101 MHz, Chloroform-d) δ 160.81, 151.45, 130.32, 109.86, 106.53, 103.90, 55.39, 46.99, 43.47.

### Intermediate 28: tert-Butyl 4-(4-methoxyphenyl)piperazine-1-carboxylate

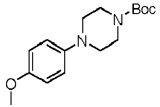

The title compound was synthesized using 1-bromo-4-methoxybenzene (500 mg, 2.67 mmol, 1 eq), *tert*-butyl piperazine-1-carboxylate (597 mg, 3.21 mmol, 1.2 eq), cesium carbonate (1307 mg, 4.01 mmol, 1.5 eq), rac-BINAP (106 mg, 0.16 mmol, 0.06 eq) and palladium diacetate (24 mg, 0.11 mmol, 0.04 eq) according to general procedure A. This yielded the product (438 mg, 1.50 mmol, 56 %). 1H NMR (CDCl3, 400 MHz) δ 6.91 - 6.72 (m, 4H), 3.70 (s, 3H), 3.60 - 3.46 (m, 4H), 3.01 - 2.87 (m, 4H), 1.45 (s, 9H). 13C NMR (CDCl3, 101 MHz) δ 154.52, 154.07, 145.48, 118.68, 114.30, 79.57, 55.31, 50.76, 28.32.

### Intermediate 29: 1-(4-Methoxyphenyl)piperazine

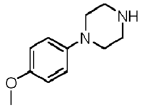

The title compound was synthesized using tert-butyl 4-(4-methoxyphenyl)piperazine-1-carboxylate (438 mg, 1.50 mmol, 1 eq) according to general procedure B. This yield the product (260 mg, 1.35 mmol, 90%). 1H NMR (CDCl3, 400 MHz) δ 6.89 - 6.77 (m, 4H), 3.70 (s, 3H), 2.96 (m, 8H). 13C NMR (CDCl3, 101 MHz) δ 153.66, 146.20, 118.07, 114.30, 55.38, 51.76, 46.20.

### Intermediate 30: tert-Butyl 4-(3-nitrophenyl)piperazine-1-carboxylate

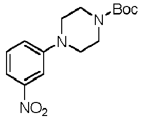

The title compound was synthesized using 1-bromo-3-nitrobenzene (202 mg, 1.00 mmol, 1 eq), tert-butyl piperazine-1-carboxylate (186 mg, 1 mmol, 1.00 eq), sodium tert-butoxide (144 mg, 1.50 mmol, 1.5 eq), rac-BINAP (37 mg, 0.06 mmol, 0.06 eq) and palladium diacetate (9 mg, 0.04 mmol, 0.04 eq) according to general procedure A. This yielded the product (150 mg, 0.49 mmol, 49 %). 1H NMR (400 MHz, Chloroform-d) δ 7.80 - 7.55 (m, 2H), 7.39 (t, J = 8.2 Hz, 1H), 7.20 (dd, J = 8.4, 2.5 Hz, 1H), 4.03 - 3.44 (t, J = 5.2 Hz, 4H), 3.25 (t, J = 5.2 Hz, 4H), 1.50 (s, 9H). 13C NMR (101 MHz, Chloroform-d) δ 154.57, 151.71, 149.17, 129.79, 121.61, 114.61, 110.02, 80.13, 48.44, 28.38.

### Intermediate 31: 1-(3-Nitrophenyl)piperazine

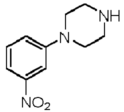

The title compound was synthesized using tert-butyl 4-(3-nitrophenyl)piperazine-1-carboxylate (100 mg, 0.33 mmol, 1 eq) according to general procedure B. This yield the product (66 mg, 0.32 mmol, 98%). 1H NMR (400 MHz, Chloroform-d) δ 7.71 (d, J = 2.1 Hz, 1H), 7.65 (d, J = 8.0 Hz, 1H), 7.38 (t, J = 8.2 Hz, 1H), 7.19 (d, J = 8.3, 2.5 Hz, 1H), 3.40 - 3.16 (m, 4H), 3.13 - 2.95 (m, 4H). 13C NMR (101 MHz, Chloroform-d) δ 152.31, 149.32, 129.76, 121.27, 113.80, 109.74, 49.48, 45.88.

### Intermediate 32: tert-Butyl 4-([1,1'-biphenyl]-3-yl)piperazine-1-carboxylate

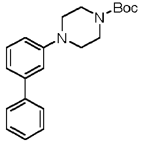

To a mixture of phenylboronic acid (107 mg, 0.88 mmol, 1.5 eq) and tert-butyl 4-(3-bromophenyl)piperazine-1-carboxylate (200 mg, 0.59 mmol, 1 eq) in 5ml 1.4-dioxane was added cesium carbonate (573 mg, 1.76 mmol, 3 eq) and tetra is(triphenylphosphine)palladium (14 mg, 0.012 mmol, 0.02 eq). Then the reaction mixture was degassed for 30 min. The mixture was heated to 80 °C for 5h. The reaction progress was monitored by TLC. Once completed, the mixture was diluted with DCM and washed with water, dried over anhydrous MgSO₄. After filtration, the filtrate was concentrated under reduced pressure. The residue was purified by silica gel column chromatography ( Et₂O / pentane, 0 - 10 %) to yield the product (181 mg, 0.53 mmol, 91 %). 1H NMR (400 MHz, Chloroform-d) δ 7.55 (dt, J = 6.3, 1.2 Hz, 2H), 7.39 (dd, J = 8.4, 6.8 Hz, 2H), 7.30 (td, J = 7.6, 2.6 Hz, 2H), 7.14 - 7.05 (m, 2H), 6.88 (dd, J = 8.1, 2.5 Hz, 1H), 3.57 (t, J = 5.2 Hz, 4H), 3.15 (t, J = 5.1 Hz, 4H), 1.48 (s, 9H). 13C NMR (101 MHz, Chloroform-d) δ 154.66, 151.64, 142.31, 141.56, 129.51, 128.67, 127.26, 127.19, 119.31, 115.59, 115.54, 79.81, 49.44, 28.42.

### Intermediate 33: 1-([1,1'-Biphenyl]-3-yl)piperazine

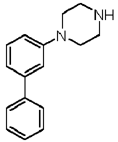

The title compound was synthesized using tert-butyl 4-([1,1'-biphenyl]-3-yl)piperazine-1-carboxylate (100 mg, 0.30mmol, 1 eq) according to general procedure B. The obtained crude was used directly without any purification.

### Intermediate 34: tert-Butyl 4-(3,4-dichlorophenyl)piperazine-1-carboxylate

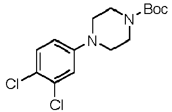

The title compound was synthesized using 4-bromo-1,2-dichlorobenzene (226 mg, 1.00 mmol, 1 eq), *tert*-butyl piperazine-1-carboxylate (186 mg, 1.00 mmol, 1 eq), sodium *tert-*butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (209 mg, 0.63 mmol, 63%). ¹H NMR (400 MHz, Chloroform-*d*) δ 7.37 (d, *J* = 2.4 Hz, 1H), 7.19 (dd, *J* = 8.6, 2.4 Hz, 1H), 6.93 (d, *J* = 8.6 Hz, 1H), 3.65 - 3.54 (m, 4H), 2.95 (t, *J* = 4.9 Hz, 4H), 1.49 (s, 9H). ¹³C NMR (101 MHz, Chloroform-*d*) δ 154.86, 147.93, 130.45, 129.71, 128.67, 127.76, 121.35, 79.97, 51.29, 28.52.

### Intermediate 35: 1-(3,4-Dichlorophenyl)piperazine

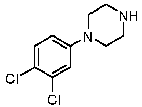

The title compound was synthesized using tert-Butyl 4-(3,4-dichlorophenyl)piperazine-1-carboxylate (100 mg, 0.30 mmol, 1 eq) according to general procedure B. This yield the product (63 mg, 0.27 mmol, 90%). 1H NMR (400 MHz, Chloroform-d) δ 7.37 (d, J = 2.4 Hz, 1H), 7.22 - 7.18 (m, 1H), 6.96 (d, J = 8.6 Hz, 1H), 3.58 (br, 1H), 3.12 (m, 4H), 3.05 (m, 4H).13C NMR (101 MHz, Chloroform-d) δ 148.07, 130.45, 129.69, 128.64, 127.80, 121.39, 51.69, 45.71.

### Intermediate 36: tert-Butyl 4-(3,5-dichlorophenyl)piperazine-1-carboxylate

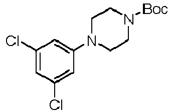

The title compound was synthesized using 1-bromo-3,5-dichlorobenzene (226 mg, 1.00 mmol, 1 eq), *tert*-butyl piperazine-1-carboxylate (186 mg, 1.00 mmol, 1 eq), sodium *tert-*butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (192 mg, 0.58 mmol, 58%). ¹H NMR (400 MHz, Chloroform-d) δ 6.82 (t, *J* = 1.7 Hz, 1H), 6.74 (d, *J* = 1.7 Hz, 2H), 3.62 - 3.48 (m, 4H), 3.15 (t, *J* = 5.2 Hz, 4H), 1.48 (s, 9H). ¹³C NMR (101 MHz, Chloroform-d) δ 154.71, 152.65, 135.61, 119.44, 114.35, 80.28, 48.48, 28.52.

### Intermediate 37: 1-(3,5-Dichlorophenyl)piperazine

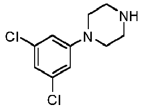

The title compound was synthesized using tert-Butyl 4-(3,5-dichlorophenyl)piperazine-1-carboxylate (130 mg, 0.39 mmol, 1 eq) according to general procedure B. This yield the product (90 mg, 0.39 mmol, 99%). ¹H NMR (400 MHz, Chloroform-d) δ 6.79 (t, J = 1.7 Hz, 1H), 6.74 (d, J = 1.8 Hz, 2H), 3.17 - 3.11 (m, 4H), 3.04 - 2.96 (m, 4H), 1.42 (br, 1H). ¹³C NMR (101 MHz, Chloroform-d) δ 153.05, 135.47, 118.90, 113.87, 49.25, 45.74.

### Intermediate 38: tert-Butyl 4-(2,4-dichlorophenyl)piperazine-1-carboxylate

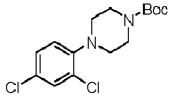

The title compound was synthesized using 1-bromo-2,4-dichlorobenzene (226 mg, 1.00 mmol, 1 eq), *tert*-butyl piperazine-1-carboxylate (186 mg, 1.00 mmol, 1 eq), sodium *tert-*butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (200 mg, 0.60 mmol, 60%). ¹H NMR (400 MHz, Chloroform-d) δ 7.37 (d, J = 2.5 Hz, 1H), 7.19 (dd, J = 8.6, 2.4 Hz, 1H), 6.93 (d, J = 8.6 Hz, 1H), 3.59 (t, 4H), 2.95 (t, 4H), 1.49 (s, 9H). ¹³C NMR (101 MHz, Chloroform-*d*) δ 154.87, 147.96, 130.46, 129.72, 128.66, 127.76, 121.35, 79.97, 51.30, 28.53.

### Intermediate 39: 1-(2,4-Dichlorophenyl)piperazine

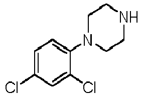

The title compound was synthesized using tert-Butyl 4-(2,4-dichlorophenyl)piperazine-1-carboxylate (100 mg, 0.30 mmol, 1.00 eq) according to general procedure B. This yield the product (66 mg, 0.28 mmol, 96%). 1H NMR (400 MHz, Chloroform-d) δ 7.35 (s, 1H, Aromatic, CH), 7.18 (d, J = 8.6 Hz, 1H), 6.95 (d, J = 8.6, 2.0 Hz, 1H), 3.06 - 3.02 (m, 4H), 3.01 - 2.90 (m, 4H), 2.14 (s, 1H). 13C NMR (101 MHz, CDCl3) δ 148.48, 130.33, 129.55, 128.17, 127.66, 121.24, 52.56, 46.19.

### Intermediate 40: tert-Butyl 4-(2,6-dichlorophenyl)piperazine-1-carboxylate

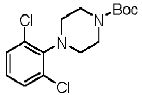

The title compound was synthesized using 2-bromo-1,3-dichlorobenzene (226 mg, 1.00 mmol, 1 eq), *tert*-butyl piperazine-1-carboxylate (186 mg, 1.00 mmol, 1 eq), sodium *tert-*butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (35 mg, 0.11 mmol, 11%). ¹H NMR (400 MHz, Chloroform-*d*) δ 7.27 (d, J = 8.0 Hz, 2H), 6.99 (t, J = 8.3, 7.8 Hz, 1H), 3.56 (m, 4H), 3.17 (m, 4H), 1.49 (s, 9H).13C NMR (101 MHz, Chloroform-*d*) δ 155.08, 135.18, 129.25, 126.32, 79.76, 49.56, 28.59.

### Intermediate 41: 1-(2,6-Dichlorophenyl)piperazine

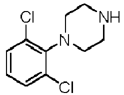

The title compound was synthesized using tert-Butyl 4-(2,6-dichlorophenyl)piperazine-1-carboxylate (35 mg, 0.11 mmol, 1.00 eq) according to general procedure B. This yield the product (22 mg, 0.10 mmol, 90%). ¹H NMR (400 MHz, Chloroform-d) δ 7.26 (d, J = 8.1 Hz, 2H), 6.97 (t, 1H), 3.19 (t, 4H), 3.01 (t, 4H), 2.19 (s, 1H). 13C NMR (101 MHz, CDCl3) δ 145.41, 135.29, 129.22, 126.03, 50.81, 46.81.

### Intermediate 42: tert-Butyl (S)-3-methyl-4-(m-tolyl)piperazine-1-carboxylate

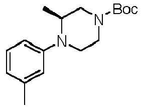

The title compound was synthesized using 1-bromo-3-methylbenzene (100 mg, 0.59 mmol, 1 eq), *tert*-butyl (*S*)-3-methylpiperazine-1-carboxylate (176 mg, 0.88 mmol, 1.5 eq), Cs₂CO₃ (286 mg, 0.88 mmol, 1.5 eq), rac-BINAP (23.25 mg, 0.04 mmol, 0.06 eq) and palladium diacetate (5.25 mg, 0.02 mmol, 0.04 eq) according to general procedure A. This yielded the product (130 mg, 0.45 mmol, 77%). ¹H NMR (400 MHz, Chloroform-d) δ 7.14 (t, J = 7.7 Hz, 1H), 6.70 (dd, J = 11.5, 4.4 Hz, 3H), 4.04 - 3.66 (m, 3H), 3.44 - 2.99 (m, 4H), 2.30 (s, 3H), 1.48 (s, 9H), 0.98 (d, J = 6.5 Hz, 3H). ¹³C NMR (101 MHz, Chloroform-d) δ 155.13, 150.22, 138.86, 129.07, 120.93, 118.05, 114.33, 79.70, 51.65, 49.45, 48.25, 44.30, 28.50, 21.85, 12.25.

### Intermediate 43: (S)-2-Methyl-1-(m-tolyl)piperazine

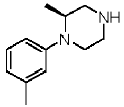

The title compound was synthesized using tert-Butyl (S)-3-methyl-4-(m-tolyl)piperazine-1-carboxylate (150 mg, 0.52 mmol, 1.00 eq) according to general procedure B. This yield the product (90 mg, 0.47 mmol, 92%). 1H NMR (400 MHz, Chloroform-d) δ 7.14 (td, J = 7.5, 1.1 Hz, 1H), 6.78 - 6.62 (m, 3H), 3.73 (qq, J = 6.7, 3.7, 3.3 Hz, 1H), 3.15 - 2.78 (m, 6H), 2.31 (s, 3H), 1.03 (d, J = 6.5 Hz, 3H).13C NMR (101 MHz, Chloroform-d) δ 150.78, 138.76, 128.94, 120.80, 118.44, 114.69, 51.85, 51.40, 46.46, 45.97, 21.83, 12.64.

### Intermediate 44: tert-Butyl (R)-3-methyl-4-(m-tolyl)piperazine-1-carboxylate

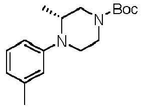

The title compound was synthesized using 1-bromo-3-methylbenzene (1000 mg, 5.85 mmol, 1 eq), *tert*-butyl (R)-3-methylpiperazine-1-carboxylate (1171 mg, 5.85 mmol, 1 eq), Cs₂CO₃ (2860 mg, 8.77 mmol, 1.5 eq), rac-BINAP (233 mg, 0.35 mmol, 0.06 eq) and palladium diacetate (52.53 mg, 0.23 mmol, 0.04 eq) according to general procedure A. This yielded the product (1050 mg, 3.62 mmol, 62%).

### Intermediate 45: (R)-2-Methyl-1-(m-tolyl)piperazine

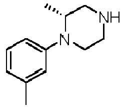

The title compound was synthesized using tert-Butyl (R)-3-methyl-4-(*m*-tolyl)piperazine-1-carboxylate (750 mg, 2.58 mmol, 1.00 eq) according to general procedure B. This yield the product (405 mg, 2.13 mmol, 82%). 1H NMR (400 MHz, Chloroform-d) δ 7.20 - 7.08 (m, 1H), 6.80 - 6.68 (m, 3H), 3.72 (m, 1H), 3.67 - 3.50 (br, 1H), 3.19 - 2.82 (m, 6H), 2.31 (s, 3H), 1.03 (d, J = 6.5 Hz, 3H). 13C NMR (101 MHz, Chloroform-d) δ 150.56, 138.84, 128.98, 121.47, 119.16, 115.41, 51.38, 51.30, 46.09, 45.93, 21.78, 13.00.

### Intermediate 46: tert-Butyl (S)-4-(3-fluorophenyl)-3-methylpiperazine-1-carboxylate

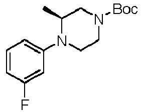

The title compound was synthesized using 1-bromo-3-fluorobenzene (175 mg, 1.00 mmol, 1 eq), *tert*-butyl (*S*)-3-methylpiperazine-1-carboxylate (200 mg, 1.00 mmol, 1 eq), sodium *tert*-butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (177 mg, 0.60 mmol, 60%). ¹H NMR (400 MHz, Chloroform-*d*) δ 7.18 (td, *J* = 8.2, 7.0 Hz, 1H), 6.62 (dd, *J* = 8.3, 2.4 Hz, 1H), 6.58 - 6.44 (m, 2H), 4.36 - 3.70 (m, 3H), 3.40 - 2.96 (m, 4H), 1.49 (s, 9H), 1.02 (d, *J* = 6.5 Hz, 3H). ¹³C NMR (101 MHz, Chloroform-*d*) δ 164.00 (d, *J* = 243.2 Hz), 155.09, 151.72 (d, *J* = 9.8 Hz), 130.26 (d, *J* = 10.0 Hz), 111.50, 105.71 (d, *J* = 21.4 Hz), 103.05 (d, *J* = 25.3 Hz), 79.86, 51.12, 48.96, 47.69, 42.36, 28.43, 12.05.

### Intermediate 47: (S)-1-(3-Fluorophenyl)-2-methylpiperazine

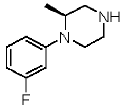

The title compound was synthesized using tert-Butyl (*S*)-4-(3-fluorophenyl)-3-methylpiperazine-1-carboxylate (100 mg, 0.34 mmol, 1.00 eq) according to general procedure B. The obtained crude was used directly without any purification.

### Intermediate 48: tert-Butyl (R)-4-(3-chlorophenyl)-3-methylpiperazine-1-carboxylate

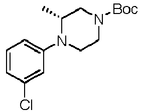

The title compound was synthesized using 1-bromo-3-chlorobenzene (191 mg, 1.00 mmol, 1 eq), *tert*-butyl (*R*)-3-methylpiperazine-1-carboxylate (200 mg, 1.00 mmol, 1 eq), sodium *tert*-butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (233 mg, 0.75 mmol, 75%). ¹H NMR (400 MHz, Chloroform-*d*) δ 7.15 (t, *J* = 8.1 Hz, 1H), 6.95 - 6.63 (m, 3H), 4.27 - 3.70 (m, 3H), 3.40 - 2.89 (m, 4H), 1.48 (s, 9H), 1.01 (d, *J* = 6.5 Hz, 3H). ¹³C NMR (101 MHz, Chloroform-*d*) δ 155.07, 151.17, 135.09, 130.19, 119.25, 116.31, 114.40, 79.87, 51.22, 49.11, 47.81, 42.84, 28.46, 12.16.

### Intermediate 49: (R)-1-(3-Chlorophenyl)-2-methylpiperazine

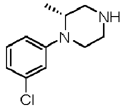

The title compound was synthesized using tert-Butyl (*R*)-4-(3-chlorophenyl)-3-methylpiperazine-1-carboxylate (100 mg, 0.34 mmol, 1.00 eq) according to general procedure B. The obtained crude was used directly without any purification.

### Intermediate 50: tert-Butyl (S)-4-(3-chlorophenyl)-3-methylpiperazine-1-carboxylate

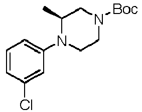

The title compound was synthesized using 1-bromo-3-chlorobenzene (191 mg, 1.00 mmol, 1 eq), *tert*-butyl (*S*)-3-methylpiperazine-1-carboxylate (200 mg, 1.00 mmol, 1 eq), sodium *tert*-butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (117 mg, 0.38 mmol, 38%). ¹H NMR (400 MHz, Chloroform-d) δ 7.16 (t, *J* = 8.1 Hz, 1H), 6.93 - 6.44 (m, 3H), 4.23 - 3.71 (m, 3H), 3.41 - 2.92 (m, 4H), 1.48 (s, 9H), 1.02 (d, *J* = 6.5 Hz, 3H). ¹³C NMR (101 MHz, Chloroform-*d*) δ 155.12, 151.18, 135.12, 130.20, 119.31, 116.38, 114.51, 79.93, 51.25, 49.15, 42.92, 28.48, 12.19.

### Intermediate 51: (S)-1-(3-Chlorophenyl)-2-methylpiperazine

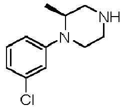

The title compound was synthesized using tert-Butyl (*S*)-4-(3-chlorophenyl)-3-methylpiperazine-1-carboxylate (100 mg, 0.34 mmol, 1.00 eq) according to general procedure B. This yield the product (56 mg, 0.26 mmol, 82%). ¹H NMR (400 MHz, Chloroform-d) δ 7.16 (t, J = 8.1 Hz, 1H), 6.92 - 6.67 (m, 3H), 3.82 (tt, J = 6.7, 3.2 Hz, 1H), 3.59 (br, 1H), 3.29 - 2.78 (m, 6H), 1.08 (d, J = 6.6 Hz, 3H). ¹³C NMR (101 MHz, Chloroform-d) δ 151.54, 135.04, 130.15, 119.50, 116.88, 114.99, 50.97, 50.72, 45.84, 44.22, 12.47.

### Intermediate 52: tert-Butyl (S)-4-(3-bromophenyl)-3-methylpiperazine-1-carboxylate

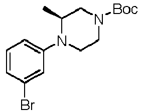

The title compound was synthesized using 1,3-dibromobenzene (236 mg, 1.00 mmol, 1 eq ), *tert*-butyl (*S*)-3-methylpiperazine-1-carboxylate (200 mg, 1.00 mmol, 1 eq), sodium *tert-*butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (154 mg, 0.43 mmol, 43%). ¹H NMR (400 MHz, Chloroform-*d*) δ 7.09 (t, *J* = 8.1 Hz, 1H), 7.02 - 6.89 (m, 2H), 6.78 (dd, *J* = 8.3, 2.4 Hz, 1H), 4.03 - 3.72 (m, 2H), 3.36 - 2.99 (m, 5H), 1.48 (s, 9H), 1.01 (d, *J* = 6.5 Hz, 3H). ¹³C NMR (101 MHz, Chloroform-*d*) δ 155.05, 151.31, 130.46, 123.37, 122.19, 119.26, 114.87, 79.87, 51.23, 49.12, 47.69, 42.78, 28.44, 12.18.

### Intermediate 53: (S)-1-(3-Bromophenyl)-2-methylpiperazine

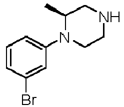

The title compound was synthesized using tert-Butyl (S)-4-(3-bromophenyl)-3-methylpiperazine-1-carboxylate (100 mg, 0.28 mmol, 1.00 eq) according to general procedure B. This yield the product (72 mg, 0.28 mmol, 98%). ¹H NMR (400 MHz, Chloroform-d) δ 7.10 (t, J = 8.1 Hz, 1H), 7.01 (t, J = 2.1 Hz, 1H), 6.95 (ddd, J = 7.9, 1.9, 0.9 Hz, 1H), 6.81 (ddd, J = 8.3, 2.5, 0.9 Hz, 1H), 3.81 (m, 1H), 3.77 (br, 1H), 3.19 - 2.86 (m, 6H), 1.07 (d, J = 6.6 Hz, 3H). ¹³C NMR (101 MHz, Chloroform-d) δ 151.66, 130.41, 123.30, 122.36, 119.73, 115.42, 50.92, 50.68, 45.79, 44.17, 12.44.

### Intermediate 54: tert-Butyl (R)-4-(3-bromophenyl)-3-methylpiperazine-1-carboxylate

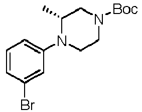

The title compound was synthesized using 1,3-dibromobenzene (236 mg, 1.00 mmol, 1 eq ), *tert*-butyl (*R*)-3-methylpiperazine-1-carboxylate (200 mg, 1.00 mmol, 1 eq), sodium *tert-*butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (257 mg, 0.72 mmol, 72%). ¹H NMR (400 MHz, Chloroform-*d*) δ 7.10 (t, *J* = 8.1 Hz, 1H), 7.03 - 6.90 (m, 2H), 6.78 (dd, *J* = 8.6, 2.4 Hz, 1H), 4.26 - 3.70 (m, 3H), 3.44 - 2.93 (m, 4H), 1.48 (s, 9H), 1.01 (d, *J* = 6.5 Hz, 3H). ¹³C NMR (101 MHz, Chloroform-d) δ 155.10, 151.35, 130.50, 123.41, 122.25, 119.30, 114.92, 79.93, 51.28, 49.20, 47.76, 42.95, 28.48, 12.22.

### Intermediate 55: (R)-1-(3-Bromophenyl)-2-methylpiperazine

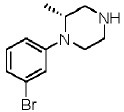

The title compound was synthesized using tert-Butyl (R)-4-(3-bromophenyl)-3-methylpiperazine-1-carboxylate (100 mg, 0.28 mmol, 1.00 eq) according to general procedure B. This yield the product (68 mg, 0.27 mmol, 95%). ¹H NMR (400 MHz, Chloroform-d) δ 7.11 (t, J = 8.1 Hz, 1H), 7.06 - 6.97 (m, 2H), 6.83 (ddd, J = 8.3, 2.4, 0.9 Hz, 1H), 4.17 (s, 1H), 3.82 (qt, J = 6.7, 3.7 Hz, 1H), 3.24 - 2.90 (m, 6H), 1.09 (d, J = 6.6 Hz, 3H). ¹³C NMR (101 MHz, Chloroform-d) δ 151.56, 130.49, 123.33, 123.00, 120.42, 116.07, 50.75, 50.61, 45.50, 44.31, 12.79.

### Intermediate 56: tert-Butyl (S)-3-methyl-4-(3-(trifluoromethyl)phenyl)piperazine-1-carboxylate

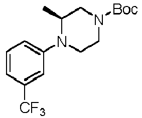

The title compound was synthesized using 1-bromo-3-(trifluoromethyl)benzene (225 mg, 1.00 mmol, 1 eq), *tert*-butyl (*S*)-3-methylpiperazine-1-carboxylate (200 mg, 1.00 mmol, 1 eq), sodium *tert*-butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (176 mg, 0.51 mmol, 51%). ¹H NMR (400 MHz, Chloroform-d) δ 7.34 (t, *J* = 7.8 Hz, 1H), 7.15 - 6.90 (m, 3H), 4.03 - 3.77 (m, 3H), 3.41 - 2.98 (m, 4H), 1.49 (s, 9H), 1.03 (d, *J* = 6.5 Hz, 3H). ¹³C NMR (101 MHz, Chloroform-d) δ 155.06, 150.22, 131.55 (q, *J* = 31.7 Hz), 129.70, 124.36 (q, *J* = 272.4 Hz), 119.27, 115.75, 112.67, 79.90, 51.21, 49.09, 47.81, 42.81, 28.38, 12.09.

### Intermediate 57: (S)-2-Methyl-1-(3-(trifluoromethyl)phenyl)piperazine

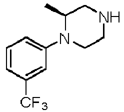

The title compound was synthesized using tert-Butyl (S)-3-methyl-4-(3-(trifluoromethyl)phenyl)piperazine-1-carboxylate (100 mg, 0.29 mmol, 1.00 eq) according to general procedure B. This yield the product (62 mg, 0.26 mmol, 95%). 1H NMR (400 MHz, Chloroform-d) δ 7.42 - 7.24 (m, 1H), 7.19 - 6.94 (m, 3H), 3.87 (qt, J = 6.6, 3.3 Hz, 1H), 3.32 - 2.80 (m, 6H), 2.50 (s, 1H), 1.08 (d, J = 6.6 Hz, 3H). 13C NMR (101 MHz, Chloroform-d) δ 150.63, 131.39 (q, J = 31.5 Hz), 129.57, 124.42 (q, J = 272.5 Hz), 119.28, 115.46 (q, J = 3.8 Hz), 112.66 (q, J = 3.9 Hz), 51.18, 50.60, 46.02, 44.09, 12.06.

### Intermediate 58: tert-Butyl (R)-3-methyl-4-(3-(trifluoromethyl)phenyl)piperazine-1-carboxylate

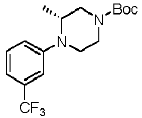

The title compound was synthesized using 1-bromo-3-(trifluoromethyl)benzene (225 mg, 1.00 mmol, 1 eq), *tert*-butyl (R)-3-methylpiperazine-1-carboxylate (200 mg, 1.00 mmol, 1 eq), sodium *tert*-butoxide (144 mg, 1.50 mmol, 1.5 eq), rac-BINAP (37.4 mg, 0.06 mmol, 0.06 eq) and palladium diacetate (8.96 mg, 0.04 mmol, 0.04 eq) according to general procedure A. This yielded the product (176 mg, 0.51 mmol, 51%). ¹H NMR (400 MHz, Chloroform-*d*) δ 7.40 - 7.29 (m, 1H), 7.18 - 6.91 (m, 3H), 4.28 - 3.70 (m, 3H), 3.47 - 3.00 (m, 4H), 1.49 (s, 9H), 1.03 (d, *J* = 6.5 Hz, 3H). ¹³C NMR (101 MHz, Chloroform-*d*) δ 155.10, 150.24, 131.58 (q, *J* = 31.6 Hz), 129.73, 124.38 (q, *J* = 272.4 Hz), 119.26, 115.79, 112.70, 79.94, 51.23, 49.14, 47.83, 42.84, 28.42, 12.15.

### Intermediate 59: (R)-2-Methyl-1-(3-(trifluoromethyl)phenyl)piperazine

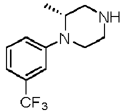

The title compound was synthesized using tert-Butyl (R)-3-methyl-4-(3-(trifluoromethyl)phenyl)piperazine-1-carboxylate (100 mg, 0.29 mmol, 1.00 eq) according to general procedure B. This yield the product (70 mg, 0.29 mmol, 99%). ¹H NMR (400 MHz, Chloroform-d) δ 7.34 (t, J = 8.0 Hz, 1H), 7.13 - 7.00 (m, 3H), 3.88 (qt, J = 6.7, 3.4 Hz, 1H), 3.25 - 2.89 (m, 6H), 1.08 (d, J = 6.6 Hz, 3H). ¹³C NMR (101 MHz, Chloroform-d) δ 150.64, 131.50 (q, J = 31.6 Hz), 129.66, 124.43 (q, J = 272.4 Hz), 119.68, 115.87 (q, J = 3.8 Hz), 113.11 (q, J = 3.9 Hz), 51.07, 50.70, 45.90, 44.29, 12.32.

### Example 2: Ethyl 2-((4-((S)-3-methyl-4-(m-tolyl)piperazine-1-carbonyl)-2-nitrophenyl)sulfinyl)acetate

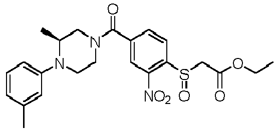

The title compound was synthesized using (*S*)-2-methyl-1-(m-tolyl)piperazine ( 57.4 mg, 0.30 mmol, 1 eq), 4-((2-ethoxy-2-oxoethyl)sulfinyl)-3-nitrobenzoic acid (100 mg, 0.33 mmol, 1.1 eq), HATU (140 mg, 0.45 mmol, 1.5 eq) and DIPEA (117 mg, 0.91 mmol, 3 eq) according to general procedure H. This yielded the product (140 mg, 0.30 mmol, 98%). HRMS: calculated for [C₂₃H₂₇N₃O₆S + H] = 474.16933, found = 474.16801.

### Example 3: Ethyl 2-((4-((R)-3-methyl-4-(m-tolyl)piperazine-1-carbonyl)-2-nitrophenyl)sulfinyl)acetate

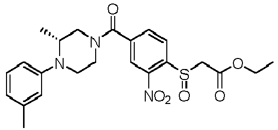

The title compound was synthesized using (*R*)-2-methyl-1-(m-tolyl)piperazine ( 57.4 mg, 0.30 mmol, 1 eq), 4-((2-ethoxy-2-oxoethyl)sulfinyl)-3-nitrobenzoic acid (100 mg, 0.33 mmol, 1.1 eq), HATU (140 mg, 0.45 mmol, 1.5 eq) and DIPEA (117 mg, 0.91 mmol, 3 eq) according to general procedure H. This yielded the product (98 mg, 0.21 mmol, 69%). ¹H NMR (850 MHz, Chloroform-d) δ 8.47 - 8.34 (m, 2H), 8.07 - 7.97 (m, 1H), 7.18 (t, J = 7.8 Hz, 1H), 6.83 - 6.68 (m, 3H), 4.47 - 4.08 (m, 4H), 3.78 (d, J = 13.8 Hz, 2H), 3.72 - 3.06 (m, 5H), 2.33 (s, 3H), 1.28 (td, J = 7.1, 1.7 Hz, 3H), 1.12 - 0.92 (m, 3H). ¹³C NMR (214 MHz, Chloroform-d) δ 166.88, 164.60, 149.51, 144.89, 143.86, 139.88, 139.17, 133.69, 129.20, 127.89, 124.24, 121.93, 119.36, 115.60, 62.44, 60.04, 52.25, 47.72, 45.62, 42.59, 21.79, 14.18, 12.71.

### Example 4: Ethyl 2-((2-nitro-4-(4-(m-tolyl)piperazine-1-carbonyl)phenyl)sulfinyl)acetate

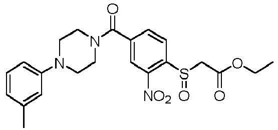

The title compound was synthesized using 1-(*m*-tolyl)piperazine (29.3 mg, 0.17 mmol, 1 eq), 4-((2-ethoxy-2-oxoethyl)sulfinyl)-3-nitrobenzoic acid (50 mg, 0.17 mmol, 1 eq), HATU (95 mg, 0.25 mmol, 1.5 eq) and DIPEA (64.4 mg, 0.50 mmol, 3 eq) according to general procedure H. This yielded the product (53.4 mg, 0.12 mmol, 70%). ¹H NMR (850 MHz, Chloroform-d) δ 8.44 (d, J = 1.7 Hz, 1H), 8.37 (d, J = 8.1 Hz, 1H), 8.07 (dd, J = 8.0, 1.6 Hz, 1H), 7.35 (t, J = 7.9 Hz, 1H), 7.24 - 7.17 (m, 2H), 7.15 (dd, J = 7.5, 1.5 Hz, 1H), 4.32 - 4.08 (m, 5H), 3.90 (m, 2H), 3.77 (d, J = 14.2 Hz, 1H), 3.54 (m, 4H), 2.39 (s, 3H), 1.26 (t, J = 7.7Hz, 3H). ¹³C NMR (214 MHz, Chloroform-d) δ 166.90, 164.84, 144.96, 144.18, 143.94, 140.85, 138.59, 133.88, 130.21, 128.65, 128.02, 124.49, 120.28, 116.68, 62.62, 59.96, 53.48, 53.30, 45.78, 40.63, 21.56, 14.08.

### Example 5: Ethyl (S)-2-((4-(3-methyl-4-(m-tolyl)piperazine-1-carbonyl)-2-nitrophenyl)thio)acetate

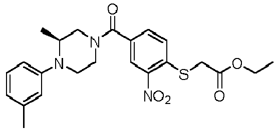

The title compound was synthesized using (*R*)-2-methyl-1-(*m*-tolyl)piperazine ( 60.6 mg, 0.32 mmol, 1 eq), 4-((2-ethoxy-2-oxoethyl)thio)-3-nitrobenzoic acid (100 mg, 0.35 mmol, 1.1 eq), HATU (147 mg, 0.48 mmol, 1.5 eq) and DIPEA (124 mg, 0.96 mmol, 3 eq) according to general procedure H. This yielded the product (108 mg, 0.24 mmol, 74%). ¹H NMR (400 MHz, Chloroform-d) δ 8.34 (d, J = 1.9 Hz, 1H), 7.70 (dd, J = 8.4, 1.9 Hz, 1H), 7.60 (d, J = 8.4 Hz, 1H), 7.17 (t, J = 7.9 Hz, 1H), 6.75 (d, J = 5.8 Hz, 3H), 4.49-3.01 (m, 7H), 4.24 (q, J = 7.1 Hz, 2H), 3.79 (s, 2H), 2.32 (s, 3H), 1.29 (t, J = 7.1 Hz, 3H), 1.01 (m, 3H). ¹³C NMR (101 MHz, Chloroform-d) δ 168.26, 167.70, 149.38, 144.90, 138.78, 138.48, 132.42, 132.36, 128.85, 126.69, 124.90, 121.48, 118.91, 115.14, 61.98, 52.72, 51.87, 47.65, 45.06, 34.72, 21.49, 13.86, 12.47. HRMS: Calculated for [ C₂₃H₂₇N₃O₅S + H]⁺ = 458.17442, found = 458.17432.

### Example 6: Ethyl (S)-2-((4-(3-methyl-4-(m-tolyl)piperazine-1-carbonyl)-2-nitrophenyl)sulfonyl)acetate

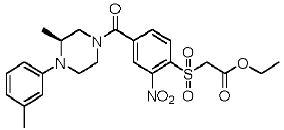

The title compound was synthesized using (S)-2-methyl-1-(m-tolyl)piperazine (12 mg, 0.06 mmol, 1 eq), 4-((2-ethoxy-2-oxoethyl)sulfonyl)-3-nitrobenzoic acid (20 mg, 0.06 mmol, 1 eq), HATU (36 mg, 0.10 mmol, 1.5 eq) and DIPEA (24 mg, 0.19 mmol, 3 eq) according to general procedure H. This yielded the product (2 mg, 0.004 mmol, 6%). 1H NMR (400 MHz, Chloroform-d) δ 8.30 (d, J = 8.0 Hz, 1H), 7.94 (d, J = 3.9 Hz, 1H), 7.83 (d, J = 8.0 Hz, 1H), 7.18 (t, J = 7.6 Hz, 1H), 6.76 (s, 3H), 4.68 (s, 2H), 4.45-3.05 (m, 7H), 4.22 (q, J = 7.1 Hz, 2H), 2.33 (s, 3H), 1.27 (t, J = 7.0 Hz, 4H), 1.02 (m, 3H).

### Example 7: Ethyl 2-((4-((S)-3-methyl-4-(m-tolyl)piperazine-1-carbonyl)phenyl)sulfinyl)acetate

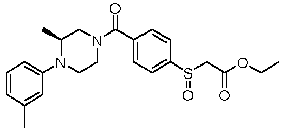

The title compound was synthesized using (*S*)-2-methyl-1-(*m*-tolyl)piperazine (22 mg, 0.12 mmol, 1 eq), 4-((2-ethoxy-2-oxoethyl)sulfinyl)benzoic acid (30 mg, 0.12 mmol, 1 eq), oxalyl chloride (16.34 mg, 0.13 mmol, 1.1 eq) and DIPEA (45 mg, 0.35 mmol, 3 eq ) according to general procedure G. This yielded the product (21 mg, 0.05 mmol, 42%).

### Example 8: Ethyl 2-((2-nitro-4-(4-phenylpiperazine-1-carbonyl)phenyl)sulfinyl)acetate

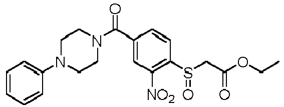

The title compound was synthesized using 1-phenylpiperazine (25 mg, 0.16 mmol, 1 eq), 4-((2-ethoxy-2-oxoethyl)sulfinyl)benzoic acid (70 mg, 0.23 mmol, 1.5 eq), HATU (140 mg, 0.37 mmol, 2.3 eq) and DIPEA (65 µl 0.37 mmol, 2.3 eq) according to general procedure H. This yielded the product (64 mg, 0.14 mmol, 89%). ¹H NMR (400 MHz, Chloroform-d) δ 8.43 - 8.37 (m, 2H), 8.01 (dd, J = 8.0, 1.6 Hz, 1H), 7.34 - 7.25 (m, 2H), 6.98 - 6.91 (m, 3H), 4.28 - 4.17 (m, 2H), 4.14 (d, J = 13.7 Hz, 1H), 3.98 (bs, 2H), 3.77 (d, J = 13.7 Hz, 1H), 3.59 (bs, 2H), 3.31 (bs, 2H), 3.18 (bs, 2H), 1.28 (t, J = 7.1 Hz, 3H). ¹³C NMR (101 MHz, CDCl3) δ 166.54, 164.58, 150.65, 144.91, 143.98, 139.79, 133.72, 129.41, 127.93, 124.22, 121.13, 117.00, 62.44, 60.08, 50.12, 49.62, 47.82, 42.51, 38.68, 14.18. HRMS: calculated for [C₂₁H₂₃N₃O₆S+H]⁺ = 446.13803, found = 446.13787.

### Example 9: Ethyl 2-((4-(4-(3-fluorophenyl)piperazine-1-carbonyl)-2-nitrophenyl)sulfinyl)acetate

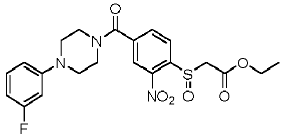

The title compound was synthesized using 1-(3-fluorophenyl)piperazine (18 mg, 0.10 mmol, 1 eq), 4-((2-ethoxy-2-oxoethyl)sulfinyl)benzoic acid (30 mg, 0.12 mmol, 1 eq ), oxalyl chloride (13.90 mg, 0.11 mmol, 1.1 eq) and DIPEA (39 mg, 0.30 mmol, 3 eq) according to general procedure G. This yielded the product (30 mg, 0.07 mmol, 42%). 1H NMR (400 MHz, Chloroform-d) δ 8.41 (dd, J = 4.8, 3.2 Hz, 2H), 8.02 (dd, J = 8.1, 1.6 Hz, 1H), 7.23 (ddd, J = 8.3, 6.6, 1.4 Hz, 1H), 6.77 - 6.66 (m, 1H), 6.65 - 6.56 (m, 2H), 4.22 (qq, J = 7.4, 3.6 Hz, 2H), 4.14 (d, J = 13.7 Hz, 1H), 3.98 (bs, 2H), 3.78 (d, J = 13.6 Hz, 1H), 3.59 (bs, 2H), 3.26 (bd, J = 50.9 Hz, 4H), 1.29 (t, J = 7.1 Hz, 1H). ¹³C NMR (101 MHz, Chloroform-d) δ 166.63, 165.09, 163.63 (d, J = 196.4 Hz), 152.26 (d, J = 9.6 Hz), 144.98, 144.18, 139.69, 133.76, 130.58 (d, J = 9.8 Hz), 128.06, 124.28, 112.09 (d, J = 2.4 Hz), 107.46 (d, J = 21.3 Hz), 103.81 (d, J = 24.9 Hz), 62.53, 60.12, 49.55, 42.32, 14.24.

### Example 10: Ethyl 2-((4-(4-(3-chlorophenyl)piperazine-1-carbonyl)-2-nitrophenyl)sulfinyl)acetate

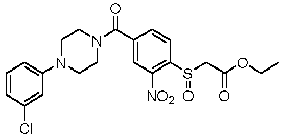

The title compound was synthesized using 1-(3-chlorophenyl)piperazine (20 mg, 0.10 mmol, 1 eq), 4-((2-ethoxy-2-oxoethyl)sulfinyl)benzoic acid (30 mg, 0.12 mmol, 1 eq ), oxalyl chloride (13.90 mg, 0.11 mmol, 1.1 eq) and DIPEA (39 mg, 0.30 mmol, 3 eq) according to general procedure G. This yielded the product (28 mg, 0.06 mmol, 57%). 1H NMR (400 MHz, Chloroform-d) δ 8.51 - 8.31 (m, 2H), 8.01 (dd, J = 8.0, 1.6 Hz, 1H), 7.21 (t, J = 8.4 Hz, 1H), 6.92 - 6.86 (m, 2H), 6.80 (ddd, J = 8.3, 2.3, 1.0 Hz, 1H), 4.22 (qd, J = 7.2, 3.8 Hz, 2H), 4.13 (d, J = 13.7 Hz, 1H), 3.97 (br, 2H), 3.78 (d, J = 13.7Hz, 1H), 3.59 (br, 2H), 3.25 (br, 4H), 1.33 - 1.19 (t, J = 7.2, 3H). ¹³C NMR (101 MHz, Chloroform-d) δ 166.62, 164.58, 151.75, 144.98, 144.18, 139.69, 135.24, 133.74, 130.40, 128.04, 124.25, 120.83, 116.86, 114.87, 62.51, 60.13, 49.58, 49.18, 47.62, 42.38, 14.24.

### Example 11: Ethyl 2-((4-(4-(4-chlorophenyl)piperazine-1-carbonyl)-2-nitrophenyl)sulfinyl)acetate

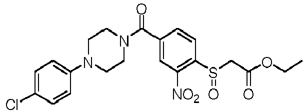

The title compound was synthesized using 1-(4-chlorophenyl)piperazine (19mg, 0.10 mmol, 1 eq), 4-((2-ethoxy-2-oxoethyl)sulfinyl)benzoic acid (30 mg, 0.12 mmol, 1 eq ), oxalyl chloride (13.90 mg, 0.11 mmol, 1.1 eq) and DIPEA (39 mg, 0.30 mmol, 3 eq) according to general procedure G. This yielded the product (8 mg, 0.02 mmol, 18%). ¹H NMR (850 MHz, Chloroform-d) δ 8.47 - 8.36 (m, 2H), 8.02 (dd, J = 8.0, 1.6 Hz, 1H), 7.30 - 7.27 (m, 2H), 6.97 (m, 2H), 4.26 - 4.18 (m, 2H), 4.14 (d, J = 13.8 Hz, 1H), 4.04 (m, 2H), 3.79 (d, J = 13.8 Hz, 1H), 3.65 (m, 2H), 3.38 - 3.11 (m, 4H), 1.29 (t, J = 7.2 Hz, 3H). ¹³C NMR (214 MHz, Chloroform-d) δ 166.66, 164.61, 148.42, 145.00, 144.25, 139.55, 133.78, 129.55, 128.12, 127.21, 124.30, 118.79, 62.57, 60.09, 50.68, 50.15, 47.41, 42.17, 14.26.

### Example 12: Ethyl 2-((4-(4-(2-chlorophenyl)piperazine-1-carbonyl)-2-nitrophenyl)sulfinyl)acetate

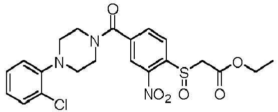

The title compound was synthesized using 1-(2-chlorophenyl)piperazine (26mg, 0.13 mmol, 1 eq), 4-((2-ethoxy-2-oxoethyl)sulfinyl)benzoic acid (40 mg, 0.13 mmol, 1 eq ), oxalyl chloride (19 mg, 0.15 mmol, 1.1 eq) and DIPEA (52 mg, 0.40 mmol, 3 eq) according to general procedure G. This yielded the product (41 mg, 0.09 mmol, 64%). ¹H NMR (400 MHz, Chloroform-d) δ 8.54 - 8.26 (m, 2H), 8.03 (dd, J = 8.0, 1.6 Hz, 1H), 7.39 (dd, J = 8.2, 1.5 Hz, 1H), 7.29 - 7.22 (m, 2H), 7.11 - 6.94 (m, 2H), 4.22 (qd, J = 7.2, 3.9 Hz, 2H), 4.14 (d, J = 13.7 Hz, 1H), 4.02 (s, 2H), 3.78 (d, J = 13.7 Hz, 1H), 3.62 (s, 2H), 3.11 (d, J = 54.3 Hz, 4H), 1.28 (t, J = 7.1 Hz, 3H). ¹³C NMR (101 MHz, Chloroform-d) δ 166.68, 164.61, 148.28, 144.93, 143.95, 139.99, 133.76, 130.91, 129.08, 127.97, 127.90, 124.77, 124.24, 120.73, 62.49, 60.12, 51.74, 50.99, 48.22, 42.87, 14.22. HRMS: Calculated for [C₂₁H₂₂ClN₃O₆S + H]⁺ = 480.09906, found = 480.09907.

### Example 13: Ethyl 2-((4-(4-(3-bromophenyl)piperazine-1-carbonyl)-2-nitrophenyl)sulfinyl)acetate

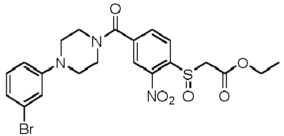

The title compound was synthesized using 1-(3-bromophenyl)piperazine (23mg, 0.10 mmol, 1 eq), 4-((2-ethoxy-2-oxoethyl)sulfinyl)benzoic acid (30 mg, 0.10 mmol, 1 eq ), oxalyl chloride (13.90 mg, 0.11 mmol, 1.1 eq) and DIPEA (39 mg, 0.30 mmol, 3 eq) according to general procedure G. This yielded the product (26 mg, 0.05 mmol, 51%). 1H NMR (500 MHz, Chloroform-d) δ 8.48 - 8.34 (m, 2H), 8.01 (dd, J = 8.1, 1.6 Hz, 1H), 7.15 (dd, J = 8.6, 7.4 Hz, 1H), 7.05 (dd, J = 8.0, 1.2 Hz, 2H), 6.90 - 6.78 (m, 1H), 4.28 - 4.18 (m, 2H), 4.14 (d, J = 13.7 Hz, 1H), 3.97 (bs, 2H), 3.79 (d, J = 13.7 Hz, 1H), 3.70 - 3.02 (m, 6H), 1.29 (t, J = 7.1 Hz, 3H). 13C NMR (126 MHz, Chloroform-d) δ 166.66, 164.60, 151.93, 145.01, 144.21, 139.70, 133.76, 130.71, 128.09, 124.28, 123.85, 123.47, 119.85, 115.43, 62.55, 60.13, 49.65, 49.20, 47.65, 42.42, 14.27.

### Example 14: Ethyl 2-((4-(4-(4-bromophenyl)piperazine-1-carbonyl)-2-nitrophenyl)sulfinyl)acetate

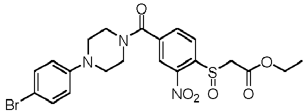

The title compound was synthesized using 1-(4-bromophenyl)piperazine (23mg, 0.10 mmol, 1 eq), 4-((2-ethoxy-2-oxoethyl)sulfinyl)benzoic acid (30 mg, 0.10 mmol, 1 eq ), oxalyl chloride (13.90 mg, 0.11 mmol, 1.1 eq) and DIPEA (39 mg, 0.30 mmol, 3 eq) according to general procedure G. This yielded the product (31 mg, 0.06 mmol, 61%). ¹H NMR (850 MHz, Chloroform-d) δ 8.42 (dd, J = 4.8, 3.1 Hz, 2H), 8.02 (dd, J = 8.0, 1.6 Hz, 1H), 7.41 - 7.39 (m, 2H), 6.85 (d, J = 8.4 Hz, 2H), 4.27 - 4.17 (m, 3H), 4.14 (d, J = 13.7 Hz, 1H), 4.00 (m, 2H), 3.79 (d, J = 13.8 Hz, 1H), 3.61 (m, 2H), 3.34 - 3.09 (m, 6H), 1.29 (t, J = 7.2 Hz, 3H). ¹³C NMR (214 MHz, Chloroform-d) δ 166.65, 164.61, 149.31, 145.00, 144.22, 139.63, 133.78, 132.40, 128.11, 124.30, 118.91, 62.57, 60.10, 50.31, 49.75, 47.57, 42.25, 14.26.

### Example 15: Ethyl 2-((2-nitro-4-(4-(3-(trifluoromethyl)phenyl)piperazine-1-carbonyl)phenyl)sulfinyl)acetate

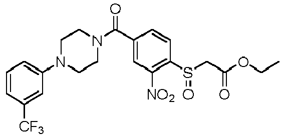

The title compound was synthesized using 1-(3-(trifluoromethyl)phenyl)piperazine (31mg, 0.13 mmol, 1 eq), 4-((2-ethoxy-2-oxoethyl)sulfinyl)benzoic acid (40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (42 mg, 0.08 mmol, 62%). ¹H NMR (400 MHz, Chloroform-d) δ 8.55 - 8.29 (m, 2H), 8.02 (dd, J = 8.1, 1.6 Hz, 1H), 7.40 (t, J = 8.0 Hz, 1H), 7.21 - 7.07 (m, 4H), 4.22 (qq, J = 7.3, 3.6 Hz, 2H), 4.14 (d, J = 13.7 Hz, 1H), 4.00 (br, 2H), 3.79 (d, J = 13.7 Hz, 1H), 3.62 (br, 2H), 3.30 (br, 4H), 1.29 (t, J = 7.1 Hz, 3H). ¹³C NMR (101 MHz, Chloroform-d) δ 166.67, 164.60, 150.87, 145.00, 144.23, 139.65, 133.76, 131.81 (q, J = 32.3 Hz), 129.99, 128.09, 124.28, 122.85(q, J = 273.71 Hz) 119.87, 117.48 (q, J = 4.04 Hz), 113.28(q, J = 3.03 Hz), 62.54, 60.11, 49.57, 47.57, 14.25. HRMS: Calculated for [C₂₂H₂₂F₃N₃O₆S + H]⁺ = 514.12542, found = 514.12520.

### Example 16: Ethyl 2-((2-nitro-4-(4-(4-(trifluoromethyl)phenyl)piperazine-1-carbonyl)phenyl)sulfinyl)acetate

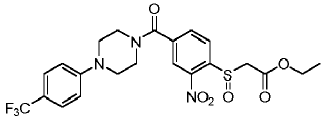

The title compound was synthesized using 1-(4-(trifluoromethyl)phenyl)piperazine (31mg, 0.13 mmol, 1 eq), 4-((2-ethoxy-2-oxoethyl)sulfinyl)benzoic acid (40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (39 mg, 0.08 mmol, 57%). *1*H NMR (400 MHz, Chloroform-d) δ 8.53 - 8.29 (m, 2H), 8.02 (dd, J = 8.1, 1.6 Hz, 1H), 7.62 - 7.45 (m, 2H), 6.96 (d, J = 8.5 Hz, 2H), 4.22 (m, 2H), 4.14 (d, J = 13.7 Hz, 1H), 3.99 (br, 2H), 3.78 (d, J = 13.7 Hz, 1H), 3.62 (br, 2H), 3.35 (br, 4H), 1.28 (t, J = 7.2 Hz, 3H). ¹³C NMR (101 MHz, Chloroform-d) δ 166.57, 164.52, 152.66, 144.88, 144.14, 139.49, 133.67, 127.97, 126.62 (q, J = 3.75), 124.48(q, J = 271.94 Hz), 122.32 (q, J = 32.32 Hz) 124.19, 115.49, 62.44, 60.01, 48.84, 48.31, 47.51, 42.15, 14.14. HRMS: Calculated for [ C₂₂H₂₂F₃N₃O₆S + H]⁺ = 514.12542, found = 514.12530.

### Example 17: Ethyl 2-((4-(4-(3-methoxyphenyl)piperazine-1-carbonyl)-2-nitrophenyl)sulfinyl)acetate

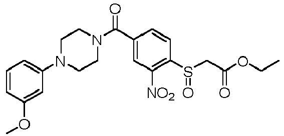

The title compound was synthesized using 1-(3-methoxyphenyl)piperazine ( 19mg, 0.10 mmol, 1 eq), 4-((2-ethoxy-2-oxoethyl)sulfinyl)benzoic acid (30 mg, 0.10 mmol, 1 eq), oxalyl chloride (14 mg, 0.11 mmol, 1.1 eq) and DIPEA (39 mg, 0.30 mmol, 3 eq) according to general procedure G. This yielded the product (29 mg, 0.06 mmol, 55%). ¹H NMR (850 MHz, Chloroform-d) δ 8.43 - 8.39 (m, 2H), 8.02 (dd, J = 8.0, 1.7 Hz, 1H), 7.23 (t, J = 8.2 Hz, 1H), 6.68 - 6.45 (m, 3H), 4.26 - 4.17 (m, 2H), 4.14 (d, J = 13.8 Hz, 1H), 4.01 (m, 2H), 3.81 (s, 3H), 3.78 (d, J = 13.8 Hz, 1H), 3.60 (m, 2H), 3.39 - 3.15 (m, 4H), 1.28 (t, J = 7.2 Hz, 3H). ¹³C NMR (214 MHz, Chloroform-d) δ 166.51, 164.53, 160.67, 151.51, 144.87, 143.99, 139.62, 133.69, 130.19, 127.94, 124.21, 109.75, 106.14, 103.75, 62.44, 60.01, 55.32, 50.26, 49.76, 47.49, 42.20, 14.14.

### Example 18: Ethyl 2-((4-(4-(4-methoxyphenyl)piperazine-1-carbonyl)-2-nitrophenyl)sulfinyl)acetate

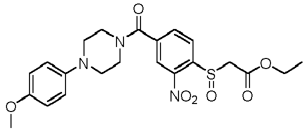

The title compound was synthesized using 1-(4-methoxyphenyl)piperazine (32 mg, 0.17 mmol, 1 eq), 4-((2-ethoxy-2-oxoethyl)sulfonyl)-3-nitrobenzoic acid (50 mg, 0.17 mmol, 1 eq), HATU (95 mg, 0.25 mmol, 1.5 eq) and DIPEA (64 mg, 0.50 mmol, 3 eq) according to general procedure H. This yielded the product (71 mg, 0.15 mmol, 90%). ¹H NMR (850 MHz, Chloroform-d) δ 8.41 (d, J = 1.6 Hz, 1H), 8.40 (d, J = 8.1 Hz, 1H), 8.02 (dd, J = 8.0, 1.6 Hz, 1H), 6.94 (d, J = 8.7 Hz, 2H), 6.89 - 6.82 (m, 2H), 4.26 - 4.16 (m, 2H), 4.14 (d, J = 13.9 Hz, 1H), 3.99 (m, 2H), 3.81 - 3.74 (m, 4H), 3.59 (m, 2H), 3.23 - 2.98 (m, 4H), 1.28 (t, J = 7.6Hz, 3H). ¹³C NMR (214 MHz, Chloroform-d) δ 166.50, 164.61, 154.84, 144.87, 144.65, 143.88, 139.82, 133.73, 127.89, 124.22, 119.31, 114.63, 62.45, 60.06, 55.60, 51.59, 51.04, 47.89, 42.56, 14.17.

### Example 19: Ethyl 2-((2-nitro-4-(4-(3-nitrophenyl)piperazine-1-carbonyl)phenyl)sulfinyl)acetate

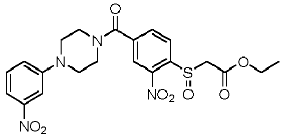

The title compound was synthesized using 1-(3-nitrophenyl)piperazine (21mg, 0.10 mmol, 1 eq), 4-((2-ethoxy-2-oxoethyl)sulfinyl)benzoic acid (30 mg, 0.10 mmol, 1 eq ), oxalyl chloride (14 mg, 0.11 mmol, 1.1 eq) and DIPEA (39 mg, 0.30 mmol, 3 eq) according to general procedure G. This yielded the product (29 mg, 0.05 mmol, 45%). ¹H NMR (850 MHz, Chloroform-d) δ 8.44 - 8.40 (m, 2H), 8.04 (dd, J = 8.0, 1.6 Hz, 1H), 7.76 - 7.73 (m, 2H), 7.46 - 7.42 (m, 1H), 7.24 (ddd, J = 8.3, 2.5, 1.0 Hz, 1H), 4.26 - 4.18 (m, 2H), 4.15 (d, J = 13.8 Hz, 1H), 4.02 (M, 2H), 3.79 (d, J = 13.8 Hz, 1H), 3.71 - 3.60 (m, 2H), 3.50 - 3.25 (m, 4H), 1.29 (t, J = 7.2 Hz, 3H). ¹³C NMR (214 MHz, Chloroform-d) δ 166.69, 164.63, 151.23, 149.29, 144.95, 144.20, 139.47, 133.76, 130.15, 128.05, 124.28, 122.14, 115.19, 110.69, 62.52, 60.08, 49.17, 48.70, 47.39, 42.20, 14.21.

### Example 20: Ethyl 2-((4-(4-([1,1'-biphenyl]-3-yl)piperazine-1-carbonyl)-2-nitrophenyl)sulfinyl)acetate

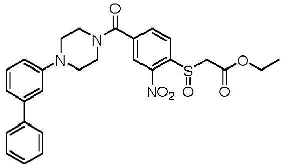

The title compound was synthesized using 1-([1,1'-biphenyl]-3-yl)piperazine ( 32mg, 0.13 mmol, 1 eq), 4-((2-ethoxy-2-oxoethyl)sulfinyl)benzoic acid (40 mg, 0.13 mmol, 1 eq), oxalyl chloride (19 mg, 0.15 mmol, 1.1 eq) and DIPEA (52 mg, 0.4 mmol, 3 eq) according to general procedure G. This yielded the product (40 mg, 0.08 mmol, 58%). ¹H NMR (400 MHz, Chloroform-d) δ 8.56 - 8.26 (m, 2H), 8.02 (dd, J = 8.0, 1.6 Hz, 1H), 7.59 - 7.54 (m, 2H), 7.48 - 7.41 (m, 2H), 7.40 - 7.33 (m, 2H), 7.21 - 7.13 (m, 2H), 7.01 - 6.86 (m, 1H), 4.21 (m, 2H), 4.01 (br, 2H), 3.78 (d, J = 13.7 Hz, 1H), 3.62 (br, 2H), 3.31 (br, 4H), 1.28 (t, J = 7.2 Hz, 3H). ¹³C NMR (101 MHz, Chloroform-d) δ 166.62, 164.61, 151.12, 144.98, 144.09, 142.73, 141.42, 139.83, 133.77, 129.84, 128.88, 128.03, 127.60, 127.33, 124.29, 120.38, 116.16, 116.03, 62.52, 60.13, 50.26, 49.85, 47.82, 42.54, 14.25. HRMS: Calculated for [C₂₇H₂₇N₃O₆S + H]⁺ = 522.16933, found = 522.16902.

### Example 21: Ethyl 2-((4-(4-(3,5-dichlorophenyl)piperazine-1-carbonyl)-2-nitrophenyl)sulfinyl)acetate

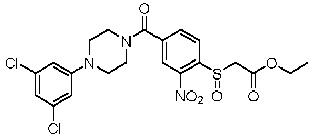

The title compound was synthesized using 1-(3,5-dichlorophenyl)piperazine ( 23mg, 0.10 mmol, 1 eq), 4-((2-ethoxy-2-oxoethyl)sulfinyl)benzoic acid (30 mg, 0.10 mmol, 1 eq), oxalyl chloride (14 mg, 0.11 mmol, 1.1 eq) and DIPEA (39 mg, 0.3 mmol, 3 eq) according to general procedure G. This yielded the product (12 mg, 0.02 mmol, 23%). ¹H NMR (850 MHz, Chloroform-d) δ 8.43 - 8.40 (m, 2H), 8.02 (dd, J = 8.0, 1.6 Hz, 1H), 6.90 (t, J = 1.7 Hz, 1H), 6.79 (d, J = 1.7 Hz, 2H), 4.26 - 4.18 (m, 2H), 4.14 (d, J = 13.8 Hz, 1H), 3.97 (m, 2H), 3.79 (d, J = 13.8 Hz, 1H), 3.60 (m, 2H), 3.27 (m, 4H), 1.29 (t, J = 7.2 Hz, 3H). ¹³C NMR (214 MHz, Chloroform-d) δ 166.67, 164.61, 151.92, 144.97, 144.23, 139.47, 135.80, 133.75, 128.08, 124.28, 120.56, 114.91, 62.54, 60.08, 49.16, 48.70, 47.34, 42.16, 14.23.

### Example 22: Ethyl 2-((4-(4-(3,4-dichlorophenyl)piperazine-1-carbonyl)-2-nitrophenyl)sulfinyl)acetate

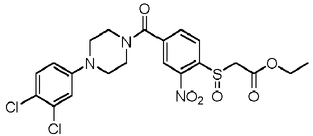

The title compound was synthesized using 1-(3,4-dichlorophenyl)piperazine ( 23mg, 0.10 mmol, 1 eq), 4-((2-ethoxy-2-oxoethyl)sulfinyl)benzoic acid (30 mg, 0.10 mmol, 1 eq), oxalyl chloride (14 mg, 0.11 mmol, 1.1 eq) and DIPEA (39 mg, 0.3 mmol, 3 eq) according to general procedure G. This yielded the product (36 mg, 0.07 mmol, 72%). 1H NMR (500 MHz, Chloroform-d) δ 8.50 - 8.33 (m, 2H), 8.02 (dd, J = 8.1, 1.6 Hz, 1H), 7.41 (d, J = 2.4 Hz, 1H), 7.23 (dd, J = 8.6, 2.4 Hz, 1H), 6.96 (d, J = 8.6 Hz, 1H), 4.28 - 4.16 (m, 2H), 4.14 (d, J = 13.6 Hz, 1H), 4.01 (bs, 2H), 3.78 (d, J = 13.7 Hz, 1H), 3.61 (bs, 2H), 3.08 (bd, J = 69.4 Hz, 4H), 1.28 (t, J = 7.1 Hz, 3H). 13C NMR (126 MHz, Chloroform-d) δ 166.70, 164.56, 147.05, 144.95, 144.06, 139.86, 133.73, 130.63, 129.85, 129.45, 128.03, 127.95, 124.21, 121.49, 62.50, 60.09, 51.70, 50.99, 48.13, 42.74, 14.21.

### Example 23: Ethyl 2-((4-(4-(2,4-dichlorophenyl)piperazine-1-carbonyl)-2-nitrophenyl)sulfinyl)acetate

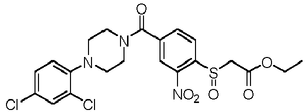

The title compound was synthesized using 1-(2,4-dichlorophenyl)piperazine (23 mg, 0.10 mmol, 1 eq), 4-((2-ethoxy-2-oxoethyl)sulfonyl)-3-nitrobenzoic acid (30 mg, 0.10 mmol, 1 eq), HATU (57 mg, 0.15 mmol, 1.5 eq) and DIPEA (39 mg, 0.30 mmol, 3 eq) according to general procedure H. This yielded the product (41 mg, 0.08 mmol, 84%). ¹H NMR (400 MHz, Chloroform-d) δ 8.44 - 8.37 (m, 2H), 8.02 (dd, J = 8.0, 1.6 Hz, 1H), 7.40 (d, J = 2.4 Hz, 1H), 7.23 (dd, J = 8.6, 2.4 Hz, 1H), 6.97 (d, J = 8.7 Hz, 1H), 4.28 - 4.15 (m, 2H), 4.14 (d, J = 13.7 Hz, 1H), 4.00 (s, 2H), 3.78 (d, J = 13.7 Hz, 1H), 3.61 (s, 2H), 3.15 (s, 2H), 3.01 (s, 2H), 1.28 (t, J = 7.1 Hz, 3H). ¹³C NMR (101 MHz, CDCl3) δ 166.66, 164.58, 147.05, 144.91, 143.99, 139.83, 133.72, 130.57, 129.79, 129.35, 127.96, 127.92, 124.20, 121.50, 62.45, 60.08, 50.93, 48.10, 14.19. HRMS: [M+H]+ calculated for C21H21Cl2N3O6S: 514.06009; found: 514.06015. HRMS: Calculated for [C₂₁H₂₁Cl₂N₃O₆S + H]⁺ = 514.06009, found = 514.06016.

### Example 24: Ethyl 2-((4-(4-(2,6-dichlorophenyl)piperazine-1-carbonyl)-2-nitrophenyl)sulfinyl)acetate

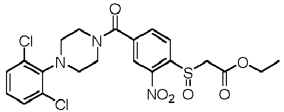

The title compound was synthesized using 1-(2,6-dichlorophenyl)piperazine (20 mg, 0.09 mmol, 1 eq), 4-((2-ethoxy-2-oxoethyl)sulfonyl)-3-nitrobenzoic acid (39 mg, 0.14 mmol, 1.5 eq), HATU (53 mg, 0.14 mmol, 1.5 eq) and DIPEA (38 mg, 0.30 mmol, 3 eq) according to general procedure H. This yielded the product (63 mg, 0.08 mmol, 89%). ¹H NMR (400 MHz, Chloroform-d) δ 8.43 - 8.37 (m, 2H), 8.01 (dd, J = 8.0, 1.6 Hz, 1H), 7.34 - 7.25 (m, 2H), 6.98 - 6.91 (m, 3H), 4.28 - 4.17 (m, 2H), 4.14 (d, J = 13.7 Hz, 1H), 3.98 (s, 2H), 3.77 (d, J = 13.7 Hz, 1H), 3.59 (s, 2H), 3.31 (s, 2H), 3.18 (s, 2H), 1.28 (t, J = 7.1 Hz, 3H). 13C NMR (101 MHz, CDCl3) δ 166.54, 164.58, 150.65, 144.91, 143.98, 139.79, 133.72, 129.41, 127.93, 124.22, 121.13, 117.00, 62.44, 60.08, 50.12, 49.62, 47.82, 42.51, 38.68, 14.18. HRMS: Calculated for [C₂₁H₂₁Cl₂N₃O₆S + H]⁺ = 514.06009, found = 514.05997.

### Example 25: Ethyl 2-((4-((S)-4-(3-fluorophenyl)-3-methylpiperazine-1-carbonyl)-2-nitrophenyl)sulfinyl)acetate

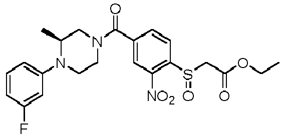

The title compound was synthesized using (S)-1-(3-fluorophenyl)-2-methylpiperazine (26mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (38 mg, 0.08 mmol, 60%). ¹H NMR (400 MHz, Chloroform-d) δ 8.60 - 8.27 (m, 3H), 8.06 - 7.91 (m, 1H), 7.22 (td, J = 8.4, 6.8 Hz, 1H), 6.76 - 6.46 (m, 4H), 4.47 (m, 1H), 4.21 (m, 2H), 4.14 (d, J = 13.7 Hz, 1H), 3.94 (m, 1H), 3.78 (d, J = 13.7 Hz, 1H), 3.74 - 2.99 (m, 5H), 1.29 (t, J = 7.1 Hz, 3H), 1.08 (m, 3H). ¹³C NMR (101 MHz, Chloroform-d) δ 167.37, 163.97 (d, J = 244.1 Hz), 164.59, 151.16 (d, J = 9.5 Hz), 144.96, 144.05, 139.73, 133.74, 130.55 (d, J = 9.9 Hz), 128.02, 124.22, 112.38 (d, J = 2.4 Hz), 106.99(d, J = 21.3 Hz), 104.00(d, J = 24.9 Hz), 62.50, 60.09, 52.53, 51.68, 47.39, 42.43, 14.23, 12.52. HRMS: Calculated for [ C₂₂H₂₄FN₃O₆S + H]⁺ = 478.14426, found = 478.14397.

### Example 26: Ethyl 2-((4-((R)-4-(3-chlorophenyl)-3-methylpiperazine-1-carbonyl)-2-nitrophenyl)sulfinyl)acetate

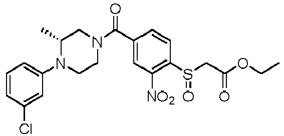

The title compound was synthesized using (R)-1-(3-fluorophenyl)-2-methylpiperazine (28mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (22 mg, 0.05 mmol, 34%). ¹H NMR (400 MHz, Chloroform-d) δ 8.43 - 8.38 (m, 2H), 8.11 - 7.94 (m, 1H), 7.20 (t, J = 8.3 Hz, 1H), 6.90 - 6.74 (m, 3H), 4.63 - 4.27 (m, 1H), 4.22 (m, 2H), 4.14 (d, J = 13.7 Hz, 1H), 3.92 (m, 1H), 3.78 (d, J = 13.7 Hz, 1H), 3.74 - 3.00 (m, 5H), 1.28 (t, J = 7.1 Hz, 3H), 1.06 (m, 3H). ¹³C NMR (101 MHz, Chloroform-d) δ 167.35, 164.59, 150.62, 144.95, 144.04, 139.71, 135.24, 133.73, 130.40, 128.01, 124.23, 120.43, 117.19, 115.18, 62.50, 60.10, 52.61, 51.81, 47.61, 42.41, 14.23, 12.63. HRMS: Calculated for [ C₂₂H₂₄ClN₃O₆S + H]⁺ = 494.11471, found = 494.11423.

### Example 27: Ethyl 2-((4-((S)-4-(3-chlorophenyl)-3-methylpiperazine-1-carbonyl)-2-nitrophenyl)sulfinyl)acetate

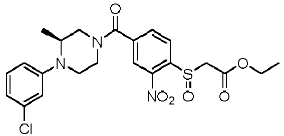

The title compound was synthesized using (*S*)-1-(3-fluorophenyl)-2-methylpiperazine (28mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (50 mg, 0.10 mmol, 76%). ¹H NMR (600 MHz, Chloroform-d) δ 8.51 - 8.33 (m, 2H), 8.04 (dd, J = 8.0, 1.6 Hz, 1H), 7.24 (d, J = 8.2 Hz, 1H), 6.95 (m, 3H), 4.57 - 4.09 (m, 4H), 4.05 - 3.08 (m, 7H), 1.28 (d, J = 7.2Hz, 3H), 1.09 (m, 3H). ¹³C NMR (151 MHz, Chloroform-d) δ 167.39, 164.59, 149.17, 144.98, 143.92, 139.42, 135.45, 133.77, 130.63, 128.09, 124.29, 121.66, 117.78, 115.87, 62.56, 60.02, 52.87, 52.41, 47.21, 42.19, 14.19, 12.81. HRMS: Calculated for [C₂₂H₂₄ClN₃O₆S + H]⁺ = 494.11471, found = 494.11432.

### Example 28: Ethyl 2-((4-((R)-4-(3-bromophenyl)-3-methylpiperazine-1-carbonyl)-2-nitrophenyl)sulfinyl)acetate

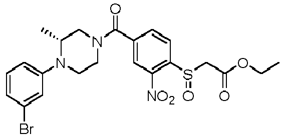

The title compound was synthesized using (*R*)-1-(3-bromophenyl)-2-methylpiperazine (34mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (50mg, 0.09 mmol, 70%). ¹H NMR (600 MHz, Chloroform-d) δ 8.51 - 8.35 (m, 2H), 8.03 (dd, J = 8.0, 1.6 Hz, 1H), 7.23 - 7.09 (m, 3H), 6.96 (m, 1H), 4.50 - 4.04 (m, 4H), 3.99 - 3.13 (m, 7H), 1.27 (t, J = 7.1Hz, 3H), 1.16 - 0.92 (m, 3H). ¹³C NMR (151 MHz, Chloroform-d) δ 167.42, 164.59, 149.38, 144.99, 143.89, 139.36, 133.78, 130.94, 128.10, 124.86, 124.31, 121.41, 117.19, 62.58, 60.01, 53.10, 52.37, 47.17, 42.14, 14.19, 12.89. HRMS: Calculated for [C₂₂H₂₄BrN₃O₃S + H]⁺ = 538.06420, found = 538.06390.

### Example 29: Ethyl 2-((4-((R)-4-(3-bromophenyl)-3-methylpiperazine-1-carbonyl)-2-nitrophenyl)sulfinyl)acetate

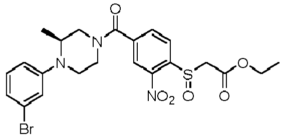

The title compound was synthesized using (S)-1-(3-bromophenyl)-2-methylpiperazine (34mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (40mg, 0.07 mmol, 56%). ¹H NMR (400 MHz, Chloroform-d) δ 8.47 - 8.34 (m, 2H), 8.14 - 7.89 (m, 1H), 7.14 (m, 1H), 7.02 (m, 2H), 6.83 (d, J = 8.3 Hz, 1H), 4.42 (m, 1H), 4.22 (m, 1H), 4.14 (d, J = 13.7 Hz, 1H), 4.01 (m, 1H), 3.78 (d, J = 13.6 Hz, 1H), 3.72 - 3.02 (m, 5H), 1.28 (d, J = 7.1 Hz, 3H), 1.07 (m, 3H). ¹³C NMR (101 MHz, Chloroform-d) δ 167.36, 164.58, 150.78, 144.94, 144.06, 139.70, 133.73, 130.69, 128.00, 124.23, 123.47, 123.40, 120.06, 115.66, 62.49, 60.08, 52.62, 51.84, 47.38, 42.40, 14.23, 12.66. HRMS: Calculated for [ C₂₂H₂₄BrN₃O₆S + H]⁺ = 538.06420, found = 538.06382.

### Example 30: Ethyl 2-((4-((R)-3-methyl-4-(3-(trifluoromethyl)phenyl)piperazine-1-carbonyl)-2-nitrophenyl)sulfinyl)acetate

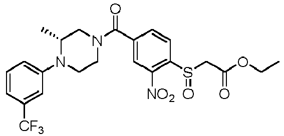

The title compound was synthesized using (*R*)-1-(3-bromophenyl)-2-methylpiperazine (32mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (60mg, 0.11 mmol, 86%). ¹H NMR (600 MHz, Chloroform-*d*) δ 8.51 - 8.33 (m, 2H), 8.05 (dd, *J* = 8.0, 1.6 Hz, 1H), 7.44 (m, 1H), 7.20 (m, 3H), 4.61 - 4.13 (m, 4H), 4.11 - 3.16 (m, 7H), 1.28 (t, *J* = 7.2Hz, 3H), 1.19 - 1.01 (m, 3H). ¹³C NMR (151 MHz, Chloroform-*d*) δ 167.47, 164.60, 148.82, 145.00, 143.79, 139.39, 133.78, 131.97 (q, *J* = 32.0 Hz), 130.18, 128.10, 124.09 (q, *J* = 271.8Hz), 124.31, 120.76, 118.53, 114.60, 62.59, 59.99, 52.98, 52.47, 47.33, 42.28, 14.17, 12.81. HRMS: Calculated for [C₂₃H₂₄F₃N₃O₆S + H]⁺ = 528.14107, found = 528.14085.

### Example 31: Ethyl 2-((4-((S)-3-methyl-4-(3-(trifluoromethyl)phenyl)piperazine-1-carbonyl)-2-nitrophenyl)sulfinyl)acetate

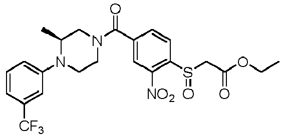

The title compound was synthesized using (*S*)-1-(3-bromophenyl)-2-methylpiperazine (32mg, 0.13 mmol, 1 eq), 4-((2-ethoxy-2-oxoethyl)sulfinyl)benzoic acid ( 40 mg, 0.13 mmol, 1 eq), oxalyl chloride (19 mg, 0.15 mmol, 1.1 eq) and DIPEA (52 mg, 0.40 mmol, 3 eq) according to general procedure G. This yielded the product (57mg, 0.11 mmol, 82%). ¹H NMR (400 MHz, Chloroform-d) δ 8.46 - 8.36 (m, 2H), 8.03 (dt, J = 8.0, 1.9 Hz, 1H), 7.40 (t, J = 7.9 Hz, 1H), 7.20 - 6.98 (m, 3H), 4.62 - 4.29 (m, 1H), 4.22 (qdd, J = 7.1, 3.7, 1.4 Hz, 2H), 4.14 (d, J = 13.7 Hz, 1H), 4.11 - 3.83 (m, 1H), 3.79 (d, J = 13.7 Hz, 1H), 3.74 - 3.06 (m, 5H), 1.29 (t, J = 7.2 Hz, 3H), 1.20 - 0.93 (m, 3H). ¹³C NMR (101 MHz, Chloroform-d) δ 167.43, 164.61, 149.71, 144.96, 144.08, 139.68, 133.74, 131.78 (q, J = 31.8 Hz), 129.98, 128.02, 124.24, 124.22 (q, J = 273.71 Hz), 120.16, 117.04, 113.59, 62.49, 60.08, 52.61, 51.80, 47.38, 42.38, 14.21, 12.66. HRMS: Calculated for [C₂₃H₂₄F₃N₃O₆S + H]⁺ = 528.14107, found = 528.14105.

### Example 32: Methyl 2-((4-((R)-4-(3-chlorophenyl)-3-methylpiperazine-1-carbonyl)-2-nitrophenyl)sulfinyl)acetate

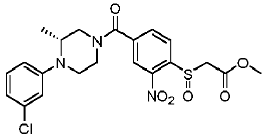

Step A: To a solution of ethyl 2-((4-((R)-4-(3-chlorophenyl)-3-methylpiperazine-1-carbonyl)-2-nitrophenyl)sulfinyl)acetate (38 mg, 0.08 mmol, 1 eq) in 2ml MeOH was added 2 ml TEA and 1ml water. Then the reaction mixture was stirred overnight at room temperature. The reaction progress was monitored by TLC. Once completed, the pH of resulted mixture was adjusted to 1 with 1M HCl solution. Then the mixture was diluted with EtOAc and the organic layer was washed with water and dried over anhydrous magnesium sulfate. After filtration, the filtrate was concentrated under reduced pressure. The residue was purified by silica gel column chromatography (MeOH/DCM, 1%→2%) to afford the product (31 mg, 0.07 mmol, 86 %).
Step B: The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq ), MeOH (55 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (28mg, 0.061 mmol, 68%). ¹H NMR (600 MHz, Chloroform-d) δ 8.50 - 8.30 (m, 2H), 8.04 (dd, J = 8.0, 1.6 Hz, 1H), 7.24 (t, J = 8.2 Hz, 1H), 7.05 - 6.79 (m, 3H), 4.57 - 3.61 (m, 8H), 3.60 - 3.12 (m, 4H), 1.09 (d, J = 78.9 Hz, 3H). ¹³C NMR (151 MHz, Chloroform-d) δ 167.42, 165.03, 149.47, 145.03, 143.88, 139.53, 135.46, 133.81, 130.62, 128.05, 124.33, 122.12, 118.30, 116.40, 59.90, 53.18, 52.71, 52.44, 46.22, 42.24, 12.80. HRMS: Calculated for [C₂₁H₂₂ClN₃O₆S + H]⁺ = 480.09906, found = 480.09894.

### Example 33: 2-((4-((R)-4-(3-Chlorophenyl)-3-methylpiperazine-1-carbonyl)-2-nitrophenyl)sulfinyl)-N-ethyl-N-methylacetamide

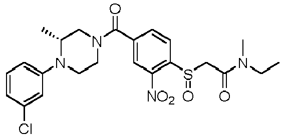

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), *N*-methylethanamine (102 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (18mg, 0.036 mmol, 41%). ¹H NMR (600 MHz, Chloroform-d) δ 8.39 (m, 2H), 8.01 (d, J = 7.8 Hz, 1H), 7.22 (t, J = 8.0 Hz, 1H), 6.88 (m, 3H), 4.36 - 3.91 (m, 2H), 3.76 (m, 3H), 3.58 - 3.32 (m, 4H), 3.32 - 3.12 (m, 2H), 3.03 (dd, J = 76.4, 3.6 Hz, 3H), 1.27 - 0.91 (m, 6H). ¹³C NMR (151 MHz, Chloroform-d) δ 167.22, 163.56, 150.06, 145.10, 144.32, 139.36, 135.37, 133.70, 130.52, 128.10, 124.16, 121.00, 117.45, 115.51, 59.51, 52.40, 47.45, 45.51, 43.43, 42.30, 33.36, 13.72, 12.88. HRMS: Calculated for [ C₂₃H₂₇ClN₄O₅S + H]⁺ = 507.14634, found = 507.14629.

### Example 34: Isopropyl 2-((4-((R)-4-(3-chlorophenyl)-3-methylpiperazine-1-carbonyl)-2-nitrophenyl)sulfinyl)acetate

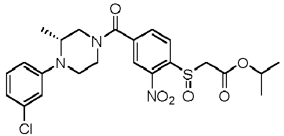

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), propan-2-ol (103 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (16mg, 0.031 mmol, 37%). ¹H NMR (600 MHz, Chloroform-d) δ 8.50 - 8.33 (m, 2H), 8.03 (dd, J = 8.0, 1.6 Hz, 1H), 7.22 (t, J = 8.1 Hz, 1H), 6.88 (m, 3H), 5.07 (m, 1H), 4.39 (d, J = 143.2 Hz, 1H), 4.13 (d, J = 13.8 Hz, 1H), 3.90 (m, 1H), 3.76 (d, J = 13.8 Hz, 1H), 3.74 - 3.10 (m, 5H), 1.31 - 1.22 (m, 6H), 1.08 (d, J = 75.1 Hz, 3H). ¹³C NMR (151 MHz, Chloroform-d) δ 167.13, 164.17, 150.12, 145.00, 144.12, 139.55, 135.39, 133.76, 130.54, 128.19, 124.28, 121.53, 117.52, 115.53, 70.72, 60.24, 52.36, 47.38, 45.08, 42.35, 21.83, 12.75. HRMS: Calculated for [C₂₃H₂₆ClN₃O₆S + H]⁺ = 508.13036, found = 508.13049.

### Example 35: 2,2,2-Trifluoroethyl 2-((4-((R)-4-(3-chlorophenyl)-3-methylpiperazine-1-carbonyl)-2-nitrophenyl)sulfinyl)acetate

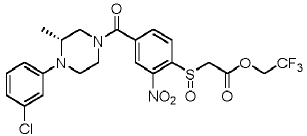

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), 2,2,2-trifluoroethan-1-ol (172 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (8mg, 0.015 mmol, 17%). ¹H NMR (600 MHz, Chloroform-d) δ 8.50 - 8.33 (m, 2H), 8.10 - 7.95 (m, 1H), 7.22 (t, J = 8.0 Hz, 1H), 6.86 (d, J = 52.7 Hz, 3H), 4.52 (qd, J = 8.2, 1.8 Hz, 2H), 4.35 - 3.05 (m, 9H), 1.07 (m, 3H). ¹³C NMR (151 MHz, Chloroform-d) δ 167.35, 163.16, 150.41, 145.00, 143.45, 140.02, 135.38, 133.95, 130.51, 128.18, 124.42, 122.56 (q, J = 277.4 Hz), 120.88, 117.47, 115.46, 61.54 (q, J = 37.3 Hz), 59.13, 52.62, 52.19, 47.49, 42.44, 12.72. HRMS: Calculated for [C₂₂H₂₁ClF₃N₃O₆S + H]⁺ = 548.08644, found = 548.08643.

### Example 36: Propyl 2-((4-((R)-4-(3-chlorophenyl)-3-methylpiperazine-1-carbonyl)-2-nitrophenyl)sulfinyl)acetate

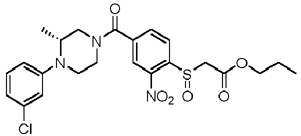

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), propan-1-ol (103 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (26mg, 0.051 mmol, 60%). ¹H NMR (850 MHz, Chloroform-d) δ 8.42 (d, J = 7.9 Hz, 2H), 8.04 (d, J = 8.1 Hz, 1H), 7.24 (s, 1H), 6.94 (t, J = 40.3 Hz, 3H), 4.50 - 3.14 (m, 11H), 1.69 (p, J = 7.0 Hz, 2H), 1.20 - 0.98 (m, 3H), 0.96 (td, J = 7.4, 1.1 Hz, 3H). ¹³C NMR (214 MHz, Chloroform-d) δ 167.08, 164.70, 149.57, 145.00, 144.09, 139.47, 135.46, 133.78, 130.63, 128.10, 124.32, 121.58, 118.31, 116.32, 68.10, 60.15, 52.73, 52.45, 47.17, 42.22, 21.94, 12.77, 10.41. HRMS: Calculated for [C₂₃H₂₆ClN₃O₆S + H]⁺ = 508.13036, found = 508.13022.

### Example 37: Butyl 2-((4-((R)-4-(3-chlorophenyl)-3-methylpiperazine-1-carbonyl)-2-nitrophenyl)sulfinyl)acetate

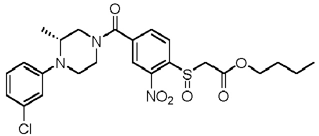

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), butan-1-ol (127 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (22mg, 0.042 mmol, 49%). ¹H NMR (850 MHz, Chloroform-d) δ 8.45 - 8.36 (m, 2H), 8.03 (dd, J = 8.0, 1.6 Hz, 1H), 7.21 (t, J = 8.1 Hz, 1H), 6.94 - 6.86 (m, 2H), 6.82 (d, J = 24.0 Hz, 1H), 4.41 - 3.11 (m, 11H), 1.67 - 1.61 (m, 2H), 1.39 (m, 2H), 1.17 - 0.98 (m, 3H), 0.94 (td, J = 7.4, 1.6 Hz, 3H). ¹³C NMR (214 MHz, Chloroform-d) δ 167.08, 164.74, 150.24, 144.97, 143.99, 139.60, 135.34, 133.77, 130.50, 128.05, 124.29, 120.80, 117.87, 115.94, 66.42, 60.17, 52.14, 47.37, 44.79, 42.36, 30.52, 19.11, 13.77, 12.64. HRMS: Calculated for [C₂₄H₂₈ClN₃O₆S + H]⁺ = 522.14601, found = 522.14572.

### Example 38: sec-Butyl 2-((4-((R)-4-(3-chlorophenyl)-3-methylpiperazine-1-carbonyl)-2-nitrophenyl)sulfinyl)acetate

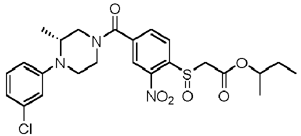

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), butan-2-ol (127 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (12mg, 0.023 mmol, 27%). ¹H NMR (850 MHz, Chloroform-d) δ 8.48 - 8.35 (m, 2H), 8.08 - 8.01 (m, 1H), 7.28 (m, 1H), 7.01 (t, J = 48.6 Hz, 3H), 4.93 (m, 1H), 4.47-3.20 (m, 9H), 1.65 (m, 1H), 1.62 - 1.53 (m, 1H), 1.25 (m, 3H), 1.11 (d, J = 120.8 Hz, 3H), 0.95 - 0.90 (m, 3H). ¹³C NMR (214 MHz, Chloroform-d) δ 167.18, 164.32, 147.91, 145.00, 144.07, 139.13, 135.67, 133.83, 130.84, 128.24, 124.38, 123.57, 118.95, 117.16, 75.40, 60.20, 54.46, 52.24, 46.99, 41.97, 28.78, 19.44, 13.27, 9.70. HRMS: Calculated for [ C₂₄H₂₈ClN₃O₆S + H]⁺ = 522.14601, found = 522.14612.

### Example 39: tert-Pentyl 2-((4-((R)-4-(3-chlorophenyl)-3-methylpiperazine-1-carbonyl)-2-nitrophenyl)sulfinyl)acetate

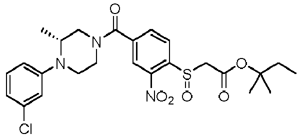

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), 2-methylbutan-2-ol (151 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (12 mg, 0.022 mmol, 26%). ¹H NMR (850 MHz, Chloroform-d) δ 8.49 - 8.38 (m, 2H), 8.04 (d, J = 8.0 Hz, 1H), 7.28 (s, 1H), 7.05 (s, 3H), 4.51 - 3.19 (m, 15H), 1.80 (tt, J = 14.1, 6.8 Hz, 2H), 1.47 (d, J = 13.0 Hz, 6H), 1.12 (d, J = 123.0 Hz, 3H), 0.93 (t, J = 7.5 Hz, 3H). ¹³C NMR (214 MHz, Chloroform-d) δ 167.13, 163.73, 145.01, 144.43, 139.13, 135.68, 133.80, 130.84, 128.26, 124.36, 86.81, 61.28, 53.78, 52.28, 46.94, 41.87, 33.64, 25.56, 13.34, 8.34. (4C missed). HRMS: Calculated for [C₂₅H₃₀ClN₃O₆S + H]⁺ = 536.16166, found = 536.16167.

### Example 40: tert-Butyl 2-((4-((R)-4-(3-chlorophenyl)-3-methylpiperazine-1-carbonyl)-2-nitrophenyl)sulfinyl)acetate

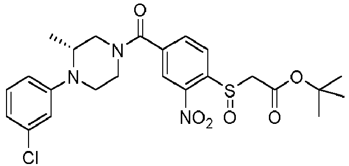

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), *tert*-butanol (127 mg, 1.72 mmol, 20 eq), oxalyl chloride (12 mg, 0.94 mmol, 1.1 eq) and DIPEA (33 mg, 0.26 mmol, 3 eq) according to general procedure G. This yielded the product (5mg, 0.001mmol, 11%). LC/MS: calculated for [C24H28ClN3O6S + H] = 522.19, found = 522.26.

### Example 41: 3-Hydroxypropyl 2-((4-((R)-4-(3-chlorophenyl)-3-methylpiperazine-1-carbonyl)-2-nitrophenyl)sulfinyl)acetate

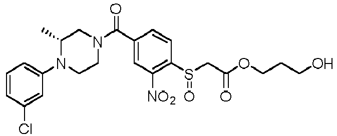

The title compound was synthesized using 2-((4-((*R*)-4-(3-chlorophenyl)-3-methylpiperazine-1-carbonyl)-2-nitrophenyl)sulfinyl)acetic acid (40 mg, 0.09 mmol, 1 eq), propane-1,3-diol (131 mg, 1.72 mmol, 20 eq), oxalyl chloride (12 mg, 0.94 mmol, 1.1 eq ) and DIPEA (33 mg, 0.26 mmol, 3 eq) according to general procedure G. This yielded the product (12 mg, 0.040 mmol, 47%). ¹H NMR (850 MHz, Chloroform-d) δ 8.50 - 8.34 (m, 2H), 8.05 (s, 1H), 7.25 (s, 1H), 6.97 (m, 3H), 4.59 - 3.12 (m, 13H), 3.01 (br, 1H), 1.93 - 1.75 (m, 2H), 1.21 - 0.97 (m, 3H). ¹³C NMR (214 MHz, Chloroform-d) δ 167.21, 164.38, 149.95, 145.04, 143.65, 139.57, 135.51, 133.86, 130.67, 128.26, 124.29, 121.56, 118.46, 116.56, 64.61, 63.54, 59.10, 58.95, 52.47, 47.20, 42.19, 31.18, 13.06.

### Example 42: Ethyl 2-((4-(4-(3-chlorophenyl)-3,5-dimethylpiperazine-1-carbonyl)-2-nitrophenyl)sulfinyl)acetate

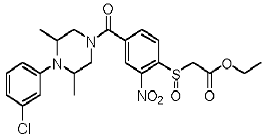

Step A: To a solution of 1-bromo-3-chlorobenzene (191 mg, 1 mmol, 1 eq) and tert-butyl 3,5-dimethylpiperazine-1-carboxylate (257 mg, 1.2 mmol, 1.2 eq) in 2ml dry 1,4-dioxane was added KHMDS (239 mg, 1,2 mmol, 1.2 eq) and the reaction mixture was heated to 100°C and stirred overnight. The reaction progress was monitored by TLC. Once completed, the mixture was cool to room temperature, diluted with EtOAc, washed with water and dried over anhydrous magnesium sulfate. After filtration, the filtrate was concentrated under reduced pressure. The residue was purified by silica gel column chromatography (Diethyl ether/Pentane, 5%→20%) to afford the product (70mg, 0.22 mmol, 22%).¹H NMR (400 MHz, Chloroform-d) δ 7.22 (td, J = 8.2, 1.4 Hz, 1H), 7.06 (dt, J = 8.1, 1.3 Hz, 2H), 6.94 (dq, J = 8.0, 1.4 Hz, 1H), 3.80 (br, 2H), 3.38 - 2.81 (m, 4H), 1.50 (s, 9H), 0.84 (dd, J = 6.3, 1.3 Hz, 6H). ¹³C NMR (101 MHz, Chloroform-d) δ 154.75, 150.43, 134.70, 129.99, 124.18, 124.06, 122.50, 54.31, 28.54, 18.12.
Step B: tert-butyl 4-(3-chlorophenyl)-3,5-dimethylpiperazine-1-carboxylate (70 mg, 0.22 mmol, 1 eq) was used according to general procedure B. This yield the product (42 mg, 0.19 mmol, 86%). 1H NMR (400 MHz, Chloroform-d) δ 7.36 - 7.27 (m, 2H), 7.25 (t, J = 1.9 Hz, 1H), 7.16 (dt, J = 7.7, 1.6 Hz, 1H), 3.52 (d, J = 11.0 Hz, 2H), 3.44 - 3.36 (m, 2H), 2.99 (t, J = 11.8 Hz, 2H), 0.84 (d, J = 6.3 Hz, 6H).13C NMR (101 MHz, Chloroform-d) δ 134.97, 130.45, 127.74, 54.18, 49.39, 17.80.
Step C: The title compound was synthesized using 4-((2-ethoxy-2-oxoethyl)sulfinyl)-3-nitrobenzoic acid (40 mg, 0.13 mmol, 1 eq), 1-(3-chlorophenyl)-2,6-dimethylpiperazine (30 mg, 0.13 mmol, 1 eq), oxalyl chloride (19 mg, 0.15 mmol, 1.1 eq ) and DIPEA (52 mg, 0.40 mmol, 3 eq) according to general procedure G. This yielded the product (36 mg, 0.07 mmol, 53%). ¹H NMR (850 MHz, Chloroform-d) δ 8.49 (s, 1H), 8.42 (d, J = 8.0 Hz, 1H), 8.09 (d, J = 8.0 Hz, 1H), 7.55 - 7.39 (m, 4H), 4.76 (bs, 1H), 4.25 - 4.12 (m, 3H), 3.92 (bs, 1H), 3.80 (d, J = 13.9 Hz, 1H), 3.75 - 3.33 (m, 4H), 1.28 (t, J = 7.2 Hz, 3H), 1.06 (m, 6H). ¹³C NMR (214 MHz, Chloroform-d) δ 166.57, 164.68, 145.16, 144.39, 142.76, 138.73, 136.19, 133.88, 131.35, 129.33, 128.14, 124.78, 123.92, 123.03, 62.61, 60.63, 60.03, 51.89, 46.59, 16.21, 14.21. HRMS: Calculated for [C₂₃H₂₆ClN₃O₆S + H]⁺ = 508.13036, found = 508.13032.

### Example 43: Ethyl 2-((4-((R)-4-(3-chlorophenyl)-3-methylpiperazine-1-carbonyl)-2-fluorophenyl)sulfinyl)acetate

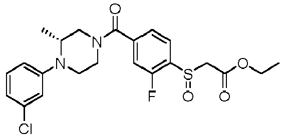

Step A: 3,4-difluorobenzoic acid (200 mg, 1.27 mmol, 1 eq) was used according to procedure general procedure G. This yield the product (198 mg, 0.92 mmol, 73%). ¹H NMR (400 MHz, Chloroform-d) δ 7.90 - 7.67 (m, 2H), 7.18 (q, J = 9.5, 8.8 Hz, 1H), 1.60 (S, 9H). ¹³C NMR (101 MHz, Chloroform-d) δ 163.74, 153.31 (dd, J = 254.9, 12.8 Hz), 150.00 (dd, J = 249.3, 13.0 Hz), 129.14 (dd, J = 5.3, 3.6 Hz), 126.31 (dd, J = 7.3, 3.6 Hz), 118.75 (d, J = 18.6 Hz), 117.06 (d, J = 17.8 Hz), 81.83, 28.07.
Step B: ethyl 2-mercaptoacetate (101 mg, 0.84 mmol, 1 eq) and *tert*-butyl 3,4-difluorobenzoate (198 mg, 0.92 mmol, 1.1eq) were used according to general procedure D. This yielded the product (188 mg, 0.60 mmol, 71%). 1H NMR (400 MHz, Chloroform-d) δ 7.73 (dd, J = 8.1, 1.8 Hz, 1H), 7.64 (dd, J = 10.4, 1.7 Hz, 1H), 7.42 (dd, J = 8.2, 7.3 Hz, 1H), 4.17 (q, J = 7.1 Hz, 2H), 3.71 (s, 2H), 1.59 (s, 9H), 1.23 (t, J = 7.1 Hz, 3H). 13C NMR (101 MHz, Chloroform-d) δ 168.86, 164.20, 160.22 (d, J = 246.1 Hz), 132.42 (d, J = 7.1 Hz), 130.11 (d, J = 1.9 Hz), 127.99 (d, J = 17.4 Hz), 125.48 (d, J = 3.4 Hz), 116.27 (d, J = 23.7 Hz), 81.72, 61.83, 34.70, 28.14, 14.10.
Step C: tert-butyl 4-((2-ethoxy-2-oxoethyl)thio)-3-fluorobenzoate (40 mg, 0.13 mmol, 1.00 eq) was used according to general procedure E. This yielded the product (38 mg, 0.12 mmol, 90%). ¹H NMR (400 MHz, Chloroform-d) δ 8.01 (dd, J = 8.1, 1.4 Hz, 1H), 7.91 (dd, J = 8.1, 6.6 Hz, 1H), 7.74 (dd, J = 10.1, 1.4 Hz, 1H), 4.20 (qd, J = 7.2, 1.2 Hz, 2H), 3.97 (d, J = 13.7 Hz, 1H), 3.78 (d, J = 13.7 Hz, 1H), 1.61 (s, 9H), 1.25 (t, J = 7.1 Hz, 3H). ¹³C NMR (101 MHz, Chloroform-d) δ 164.23, 163.55, 157.35 (d, J = 247.8 Hz), 137.41 (d, J = 6.9 Hz), 134.60 (d, J = 17.2 Hz), 126.37, 126.32 (d, J = 4.1 Hz), 116.73 (d, J = 22.0 Hz), 82.65, 62.44, 58.64, 28.18, 14.17.
Step D: tert-butyl 4-((2-ethoxy-2-oxoethyl)sulfinyl)-3-fluorobenzoate (30mg, 0.09 mmol, 1 eq) was used according to general procedure F. This yielded the product (18 mg, 0.7 mmol, 72%). ¹H NMR (400 MHz, Methanol-d4) δ 8.10 (dd, J = 8.1, 1.5 Hz, 1H), 7.95 - 7.80 (m, 2H), 4.27 - 4.05 (m, 3H), 3.95 (d, J = 14.2 Hz, 1H), 1.19 (t, J = 7.1 Hz, 3H). ¹³C NMR (101 MHz, Methanol-d4) δ 167.20, 165.75, 159.03 (d, J = 247.7 Hz), 137.86 (d, J = 7.1 Hz), 135.69 (d, J = 16.9 Hz), 127.66 (d, J = 3.3 Hz), 127.53 (d, J = 2.1 Hz), 117.91 (d, J = 22.2 Hz), 63.17, 59.38, 14.31.
Step E: The title compound was synthesized using 4-((2-ethoxy-2-oxoethyl)sulfinyl)-3-fluorobenzoic acid (40 mg, 0.15 mmol, 1 eq), (*R*)-1-(3-chlorophenyl)-2-methylpiperazine (31 mg, 0.15 mmol, 1 eq), oxalyl chloride (20 mg, 0.16 mmol, 1.1 eq ) and DIPEA (57 mg, 0.44 mmol, 3 eq) according to general procedure G. This yielded the product (36 mg, 0.07 mmol, 53%). ¹H NMR (850 MHz, Chloroform-d) δ 7.95 (t, J = 7.2 Hz, 1H), 7.51 (d, J = 8.4 Hz, 1H), 7.31 (t, J = 11.5 Hz, 2H), 7.24 - 6.98 (m, 3H), 4.35 - 3.17 (m, 11H), 1.25 (t, J = 7.2 Hz, 3H), 1.22 - 0.94 (m, 3H). ¹³C NMR (214 MHz, Chloroform-d) δ 168.20, 164.20, 157.65 (d, J = 246.3 Hz), 146.52, 140.01 (d, J = 29.3 Hz), 135.80, 132.01, 131.01, 127.15, 124.17, 124.01, 118.82, 117.08, 115.23 (d, J = 22.0 Hz), 62.61, 58.57, 55.09, 51.80, 46.42, 41.44, 14.10, 13.39. HRMS: Calculated for [ C₂₂H₂₄ClFN₂O₄S + H]⁺ = 467.12021, found = 467.12018.

### Example 44: Ethyl 2-((4-(4-(3-chlorophenyl)-2-methylpiperazine-1-carbonyl)-2-fluorophenyl)sulfinyl)acetate

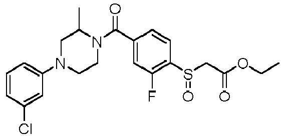

Step A: 1-bromo-3-chlorobenzene (96 mg, 0.50 mmol, 1 eq), *tert*-butyl 2-methylpiperazine-1-carboxylate (100 mg, 0.50 mmol, 1 eq), sodium *tert*-butoxide (72 mg, 0.75 mmol, 1.5 eq), rac-BINAP (19 mg, 0.03 mmol, 0.06 eq) and palladium diacetate ( 4.45 mg, 0.02 mmol, 0.04 eq) were reacted together according to general procedure A. This yielded the product (121 mg, 0.39 mmol, 78%).
Step B: tert-Butyl 4-(3-chlorophenyl)-2-methylpiperazine-1-carboxylate (104mg, 0.34 mmol, 1 eq) was used in general procedure B. This yield the product (66 mg, 0.31 mmol, 93%). ¹H NMR (400 MHz, Chloroform-d) δ 7.15 (t, J = 8.1 Hz, 1H), 6.87 (t, J = 2.2 Hz, 1H), 6.79 (ddt, J = 9.6, 7.5, 1.2 Hz, 2H), 3.50 (dq, J = 11.5, 1.5 Hz, 2H), 3.13 (ddd, J = 12.0, 3.3, 2.3 Hz, 1H), 3.07 - 2.92 (m, 2H), 2.85 - 2.69 (m, 3H), 2.41 (dd, J = 11.9, 10.2 Hz, 1H), 1.16 (d, J = 6.4 Hz, 3H). ¹³C NMR (101 MHz, Chloroform-d) δ 152.47, 134.96, 130.08, 119.33, 115.89, 114.07, 56.18, 50.59, 48.75, 45.60, 19.58.
Step C: The title compound was synthesized using 4-((2-ethoxy-2-oxoethyl)sulfinyl)-3-fluorobenzoic acid (18 mg, 0.07 mmol, 1 eq), 1-(3-chlorophenyl)-3-methylpiperazine (14 mg, 0.07 mmol, 1 eq), oxalyl chloride (9 mg, 0.07 mmol, 1.1 eq) and DIPEA (25 mg, 0.20 mmol, 3 eq) according to general procedure G. This yielded the product (15 mg, 0.03 mmol, 49%). ¹H NMR (850 MHz, Chloroform-d) δ 8.09 - 7.88 (m, 1H), 7.56 - 7.38 (m, 1H), 7.22 (m, 2H), 7.02 - 6.75 (m, 3H), 4.79 (m, 1H), 4.36 - 4.18 (m, 2H), 3.97 (d, J = 13.8 Hz, 1H), 3.81 (d, J = 13.9 Hz, 1H), 3.51 (m, 3H), 3.13 - 2.67 (m, 3H), 1.45 (m, 3H), 1.27 (t, J = 7.0 Hz, 3H). ¹³C NMR (214 MHz, Chloroform-d) δ 167.75, 164.30, 157.69 (d, J = 250.3 Hz), 152.28, 141.31, 135.19, 131.92 (d, J = 16.9 Hz), 130.38, 127.07, 123.74, 120.68, 116.95, 114.98, 114.77, 62.54, 58.99, 54.51, 49.37, 42.94, 37.36, 16.45, 14.19.

### Example 45: (±)Ethyl 2-((4-(4-(3-chlorophenyl)-cis-2,3-dimethylpiperazine-1-carbonyl)-2-fluorophenyl)sulfinyl)acetate

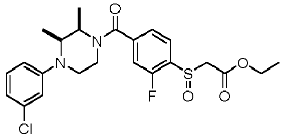

Step A: A solution of ethylenediamine (6.6 mL, 100 mmol) in 250 mL Et2O was cooled to 0°C using an ice bath. A solution of 2,3-butanedione (8.8 mL, 100 mmol) in 250 mL Et2O was added dropwise and the resulting suspension was allowed to stir for 16h. The resulting clear liquid was dried using potassium hydroxide for 30 min. After filtration, the mixture was concentrated, and the residue was purified by short-neck distillation which yielded the product (9.36 g; 85 mmol; 85%). ¹H NMR (400 MHz, CDCl3) δ 3.36 (s, 4H), 2.15 (s, 6H). ¹³C NMR (101 MHz, CDCl3) δ 159.5, 44.9, 23.4.
Step B: A solution of 5,6-dimethyl-2,3-dihydropyrazine (3.49 g, 31.7 mmol, 1 eq) in 100 ml EtOH was degassed for 30 min by bubbling argon through the solution. Solid 10% palladium loaded on carbon (3.2 g, 30.1 mmol) was added under constant bubbling. The solution was flushed three times with hydrogen after which the pressure was increased to 40 bar. The reaction mixture was stirred for 72 h at 40 bar. The suspension was filtered over Celite and rinsed three times with EtOH (3x 50 mL). After concentrating the residue was purified by column chromatography (Et₂O:MeOH:NH₄OH, 10:4:1) to obtain the product (197 mg, 1.73 mmol, 5%). ¹H NMR (500 MHz, CDCl3) δ 5.00 (bs, 2H), 3.29-3.21 (m, 2H), 3.09-2.94 (m, 4H), 1.18 (d, J = 6.7 Hz, 6H). ¹³C NMR (126 MHz, CDCl3) δ 51.5, 40.9, 13.7.
Step C: To a solution of 1-bromo-3-chlorobenzene (335 mg, 1.75 mmol, 1 eq) in 2ml 1,4-dioxane was added cis-2,3-dimethylpiperazine (200 mg, 1.75 mmol, 1eq) and KHMDS (524mg, 2.63mmol, 1.5eq). Then the reaction mixture was stirred for 2h at room temperature. The reaction progress was monitored by TLC. Once completed, the mixture was diluted with DCM, washed with water and dried over anhydrous magnesium sulfate. After filtration, the filtrate was concentrated under reduced pressure. The residue was purified by silica gel column chromatography (MeOH/DCM, 1%→5%) to afford the product (43 mg, 0.12 mmol, 11%). ¹H NMR (400 MHz, Chloroform-d) δ 7.14 (t, J = 8.1 Hz, 1H), 6.81 (t, J = 2.2 Hz, 1H), 6.74 (tdd, J = 8.0, 2.2, 1.0 Hz, 2H), 3.76 (m, 1H), 3.26 - 2.59 (m, 5H), 1.11 (d, J = 6.7 Hz, 3H), 0.97 (d, J = 6.7 Hz, 3H). ¹³C NMR (101 MHz, Chloroform-d) δ 151.40, 135.15, 130.16, 118.36, 115.43, 113.54, 54.95, 54.04, 46.07, 41.26, 19.03, 6.51.
Step D: The title compound was synthesized using 4-((2-ethoxy-2-oxoethyl)sulfinyl)-3-fluorobenzoic acid (30 mg, 0.11 mmol, 1 eq), *cis*-1-(3-chlorophenyl)-2,3-dimethylpiperazine (25 mg, 0.11 mmol, 1 eq), oxalyl chloride (15 mg, 0.12 mmol, 1.1 eq) and DIPEA (42 mg, 0.33 mmol, 3 eq) according to general procedure G. This yielded the product (12 mg, 0.03 mmol, 23%). 1H NMR (400 MHz, Chloroform-d) δ 7.95 (t, J = 7.2 Hz, 1H), 7.45 (d, J = 7.8 Hz, 1H), 7.32 - 7.14 (m, 4H), 7.10 (d, J = 7.9 Hz, 1H), 4.50 - 2.90 (m, 10H), 1.40 (dd, J = 6.6, 1.5 Hz, 3H), 1.26 (t, J = 7.1 Hz, 3H), 0.88 (d, J = 6.3 Hz, 3H). HRMS: Calculated for [C₂₂H₂₄ClFN₂O₄S + H]⁺ = 481.13586, found = 481.13566.

### Example 46: (±)Ethyl 2-((4-(4-(3-chlorophenyl)-trans-2,3-dimethylpiperazine-1-carbonyl)-2-fluorophenyl)sulfinyl)acetate

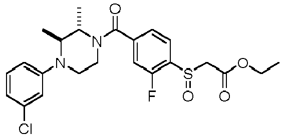

Step A: A solution of ethylenediamine (6.6 mL, 100 mmol) in 250 mL Et2O was cooled to 0°C using an ice bath. A solution of 2,3-butanedione (8.8 mL, 100 mmol) in 250 mL Et2O was added dropwise and the resulting suspension was allowed to stir for 16h. The resulting clear liquid was dried using potassium hydroxide for 30 min. After filtration, the mixture was concentrated, and the residue was purified by short-neck distillation which yielded the product (9.36 g; 85 mmol; 85%). ¹H NMR (400 MHz, CDCl3) δ 3.36 (s, 4H), 2.15 (s, 6H). ¹³C NMR (101 MHz, CDCl3) δ 159.5, 44.9, 23.4.
Step B: To a solution of 5,6-dimethyl-2,3-dihydropyrazine (9.36 g, 85 mmol, 1 eq) in 300ml absolute ethanol was added sodium metal (23 g, 1 mol, 11.8 eq) portion wise over six hours, after which the solution was refluxed for an additional 16h. The slurry was neutralized by addition of acetic acid (50 mL) at 0°C. The suspension was diluted with DCM, after stirring for 30 mins the precipitated sodium acetate was filtered off. The filtrate was concentrated under reduced pressure and the residue was purified by silica gel column chromatography (Et₂O:MeOH:NH₄OH, 10:4:1) to afford the product (3.71 g, 32.5 mmol, 38%). ¹H NMR (500 MHz, CDCl₃) δ 3.90 (bs, 1H), 2.98 (m, 4H), 2.53 (m, 2H), 1.12-1.09 (m, 6H). ¹³C NMR (126 MHz, CDCl₃) δ 57.2, 45.8, 18.5.
Step C: To a of (±) *trans*-2,3-dimethylpiperazine (0.70 g, 6.1 mmol, 1 eq) in 17 ml anhydrous dioxane was added 1-bromo-2-chlorobenzene (0.60 ml, 6.1 mmol, 1 eq) and 1M KHMDS solution (6.1 ml, 6.1 mmol, 1 eq). The reaction mixture was stirred at RT for 2h. The reaction progress was monitored by TLC. Once completed, the mixture was diluted with DCM, washed with water, extracted with DCM, dried over anhydrous magnesium sulfate, filtered and concentrated. The crude product was purified using column chromatography (1% -> 10% MeOH in DCM with 1% TEA) to yield the product (0.51 mg, 2.0 mmol, 32%). ¹H NMR (CDCl₃, 400 MHz): δ 7.20 (t, *J* = 8.1 Hz, 1H), 6.97 (t, *j* = 2.2 Hz, 1H), 6.89 (dddd, *J* = 18.0, 8.3, 2.1, 0.9 Hz, 2H), 3.24 - 2.82 (m, 6H), 1.31 (d, *J* = 6.6 Hz, 3H), 1.05 (d, *J* = 6.4 Hz, 3H). ¹³C NMR (400 MHz, CDCl₃) δ 153.01, 134.81, 130.07, 121.22, 120.23, 118.30, 57.84, 54.59, 48.88, 42.59, 19.04, 14.91.
Step D: The title compound was synthesized using 4-((2-ethoxy-2-oxoethyl)sulfinyl)-3-fluorobenzoic acid (30 mg, 0.11 mmol, 1 eq), (±) *trans*-1-(3-chlorophenyl)-2,3-dimethylpiperazine (25 mg, 0.11 mmol, 1 eq), oxalyl chloride (15 mg, 0.12 mmol, 1.1 eq) and DIPEA (42 mg, 0.33 mmol, 3 eq) according to general procedure G. This yielded the product (18 mg, 0.04 mmol, 34%). ¹H NMR (400 MHz, Chloroform-d) δ 8.00 - 7.90 (m, 1H), 7.44 (q, J = 7.5 Hz, 1H), 7.26 - 7.12 (m, 2H), 6.90 - 6.61 (m, 3H), 4.88 - 4.55 (m, 1H), 4.23 (q, J = 7.1 Hz, 2H), 3.97 (d, J = 13.8 Hz, 1H), 3.81 (d, J = 13.9 Hz, 1H), 3.71 - 3.45 (m, 2H), 3.38 - 3.05 (m, 3H), 1.56 - 1.41 (m, 3H), 1.27 (t, J = 7.1 Hz, 3H), 1.17 - 0.96 (m, 3H). HRMS: Calculated for [C₂₂H₂₄ClFN₂O₄S + H]⁺ = 481.13586, found = 481.13577.

### Example 47: Ethyl 2-((4-(4-(3-chlorophenyl)-3,3-dimethylpiperazine-1-carbonyl)-2-fluorophenyl)sulfinyl)acetate

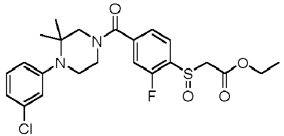

Step A: To a of tert-butyl 3,3-dimethylpiperazine-1-carboxylate (0.20 g, 0.93 mmol, 1 eq) in 3 ml anhydrous dioxane was added 1-bromo-2-chlorobenzene (179 mg, 0.93 mmol, 1 eq) and 1M KHMDS solution (1.1 ml, 1.1 mmol, 1.2 eq). The reaction mixture was stirred at RT for 2h. The reaction progress was monitored by TLC. Once completed, the mixture was diluted with DCM, washed with water, extracted with DCM, dried over anhydrous magnesium sulfate, filtered and concentrated. The crude product was purified using column chromatography (Diethyl ether/Pentane, 5%-15%) to yield the product (86 mg, 0.27 mmol, 28%). ¹H NMR (400 MHz, Chloroform-d) δ 7.18 (t, J = 8.1 Hz, 1H), 7.09 (d, J = 7.5 Hz, 2H), 6.98 (d, J = 8.0 Hz, 1H), 3.55 (t, J = 7.0 Hz, 2H), 3.32 (s, 2H), 3.05 (t, J = 5.2 Hz, 2H), 1.48 (s, 9H), 1.03 (s, 6H). ¹³C NMR (101 MHz, Chloroform-d) δ 154.88, 150.41, 133.71, 129.06, 127.58, 125.80, 124.78, 79.69, 56.45, 55.07, 46.91, 43.94, 28.49, 21.75.
Step B: *tert*-butyl 4-(3-chlorophenyl)-3,3-dimethylpiperazine-1-carboxylate (86 mg, 0.27 mmol, 1 eq) was used according to general procedure B. This yield the product ( 57 mg, 0.25 mmol, 96 %). ¹H NMR (400 MHz, Chloroform-*d*) δ 7.18 (ddd, *J* = 8.2, 7.2, 1.2 Hz, 1H), 7.09 (dd, *J* = 7.2, 1.3 Hz, 2H), 7.02 - 6.95 (m, 1H), 3.99 (s, 1H), 3.19 - 2.96 (m, 4H), 2.81 (s, 2H), 1.07 (s, 6H). ¹³C NMR (101 MHz, Chloroform-*d*) δ 150.68, 133.70, 129.04, 127.54, 125.91, 124.71, 58.40, 54.59, 47.59, 46.72, 22.22.
Step C: To a solution of 1-bromo-3-chlorobenzene (335 mg, 1.75 mmol, 1 eq) and 2,2-dimethylpiperazine (200 mg, 1.75 mmol, 1 eq) was added 1M KHMDS solution ( 2.1 ml, 2.1 mmol, 1.2 eq). Then the reaction mixture was stirred at room temperature for 2h. The reaction progress was monitored by TLC. Once completed, the mixture was diluted with DCM, washed with water, extracted with DCM, dried over anhydrous magnesium sulfate, filtered and concentrated. The crude product was purified using column chromatography (MeOH/DCM, 1%-10%) to yield the product (96 mg, 0.27 mmol, 28%). 1H NMR (500 MHz, Chloroform-d) δ 7.17 - 7.10 (m, 1H), 6.84 (t, J = 2.2 Hz, 1H), 6.76 (dddd, J = 16.4, 8.4, 2.2, 0.9 Hz, 2H), 3.11 - 2.95 (m, 4H), 2.89 (s, 2H), 1.22 (s, 6H). 13C NMR (126 MHz, Chloroform-d) δ 153.20, 134.98, 130.09, 119.12, 116.04, 114.21, 60.82, 49.41, 48.19, 41.20, 26.29.
Step D: The title compound was synthesized using 4-((2-ethoxy-2-oxoethyl)sulfinyl)-3-fluorobenzoic acid (30 mg, 0.11 mmol, 1 eq), 1-(3-chlorophenyl)-2,2-dimethylpiperazine (25 mg, 0.11 mmol, 1 eq), oxalyl chloride (15 mg, 0.12 mmol, 1.1 eq ) and DIPEA (42 mg, 0.33 mmol, 3 eq) according to general procedure G. This yielded the product (26 mg, 0.05 mmol, 49%). 1H NMR (400 MHz, Chloroform-d) δ 7.96 (t, J = 7.2 Hz, 1H), 7.50 (s, 1H), 7.36 (d, J = 6.5 Hz, 2H), 7.29 (m, 3H), 4.27 - 3.30 (m, 10H), 1.27 (m, 6H), 1.12 (bs, 3H). HRMS: Calculated for [C₂₂H₂₄ClFN₂O₄S + H]⁺ = 481.13586, found = 481.13580.

### Example 48: Ethyl 2-((4-(4-(3-chlorophenyl)-2,2-dimethylpiperazine-1-carbonyl)-2-fluorophenyl)sulfinyl)acetate

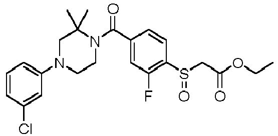

The title compound was synthesized using 4-((2-ethoxy-2-oxoethyl)sulfinyl)-3-fluorobenzoic acid (30 mg, 0.11 mmol, 1 eq), 1-(3-chlorophenyl)-3,3-dimethylpiperazine ( 25 mg, 0.11 mmol, 1 eq), oxalyl chloride (15 mg, 0.12 mmol, 1.1 eq) and DIPEA (42 mg, 0.33 mmol, 3 eq) according to general procedure G. This yielded the product (28 mg, 0.06 mmol, 53%). 1H NMR (400 MHz, Chloroform-d) δ 7.95 (t, J = 7.0 Hz, 1H), 7.47 (s, 1H), 7.32 - 7.05 (m, 5H), 4.22 (q, J = 7.1 Hz, 2H), 4.07 - 3.15 (m, 8H), 1.26 (t, J = 7.1 Hz, 3H), 1.21 (bs, 3H), 1.03 (bs, 3H). HRMS: Calculated for [C₂₂H₂₄ClFN₂O₄S + H]⁺ = 481.13586, found = 481.13580.

### Example 49: (±) Ethyl 2-((2-chloro-4-(4-(3-chlorophenyl)-trans-2,3-dimethylpiperazine-1-carbonyl)phenyl)sulfinyl)acetate

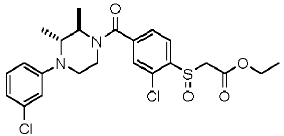

Step A: 4-fluoro-3-chlorobenzoic acid (1000 mg, 5.73 mmol, 1 eq), Boc₂O (3.13 g, 14.3 mmol, 2.5 eq) and DMAP (210 mg, 1.72 mmol, 0.30 eq) were used according to general procedure C. This yield the product (1.16 g, 5.04 mmol, 88%). ¹H NMR (500 MHz, CDCl₃) δ 8.03 (dd, 3,4J = 7.2, 2.2 Hz, 1H), 7.88 (ddd, J = 8.6, 4.7, 2.2 Hz, 1H), 7.17 (t, 3J = 8.6 Hz, 1H), 1.59 (s, 9H). ¹³C NMR (126 MHz, CDCl₃) δ 163.9, 160.9 (d, J = 255.2 Hz), 132.3, 129.9 (d, J = 8.4 Hz), 129.3 (d, J = 3.6 Hz), 121.3 (d, J = 18.2 Hz), 116.5 (d, J = 21.6 Hz), 82.1, 28.3.
Step B: ethyl 2-mercaptoacetate (1.21 g, 10.1 mmol, 2 eq) and *tert*-butyl 3-chloro-4-fluorobenzoate (1.16 g, 5.04 mmol, 1eq) were used according to general procedure D. This yielded the product (1.62 g, 4.60 mmol, 91%). ¹H NMR (400 MHz, CDCl₃) δ 7.94 (d, 4J = 1.8 Hz, 1H), 7.83 (dd, 3,4J = 8.3, 1.8 Hz, 1H), 7.32 (d, 3J = 8.3 Hz, 1H), 4.20 (q, 3J = 7.1 Hz, 2H), 3.74 (s, 2H), 1.58 (s, 9H), 1.26 (t, 3J = 7.1 Hz, 3H). ¹³C NMR (101 MHz, CDCl₃) δ 168.8, 164.4, 140.6, 132.2, 130.6, 130.5, 128.2, 126.6, 81.8, 62.2, 34.5, 28.3, 14.2.
Step C: tert-butyl 3-chloro-4-((2-ethoxy-2-oxoethyl)thio)benzoate (140 mg, 0.42 mmol, 1.00 eq) was used according to general procedure E. This yielded the product (147 mg, 0.42 mmol, quant.). ¹H NMR (400 MHz, CDCl₃) δ 8.12 (d, 3J = 8.1 Hz, 1H), 8.06-7.92 (m, 2H), 4.29 - 4.18 (m, 2H), 4.03 (d, J = 13.1 Hz, 1H), 3.69 (d, J = 13.8 Hz, 1H), 1.61 (s, 9H), 1.26 (t, 3J = 7.0 Hz, 3H). ¹³C NMR (126 MHz, CDCl₃) δ = 164.5, 163.6, 145.2, 136.5, 130.8, 130.1, 128.8, 126.5, 82.7, 62.4, 58.1, 28.2, 14.2.
Step D: tert-butyl 3-chloro-4-((2-ethoxy-2-oxoethyl)sulfinyl)benzoate (1.39 g, 4.00 mmol, 1 eq) was used according to general procedure F. This yielded the product (1.04 g, 3.59 mmol, 90%). ¹H NMR (500 MHz, CDCl₃) δ 8.17 (dd, 3,4J = 8.1, 1.5 Hz, 1H), 8.07 (d, 4J = 1.5, 1H), 7.99 (d, 3J = 8.1, 1H), 4.26 - 4.14 (m, 2H), 4.04 (d, J = 14.0 Hz, 1H), 3.72 (d, J = 14.0 Hz, 1H), 1.23 (t, 3J = 7.1 Hz, 3H). ¹³C NMR (126 MHz, CDCl₃) δ 166.5, 164.5, 145.4, 135.0, 131.2, 130.2, 129.3, 126.7, 62.5, 58.0, 14.1.
Step E: The title compound was synthesized using (±) *trans*-1-(3-Chlorophenyl)-2,3-dimethylpiperazine (23.2 mg, 0.10 µmol, 1 eq), 3-chloro-4-((2-ethoxy-2-oxoethyl)sulfinyl)benzoic acid (30 mg, 0.10 mmol, 1 eq), HATU (39.2 mg, 0.10 mmol, 1.00 eq) and DIPEA (31 mg, 0.30 mmol, 3 eq) according to general procedure H. This yield the product (38.3 mg, 77.3 µmol, 75 %). ¹H NMR (500 MHz, CDCl₃) δ 8.01 (d, *J* = 8.0 Hz, 1H), 7.54 (dd, *J* = 8.0, 1.6 Hz, 1H), 7.51 - 7.42 (m, 1H), 7.16 (t, *J* = 8.0 Hz, 1H), 6.83 - 6.78 (m, 2H), 6.70 (d, *J* = 8.4 Hz, 1H), 4.80 (t, J = 6.7 Hz, 1H), 4.62 (s, 1H), 4.29 - 4.17 (m, 2H), 4.04 (dd, 3,4J = 14.1, 1.7 Hz, 1H), 3.87 (d, J = 7.0 Hz, 1H), 3.69 (dd, 3,4J = 14.0, 1.2 Hz, 1H), 3.67 - 3.60 (m, 1H), 3.57 - 3.49 (m, 1H), 3.37 - 3.06 (m, 3H), 1.52 - 1.44 (m, 3H), 1.27 (t, J = 7.1 Hz, 3H), 1.16 - 097 (m, 3H). ¹³C NMR (126 MHz, CDCl₃) δ 168.8, 168.3, 164.5, 151.3, 142.4, 140.5, 135.3, 130.8, 130.4, 128.4, 127.7, 127.1, 126.3, 125.8, 119.5, 116.3, 114.3, 62.5, 58.4, 56.2, 55.6, 49.8, 42.4, 41.3, 40.5, 36.6, 17.8, 16.8, 14.2, 12.8, 12.6. HRMS: Calculated for [C₂₃H₂₇Cl₂N₂O₄S + H]⁺ = 497.1063, found = 497.1065.

### Example 50: (±) Ethyl 2-((2-bromo-4-(4-(3-chlorophenyl)-trans-2,3-dimethylpiperazine-1-carbonyl)phenyl)sulfinyl)acetate

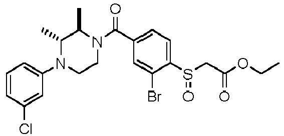

Step A: 3-bromo-4-fluorobenzoic acid (212 mg, 1 mmol, 1 eq) was used according to general procedure C. This yield the product (228 mg, 0.83 mmol, 83%). ¹H NMR (300 MHz, CDCl₃) δ 8.17 (dd, J = 6.7, 2.0 Hz, 1H), 8.04 - 7.81 (m, 1H), 7.13 (t, J = 8.4 Hz, 1H), 1.58 (s, 9H). ¹³C NMR (75 MHz, Chloroform-d) δ 163.10, 161.38 (d, J = 247.8 Hz), 134.84, 130.37 (d, J = 8.4 Hz), 129.38 (d, J = 3.4 Hz), 115.93 (d, J = 22.8 Hz), 108.70 (d, J = 21.6 Hz), 81.43, 27.86.
Step B: ethyl 2-mercaptoacetate (36 mg, 0.30 mmol, 1.1 eq) and *tert*-butyl 3-bromo-4-fluorobenzoate (100 mg, 0.27 mmol, 1eq) was used according to general procedure D. This yielded the product (34 mg, 0.09 mmol, 34%). 1H NMR (300 MHz, CDCl₃) δ 8.13 (d, J = 1.8 Hz, 1H), 7.90 (dt, J = 8.3, 4.3 Hz, 1H), 7.37 - 7.20 (m, 1H), 4.31 - 4.10 (m, 2H), 3.76 (s, 2H), 1.61 (d, J = 7.1 Hz, 9H), 1.28 (t, J = 7.1 Hz, 3H).13C NMR (75 MHz, CDCl3) δ 168.58, 164.09, 142.52, 133.63, 130.40, 128.67, 126.12, 121.48, 81.58, 62.02, 34.90, 28.14, 13.99.
Step C: tert-butyl 3-bromo-4-((2-ethoxy-2-oxoethyl)thio)benzoate (110 mg, 0.29 mmol, 1.00 eq) was used according to general procedure E. This yielded the product (92 mg, 0.23 mmol, 81%.). 1H NMR (300 MHz, CDCl3) δ 8.16 (dd, J = 7.4, 1.2 Hz, 2H), 7.98 (d, J = 8.6 Hz, 1H), 4.22 (m, 2H), 4.07 (d, J = 13.8 Hz, 1H), 3.69 (d, J = 13.8 Hz, 1H), 1.61 (s, 9H), 1.27 (t, J = 7.1 Hz, 3H). 13C NMR (75 MHz, CDCl3) δ 164.45, 163.31, 146.81, 136.39, 133.80, 129.24, 126.75, 118.32, 82.58, 62.32, 58.24, 28.07, 14.11.
Step D: tert-butyl 3-bromo-4-((2-ethoxy-2-oxoethyl)sulfinyl)benzoate (167 mg, 0.43 mmol, 1 eq) was used according to general procedure F. This yielded the product ( 60 mg, 0.18 mmol, 43 %). ¹H NMR (300 MHz, MeOD) δ 8.32 - 8.12 (m, 2H), 7.96 (d, J = 8.5 Hz, 1H), 4.20 (ddd, J = 14.0, 9.5, 8.4 Hz, 3H), 3.86 (d, J = 14.3 Hz, 1H), 1.31 - 1.10 (m, 3H). ¹³C NMR (75 MHz, MeOD) δ 165.64, 164.61, 146.54, 135.50, 133.81, 129.35, 126.54, 118.38, 61.85, 57.72, 12.98.
Step E: The title compound was synthesized using 3-bromo-4-((2-ethoxy-2-oxoethyl)sulfinyl)benzoic acid (50.0 mg, 0.150 mmol, 1eq), (±)-1-(3-chlorophenyl)-trans-2,3-dimethylpiperazine (33.6 mg, 0.150 mmol, 1 eq), HATU (85.0 mg, 0.23 mmol, 1.5 eq ) and DIPEA (58.0 mg, 0.450 mmol) according to general procedure H. This yielded the product (68.4 mg, 0.126 mmol, 85%). ¹H NMR (400 MHz, CDCl3) δ 8.02 (d, J = 8.0 Hz, 1H), 7.67 (d, J = 1.2 Hz, 1H), 7.62 (dd, J = 8.0, 1.4 Hz, 1H), 7.19 (t, J = 8.2 Hz, 1H), 6.83 (dd, J = 8.1, 1.2 Hz, 2H), 6.73 (d, J = 9.0 Hz, 1H), 4.93 - 4.53 (m, 1H), 4.26 (qd, J = 7.1, 1.4 Hz, 2H), 4.10 (dd, J = 14.1, 0.8 Hz, 1H), 3.97 - 3.02 (m, 6H), 1.51 (s, 3H), 1.30 (t, J = 7.1 Hz, 3H), 1.18 - 0.99 (m, 3H). ¹³C NMR (101 MHz, CDCl3) δ 164.43, 158.30, 151.18, 143.91, 140.39, 135.19, 131.26, 130.29, 127.30, 126.73, 119.48, 119.26, 116.19, 114.20, 62.50, 58.46, 56.08, 49.83, 40.37, 36.62, 17.73, 14.10, 12.47.

### Example 51: (±) 2-((2-Chloro-4-(4-(3-chlorophenyl)-trans-2,3-dimethylpiperazine-1-carbonyl)phenyl)sulfinyl)acetic acid

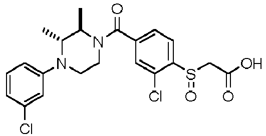

To a solution of (±) ethyl 2-((2-chloro-4-(4-(3-chlorophenyl)-trans-2,3-dimethylpiperazine-1-carbonyl)phenyl)sulfinyl)acetate (180 mg, 0.36 mmol, 1 eq) in 2 ml MeOH was added 2ml TEA and 2ml water. The reaction mixture was stirred at room temperature overnight. The reaction progress was monitored by TLC. Once completed, the mixture was acidified with 3M HCl solution to pH 2, extracted with EtOAc, dried over anhydrous magnesium sulfate. After filtration, the filtrate was concentrated under reduced pressure. The residue was purified by silica gel column chromatography (MeOH/DCM, 1-5%) to afford the product (0.16 g, 0.35 mmol, 97%). 1H NMR (500 MHz, CDCl3) δ 8.03 (d, J = 8.1 Hz, 1H), 7.63 - 7.43 (m, 2H), 7.17 (t, J = 8.3 Hz, 1H), 6.85 - 6.77 (m, 2H), 6.73 - 6.69 (m, 1H), 4.85 - 4.59 (m, 1H), 4.08 (dd, J = 14.2, 3.0 Hz, 1H), 3.92 - 3.49 (m, 3H), 3.36 - 3.11 (m, 2H), 1.54 - 1.44 (m, 3H), 1.15 - 0.98 (m, 3H). 13C NMR (126 MHz, CDCl3) δ 176.17, 169.06, 151.35, 141.69, 140.54, 135.28, 130.87, 130.30, 128.51, 127.15, 126.01, 119.52, 116.25, 114.25, 57.76, 56.16, 49.88, 40.47, 36.69, 17.81, 12.84 (carbon spectrum shows a mixture of rotamers). HRMS: Calculated for [C₂₁H₂₂Cl₂N₂O₄S + H]+ = 496.0750, found = 496.0746.

### Example 52: (±) Ethyl 2-((2-chloro-4-(4-(3-chlorophenyl)-trans-2,3-dimethylpiperazine-1-carbonyl)phenyl)thio)acetate

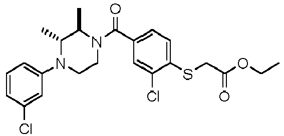

Step A: tert-butyl 3-chloro-4-((2-ethoxy-2-oxoethyl)sulfinyl)benzoate (62 mg, 0.19 mmol, 1 eq) was used according to general procedure F. This yielded the product (51 mg, 0.19 mmol, 92 %). ¹H NMR (400 MHz, CDCl3) δ 8.07 (s, 1H), 7.95 (d, J = 8.3 Hz, 1H), 7.37 (d, J = 8.2 Hz, 1H), 4.23 (q, J = 7.2 Hz, 2H), 3.78 (s, 2H), 1.28 (t, J = 7.1 Hz, 3H). ¹³C NMR (400 MHz, CDCl3) δ 170.34, 168.37, 142.67, 130.76, 128.58, 127.15, 125.87, 61.98, 34.03, 13.91.
Step B: The title compound was synthesized using 3-chloro-4-((2-ethoxy-2-oxoethyl)thio)benzoic acid (33 mg, 0.12 mmol, 1 eq), (±)1-(3-chlorophenyl)-trans-2,3-dimethylpiperazine (27 mg, 0.12 mmol, 1 eq), HATU (68 mg, 0.18 mmol, 1.5 eq) and DIPEA (46 mg, 0.36 mmol, 3 eq) according to general procedure H. This yielded the product (11 mg, 0.02 mmol, 19 %). 1H NMR (400 MHz, CDCl3) δ 7.48 - 7.36 (m, 2H), 7.34 - 7.27 (m, 1H), 7.17 (t, J = 8.3 Hz, 1H), 6.80 (d, J = 6.1 Hz, 2H), 6.71 (d, J = 8.4 Hz, 1H), 4.84 - 4.53 (m, 1H), 4.21 (q, J = 7.1 Hz, 2H), 3.73 (s, 2H), 3.69 - 3.01 (m, 5H), 1.51 - 1.41 (m, 3H), 1.27 (t, J = 7.2 Hz, 3H), 1.06 (dd, J = 46.6, 6.5 Hz, 3H). 13C NMR (101 MHz, CDCl3) δ 169.52, 168.85, 151.39, 137.10, 135.16, 134.77, 133.25,130.25, 128.12, 128.87, 125.47, 119.18, 116.02, 114.05, 61.99, 56.06, 49.45, 40.43, 36.42, 34.69, 17.69, 14.13, 12.49 (carbon spectrum shows a mixture of rotamers). HRMS: Calculated for [C₂₃H₂₆Cl₂N₂O₃S + H]⁺ = 483.1084, found = 483.1079.

### Example 53: (±) Ethyl 2-((2-chloro-4-(4-(3-chlorophenyl)-trans-2,3-dimethylpiperazine-1-carbonyl)phenyl)sulfonyl)acetate

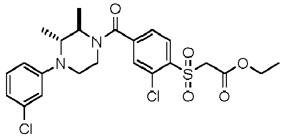

Step A: To a cooled (0°C) solution of tert-butyl-3-chloro-4-((2-ethyoxy-2oxoethyl)sulfinyl)benzoate (70 mg, 0.21 mmol, 1 eq.) in 7 ml MeOH was added Oxone ( 390 mg, 0.64 mmol, 3 eq.) / 3.5 ml H₂O solution. The reaction mixture was stirred at RT overnight. The reaction progress was monitored by TLC. Once completed, the reaction mixture was extracted with DCM, dried over anhydrous magnesium sulfate. After filtration, the filtrate was concentrated under reduced pressure. The residue was purified by silica gel column chromatography (EtOAc/Pentane, 0-15%) to afford the product (65 mg, 0.18 mmol, 85%). 1H NMR (400 MHz, CDCl3) δ 8.18 (d, J = 8.2 Hz, 1H), 8.14 (d, J = 1.5 Hz, 1H), 8.05 (dd, J = 8.3, 1.6 Hz, 1H), 4.47 (s, 2H), 4.12 (q, J = 7.1 Hz, 2H), 1.61 (s, 9H), 1.17 (t, J = 7.2 Hz, 3H). 13C NMR (400 MHz, CDCl3) δ 162.97, 162.11, 139.35, 138.42, 132.84, 132.72, 132.25, 128.11, 83.23, 62.70, 58.74, 28.17, 13.96.
Step B: *tert*-butyl 3-chloro-4-((2-ethoxy-2-oxoethyl)sulfonyl)benzoate (65 mg, 0.18 mmol, 1 eq) was used according to general procedure F. This yielded the product ( 50.2 mg, 0.16 mmol, 91%). ¹H NMR (400 MHz, MeOD) δ 8.28 - 8.05 (m, 3H), 4.61 (s, 2H), 4.07 (q, J = 7.1 Hz, 2H), 1.08 (t, J = 7.1 Hz, 3H). ¹³C NMR (101 MHz, MeOD) δ 165.24, 162.16, 139.79, 137.42, 132.58, 132.39, 131.98, 128.13, 61.91, 58.48, 12.67.
Step C: The title compound was synthesized using tert-butyl 3-chloro-4-((2-ethoxy-2-oxoethyl)sulfonyl)benzoate (27 mg, 0.09 mmol, 1 eq), (±)1-(3-chlorophenyl)-trans-2,3-dimethylpiperazine (20 mg, 0.09 mmol, 1 eq), HATU (51 mg, 0.13 mmol, 1.5 eq ) and DIPEA (35 mg, 0.27 mmol, 3 eq) according to general procedure H. This yielded the product (8 mg, 0.02 mmol, 18 %). ¹H NMR (500 MHz, CDCl3) δ 8.21 (d, J = 8.1 Hz, 1H), 7.67 - 7.42 (m, 2H), 7.18 (t, J = 8.0 Hz, 1H), 6.86 - 6.78 (m, 2H), 6.72 (d, J = 8.5 Hz, 1H), 4.47 (s, 2H), 4.15 (q, J = 7.1 Hz, 2H), 3.95 - 3.00 (m, 6H), 1.49 (dd, J = 16.0, 6.7 Hz, 3H), 1.19 (td, J = 7.2, 3.0 Hz, 3H), 1.13 (d, J = 6.6 Hz, 1H), 1.02 (d, J = 6.5 Hz, 1H).

### Example 54: (±) Ethyl 2-((2-chloro-4-((4-(3-chlorophenyl)-trans-2,3-dimethylpiperazin-1-yl)methyl)phenyl)sulfinyl)acetate

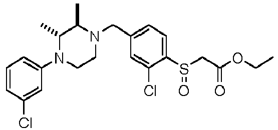

Step A: To a solution of 3-chloro-4-fluorobenzaldehyde (300 mg, 1.89 mmol, 1eq) in 5ml DMF was added K₂CO₃ (523 mg, 3.78 mmol, 2 eq) and ethyl 2-mercaptoacetate ( 227 mg, 1.89 mmol, 2 eq) and stirred at rt overnight. The reaction progress was monitored by TLC. Once completed, the reaction mixture was diluted with diethyl ether, washed with water, dried over anhydrous magnesium sulfate. After filtration, the filtrate was concentrated under reduced pressure. The residue was purified by silica gel column chromatography (Et₂O /Pentane, 20-35%) to afford the product (446 mg, 1.72 mmol, 91%,). ¹H NMR (400 MHz, CDCl3) δ 9.89 (s, 1H), 7.84 (d, 4J = 1.8 Hz, 1H), 7.72 (dd, 3,4J = 8.3, 1.8 Hz, 1H), 7.42 (d, 3J = 8.3 Hz, 1H), 4.22 (q, 3J = 7.1 Hz, 2H), 3.78 (s, 2H), 1.27 (t, 3J = 7.1 Hz, 3H). ¹³C NMR (126 MHz, CDCl3) δ 190.1, 168.5, 143.9, 134.6, 132.6, 130.1, 128.4, 126.3, 62.3, 34.2, 14.2.
Step B: To a solution of ethyl ethyl-2-((2-chloro-4-formylphenyl)thio)acetate (31.8 mg, 0.12 mmol, 1.2 eq) in 1,2-dichloroethane (1.0 ml) was added (±)1-(3-chlorophenyl)-trans-2,3-dimethylpiperazine (23.0 mg, 0.10 mmol, 1 eq) and the reaction mixture was stirred at rt for 30min. Then sodium triacetoxyborohydride (65.1 mg, 0.31 mmol, 3 eq) was added and the reaction mixture was stirred further at rt overnight. The reaction progress was monitored by TLC. Once completed, the reaction was quenched by addition of H₂O, followed by extraction with DCM. The combined organic layers were washed with brine, dried over Na₂SO₄ and concentrated under reduced pressure. Purification by flash column chromatography (EtOAc/Pentane, 20%) yielded the product (19 mg, 0.04 mmol, 40 %). ¹H NMR (400 MHz, CDCl3) δ 7.46 (s, 1H), 7.39 (d, J = 8.0 Hz, 1H), 7.30 - 7.27 (m, 1H), 7.15 (t, J = 8.0 Hz, 1H), 6.84 - 6.79 (m, 1H), 6.73 (t, J = 8.0 Hz, 1H), 4.20 (q, J = 7.2 Hz, 2H), 3.75 - 3.67 (m, 3H), 3.57 (q, J = 13.9 Hz, 2H), 3.27 - 3.09 (m, 2H), 2.91 - 2.81 (m, 1H), 2.75 (td, J = 11.4 Hz, 1H), 2.52 (d, J = 11.5 Hz, 1H), 1.27 (d, J = 7.2 Hz, 3H), 1.24 - 1.19 (m, 3H), 1.15 (d, 3J = 6.3 Hz, 3H). ¹³C NMR (101 MHz, CDCl3) δ 169.4, 152.1, 140.2, 135.1, 134.6, 132.3, 130.5, 130.1, 129.9, 127.6, 118.0, 115.5, 113.6, 61.8, 58.0, 56.7, 56.6, 44.9, 42.0, 35.6, 14.2, 13.0, 9.5.
Step C: The title compound was synthesized using (±) ethyl 2-((2-chloro-4-((4-(3-chlorophenyl)-*trans*-2,3-dimethylpiperazin-1-yl)methyl)phenyl)thio)acetate (19 mg, 0.04 mmol, 1 eq) according to general procedure E. This yielded the product (6.3 mg, 0.01 mmol, 32 %). ¹H NMR (500 MHz, CDCl3) δ 7.89 (d, 3J = 8.0 Hz, 1H), 7.55 (ddd, J = 8.0, 3.0, 1.5 Hz, 1H), 7.49 (dd, J = 3.9, 1.5 Hz, 1H), 7.14 (t, 3J = 8.0 Hz, 1H), 6.80 (t, J = 2.2 Hz, 1H), 6.72 (dddd, J = 14.5, 8.5, 2.3, 0.8 Hz, 2H), 4.26 - 4.18 (m, 2H), 4.02 (dd, J = 13.7, 2.8 Hz, 1H), 3.75 - 3.66 (m, 3H), 3.61 (d, J = 14.3 Hz, 1H), 3.24 - 3.19 (m, 1H), 3.15 (dd, J = 4.0, 1.5 Hz, 1H), 2.85 (q, J = 6.4 Hz, 1H), 2.78 (td, J = 11.5, 4.2 Hz, 1H), 2.51 (dt, J = 11.5, 1.8 Hz, 1H), 1.27 (td, J = 7.2, 1.0 Hz, 3H), 1.22 (dd, J = 6.6, 1.4 Hz, 3H), 1.17 (d, 3J = 6.5 Hz, 3H). ¹³C NMR (126 MHz, CDCl3) δ 169.4, 152.1, 145.6, 139.3, 135.2, 130.2, 130.2, 129.8, 128.2, 126.4, 118.2, 115.6, 113.7, 62.3, 58.7, 58.2, 57.0, 56.8, 45.0, 42.0, 14.3, 13.0, 9.6. HRMS: Calculated for [C₂₃H₂₈Cl₂N₂O₃S + H]⁺ = 483.1270, found = 483.1273.

### Example 55: (±) Ethyl 3-(2-chloro-4-(4-(3-chlorophenyl)-trans-2,3-dimethylpiperazine-1-carbonyl)phenyl)-3-oxopropanoate

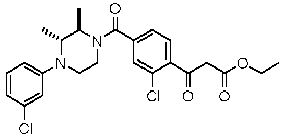

Step A: To a solution of 4-bromo-3-chlorobenzoic acid (300 mg, 1.27 mmol, 1.00 equiv.) in abs. THF (5.0 mL) at -78 C under an inert atmosphere was added Turbo-GRIGNARD (1.3 M in THF, 2.94 ml, 3.82 mmol, 3.00 equiv.). The temperature was raised to 0 C after 10 min and the reaction mixture was stirred at 0 °C for 1 h. Subsequently DMF (6.37 ml, 0.49 mmol, 5.00 equiv.) was added, the reaction mixture warmed up to room temperature and stirred further for 1.5 h. The reaction was quenched by addition of sat. aq. NH₄Cl, followed by washing with EtOAc. Then the pH of the aqueous layer was adjusted with aq. 1 N HCl and extracted with EtOAc. The combined organic layers were neutralized with sat. aq. NaHCO₃, washed with brine, dried over Na₂SO₄ and concentrated under reduced pressure. The title compound was obtained without any further purification (165 mg, 0.89 mmol, 70%). ¹H NMR (500 MHz, DMSO-d6) δ 10.38 (s, 1H), 8.07 - 8.00 (m, 2H), 8.01 - 7.95 (m, 1H). ¹³C NMR (126 MHz, DMSO-d6) δ 189.6, 165.4, 136.8, 136.1, 134.8, 131.1, 130.1, 128.3.
Step B: 3-chloro-4-formylbenzoic acid (65.0 mg, 35.0 mmol, 1.00 equiv.), (±) 1-(3-chlorophenyl)-trans-2,3-dimethylpiperazine (79.0 mg, 0.35 mmol, 1.00 equiv.), DIPEA (137 mg, 1.06 mmol, 3.00 equiv.) and HATU (161 mg, 0.42 mmol, 1.20 equiv.) were used according to general procedure H. This yielded the product (69.0 mg, 0.18 mmol, 50%). ¹H NMR (400 MHz, CDCl3) δ = 10.49 - 10.45 (m, 1H), 7.97 (d, 3J = 7.9 Hz, 1H), 7.49 (dd, J = 11.0, 1.5 Hz, 1H), 7.41 - 7.35 (m, 1H), 7.18 - 7.12 (m, 1H), 6.82 - 6.76 (m, 2H), 4.79 (qd, J = 6.9, 1.4 Hz, 1H), 4.60 (dt, J = 12.0, 2.2 Hz, 1H), 3.89 - 3.83 (m, 1H), 3.63 (dq, J = 19.7, 6.9 Hz, 1H), 3.53 - 3.46 (m, 2H), 3.33 (dt, J = 11.2, 2.7 Hz, 1H), 3.29 - 3.07 (m, 4H), 1.48 (d, J = 6.8 Hz, 1H), 1.45 (d, J = 6.8 Hz, 2H), 1.11 (d, J = 6.6 Hz, 1H), 1.00 (d, J = 6.6 Hz, 2H). ¹³C NMR (101 MHz, CDCl3) δ 188.9, 168.6, 168.2, 151.3, 142.7, 142.6, 138.4, 135.2, 132.9, 130.3, 129.9, 129.8, 129.1, 128.7, 125.6, 125.2, 119.4, 116.2, 114.2, 114.1, 60.4, 56.1, 55.4, 49.5, 42.2, 41.2, 40.4, 36.4, 17.7, 16.7, 14.2, 12.8, 12.5.
Step C: To a solution of (±) 2-Chloro-4-(4-(3-chlorophenyl)-trans-2,3-dimethylpiperazine-1-carbonyl)benzaldehyde (69.0 mg, 176 µmol, 1.00 equiv.) in DMF (1.0 ml) was added oxone (108 mg, 176 µmol, 1.00 equiv.) and the mixture was stirred at rt overnight. Procedure oxone oxidation. This yielded the product (68.0 mg, 0.15 mmol, 50%). ¹H NMR (500 MHz, CDCl3) δ 8.03 (bs, 1H, OH), 7.74 (dd, J = 8.0, 3.4 Hz, 1H), 7.39 (dd, J = 10.4, 1.5 Hz, 1H), 7.28 - 7.22 (m, 1H), 7.17 - 7.10 (m, 1H), 6.80 - 6.74 (m, 2H), 6.71 - 6.65 (m, 1H), 4.75 (q, J = 6.9 Hz, 1H), 4.61 - 4.53 (m, 1H), 3.87 - 3.79 (m, 1H), 3.55 - 2.99 (m, 3H), 1.44 (d, J = 6.8 Hz, 1H), 1.44 (m, 2H), 1.08 (d, J = 6.7 Hz, 1H), 0.95 (d, J = 6.7 Hz, 1H). ¹³C NMR (126 MHz, CDCl3) δ 171.2, 169.6, 169.1, 151.4, 138.6, 135.7, 132.7, 130.9, 130.3, 128.8, 128.4, 125.2 - 124.5, 119.3, 116.1, 114.2, 69.7, 56.1, 55.4, 49.6, 42.3 , 41.3, 40.5, 36.5, 17.7, 16.7, 14.2, 12.8, 12.5.
Step D: To a solution of (±) 2-chloro-4-(4-(3-chlorophenyl)-trans-2,3-dimethylpiperazine-1-carbonyl)benzoic acid (12.0 mg, 29.0 µmol, 1.00 equiv.) in dry THF (250 µl), carbonyl diimidazole (5.30 mg, 32.0 mmol, 1.10 eq.) was added and the reaction mixture was stirred at rt for 2 h. Then a mixture of ethyl potassium malonate (5.00 mg, 29.0 µmol, 1.00 equiv.) [which had been prepared from ethyl hydrogen malonate (1 g) and KOH (0.4 g) in abs. ethyl alcohol 4 ml], anhydr. MgCl₂ (5.61 mg, 59.0 µmol, 2.00 equiv.) and TEA (9.86 µl, 7.16 mg, 71.0 µmol, 2.40 equiv.) were added. The reaction mixture was stirred further at rt for 24 h. After concentration under reduced pressure the obtained residue was resuspended in 2 M aq. HCl and extracted with DCM. The combined organic layers were washed with sat. aq. NaHCO₃ and brine, dried over Na₂SO₄ and concentrated under reduced pressure. Purification by flash column chromatography (nPen/EtOAc 3:1) resulted in the title compound as a colourless oil (0.82 mg, 1.72 µmol, 6%). 1H NMR (500 MHz, CDCl3) δ 7.66 (dd, J = 10.0, 7.9 Hz, 1H), 7.48 (d, J = 4.5 Hz, 1H), 7.35 (dd, J = 13.4, 7.9 Hz, 1H), 7.20 - 7.14 (m, 1H), 6.84 - 6.79 (m, 2H), 6.71 (dd, J = 9.1, 2.1 Hz, 1H), 5.59 (s, 1H), 4.71 (bs, 1H), 4.29 (q, J = 7.1 Hz, 1H), 4.21 (q, J = 7.1 Hz, 1H), 4.04 (s, 2H), 3.92 - 3.46 (m, 3H), 3.38 - 3.09 (m, 3H), 1.47 (bs, 3H), 1.35 (t, J = 7.1 Hz, 1H), 1.26 (t, J = 7.1 Hz, 2H), 1.07 (bs, 3H). ¹³C NMR (126 MHz, CDCl3) δ 172.7, 169.4, 166.8, 151.4, 139.0, 138.8, 135.4, 132.9, 132.2, 130.5, 130.4, 119.6, 119.5, 116.3, 114.3, 94.0, 61.8, 60.9, 56.3, 49.2, 14.4, 14.2. HRMS: Calculated for [C₂₄H₂₈Cl₂N₂O₄S + H]⁺ = 477.1342, found = 477.1346.

### Example 56: (±) Isopropyl 2-((2-chloro-4-(4-(3-chlorophenyl)-trans-2,3-dimethylpiperazine-1-carbonyl)phenyl)sulfinyl)acetate

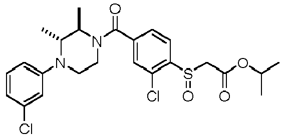

The title compound was synthesized using (±) 2-Chloro-4-(4-(3-chlorophenyl)-trans-2,3-dimethylpiperazine-1-carbonyl)benzoic acid (30.0 mg, 64.0 µmol, 1.00 equiv.), oxalyl chloride (2 M in DCM, 35.0 µl, 70.0 µmol, 1.10 equiv.), 2-propanol (19.2 mg, 320 µmol, 5.00 equiv.) and DIPEA (9.09 mg, 70.0 µmol, 1.10 equiv.) according to general procedure G. This yielded the product (14.0 mg, 27.5 µmol, 43 %). ¹H NMR (500 MHz, CDCl3) δ 8.02 (d, 3J = 7.9 Hz, 1H), 7.58 - 7.51 (m, 1H), 7.51 - 7.43 (m, 1H), 7.17 (t, 3J = 8.0 Hz, 1H), 6.84 - 6.79 (m, 2H), 6.71 (d, 3J = 8.4 Hz, 1H), 5.10 (quin., 3J = 6.3 Hz, 1H), 4.88 - 4.76 (m, 1H), 4.62 (s, 1H), 4.02 (dd, 3,4J = 14.1, 2.3 Hz, 1H), 3.87 (d, J = 6.9 Hz, 1H), 3.65 (dt, 3,4J = 14.2, 2.8 Hz, 1H), 3.53 (s, 1H), 3.40 - 3.06 (m, 3H), 1.53 - 1.44 (m, 3H), 1.32 - 1.24 (m, 7H), 1.16 - 0.99 (m, 3H). ¹³C NMR (126 MHz, CDCl3) δ 168.8, 164.2, 151.4, 142.7, 140.6, 135.4, 130.4, 126.2, 119.6, 116.3, 114.3, 70.6, 58.8, 56.3, 55.5, 49.7, 42.4, 41.4, 40.6, 36.6, 21.9, 17.9, 16.8, 12.9, 12.6. HRMS: Calculated for [C₂₄H₂₈Cl₂N₂O₄S + H]⁺ = 511.1220, found = 511.1227.

### Example 57: (±) sec-Butyl 2-((2-chloro-4-(4-(3-chlorophenyl)-trans-2,3-dimethylpiperazine-1-carbonyl)phenyl)sulfinyl)acetate

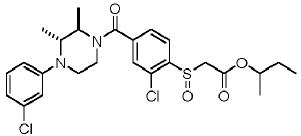

The title compound was synthesized using (±) 2-Chloro-4-(4-(3-chlorophenyl)-trans-2,3-dimethylpiperazine-1-carbonyl)benzoic acid (50.0 mg, 107 µmol, 1.00 equiv.), oxalyl chloride (2 M in DCM, 80.0 µl, 160 µmol, 1.50 equiv.), butan-2-ol (49.0 µl, 39.5 mg, 533 µmol, 5.00 equiv.) and DIPEA (28.0 µl, 20.7 mg, 160 µmol, 1.50 equiv.) according to general procedure G. This yielded the product (14.1 mg, 26.8 µmol, 25 %). ¹H NMR (400 MHz, CDCl3) δ 8.02 (dd, 3J = 7.9, 2.0 Hz, 1H), 7.59 - 7.51 (m, 1H), 7.50 - 7.42 (m, 1H), 7.17 (t, 3J = 8.0 Hz, 1H), 6.85 - 6.77 (m, 2H), 6.70 (dd, 3J = 7.9, 3.2 Hz, 1H), 4.95 (sex.d, J = 6.3, 2.7 Hz, 1H), 4.81 (t, J = 6.2 Hz, 1H), 4.67 - 4.55 (m, 1H), 4.04 (dt, 3,4J = 14.0, 1.8 Hz, 1H), 3.87 (q, J = 6.7 Hz, 1H), 3.71 - 3.59 (m, 2H), 3.52 (d, J = 6.8 Hz, 1H), 3.40 - 3.06 (m, 3H), 1.70 - 1.53 (m, 2H), 1.53 - 1.43 (m, 3H), 1.30 - 1.21 (m, 4H), 1.16 - 0.99 (m, 3H), 0.97 - 0.88 (m, 3H). ¹³C NMR (126 MHz, CDCl3) δ 164.4, 164.3, 151.4, 142.8, 140.7, 135.3, 130.8, 130.4, 128.6, 127.9, 127.0, 126.4, 126.2, 125.9, 119.6, 116.3, 114.3, 75.2, 59.0, 58.8, 56.2, 55.5, 49.7, 42.4, 41.4, 40.5, 36.6, 28.8, 19.5, 17.9, 16.8, 12.9, 12.6, 9.7. HRMS: Calculated for [C₂₅H₃₀Cl₂N₂O₄S + H]⁺ = 525.1376, found = 525.1385.

### Example 58: (±) Cyclobutyl 2-((2-chloro-4-(4-(3-chlorophenyl)-trans-2,3-dimethylpiperazine-1-carbonyl)phenyl)sulfinyl)acetate

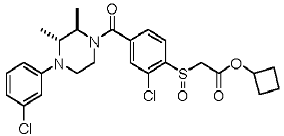

The title compound was synthesized using (±) 2-Chloro-4-(4-(3-chlorophenyl)-trans-2,3-dimethylpiperazine-1-carbonyl)benzoic acid (50.0 mg, 107 µmol, 1.00 equiv.), oxalyl chloride (2 M in DCM, 80.0 µl, 160 µmol, 1.50 equiv.), cyclobutanol (77 mg, 1.07 mmol, 10 equiv.) and DIPEA (28.0 µl, 20.7 mg, 160 µmol, 1.50 equiv.) according to general procedure G. This yielded the product (8mg, 0.015 mmol, 14%). ¹H NMR (500 MHz, CDCl3) δ 8.03 (d, J = 8.0 Hz, 1H), 7.66 - 7.40 (m, 2H), 7.18 (t, J = 8.1 Hz, 1H), 6.89 - 6.76 (m, 2H), 6.72 (d, J = 8.4 Hz, 1H), 5.06 (tt, J = 7.9, 7.1 Hz, 1H), 4.88 - 3.00 (m, 8H), 2.45 - 2.27 (m, 2H), 2.19 - 2.00 (m, 2H), 1.89 - 1.57 (m, 2H), 1.49 (d, J = 10.0 Hz, 3H), 1.20 - 0.86 (m, 3H). ¹³C NMR (126 MHz, CDCl₃) δ 169.03, 163.85, 151.33, 142.36, 140.47, 135.36, 130.95, 130.43, 128.36, 127.16, 125.85, 119.66, 116.37, 114.34, 70.27, 58.23, 56.27, 49.92, 40.54, 36.71, 30.37, 30.32, 18.42, 14.12, 12.61. HRMS: Calculated for [C₂₅H₂₈Cl₂N₂O₄S + H]⁺ = 523.12196, found = 523.12181.

### Example 59: (±) Cyclopentyl 2-((2-chloro-4-(4-(3-chlorophenyl)-trans-2,3-dimethylpiperazine-1-carbonyl)phenyl)sulfinyl)acetate

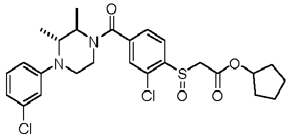

The title compound was synthesized using (±) 2-Chloro-4-(4-(3-chlorophenyl)-trans-2,3-dimethylpiperazine-1-carbonyl)benzoic acid (50.0 mg, 107 µmol, 1.00 equiv.), oxalyl chloride (2 M in DCM, 80.0 µl, 160 µmol, 1.50 equiv.), cyclopentanol (92 mg, 1.07 mmol, 10 equiv.) and DIPEA (28.0 µl, 20.7 mg, 160 µmol, 1.50 equiv.) according to general procedure G. This yielded the product (12 mg, 0.022 mmol, 21%). ¹H NMR (500 MHz, CDCl₃) δ 8.03 (d, J = 8.0 Hz, 1H), 7.63 - 7.41 (m, 2H), 7.18 (t, J = 8.1 Hz, 1H), 6.87 - 6.76 (m, 2H), 6.72 (d, J = 8.7 Hz, 1H), 5.29 - 5.19 (m, 1H), 5.12 - 3.05 (m, 8H), 1.87 (q, J = 6.9 Hz, 2H), 1.79 - 1.40 (m, 9H), 1.08 (d, J = 53.0 Hz, 3H). ¹³C NMR (126 MHz, CDCl₃) δ 169.01, 164.34, 151.32, 140.35, 135.36, 130.95, 130.43, 128.58, 127.17, 126.39, 119.68, 116.38, 114.35, 79.90, 58.42, 56.28, 49.97, 40.53, 36.75, 32.84, 32.72, 23.81, 23.78, 17.89, 12.60. HRMS: Calculated for [C₂₆H₃₀Cl₂N₂O₄S + H]⁺ = 537.13761, found = 537.13760.

### Example 60: (±) Cyclohexyl 2-((2-chloro-4-(4-(3-chlorophenyl)-trans-2,3-dimethylpiperazine-1-carbonyl)phenyl)sulfinyl)acetate

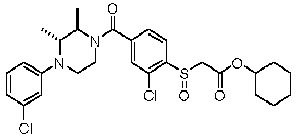

The title compound was synthesized using (±) 2-Chloro-4-(4-(3-chlorophenyl)-trans-2,3-dimethylpiperazine-1-carbonyl)benzoic acid (50.0 mg, 107 µmol, 1.00 equiv.), oxalyl chloride (2 M in DCM, 80.0 µl, 160 µmol, 1.50 equiv.), cyclohexanol (107 mg, 1.07 mmol, 10 equiv.) and DIPEA (28.0 µl, 20.7 mg, 160 µmol, 1.50 equiv.) according to general procedure G. This yielded the product (3 mg, 0.005mmol, 5%). ¹H NMR (500 MHz, CDCl₃) δ 8.03 (d, J = 8.0 Hz, 1H), 7.62 - 7.43 (m, 2H), 7.18 (t, J = 8.1 Hz, 1H), 6.87 - 6.77 (m, 2H), 6.71 (d, J = 8.3 Hz, 1H), 4.88 (tt, J = 8.9, 3.9 Hz, 1H), 4.84 - 2.94 (m, 8H), 1.70 - 1.20 (m, 13H), 1.20 - 0.96 (m, 3H). ¹³C NMR (126 MHz, CDCl₃) δ 168.06, 163.57, 151.37, 142.76, 140.55, 134.94, 130.90, 130.40, 128.34, 127.08, 126.37, 119.62, 116.35, 114.32, 75.45, 58.72, 56.27, 49.78, 40.54, 36.65, 31.58, 25.34, 23.73, 17.89, 12.62. HRMS: Calculated for [C₂₇H₃₂Cl₂N₂O₄S + H]⁺ = 551.15326, found = 551.15325.

### Example 61: (±) 2,3-Dihydroxypropyl 2-((2-chloro-4-(4-(3-chlorophenyl)-trans-2,3-dimethylpiperazine-1-carbonyl)phenyl)sulfinyl)acetate

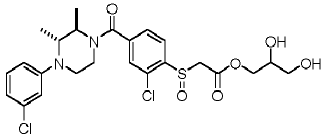

The title compound was synthesized using (±) 2-Chloro-4-(4-(3-chlorophenyl)-trans-2,3-dimethylpiperazine-1-carbonyl)benzoic acid (50.0 mg, 107 µmol, 1.00 equiv.), oxalyl chloride (2 M in DCM, 80.0 µl, 160 µmol, 1.50 equiv.), glycerol (98 mg, 1.07 mmol, 10 equiv.) and DIPEA (28.0 µl, 20.7 mg, 160 µmol, 1.50 equiv.) according to general procedure G. This yielded the product(3mg, 0.006mmol, 5%) 1H NMR (500 MHz, CDCl3) δ 7.98 - 7.87 (m, 1H), 7.60 - 7.42 (m, 2H), 7.18 (t, J = 8.1 Hz, 1H), 6.85 - 6.77 (m, 2H), 6.71 (d, J = 7.5 Hz, 1H), 4.87 - 3.05 (m, 11H), 1.95 (br, 2H), 1.50 (dd, J = 15.0, 6.7 Hz, 3H), 1.08 (m, 3H). HRMS: Calculated for [C24H28Cl2N2O6S + H]+ = 543.11179, found = 543.11171.

### Example 62: (±) Ethyl 2-((2-chloro-4-(4-(3-chlorophenyl)-trans-2,3-dimethylpiperazine-1-carbonyl)phenyl)sulfinyl)propanoate

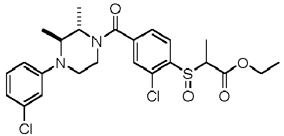

Step A: tert-butyl 3-chloro-4-fluorobenzoate (0.270 g, 1.18 mmol, 1.2 eq.) and ethyl 2-mercaptopropanoate (0.13 ml, 0.98 mmol, 1 eq.) was used according to general procedure D. This yielded the product (0.27 g, 0.78 mmol, 79 %). ¹H NMR (400 MHz, CDCl3) δ 7.96 (s, 1H), 7.82 (d, J = 8.5 Hz, 1H), 7.47 (d, J = 8.3 Hz, 1H), 4.22 - 4.10 (m, 2H), 4.07 - 3.97 (m, 1H), 1.59 (m, 12H), 1.21 (t, J = 7.1 Hz, 3H). ¹³C NMR (101 MHz, CDCl3) δ 171.99, 164.21, 139.65, 133.70, 131.23, 130.49, 129.45, 127.94, 81.76, 61.69, 43.25, 28.17, 17.18, 14.11.
Step B: tert-butyl 3-chloro-4-((1-ethoxy-1-oxopropan-2-yl)thio)benzoate (0.13 g, 0.38 mmol, 1 eq) was used according to general procedure E. This yielded the product ( 0.13 g, 0.35 mmol, 92%). ¹H NMR (400 MHz, CDCl3) δ 8.11 (dd, J = 8.2, 1.5 Hz, 1H), 8.00 (d, J = 1.6 Hz, 1H), 7.90 (d, J = 8.1 Hz, 1H), 4.31 (q, J = 8 Hz, 2H), 3.91 (q, J = 8 Hz, 1H), 1.61 (s, 9H), 1.35 (t, J = 7.1 Hz, 3H), 1.25 (d, J = 7.2 Hz, 3H). ¹³C NMR (101 MHz, CDCl3) δ 168.58, 163.61, 144.19, 136.33, 130.88, 130.32, 128.49, 127.58, 82.73, 77.48, 77.16, 76.85, 62.57, 60.49, 28.21, 14.26, 6.62.
Step C: tert-butyl 3-chloro-4-((2-ethoxy-2-oxoethyl)sulfinyl)benzoate (0.14 g, 0.40 mmol, 1 eq) was used according to general procedure F. This yielded the product (0.12 g, 0.40 mmol, 100%). ¹H NMR (MeOD, 400 MHz): δ 8.22 (dd, J = 8.1, 1.5 Hz, 1H), 8.11 (d, J = 1.5 Hz, 1H), 7.89 (d, J = 8.1 Hz, 1H), 4.30 (qd, J = 7.1, 2.3 Hz, 2H), 4.13 (q, J = 7.1 Hz, 1H), 1.32 (t, J = 7.1 Hz, 3H), 1.21 (d, J = 7.1 Hz, 3H). ¹³C NMR (MeOD, 101 MHz): δ 169.70, 144.80, 136.92, 132.16, 131.70, 129.93, 128.61, 63.50, 61.67, 48.58, 14.42, 6.78.
Step D: The title compound was synthesized using 3-chloro-4-((1-ethoxy-1-oxopropan-2-yl)sulfinyl)benzoic acid (0.10 g, 0.34 mmol, 1 eq.) according to general procedure H. This yield the product (0.15 g, 0.29 mmol, 85%). ¹H NMR (400 MHz, CDCl3) δ 7.95 - 7.85 (m, 1H), 7.60 - 7.39 (m, 2H), 7.17 (t, J = 8.3 Hz, 1H), 6.86 - 6.78 (m, 2H), 6.78 - 6.66 (m, 1H), 4.91 - 4.53 (m, 1H), 4.32 (q, J = 7.2 Hz, 1H), 4.10 - 3.05 (m, 7H), 1.56 - 1.43 (m, 3H), 1.40 - 1.31 (m, 3H), 1.31 - 1.24 (m, 3H), 1.17 - 0.97 (m, 3H). ¹³C NMR (126 MHz, CDCl3) δ 168.56, 166.53, 151.31, 140.49, 135.26, 131.61, 131.03, 130.34, 128.61, 128.10, 125.35, 119.45, 116.21, 114.20, 62.50, 60.52, 56.14, 40.47, 38.68, 36.50, 17.81, 14.21, 12.57, 6.60 (carbon spectrum shows a mixture of rotamers). HRMS: Calculated for [C₂₄H₂₈Cl₂N₂O₄S + Na]⁺ = 535.1008, found = 535.1009.

### Example 63: (±) sec-Butyl 2-((2-chloro-4-(4-(3-chlorophenyl)-trans-2,3-dimethylpiperazine-1-carbonyl)phenyl)sulfinyl)propanoate

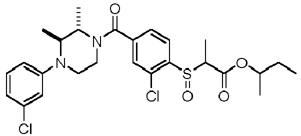

Step A: To a solution of (±) ethyl 2-((2-chloro-4-(4-(3-chlorophenyl)-trans-2,3-dimethylpiperazine-1-carbonyl)phenyl)sulfinyl)propanoate (0.15 g, 0.29 mmol, 1 eq.) in 2 ml MeOH was added 2ml TEA and 2ml water. The reaction mixture was stirred at room temperature overnight. The reaction progress was monitored by TLC. Once completed, the mixture was acidified with 3M HCl solution to pH 2, extracted with EtOAc, dried over anhydrous magnesium sulfate. After filtration, the filtrate was concentrated under reduced pressure. The residue was purified by silica gel column chromatography (MeOH/DCM, 1-5%) to afford the product the product (0.13 g, 0.28 mmol, quant.). ¹H NMR (500 MHz, CDCl3) δ 8.28 (s, 1H), 7.96 (dd, J = 23.2, 7.9 Hz, 1H), 7.58 - 7.42 (m, 2H), 7.18 (t, J = 8.1 Hz, 1H), 6.85 - 6.79 (m, 2H), 6.71 (d, J = 8.3 Hz, 1H), 4.95 - 4.58 (m, 1H), 3.96 (q, J = 7.1 Hz, 1H), 3.92 - 3.02 (m, 5H), 1.74 (d, J = 7.0 Hz, 1H), 1.54 - 1.45 (m, 3H), 1.30 (d, J = 7.2 Hz, 3H), 1.17 - 0.96 (m, 3H). ¹³C NMR (126 MHz, CDCl3) δ 170.41, 169.00, 151.30, 140.59, 140.22, 135.34, 131.47, 130.42, 128.78, 128.21, 126.10, 119.64, 116.36, 114.33, 60.57, 56.25, 39.89, 36.77, 17.88, 16.84, 12.56, 7.02 (carbon spectrum shows a mixture of rotamers).
Step B: The title compound was synthesized using (±) 2-((2-Chloro-4-(trans-4-(3-chlorophenyl)-2,3-dimethylpiperazine-1-carbonyl)phenyl)sulfinyl)propanoic acid (60 mg, 0.12 mmol, 1 eq.), butan-2-ol (1 ml, 10.87 mmol, 88 eq.), 2 M oxalyl chloride solution (0.07 ml, 0.14 mmol. 1.1 eq.) and DIPEA (0.1 ml, 0.57 mmol, 4.6 eq.) according to general procedure G. This yielded the product (8.1 mg, 0.02 mmol, 12%). 1H NMR (500 MHz, CDCl3) δ 7.93 (d, 1H), 7.57 - 7.46 (m, 2H), 7.19 (t, J = 8.0 Hz, 1H), 6.86 - 6.81 (m, 2H), 6.74 (d, J = 8.4 Hz, 1H), 5.06 - 4.99 (m, 1H), 3.90 (qd, J = 7.2, 5.0 Hz, 1H), 3.84 - 3.11 (m, 6H), 1.77 - 1.60 (m, 2H), 1.51 (d, J = 6.7 Hz, 3H), 1.31 (dd, J = 6.3, 0.9 Hz, 3H), 1.27 (ddd, J = 7.3, 6.0, 1.5 Hz, 3H), 1.14 - 1.04 (m, 3H), 0.97 (dt, J = 11.0, 7.4 Hz, 3H). 13C NMR (126 MHz, CDCl3) δ 166.53, 165.29, 151.34, 141.97, 140.49, 135.38, 131.84, 130.42, 128.30, 127.91, 125.92, 119.66, 116.37, 114.35, 74.55, 74.38, 62.00, 61.35, 56.31, 28.74, 19.34, 12.46, 12.24, 9.61. HRMS: Calculated for [C₂₃H₃₂Cl₂N₂O₄S + H]⁺ = 541.1503, found = 541.1501.

### Example 64: (±) Benzo[d][1,3]dioxol-5-ylmethyl 2-((2-chloro-4-(4-(3-chlorophenyl)-trans-2,3-dimethylpiperazine-1-carbonyl)phenyl)sulfinyl)acetate

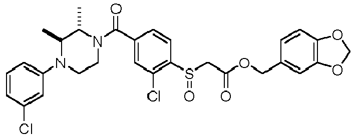

The title compound was synthesized using benzo[d][1,3]dioxol-5-ylmethanol (20 mg, 0.13 mmol, 3 eq.) according to general procedure G. The title compound was obtained as a white solid (8 mg, 0.01 mmol, 31%). ¹H NMR (500 MHz, CDCl3) δ 7.98 (d, J = 8.0 Hz, 1H), 7.57 - 7.39 (m, 2H), 7.18 (t, J = 8.0 Hz, 1H), 6.88 - 6.76 (m, 5H), 6.71 (d, J = 8.4 Hz, 1H), 5.98 (s, 2H), 5.18 - 5.04 (m, 2H), 4.16 - 3.08 (m, 8H), 1.55 - 1.43 (m, 3H), 1.30 - 1.22 (m, 3H). HRMS: Calculated for [C₂₉H₂₈Cl₂N₂O₆S + H]⁺ = 603.1118, found = 603.1116.

### Example 65: (±) 2-Methoxyethyl 2-((2-chloro-4-(4-(3-chlorophenyl)-trans-2,3-dimethylpiperazine-1-carbonyl)phenyl)sulfinyl)acetate

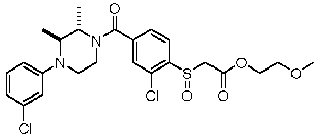

The title compound was synthesized using 2-methoxyethan-1-ol (1 ml, 12.68 mmol, 238 eq.) according to general procedure G. The title compound was obtained as a white solid (8 mg, 0.02 mmol, 29%). ¹H NMR (500 MHz, CDCl3) δ 8.03 (d, J = 8.0 Hz, 1H), 7.56 (d, J = 9.5 Hz, 1H), 7.49 (d, J = 1.5 Hz, 1H), 7.18 (t, J = 8.0 Hz, 1H), 6.83 (d, J = 9.3 Hz, 2H), 6.72 (d, J = 8.4 Hz, 1H), 4.72 (d, J = 75.1 Hz, 1H), 4.40 - 4.27 (m, 2H), 4.11 (dd, J = 14.0, 1.1 Hz, 1H), 3.87 (s, 1H), 3.75 (d, J = 14.0 Hz, 1H), 3.61 (ddd, J = 5.8, 3.8, 2.2 Hz, 4H), 3.39 (s, 3H), 3.34 - 3.08 (m, 2H), 1.50 (d, J = 6.6 Hz, 3H), 1.31 - 0.94 (m, 3H). HRMS: Calculated for [C₂₄H₂₈Cl₂N₂O₅S + H]⁺ = 527.1169, found = 527.1163.

### Example 66: (±) 1-Methoxypropan-2-yl 2-((2-chloro-4-(4-(3-chlorophenyl)-trans-2,3-dimethylpiperazine-1-carbonyl)phenyl)sulfinyl)acetate

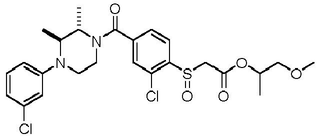

The title compound was synthesized using 1-methoxypropan-2-ol (1 ml, 10.21 mmol, 192 eq.) according to general procedure G. The title compound was obtained as a white solid (5.3 mg, 9.8 µmol, 18%). ¹H NMR (500 MHz, CDCl3) δ 8.03 (d, J = 7.9 Hz, 1H), 7.64 - 7.39 (m, 2H), 7.18 (t, J = 8.1 Hz, 1H), 6.87 - 6.77 (m, 2H), 6.72 (d, J = 8.5 Hz, 1H), 5.24 - 5.11 (m, 1H), 5.04 - 4.54 (m, 1H), 4.08 (d, J = 10.6 Hz, 1H), 3.87 (s, 1H), 3.70 (dd, J = 14.0, 3.7, 0.8 Hz, 1H), 3.52 - 3.40 (m, 3H), 3.38 (s, 3H), 3.19 (s, 3H), 1.49 (d, J = 6.7 Hz, 3H), 1.28 (dd, J = 19.0, 6.5 Hz, 3H), 1.18 - 0.96 (m, 3H). HRMS: Calculated for [C₂₅H₃₀Cl₂N₂O₅S + H]+ = 541.1325, found = 541.1323.

### Example 67: (±) tetraHydro-2H-pyran-4-yl 2-((2-chloro-4-(4-(3-chlorophenyl)-trans-2,3-dimethylpiperazine-1-carbonyl)phenyl)sulfinyl)acetate

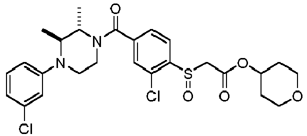

The title compound was synthesized using (±) 2-((2-Chloro-4-(4-(3-chlorophenyl)-trans-2,3-dimethylpiperazine-1-carbonyl)phenyl)sulfinyl)acetic acid (30 mg, 0,064 mmol, 1 eq), 2 M oxalyl chloride solution (8.94 mg, 0.071 mmol, 1.1 eq), tetrahydro-2H-pyran-4-ol (107 mg, 1.05 mmol, 16 eq) and DIPEA (74 mg, 0.57 mmol, 9 eq) according to general procedure G. This yielded the product (4.1 mg, 0.0074 mmol, 12%). 1H NMR (600 MHz, CDCl3) δ 8.04 (d, J = 8.0 Hz, 1H), 7.61 - 7.54 (m, 1H), 7.54 - 7.47 (m, 1H), 7.20 (t, J = 8.1 Hz, 1H), 6.87 - 6.79 (m, 2H), 6.78 - 6.70 (m, 1H), 5.11 - 5.05 (m, 1H), 4.10 (d, J = 13.9 Hz, 1H), 3.98 - 3.89 (m, 2H), 3.79 - 3.53 (m, 5H), 3.38 - 3.12 (m, 3H), 2.09 - 2.00 (m, 2H), 1.98 - 1.91 (m, 2H), 1.46 (d, J = 6.7 Hz, 3H), 1.41 (d, J = 6.7 Hz, 3H). 13C NMR (151 MHz, CDCl3) δ 164.03, 151.43, 142.68, 140.85, 135.41, 130.86, 130.43, 128.44, 127.07, 126.42, 120.98, 119.61, 116.34, 114.31, 71.75, 65.99, 65.27, 58.62, 54.19, 53.55, 42.41, 31.74, 14.18, 11.98.

### Example 68: (±) Ethyl 2-((2-chloro-4-(-4-(5-chloropyridin-3-yl)-trans-2,3-dimethylpiperazine-1-carbonyl)phenyl)sulfinyl)acetate

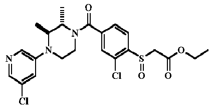

Step A: (±) trans-2,3-Dimethylpiperazine (262 mg, 2.29 mmol, 1 eq) was dissolved in DCM (100 ml) at rt. Boc₂O (500 mg, 2.29 mmol, 1 eq) dissolved in DCM (10 ml) was added slowly via a syringe pump over 45 h at rt. After adding, the reaction mixture was concentrated, and the residue was purified by column chromatography (0-10% MeOH in DCM). This yielded the product as a yellow oil (282 mg, 1.31 mmol, 57%). ¹H NMR (400 MHz, CDCl₃) δ 5.19 (br, 1H), 3.92 (dd, *J* = 5.5, 1.6 Hz, 1H), 3.80 (d, *J* = 10.5 Hz, 1H), 3.12 - 2.71 (m, 4H), 1.38 (s, 9H), 1.24 (d, J = 5.2 Hz, 3H), 1.23 (d, J = 5.1 Hz, 3H). ¹³C NMR (101 MHz, CDCl₃) δ 155.12, 79.87, 51.40, 50.70, 38.46, 37.41, 28.39, 17.21, 16.19.
Step B: 3-bromo-5-chloropyridine (55 mg, 0.29 mmol, 1 eq), (±) *tert*-butyl trans-2,3-dimethylpiperazine-1-carboxylate (61 mg, 0.29 mmol, 1 eq) was used according to general procedure A. This yielded the product (11.4 mg, 0.035 mmol, 12%). 1H NMR (400 MHz, CDCl3) δ 8.09 (d, J = 2.6 Hz, 1H), 7.98 (d, J = 1.9 Hz, 1H), 7.05 (t, J = 2.2 Hz, 1H), 4.32 - 3.85 (m, 2H), 3.77 - 3.64 (m, 1H), 3.31 - 3.07 (m, 3H), 1.48 (s, 9H), 1.31 (d, J = 6.8 Hz, 3H), 1.11 (d, J = 6.7 Hz, 3H). 13C NMR (101 MHz, CDCl3) δ 148.79, 146.97, 145.87, 138.02, 135.62, 134.21, 121.19, 80.06, 55.32, 55.11, 51.85, 40.58, 28.45, 13.61.
Step C: 3-bromo-5-chloropyridine (385 mg, 2 mmol, 2 eq) and (±) trans-2,3-dimethylpiperazine (114 mg, 1 mmol, 1 eq) was used according to general procedure J. This yielded the product (9.3 mg, 0.041 mmol, 4%). ¹H NMR (400 MHz, CDCl₃) δ 8.17 (d, J = 2.5 Hz, 1H), 8.09 (d, J = 2.0 Hz, 1H), 7.19 (t, J = 2.3 Hz, 1H), 3.36 - 3.29 (m, 1H), 3.22 - 3.09 (m, 3H), 3.04 - 2.90 (m, 2H), 2.29 (br, 1H), 1.34 (d, J = 6.7 Hz, 3H), 1.11 (d, J = 6.5 Hz, 3H). ¹³C NMR (101 MHz, CDCl3) δ 147.95, 140.23, 139.53, 132.17, 125.13, 56.92, 53.98, 46.70, 41.54, 18.74, 14.70.
Step D: The title compound was synthesized using 3-chloro-4-((2-ethoxy-2-oxoethyl)sulfinyl)benzoic acid (11.6 mg, 0.04 mmol, 1 eq) and (±) 1-(5-chloropyridin-3-yl)-trans-2,3-dimethylpiperazine (9 mg, 0.04 mmol, 1 eq) according to general procedure H. This yielded the product (12.4 mg, 0.025 mmol, 62%). ¹H NMR (400 MHz, CDCl₃) 8.40 (s, 1H), 8.08 (s, 1H), 8.04 (d, *J* = 7.9 Hz, 1H), 7.62 - 7.54 (m, 1H), 7.47 (d, *J* = 13.7 Hz, 2H), 4.98 - 4.65 (m, 1H), 4.33 - 4.17 (m, 2H), 4.06 (dd, *J* = 14.0, 1.9 Hz, 1H), 4.00 - 3.19 (m, 6H), 1.52 - 1.17 (m, 9H). ¹³C NMR (101 MHz, CDCl₃) δ 168.99, 164.36, 148.19, 142.53, 139.55, 135.34, 131.00, 130.26, 128.53, 128.24, 127.90, 127.09, 126.08, 62.57, 58.06, 54.94, 49.27, 40.23, 35.82, 17.67, 14.07, 13.76. HRMS: Calculated for [C₂₂H₂₅Cl₂N₃O₄S + H]⁺ = 498.10156, found = 498.10148.

### Example 69: (±) Ethyl 2-((2-chloro-4-(-4-(2-chloropyridin-4-yl)-trans-2,3-dimethylpiperazine-1-carbonyl) phenyl)sulfinyl) acetate

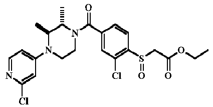

Step A: (±) trans-2,3-Dimethylpiperazine (262 mg, 2.29 mmol, 1 eq) was dissolved in DCM (100 ml) at rt. Boc₂O (500 mg, 2.29 mmol, 1 eq) dissolved in DCM (10 ml) was added slowly via a syringe pump over 45 h at rt. After adding, the reaction mixture was concentrated, and the residue was purified by column chromatography (0-10% MeOH in DCM). This yielded the product as a yellow oil (282 mg, 1.31 mmol, 57%). ¹H NMR (400 MHz, CDCl₃) δ 5.19 (br, 1H), 3.92 (dd, *J* = 5.5, 1.6 Hz, 1H), 3.80 (d, *J* = 10.5 Hz, 1H), 3.12 - 2.71 (m, 4H), 1.38 (s, 9H), 1.24 (d, J = 5.2 Hz, 3H), 1.23 (d, J = 5.1 Hz, 3H). ¹³C NMR (101 MHz, CDCl₃) δ 155.12, 79.87, 51.40, 50.70, 38.46, 37.41, 28.39, 17.21, 16.19.
Step B: 4-bromo-2-chloropyridine (45 mg, 0.23 mmol, 1 eq) and (±)-tert-butyl 2,3-dimethylpiperazine-1-carboxylate (50 mg, 0.23 mmol, 1 eq) was used according to general procedure A. This yielded the product (24 mg, 0.073 mmol, 31%). ¹H NMR (400 MHz, CDCl₃) δ 8.01 (d, *J* = 6.1 Hz, 1H), 6.59 (d, *J* = 2.4 Hz, 1H), 6.51 (dd, *J* = 6.1, 2.4 Hz, 1H), 4.28 - 3.69 (m, 3H), 3.53 - 3.07 (m, 3H), 1.48 (s, 9H), 1.23 (d, *J* = 6.8 Hz, 3H), 1.19 (d, *J* = 6.7 Hz, 3H). ¹³C NMR (101 MHz, CDCl₃) δ 156.54, 152.83, 149.66, 127.89, 107.05, 80.22, 53.61, 51.80, 40.21, 37.11, 28.41, 17.55 15.16.
Step C: The title compound was synthesized using 4-bromo-2-chloropyridine (385 mg, 2 mmol, 2 eq) and (±) trans-2,3-dimethylpiperazine (114 mg, 1 mmol, 1 eq) according to general procedure J. This yielded the product (11 mg, 0.049 mmol, 5%). ¹H NMR (400 MHz, CDCl₃) δ 7.99 (d, *J* = 6.1 Hz, 1H), 6.59 (d, *J* = 2.4 Hz, 1H), 6.52 (dd, *J* = 6.1, 2.4 Hz, 1H), 3.76 - 3.58 (m, 1H), 3.51 - 3.36 (m, 1H), 3.24 - 2.83 (m, 4H), 2.48 (br, 1H), 1.30 (d, *J* = 6.7 Hz, 3H), 1.25 (d, *J* = 6.7 Hz, 3H). ¹³C NMR (101 MHz, CDCl₃) δ 156.95, 152.91, 149.67, 107.19, 107.07, 54.06, 51.98, 40.48, 39.12, 18.70, 14.37.
Step D: The title compound was synthesized using 3-chloro-4-((2-ethoxy-2-oxoethyl)sulfinyl)benzoic acid (14.5 mg, 0.05 mmol) and (±) 1-(2-chloropyridin-4-yl)-trans-2,3-dimethylpiperazine (11 mg, 0.05 mmol, 1eq) according to general procedure H. This yielded the product (7.5 mg, 0.015 mmol, 30%). ¹H NMR (400 MHz, CDCl₃) δ 8.29 (d, *J* = 6.4 Hz, 1H), 8.04 (d, *J* = 7.9 Hz, 1H), 7.60 - 7.42 (m, 2H), 6.77 (d, *J* = 7.5 Hz, 2H), 4.96 - 4.62 (m, 1H), 4.24 (qd, *J* = 7.1, 1.5 Hz, 2H), 4.10 - 3.27 (m, 7H), 1.47 - 1.20 (m, 9H). ¹³C NMR (101 MHz, CDCl₃) δ 168.88, 164.42, 158.23, 146.19, 143.54, 142.89, 139.39, 131.04, 128.60, 127.27, 126.28, 107.48, 106.82, 62.53, 58.17, 54.62, 49.33, 40.32, 35.84, 17.78, 15.38, 14.09. HRMS: Calculated for [C₂₂H₂₅Cl₂N₃O₄S + H]⁺ = 498.10156, found = 498.10168.

### Example 70: (±) Ethyl 2-((2-chloro-4-(-4-(6-chloropyridin-2-yl)-trans-2,3-dimethylpiperazine-1-carbonyl)phenyl)sulfinyl)acetate

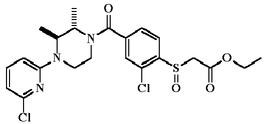

Step A: (±) trans-2,3-Dimethylpiperazine (262 mg, 2.29 mmol, 1 eq) was dissolved in DCM (100 ml) at rt. Boc₂O (500 mg, 2.29 mmol, 1 eq) dissolved in DCM (10 ml) was added slowly via a syringe pump over 45 h at rt. After adding, the reaction mixture was concentrated, and the residue was purified by column chromatography (0-10% MeOH in DCM). This yielded the product as a yellow oil (282 mg, 1.31 mmol, 57%). ¹H NMR (400 MHz, CDCl₃) δ 5.19 (br, 1H), 3.92 (dd, *J* = 5.5, 1.6 Hz, 1H), 3.80 (d, *J* = 10.5 Hz, 1H), 3.12 - 2.71 (m, 4H), 1.38 (s, 9H), 1.24 (d, J = 5.2 Hz, 3H), 1.23 (d, J = 5.1 Hz, 3H). ¹³C NMR (101 MHz, CDCl₃) δ 155.12, 79.87, 51.40, 50.70, 38.46, 37.41, 28.39, 17.21, 16.19.
Step B: 2-bromo-6-chloropyridine (45 mg, 0.23 mmol, 1 eq), (±) tert-butyl trans-2,3-dimethylpiperazine-1-carboxylate (50 mg, 0.23 mmol, 1 eq) were used according to general procedure A. This yielded the product (35 mg, 0.11 mmol, 46. ¹H NMR (400 MHz, CDCl₃) δ 7.37 (t, *J* = 7.9 Hz, 1H), 6.56 (d, *J* = 7.4 Hz, 1H), 6.42 (dd, *J* = 8.4, 2.8 Hz, 1H), 4.25 - 3.81 (m, 4H), 3.25 - 3.05 (m, 2H), 1.47 (s, 9H), 1.21 (d, *J* = 6.8 Hz, 3H), 1.17 (d, *J* = 6.7 Hz, 3H). ¹³C NMR (101 MHz, CDCl₃) δ 159.04, 155.48, 149.65, 139.84, 111.85, 104.33, 79.95, 51.9, 50.54, 38.69, 37.37, 28.55, 17.20, 15.08.
Step C: (±) *tert*-butyl-4-(6-chloropyridin-2-yl)-trans-2,3-dimethylpiperazine-1-carboxylate (35 mg, 0.11 mmol) was used according to general procedure B. This yielded the product (19 mg, 0.083 mmol, 77%). ¹H NMR (400 MHz, CDCl₃) δ 7.39 (dd, *J* = 8.3, 7.6 Hz, 1H), 6.58 (d, *J* = 7.5 Hz, 1H), 6.43 (d, *J* = 8.4 Hz, 1H), 4.34 (br, 1H), 3.54 (dq, *J* = 14.5, 7.3 Hz, 1H), 3.28 - 3.02 (m, 5H), 1.39 (d, *J* = 6.8 Hz, 3H), 1.33 (d, *J* = 6.9 Hz, 3H). ¹³C NMR (101 MHz, CDCl₃) δ 158.78, 149.66, 139.96, 112.23, 104.46, 52.06, 51.73, 38.68, 37.85, 17.39, 14.57.
Step D: 3-chloro-4-((2-ethoxy-2-oxoethyl)sulfinyl)benzoic acid (24 mg, 0.082 mmol, 1 eq) and (±) 1-(6-chloropyridin-2-yl)-trans-2,3-dimethylpiperazine (18.6 mg, 0.082 mmol) were use according to general procedure H. This yielded the product (32.6 mg, 0.065 mmol, 79%). ¹H NMR (400 MHz, CDCl₃) δ 8.05 (dd, *J* = 8.0, 2.8 Hz, 1H), 7.61 - 7.41 (m, 3H), 6.66 (dd, *J* = 7.5, 2.9 Hz, 1H), 6.49 (dd, *J* = 12.1, 8.4 Hz, 1H), 4.91 - 4.56 (m, 1H), 4.38 - 4.05 (m, 5H), 3.77 - 3.06 (m, 4H), 1.45 - 1.14 (m, 9H). ¹³C NMR (101 MHz, CDCl₃) δ 168.96, 164.51, 158.63, 149.76, 142.42, 140.45, 140.15, 130.89, 128.59, 127.19, 126.41, 112.80, 104.55, 62.59, 58.36, 51.75, 49.08, 38.85, 36.52, 17.81, 16.81, 14.21. HRMS: Calculated for [C₂₂H₂₅Cl₂N₃O₄S + H]⁺ = 498.10156, found = 498.10157.

### Example 71: (±) Ethyl 2-((2-chloro-4-(-4-(4-chloropyridin-2-yl)-trans-2,3-dimethylpiperazine-1-carbonyl)phenyl)sulfinyl)acetate

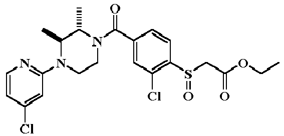

Step A: (±) trans-2,3-Dimethylpiperazine (262 mg, 2.29 mmol, 1 eq) was dissolved in DCM (100 ml) at rt. Boc₂O (500 mg, 2.29 mmol, 1 eq) dissolved in DCM (10 ml) was added slowly via a syringe pump over 45 h at rt. After adding, the reaction mixture was concentrated, and the residue was purified by column chromatography (0-10% MeOH in DCM). This yielded the product as a yellow oil (282 mg, 1.31 mmol, 57%). ¹H NMR (400 MHz, CDCl₃) δ 5.19 (br, 1H), 3.92 (dd, *J* = 5.5, 1.6 Hz, 1H), 3.80 (d, *J* = 10.5 Hz, 1H), 3.12 - 2.71 (m, 4H), 1.38 (s, 9H), 1.24 (d, J = 5.2 Hz, 3H), 1.23 (d, J = 5.1 Hz, 3H). ¹³C NMR (101 MHz, CDCl₃) δ 155.12, 79.87, 51.40, 50.70, 38.46, 37.41, 28.39, 17.21, 16.19.
Step B: 2-bromo-4-chloropyridine (45 mg, 0.23 mmol, 1 eq), (±) tert-butyl trans-2,3-dimethylpiperazine-1-carboxylate (50 mg, 0.23 mmol, 1 eq) were used according to general procedure A. This yielded the product (14.5 mg, 0.045 mmol, 19%). ¹H NMR (400 MHz, CDCl₃) δ 8.05 (d, *J* = 5.3 Hz, 1H), 6.59 (dd, *J* = 5.4, 1.6 Hz, 1H), 6.55 (s, 1H), 4.65 - 3.63 (m, 4H), 3.31 - 2.88 (m, 2H), 1.48 (s, 9H), 1.23 (d, *J* = 6.8 Hz, 3H), 1.17 (d, *J* = 6.7 Hz, 3H). ¹³C NMR (101 MHz, CDCl₃) δ 160.09, 149.01, 145.17, 128.48, 113.23, 106.50, 80.00, 52.03, 50.61, 38.93, 37.43, 28.59, 17.27, 15.20.
Step C: (±) *tert*-butyl-4-(4-chloropyridin-2-yl)-trans-2,3-dimethylpiperazine-1-carboxylate (14.5 mg, 0.045 mmol) according to general procedure B. This yielded the product (7.2 mg, 0.032 mmol, 72%). ¹H NMR (400 MHz, CDCl₃) δ 8.06 (d, *J* = 5.3 Hz, 1H), 6.66 (dd, *J* = 5.4, 1.6 Hz, 1H), 6.58 (d, *J* = 1.6 Hz, 1H), 4.38 (q, *J* = 6.4 Hz, 1H), 4.27 - 4.17 (m, 1H), 3.56 (qd, *J* = 7.0, 1.7 Hz, 1H), 3.47 - 3.23 (m, 3H), 1.54 (d, *J* = 6.8 Hz, 3H), 1.45 (d, *J* = 6.8 Hz, 3H). ¹³C NMR (101 MHz, CDCl₃) δ 159.21, 149.07, 145.56, 114.45, 106.82, 52.27, 51.02, 37.92, 36.48, 15.88, 14.68.
Step D: 3-chloro-4-((2-ethoxy-2-oxoethyl)sulfinyl)benzoic acid (9.3 mg, 0.032 mmol, 1 eq) and (±) 1-(4-chloropyridin-2-yl)-trans-2,3-dimethylpiperazine (7.2 mg, 0.032 mmol, 1 eq) were used according to general procedure H. This yielded the product (15.6 mg, 0.031 mmol, 98%). ¹H NMR (400 MHz, CDCl₃) δ 8.21 (t, *J* = 3.8 Hz, 1H), 8.06 (d, *J* = 6.1 Hz, 1H), 7.63 - 7.46 (m, 2H), 6.93 - 6.87 (m, 2H), 4.96 - 4.63 (m, 1H), 4.41 - 3.96 (m, 5H), 3.83 - 3.28 (m, 4H), 1.48 - 1.24 (m, 9H). ¹³C NMR (101 MHz, CDCl₃) δ 168.89, 164.49, 155.08, 150.38, 143.34, 142.86, 139.46, 130.96, 128.56, 127.02, 126.32, 114.69, 109.82, 62.47, 58.29, 54.03, 49.17, 40.73, 35.82, 17.50, 15.63, 14.10. HRMS: Calculated for [C₂₂H₂₅Cl₂N₃O₄S + H]⁺ = 498.10156, found = 498.10166.

### Example 72: (±) 3-((2-Chloro-4-(4-(3-chlorophenyl)-trans-2,3-dimethylpiperazine-1-carbonyl)phenyl)thio)-1,1,1-trifluoropropan-2-one

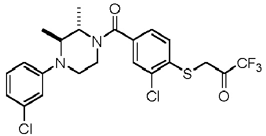

Step A: tert-butyl 3-chloro-4-fluorobenzoate (0.33 g, 1.45 mmol, 1.2 eq.) and ethyl 2-mercaptoacetate (0.13 ml, 1.21 mmol, 1 eq.) were used according to general procedure D. This yielded the product (0.31 g, 0.95 mmol, 95%). 1H NMR (CDCl3, 400 MHz): δ 7.94 (d, J = 1.8 Hz, 1H), 7.83 (dd, J = 8.3, 1.8 Hz, 1H), 7.33 (d, J = 8.3 Hz, 1H), 4.21 (q, J = 7.1 Hz, 2H), 3.75 (s, 2H), 1.58 (s, 9H), 1.26 (t, J = 7.2 Hz, 3H). 13C NMR (CDCl3, 101 MHz): δ 168.75, 164.35, 140.61, 132.17, 130.61, 130.44, 128.19, 126.62, 81.79, 62.12, 34.51, 28.25, 14.21.
Step B: tert-butyl 3-chloro-4-((2-ethoxy-2-oxoethyl)thio)benzoate (61.5 mg, 0.19 mmol) were used according to general procedure F. This yielded the product (51.1 mg, 0.19 mmol, 92%). ¹H NMR (400 MHz, CDCl₃) δ 8.07 (s, 1H), 7.95 (d, J = 8.3 Hz, 1H), 7.37 (d, J = 8.2 Hz, 1H), 4.23 (q, J = 7.2 Hz, 2H), 3.78 (s, 2H), 1.28 (t, J = 7.1 Hz, 3H). ¹³C NMR (400 MHz, CDCl₃) δ 170.34, 168.37, 142.67, 130.76, 128.58, 127.15, 125.87, 61.98, 34.03, 13.91.
Step C: 3-chloro-4-((2-ethoxy-2-oxoethyl)thio)benzoic acid (27, 0.15 mg, 0.54 mmol, 1 eq.) was used according to general procedure H. This yielded the product (0.25 g, 0.46 mmol, 85%). ¹H NMR (400 MHz, CDCl₃) δ 7.48 - 7.36 (m, 2H), 7.34 - 7.27 (m, 1H), 7.17 (t, J = 8.3 Hz, 1H), 6.80 (d, J = 6.1 Hz, 2H), 6.71 (d, J = 8.4 Hz, 1H), 4.84 - 4.53 (m, 1H), 4.21 (q, J = 7.1 Hz, 2H), 3.73 (s, 2H), 3.69 - 3.01 (m, 5H), 1.51 - 1.41 (m, 3H), 1.27 (t, J = 7.2 Hz, 3H), 1.06 (dd, J = 46.6, 6.5 Hz, 3H). ¹³C NMR (101 MHz, CDCl₃) δ 169.52, 168.85, 151.39, 137.10, 135.16, 134.77, 133.25,130.25, 128.12, 128.87, 125.47, 119.18, 116.02, 114.05, 61.99, 56.06, 49.45, 40.43, 36.42, 34.69, 17.69, 14.13, 12.49 (carbon spectrum shows a mixture of rotamers). HRMS: Calculated for [C₂₃H₂₆Cl₂N₂O₃S + H]⁺ = 483.1084, found = 483.1079.
Step D: To a solution of (±) ethyl 2-((2-chloro-4-(4-(3-chlorophenyl)-trans-2,3-dimethylpiperazine-1-carbonyl)phenyl)thio)acetate (0.36 mg, 0.75 mmol,1 eq.) in MeOH ( 10 ml) was added 2 M NaOH solution (0.75 ml, 1.25 mmol, 2 eq.) and the reaction mixture was stirred at RT for 2h. The reaction progress was monitored by TLC. Once completed, the solution was acidified to pH 3 and extracted with DCM. The organic layer was dried over anhydrous MgSO₄ and concentrated under reduced pressure. The residue was purified with column chromatography (EtOAc / pentane, 0 - 100 % with 1 % acetic acid) to yield the product (0.33 mg, 0.72 mmol, 96%). ¹H NMR (400 MHz, CDCl₃) δ 7.48 - 7.36 (m, 2H), 7.29 (d, J = 6.0 Hz, 1H), 7.17 (t, J = 8.3 Hz, 1H), 6.80 (d, J = 6.1 Hz, 2H), 6.71 (d, J = 8.4 Hz, 1H), 4.80 - 4.50 (m, 1H), 4.04 - 3.95 (m, 1H), 3.90 (s, 2H), 3.86 - 3.38 (m, 2H), 3.32 - 3.06 (m, 2H), 1.52 - 1.45 (m, 3H), 1.04 (dd, J = 55.7, 6.5 Hz, 3H). ¹³C NMR (101 MHz, CDCl₃) δ 172.24, 170.27, 151.35, 137.79, 135.25, 133.79, 132.72, 130.33, 129.11, 128.18, 125.37, 119.39, 116.17, 114.18, 56.16, 49.90, 40.48, 36.75, 34.50, 17.75, 12.49 (carbon spectrum shows a mixture of rotamers).
Step E: To a solution of trifluoroacetic anhydride (34 µl, 0.24 mmol, 2.2 eq.) in toluene (0.5 ml) was added (±) 2-((2-chloro-4-(4-(3-chlorophenyl)-trans-2,3-dimethylpiperazine-1-carbonyl)phenyl)thio)acetic acid (50 mg, 0.11 mmol, 1 eq.) and the mixture was cooled to 0 °C. Then pyridine (22µl, 0.28 mmol, 2.5 equiv.) in toluene (0.5 ml) was slowly added and the mixture was stirred at 65 °C overnight. Afterwards it was cooled to 0 °C and 1 ml water was added dropwise. Then the temperature was brought up to 45 °C and maintained for 2h. After cooling, the aqueous phase of the mixture was separated and washed with ethyl acetate. The combined organic layers were washed with water and brine, dried (MgSO₄), filtered and concentrated. The crude product was purified by prep HPLC to provide a white solid (9.4 mg, 0.11 mmol, 17%). 1H NMR (400 MHz, CDCl3) δ 7.63 - 7.27 (m, 3H), 7.17 (t, J = 8.4 Hz, 1H), 6.81 (d, J = 7.2 Hz, 2H), 6.71 (d, J = 8.5 Hz, 1H), 4.69 (dd, J = 76.4, 10.1 Hz, 1H), 4.06 (s, 1H), 3.98 - 3.02 (m, 5H), 1.47 (dd, J = 9.7, 6.7 Hz, 3H), 1.06 (dd, J = 45.1, 6.7 Hz, 3H). HRMS: Calculated for [C₂₂H₂₁Cl₂F₃N₂O₂S + H]⁺ = 523.0831, found = 523.0828.

### Example 73: (±) 3-((2-Chloro-4-(4-(3-chlorophenyl)-trans-2,3-dimethylpiperazine-1-carbonyl)phenyl)sulfinyl)-1,1,1-trifluoropropan-2-one

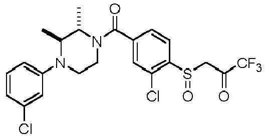

Step A: To a solution of *tert*-butyl 3-chloro-4-fluorobenzoate (0.510 g, 2.22 mmol, 1 eq.) in degassed DMF was added sodium methanethiolate (0.230 g, 3.32 mmol, 1.5 eq.) at -10 °C and the mixture was stirred at RT overnight. The reaction progress was monitored by TLC. Once completed, the mixture was diluted with Et₂O, washed with water, dried over anhydrous MgSO₄ and concentrated under reduced pressure. The residue was purified using column chromatography (Et₂O / pentane, 0-10%) to yield the product (0.24 g, 0.91 mmol, 41%). 1H NMR (400 MHz, CDCl3) δ 7.91 (d, J = 1.8 Hz, 1H), 7.85 (dd, J = 8.3, 1.8 Hz, 1H), 7.13 (d, J = 8.4 Hz, 1H), 2.49 (s, 3H), 1.59 (s, 9H). 13C NMR (400 MHz, CDCl3) δ 164.54, 143.80, 130.83, 129.94, 129.04, 128.05, 123.94, 81.49, 28.22, 14.92.
Step B: tert-butyl 3-chloro-4-(methylthio)benzoate (0.24 g, 0.91 mmol, 1 eq.) was used according to general procedure E. This yielded the product (0.290 g, 1.17 mmol, quant.). 1H NMR (500 MHz, CDCl3) δ 8.13 (dd, J = 8.0, 1.6 Hz, 1H), 8.03 - 7.98 (m, 2H), 2.86 (s, 3H), 1.62 (s, 9H). 13C NMR (500 MHz, CDCl3) δ 163.34, 147.97, 135.70, 130.48, 129.52, 128.73, 125.20, 82.24, 41.37, 27.94.
Step C: tert-butyl 3-chloro-4-(methylsulfinyl)benzoate (0.26 g, 0.94 mmol) was used according to general procedure F. This yielded the product (0.19 g, 0.87 mmol, 92% ). 1H NMR (400 MHz, MeOD) δ 8.22 (dd, J = 8.2, 1.6 Hz, 1H), 8.08 (d, J = 1.6 Hz, 1H), 7.98 (d, J = 8.1 Hz, 1H), 2.90 (s, 3H). 13C NMR (400 MHz, MeOD) δ 167.19, 148.93, 136.49, 132.03, 131.20, 130.45, 126.47, 41.68.
Step D: To a stirred suspension of 3-chloro-4-((2-ethoxy-2-oxoethyl)sulfinyl)benzoic acid (0.19 g, 0.87 mmol, 1 eq.) in DCM (10 ml) were added (±) trans-1-(3-chlorophenyl)-2,3-dimethylpiperazine (32, 0.230 g, 1.04 mmol, 1.2 eq.), DIPEA (0.34 mg, 2.59 mmol, 3 eq.), HOBt (0.18 mg, 1.30 mmol, 1.5 eq.) and EDCI (0.25 mg, 1.30 mmol, 1.5 eq.). The mixture was stirred at RT overnight. The reaction progress was monitored by TLC. Once completed, the mixture was washed with water and brine, dried (MgSO4), filtered and concentrated. The crude product was purified using column chromatography (EtOAc / pentane, 0%-60%) to yield the product (0.27 mg, 0.64 mmol, 74%). ¹H NMR (400 MHz, CDCl₃) δ 8.04 - 7.97 (m, 2H), 7.56 - 7.38 (m, 2H), 7.14 (t, J = 8.2 Hz, 1H).6.60 (d, J = 2.4 Hz, 1H), 6.56 - 6.47 (m, 1H), 4.85 - 4.49 (m, 1H), 3.98 - 3.07 (m, 5H), 2.82 (s, 3H), 1.42 - 1.33 (m, 3H), 1.28 - 1.06 (m, 3H). ¹³C NMR (126 MHz, CDCl₃) δ 168.83, 151.33, 145.25, 140.18, 135.19, 130.47, 130.33, 128.43, 127.77, 125.85, 119.34, 116.13, 114.19, 56.08, 49.57, 42.29, 41.64, 36.46, 17.76, 12.52 (carbon spectrum shows a mixture of rotamers).
Step E: The title was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl 2,2,2-trifluoroacetate (167 mg, 1.18 mmol, 10 eq) according to general procedure I. This yielded the product (48 mg, 0.09 mmol, 79%). ¹H NMR (600 MHz, CDCl₃) δ 8.07 (d, J = 8.0 Hz, 1H), 7.66 - 7.47 (m, 2H), 7.19 (t, J = 8.1 Hz, 1H), 6.86 - 6.80 (m, 2H), 6.72 (dd, J = 8.4, 2.4 Hz, 1H), 3.77 - 2.99 (m, 7H), 1.52 (d, J = 6.8 Hz, 3H), 1.07 (m, 3H). ¹³C NMR (126 MHz, CDCl₃) δ 168.76, 168.28, 151.30, 141.82, 140.78, 135.27, 130.60, 130.38, 128.68, 126.78, 121.76 (q, J = 287.3 Hz), 119.53, 116.26, 114.26, 93.88 (q, J = 33.8 Hz), 56.18, 53.97, 49.80, 40.45, 36.64, 17.81, 12.25 (carbon spectrum shows a mixture of rotamers). HRMS: Calculated for [C₂₂H₂₁Cl₂F₃N₂O₃S + H₂O + H]⁺ = 539.07804, found = 539.07794.

### Example 74: (±) 2-((2-Chloro-4-(4-(3-chlorophenyl)-trans-2,3-dimethylpiperazine-1-carbonyl)phenyl)sulfinyl)-1-(oxazol-2-yl)ethan-1-one

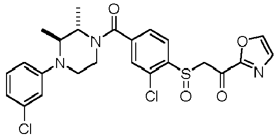

Step A: To a solution of *tert*-butyl 3-chloro-4-fluorobenzoate (0.510 g, 2.22 mmol, 1 eq.) in degassed DMF was added sodium methanethiolate (0.230 g, 3.32 mmol, 1.5 eq.) at -10 °C and the mixture was stirred at RT overnight. The reaction progress was monitored by TLC. Once completed, the mixture was diluted with Et₂O, washed with water, dried over anhydrous MgSO₄ and concentrated under reduced pressure. The residue was purified using column chromatography (Et₂O / pentane, 0-10%) to yield the product (0.24 g, 0.91 mmol, 41%). 1H NMR (400 MHz, CDCl3) δ 7.91 (d, J = 1.8 Hz, 1H), 7.85 (dd, J = 8.3, 1.8 Hz, 1H), 7.13 (d, J = 8.4 Hz, 1H), 2.49 (s, 3H), 1.59 (s, 9H). 13C NMR (400 MHz, CDCl3) δ 164.54, 143.80, 130.83, 129.94, 129.04, 128.05, 123.94, 81.49, 28.22, 14.92.
Step B: tert-butyl 3-chloro-4-(methylthio)benzoate (0.24 g, 0.91 mmol, 1 eq.) was used according to general procedure E. This yielded the product (0.290 g, 1.17 mmol, quant.). 1H NMR (500 MHz, CDCl3) δ 8.13 (dd, J = 8.0, 1.6 Hz, 1H), 8.03 - 7.98 (m, 2H), 2.86 (s, 3H), 1.62 (s, 9H). 13C NMR (500 MHz, CDCl3) δ 163.34, 147.97, 135.70, 130.48, 129.52, 128.73, 125.20, 82.24, 41.37, 27.94.
Step C: tert-butyl 3-chloro-4-(methylsulfinyl)benzoate (0.26 g, 0.94 mmol) was used according to general procedure F. This yielded the product (0.19 g, 0.87 mmol, 92% ). 1H NMR (400 MHz, MeOD) δ 8.22 (dd, J = 8.2, 1.6 Hz, 1H), 8.08 (d, J = 1.6 Hz, 1H), 7.98 (d, J = 8.1 Hz, 1H), 2.90 (s, 3H). 13C NMR (400 MHz, MeOD) δ 167.19, 148.93, 136.49, 132.03, 131.20, 130.45, 126.47, 41.68.
Step D: To a stirred suspension of 3-chloro-4-((2-ethoxy-2-oxoethyl)sulfinyl)benzoic acid (0.19 g, 0.87 mmol, 1 eq.) in DCM (10 ml) were added (±) trans-1-(3-chlorophenyl)-2,3-dimethylpiperazine (32, 0.230 g, 1.04 mmol, 1.2 eq.), DIPEA (0.34 mg, 2.59 mmol, 3 eq.), HOBt (0.18 mg, 1.30 mmol, 1.5 eq.) and EDCI (0.25 mg, 1.30 mmol, 1.5 eq.). The mixture was stirred at RT overnight. The reaction progress was monitored by TLC. Once completed, the mixture was washed with water and brine, dried (MgSO4), filtered and concentrated. The crude product was purified using column chromatography (EtOAc / pentane, 0%-60%) to yield the product (0.27 mg, 0.64 mmol, 74%). ¹H NMR (400 MHz, CDCl₃) δ 8.04 - 7.97 (m, 2H), 7.56 - 7.38 (m, 2H), 7.14 (t, J = 8.2 Hz, 1H).6.60 (d, J = 2.4 Hz, 1H), 6.56 - 6.47 (m, 1H), 4.85 - 4.49 (m, 1H), 3.98 - 3.07 (m, 5H), 2.82 (s, 3H), 1.42 - 1.33 (m, 3H), 1.28 - 1.06 (m, 3H). ¹³C NMR (126 MHz, CDCl₃) δ 168.83, 151.33, 145.25, 140.18, 135.19, 130.47, 130.33, 128.43, 127.77, 125.85, 119.34, 116.13, 114.19, 56.08, 49.57, 42.29, 41.64, 36.46, 17.76, 12.52 (carbon spectrum shows a mixture of rotamers).
Step E: The title compound was synthesized using (±) (3-chloro-4-(methylsulfinyl)phenyl)(trans-4-(3-chlorophenyl)-2,3-dimethylpiperazin-1-yl)methanone (65 mg, 0.15 mmol, 1 eq.) and ethyl oxazole-2-carboxylate (216 mg, 1.53 mmol, 10 eq) according to general procedure I. This yielded the product(3.2 mg, 0.15 mmol, 3%). ¹³C NMR (126 MHz, CDCl3) δ 178.05, 168.68, 168.22, 151.35, 142.95, 142.25, 140.76, 135.28, 131.01, 130.37, 129.97, 128.58, 126.92, 126.05, 119.49, 116.25, 114.25, 61.04, 60.50, 49.66, 42.35, 36.53, 14.29, 12.58. HRMS: Calculated for [C₂₄H₂₃Cl₂N₃O₄S + H]+ = 520.0859, found = 520.0857.

### Example 75: (±) 2-((2-Chloro-4-(4-(3-chlorophenyl)-trans-2,3-dimethylpiperazine-1-carbonyl)phenyl)sulfinyl)-1-(thiazol-2-yl)ethan-1-one

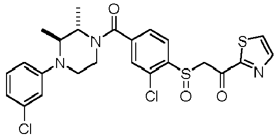

Step A: To a solution of *tert*-butyl 3-chloro-4-fluorobenzoate (0.510 g, 2.22 mmol, 1 eq.) in degassed DMF was added sodium methanethiolate (0.230 g, 3.32 mmol, 1.5 eq.) at -10 °C and the mixture was stirred at RT overnight. The reaction progress was monitored by TLC. Once completed, the mixture was diluted with Et₂O, washed with water, dried over anhydrous MgSO₄ and concentrated under reduced pressure. The residue was purified using column chromatography (Et₂O / pentane, 0-10%) to yield the product (0.24 g, 0.91 mmol, 41%). 1H NMR (400 MHz, CDCl3) δ 7.91 (d, J = 1.8 Hz, 1H), 7.85 (dd, J = 8.3, 1.8 Hz, 1H), 7.13 (d, J = 8.4 Hz, 1H), 2.49 (s, 3H), 1.59 (s, 9H). 13C NMR (400 MHz, CDCl3) δ 164.54, 143.80, 130.83, 129.94, 129.04, 128.05, 123.94, 81.49, 28.22, 14.92.
Step B: tert-butyl 3-chloro-4-(methylthio)benzoate (0.24 g, 0.91 mmol, 1 eq.) was used according to general procedure E. This yielded the product (0.290 g, 1.17 mmol, quant.). 1H NMR (500 MHz, CDCl3) δ 8.13 (dd, J = 8.0, 1.6 Hz, 1H), 8.03 - 7.98 (m, 2H), 2.86 (s, 3H), 1.62 (s, 9H). 13C NMR (500 MHz, CDCl3) δ 163.34, 147.97, 135.70, 130.48, 129.52, 128.73, 125.20, 82.24, 41.37, 27.94.
Step C: tert-butyl 3-chloro-4-(methylsulfinyl)benzoate (0.26 g, 0.94 mmol) was used according to general procedure F. This yielded the product (0.19 g, 0.87 mmol, 92% ). 1H NMR (400 MHz, MeOD) δ 8.22 (dd, J = 8.2, 1.6 Hz, 1H), 8.08 (d, J = 1.6 Hz, 1H), 7.98 (d, J = 8.1 Hz, 1H), 2.90 (s, 3H). 13C NMR (400 MHz, MeOD) δ 167.19, 148.93, 136.49, 132.03, 131.20, 130.45, 126.47, 41.68.
Step D: To a stirred suspension of 3-chloro-4-((2-ethoxy-2-oxoethyl)sulfinyl)benzoic acid (0.19 g, 0.87 mmol, 1 eq.) in DCM (10 ml) were added (±) trans-1-(3-chlorophenyl)-2,3-dimethylpiperazine (32, 0.230 g, 1.04 mmol, 1.2 eq.), DIPEA (0.34 mg, 2.59 mmol, 3 eq.), HOBt (0.18 mg, 1.30 mmol, 1.5 eq.) and EDCI (0.25 mg, 1.30 mmol, 1.5 eq.). The mixture was stirred at RT overnight. The reaction progress was monitored by TLC. Once completed, the mixture was washed with water and brine, dried (MgSO4), filtered and concentrated. The crude product was purified using column chromatography (EtOAc / pentane, 0%-60%) to yield the product (0.27 mg, 0.64 mmol, 74%). ¹H NMR (400 MHz, CDCl₃) δ 8.04 - 7.97 (m, 2H), 7.56 - 7.38 (m, 2H), 7.14 (t, J = 8.2 Hz, 1 H).6.60 (d, J = 2.4 Hz, 1H), 6.56 - 6.47 (m, 1H), 4.85 - 4.49 (m, 1H), 3.98 - 3.07 (m, 5H), 2.82 (s, 3H), 1.42 - 1.33 (m, 3H), 1.28 - 1.06 (m, 3H). ¹³C NMR (126 MHz, CDCl₃) δ 168.83, 151.33, 145.25, 140.18, 135.19, 130.47, 130.33, 128.43, 127.77, 125.85, 119.34, 116.13, 114.19, 56.08, 49.57, 42.29, 41.64, 36.46, 17.76, 12.52 (carbon spectrum shows a mixture of rotamers).
Step E: The title compound was synthesized using (±) (3-chloro-4-(methylsulfinyl)phenyl)(trans-4-(3-chlorophenyl)-2,3-dimethylpiperazin-1-yl)methanone (40 mg, 0.09 mmol, 1 eq.) and ethyl thiazole-2-carboxylate (148 mg, 0.94 mmol, 10 eq) according to general procedure I. This yielded the product (32 mg, 0.06 mmol, 63%). 1H NMR (600 MHz, CDCl3) δ 8.02 - 7.95 (m, 2H), 7.76 (dd, J = 3.0, 1.4 Hz, 1H), 7.53 - 7.44 (m, 2H), 7.18 (t, J = 8.1 Hz, 1H), 6.84 - 6.79 (m, 2H), 6.73 (d, 1H), 4.83 - 4.68 (m, 2H), 3.95 - 3.11 (m, 6H), 1.50 (d, J = 6.8 Hz, 3H), 1.16 - 0.97 (m, 3H). 13C NMR (151 MHz, CDCl3) δ 184.64, 166.56, 152.19, 146.31, 143.48, 141.28, 136.27, 132.04, 132.00, 131.32, 128.93, 127.99, 127.03, 125.25, 120.60, 117.30, 115.26, 61.84, 61.79, 57.22, 56.24, 30.72, 13.80, 1.02. HRMS: Calculated for[C₂₄H₂₃Cl₂N₃O₃S₂ + H]+ = 538.0600, found = 538.0600.

### Example 76: (±) 2-((2-Chloro-4-(4-(3-chlorophenyl)-trans-2,3-dimethylpiperazine-1-carbonyl)phenyl)sulfinyl)-1-(5-methyloxazol-2-yl)ethan-1-one

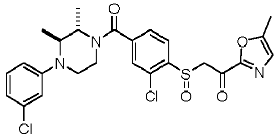

The title compound was synthesized using (±) (3-chloro-4-(methylsulfinyl)phenyl)(trans-4-(3-chlorophenyl)-2,3-dimethylpiperazin-1-yl)methanone ( 60 mg, 0.14 mmol, 1 eq.) and ethyl 5-methyloxazole-2-carboxylate (44 mg, 0.28 mmol, 2.00 eq) according to general procedure I. This yielded the product (15 mg, 0.03 mmol, 20%). ¹H NMR (400 MHz, Chloroform-d) δ 7.98 (d, J = 8.0 Hz, 1H), 7.54-7.44 (m, 2H), 7.18 (t, J = 7.7 Hz, 1H), 7.01 - 6.98 (m, 1H), 6.84 - 6.80 (m, 2H), 6.72 (d, J = 8.4 Hz, 1H), 4.87-3.05 (m, 8H), 2.44 (s, 3H), 1.53 - 1.46 (m, 3H), 1.08 (dd, J = 44.8, 6.6 Hz, 3H). HRMS: calculated for [C₂₅H₂₅Cl₂N₃O₄S + H]⁺ = 534.10156, found: 534.10166.

### Example 77: (±) 3-((2-Chloro-4-(4-(3-chlorophenyl)-trans-2,3-dimethylpiperazine-1-carbonyl)phenyl)sulfinyl)-1,1-difluoropropan-2-one

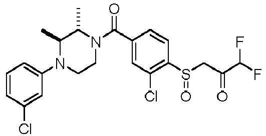

The title compound was synthesized using (±) (3-chloro-4-(methylsulfinyl)phenyl)(trans-4-(3-chlorophenyl)-2,3-dimethylpiperazin-1-yl)methanone (48 mg, 0.11 mmol, 1 eq.) and ethyl 2,2-difluoroacetate (0.024 mL, 0.23 mmol, 2.00 eq) according to general procedure I. This yielded the product (6 mg, 0.012 mmol, 10%). ¹H NMR (500 MHz, Chloroform-d) δ 8.00 (dd, J = 62.7, 8.0 Hz, 1H), 7.61 - 7.55 (m, 1H), 7.50 (d, J = 13.1 Hz, 1H), 7.18 (t, J = 8.1 Hz, 1H), 6.83 (d, J = 8.0 Hz, 2H), 6.73 (d, J = 8.3 Hz, 1H), 5.81 (t, J = 53.5 Hz, 1H), 4.54 - 4.47 (m, 1 H), 4.05 - 3.98 (m, 1 H), 3.82 - 3.12 (m, 6H), 1.50 (d, J = 6.7 Hz, 3H), 1.08 (br, 3H).

### Example 78: (±) 1-((2-Chloro-4-(4-(3-chlorophenyl)-trans-2,3-dimethylpiperazine-1-carbonyl)phenyl)sulfinyl)-3,3-difluorobutan-2-one

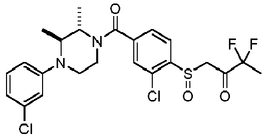

The title compound was synthesized using (±) (3-chloro-4-(methylsulfinyl)phenyl)(trans-4-(3-chlorophenyl)-2,3-dimethylpiperazin-1-yl)methanone (25 mg, 0.06 mmol, 1 eq.) and ethyl 2,2-difluoropropanoate (81 mg, 0.59 mmol, 10 eq) according to general procedure I. This yielded the product (4.9 mg, 0.06 mmol, 16%). 1H NMR (500 MHz, CDCl3) δ 8.06 (dd, J = 18.3, 8.0 Hz, 1H), 7.63 - 7.49 (m, 2H), 7.20 (t, J = 8.0 Hz, 1H), 6.88 - 6.82 (m, 2H), 6.76 (d, J = 8.4 Hz, 1H), 4.44 (dd, J = 15.1, 4.4 Hz, 1H), 4.07 (dd, J = 15.1, 3.4 Hz, 1H), 3.85-3.10 (m, 6H), 1.78 (t, J = 19.3 Hz, 3H), 1.52 (d, J = 6.8 Hz, 3H), 1.10 (s, 3H). 13C NMR (101 MHz, CDCl3) δ 168.67, 168.19, 151.36, 142.33, 140.83, 135.32, 130.67, 130.40, 128.68, 126.98, 126.52, 119.56, 117.31 (t, J = 249 Hz), 116.30, 114.28, 59.45, 56.22, 49.72, 40.52, 36.58, 18.73 (t, J = 24 Hz), 17.87, 12.60 (carbon spectrum shows a mixture of rotamers). HRMS: Calculated for [C₂₃H₂₄Cl₂F₂N₂O₃S + H₂O + H]+ = 535.1031, found = 535.1027.

### Example 79: (±) 1-((2-Chloro-4-(4-(3-chlorophenyl)-trans-2,3-dimethylpiperazine-1-carbonyl)phenyl)sulfinyl)-3,3-difluoropentan-2-one

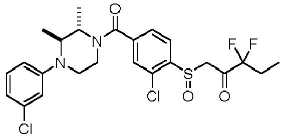

The title compound was synthesized using (±) (3-chloro-4-(methylsulfinyl)phenyl)(trans-4-(3-chlorophenyl)-2,3-dimethylpiperazin-1-yl)methanone (20 mg, 0.05 mmol, 1 eq.) and ethyl 2,2-difluorobutanoate (72 mg, 0.47 mmol, 10 eq) according to general procedure I. This yielded the product (2.5 mg, 0.05 mmol, 10%). 1H NMR (500 MHz, CDCl3) δ 8.04 (dd, J = 8.1, 4.5 Hz, 1H), 7.62 - 7.49 (m, 2H), 7.22 (t, J = 8.3 Hz, 1H), 6.89 (d, J = 7.2 Hz, 2H), 6.81 (d, J = 8.4 Hz, 1H), 4.41 (dd, J = 15.1, 4.3 Hz, 1H), 4.05 (dd, J = 15.2, 3.4 Hz, 1H), 3.85 - 3.13 (m, 8H), 2.17 - 2.02 (m, 3H), 1.54 (d, J = 6.8 Hz, 3H), 1.08 (t, J = 7.5 Hz, 3H). 13C NMR (101 MHz, CDCl3) δ 168.65, 168.17, 151.36, 142. 26, 140.93, 135.33, 130.65, 130.40, 128.67, 127.08, 126.51, 119.55, 118.14 (t, J = 253 Hz), 116.30, 114.28, 60.15, 56.22, 49.70, 40.52, 36.57, 25.52 (t, J = 23 Hz), 17.86, 12.60, 5.47 (t, J = 5 Hz) (carbon spectrum shows a mixture of rotamers). HRMS: Calculated for [C₂₄H₂₆Cl₂F₂N₂O₃S + H₂O + H]⁺ = 549.1188, found = 549.1183.

### Example 80: (±) 3-((2-Chloro-4-(4-(3-chlorophenyl)-trans-2,3-dimethylpiperazine-1-carbonyl)phenyl)sulfinyl)-1,1-difluoro-1-phenylpropan-2-one

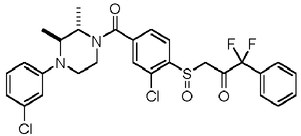

The title compound was synthesized using (±) (3-chloro-4-(methylsulfinyl)phenyl)(trans-4-(3-chlorophenyl)-2,3-dimethylpiperazin-1-yl)methanone (40 mg, 0.09 mmol, 1 eq.) and ethyl 2,2-difluoro-2-phenylacetate (188 mg, 0.94 mmol, 10 eq) according to general procedure I. This yielded the product(13.4 mg, 0.02 mmol, 25%). 1H NMR (600 MHz, CDCl3) δ 7.95 (d, J = 7.9 Hz, 1H), 7.63 - 7.42 (m, 7H), 7.17 (t, J = 8.1, 2.4 Hz, 1H), 6.85 - 6.78 (m, 2H), 6.74 - 6.68 (m, 1H), 4.69 - 4.32 (m, 1H), 4.07 - 3.01 (m, 5H), 1.54 - 1.41 (m, 3H), 1.17 - 0.97 (m, 3H). 13C NMR (151 MHz, CDCl3) δ 191.69, 191.44, 152.19, 143.10, 141.58, 136.27, 132.65 (t), 131.65, 131.31, 131.18, 130.07, 129.20, 128.43, 128.03, 126.86 (t), 120.59, 117.27, 116.39 (t), 115.24, 61.07, 61.04, 57.17, 56.53, 30.71, 13.74, 1.01. HRMS: Calculated for [C₂₈H₂₆Cl₂F₂N₂O₃S + H₂O + H]⁺ = 597.1188, found = 597.1189.

### Example 81: (±) 1-((2-chloro-4-(4-(6-chloropyridin-2-yl)-trans-2,3-dimethylpiperazine-1-carbonyl)phenyl)sulfinyl)-3,3-difluorobutan-2-one

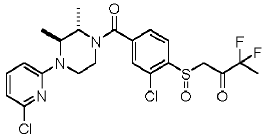

Step A: trans-1-(6-chloropyridin-2-yl)-2,3-dimethylpiperazine (70 mg, 0.31 mmol, 1.2 eq) and 3-chloro-4-(methylsulfinyl)benzoic acid (57 mg, 0.26 mmol, 1 eq) were used according to general procedure H. This yielded the product (86 mg, 0.20 mmol, 78 %). ¹H NMR (500 MHz, Chloroform-d) δ 8.04 (dd, J = 8.0, 3.8 Hz, 1H), 7.66 - 7.37 (m, 3H), 6.63 (dd, J = 7.5, 3.7 Hz, 1H), 6.48 (dd, J = 15.7, 8.4 Hz, 1H), 4.84-3.15 (m, 6H), 2.86 (d, J = 1.9 Hz, 3H), 1.47 - 1.04 (m, 6H). ¹³C NMR (126 MHz, Chloroform-d) δ 168.83, 158.60, 149.60, 145.35, 140.04, 130.46, 128.43, 127.74, 126.38, 125.85, 112.59, 104.46, 52.20, 42.10, 41.61, 36.28, 29.72, 17.74, 14.28.
Step B: The title compound was synthesized using (±) (3-chloro-4-(methylsulfinyl)phenyl)(4-(6-chloropyridin-2-yl)-trans-2,3-dimethylpiperazin-1-yl)methanone (33 mg, 0.08 mmol, 1.0 eq) and ethyl 2,2-difluoropropanoate (107 mg, 0.77 mmol, 10 eq ) according to general procedure I. This yielded the product (18 mg, 0.04 mmol, 45 %). HRMS: Calculated for [C₂₂H₂₃Cl₂F₂N₃O₃S + H₂O + H]⁺ = 536.09837, found = 536.09844.

### Example 82: (±) 1-((2-Chloro-4-(4-(6-chloropyridin-2-yl)-trans-2,3-dimethylpiperazine-1-carbonyl)phenyl)sulfinyl)-3,3-difluoropentan-2-one

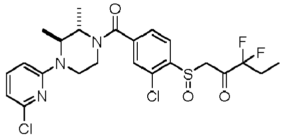

Step A: trans-1-(6-chloropyridin-2-yl)-2,3-dimethylpiperazine (70 mg, 0.31 mmol, 1.2 eq) and 3-chloro-4-(methylsulfinyl)benzoic acid (57 mg, 0.26 mmol, 1 eq) were used according to general procedure H. This yielded the product (86 mg, 0.20 mmol, 78 %). ¹H NMR (500 MHz, Chloroform-d) δ 8.04 (dd, J = 8.0, 3.8 Hz, 1H), 7.66 - 7.37 (m, 3H), 6.63 (dd, J = 7.5, 3.7 Hz, 1H), 6.48 (dd, J = 15.7, 8.4 Hz, 1H), 4.84-3.15 (m, 6H), 2.86 (d, J = 1.9 Hz, 3H), 1.47 - 1.04 (m, 6H). ¹³C NMR (126 MHz, Chloroform-d) δ 168.83, 158.60, 149.60, 145.35, 140.04, 130.46, 128.43, 127.74, 126.38, 125.85, 112.59, 104.46, 52.20, 42.10, 41.61, 36.28, 29.72, 17.74, 14.28
Step B: The title compound was synthesized using (±) (3-chloro-4-(methylsulfinyl)phenyl)(4-(6-chloropyridin-2-yl)-trans-2,3-dimethylpiperazin-1-yl)methanone (33 mg, 0.08 mmol, 1.0 eq) and ethyl 2,2-difluorobutanoate (118 mg, 0.77 mmol, 10 eq) according to general procedure I. This yielded the product (18 mg, 0.02 mmol, 29 %). HRMS: Calculated for [C₂₃H₂₅Cl₂F₂N₃O₃S + H₂O + H]⁺ = 550.11402, found = 550.11405.

### Example 83: (±) 1-((2-Chloro-4-(4-(4-chloropyridin-2-yl)-trans-2,3-dimethylpiperazine-1-carbonyl)phenyl)sulfinyl)-3,3-difluorobutan-2-one

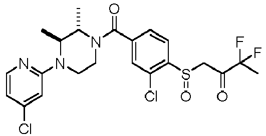

Step A: trans-1-(6-chloropyridin-2-yl)-2,3-dimethylpiperazine (40 mg, 0.18 mmol, 1.2 eq ) and 3-chloro-4-(methylsulfinyl)benzoic acid (32 mg, 0.15 mmol, 1 eq) were used according to general procedure H. This yielded the product (56 mg, 0.13 mmol, 89 %). ¹H NMR (500 MHz, Chloroform-d) δ 8.40 - 7.81 (m, 2H), 7.70 - 7.33 (m, 2H), 6.94 - 6.57 (m, 2H), 5.11 - 3.06 (m, 6H), 2.89 (d, J = 4.4 Hz, 3H), 1.44-1.19 (m, 6H). ¹³C NMR (126 MHz, Chloroform-d) δ 169.10, 156.72, 149.65, 145.50, 144.74, 139.58, 130.74, 128.58, 126.49, 126.11, 114.61, 109.18, 53.64, 49.32, 41.53, 39.62, 36.02, 17.63, 15.48.
Step B: The title compound was synthesized using (±) (3-chloro-4-(methylsulfinyl)phenyl)(4-(4-chloropyridin-2-yl)-trans-2,3-dimethylpiperazin-1-yl)methanone (55 mg, 0.13 mmol, 1.0 eq) and ethyl 2,2-difluoropropanoate (89 mg, 0.65 mmol, 5 eq) according to general procedure I. This yielded the product (26 mg, 0.05 mmol, 39 %). HRMS: Calculated for [C₂₂H₂₃Cl₂F₂N₃O₃S + H₂O + H]⁺ = 536.09837, found = 536.09828.

### Example 84: (±) 1-((2-Chloro-4-(4-(4-chloropyridin-2-yl)-trans-2,3-dimethylpiperazine-1-carbonyl)phenyl)sulfinyl)-3,3-difluoropentan-2-one

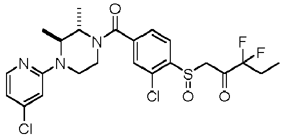

Step A: trans-1-(6-chloropyridin-2-yl)-2,3-dimethylpiperazine (40 mg, 0.18 mmol, 1.2 eq) and 3-chloro-4-(methylsulfinyl)benzoic acid (32 mg, 0.15 mmol, 1 eq) were used according to general procedure H. This yielded the product (56 mg, 0.13 mmol, 89 %). ¹H NMR (500 MHz, Chloroform-d) δ 8.40 - 7.81 (m, 2H), 7.70 - 7.33 (m, 2H), 6.94 - 6.57 (m, 2H), 5.11 - 3.06 (m, 6H), 2.89 (d, J = 4.4 Hz, 3H), 1.44-1.19 (m, 6H). ¹³C NMR (126 MHz, Chloroform-d) δ 169.10, 156.72, 149.65, 145.50, 144.74, 139.58, 130.74, 128.58, 126.49, 126.11, 114.61, 109.18, 53.64, 49.32, 41.53, 39.62, 36.02, 17.63, 15.48.
Step B: The title compound was synthesized using (±) (3-chloro-4-(methylsulfinyl)phenyl)(4-(4-chloropyridin-2-yl)-trans-2,3-dimethylpiperazin-1-yl)methanone (55 mg, 0.13 mmol, 1.0 eq) and ethyl 2,2-difluorobutanoate (98 mg, 0.65 mmol, 5 eq) according to general procedure I. This yielded the product (29 mg, 0.05 mmol, 42 %). LC/MS: Calculated for [C₂₃H₂₅Cl₂F₂N₃O₃S + H₂O + H]⁺ = 550.11, found = 549.76.

### Example 85: (±) 2-((2-Chloro-4-(4-(3-chlorophenyl)-trans-2,3-dimethylpiperazine-1-carbonyl)phenyl)sulfinyl)-1-(pyridin-3-yl)ethan-1-one

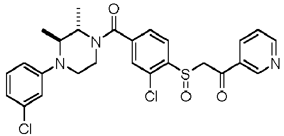

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl nicotinate (178 mg, 1.18 mmol, 10 eq) according to general procedure I. This yielded the product (5 mg, 0.01 mmol, 8%). 1H NMR (400 MHz, Chloroform-d) δ 9.41 (s, 1H), 8.99 (d, J = 5.0 Hz, 1H), 8.80 (m, 1H), 7.98 (s, 1H), 7.79 (d, J = 7.6 Hz, 1H), 7.53 (d, J = 8.8 Hz, 2H), 7.19 (t, J = 8.3 Hz, 1H), 6.85 (d, J = 6.5 Hz, 2H), 6.76 (d, J = 8.4 Hz, 1H), 4.92 (d, J = 13.0 Hz, 1H), 4.47 (d, J = 13.2 Hz, 1H), 3.88-3.13 (br, 6H), 1.52 (d, J = 6.5 Hz, 3H), 1.09 (br, 3H). HRMS: calculated for [C₂₆H₂₅Cl₂N₃O₃S + H]⁺= 530.10664, found: 530.10688.

### Example 86: (±) 2-((2-Chloro-4-(4-(3-chlorophenyl)-trans-2,3-dimethylpiperazine-1-carbonyl)phenyl)sulfinyl)-1-(4-fluorophenyl)ethan-1-one

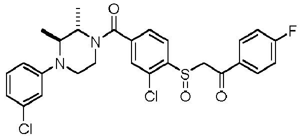

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 4-fluorobenzoate (198 mg, 1.18 mmol, 10.00 eq) according to general procedure I. This yielded the product (17 mg, 0.03 mmol, 26%). 1H NMR (400 MHz, Chloroform-d) δ 7.98 (dd, J = 5.0, 1.8 Hz, 1H), 7.97 (s, 1H), 7.95 (d, J = 3.0 Hz, 1H), 7.54 - 7.47 (m, 2H), 7.21 - 7.15 (m, 3H), 6.85 - 6.80 (m, 2H), 6.75 - 6.70 (dd, 1H), 4.64 - 4.60 (m, 1H), 4.35 (dd, J = 14.3, 1.9 Hz, 1H), 4.09 - 2.97 (m, 6H), 1.51 (d, J = 7.2 Hz, 3H), 1.07 (m, 3H). HRMS: calculated for [C₂₇H₂₅Cl₂FN₂O₃S+ H]⁺= 547.10197, found: 547.10181.

### Example 87: (±) 2-((2-Chloro-4-(4-(3-chlorophenyl)-trans-2,3-dimethylpiperazine-1-carbonyl)phenyl)sulfinyl)-1-(3-fluorophenyl)ethan-1-one

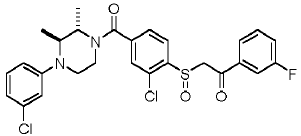

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 3-fluorobenzoate (198 mg, 1.18 mmol, 10.00 eq) according to general procedure I. This yielded the product (8.0 mg, 0.002 mmol, 12%). 1H NMR (400 MHz, Chloroform-d) δ 7.95 (d, J = 8.2 Hz, 1H), 7.72 (d, J = 7.8 Hz, 1H), 7.62 (d, J = 9.1 Hz, 1H), 7.54 - 7.46 (m, 3H), 7.34 (t, J = 8.2 Hz, 1H), 7.19 (t, J = 7.8 Hz, 1H), 6.82 (d, J = 7.5 Hz, 2H), 6.72 (d, J = 8.1 Hz, 1H), 4.67 - 4.57 (m, 1H), 4.33 (d, J = 14.3 Hz, 1H), 3.92 - 3.12 (br, 6H), 1.54 -1.45 (m, 3H), 1.16 - 1.00 (m, 3H). HRMS: calculated for [C₂₇H₂₅Cl₂FN₂O₃S+ H]⁺ = 547.10197, found: 547.10189.

### Example 88: (±) 2-((2-Chloro-4-(4-(3-chlorophenyl)-trans-2,3-dimethylpiperazine-1-carbonyl)phenyl)sulfinyl)-1-(pyridazin-3-yl)ethan-1-one

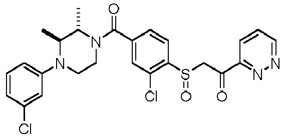

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl pyridazine-3-carboxylate (179 mg, 1.18 mmol, 10 eq) according to general procedure I. This yielded the product (13 mg, 0.024 mmol, 20%). ¹H NMR (400 MHz, Chloroform-d) δ 9.37 (d, J = 4.9 Hz, 1H), 8.21 (d, J = 8.5 Hz, 1H), 7.97 (d, J = 8.0 Hz, 1H), 7.75 (dd, J = 8.5, 5.0 Hz, 1H), 7.50 (d, J = 7.9 Hz, 1H), 7.46 (s, 1H), 7.21 (d, J = 8.3 Hz, 1H), 6.86 (d, J = 6.2 Hz, 2H), 6.77 (d, J = 6.3 Hz, 1H), 5.07 - 4.96 (m, 2H), 3.83 - 3.15 (br, 6H), 1.52 (d, J = 6.8 Hz, 3H), 1.09 (d, J = 6.2 Hz, 3H). HRMS: calculated for [C₂₅H₂₄Cl₂N₄O₃S + H]⁺= 531.10189, found: 531.10199.

### Example 89: (±) 2-((2-Chloro-4-(4-(3-chlorophenyl)-trans-2,3-dimethylpiperazine-1-carbonyl)phenyl)sulfinyl)-1-(3,5-difluorophenyl)ethan-1-one

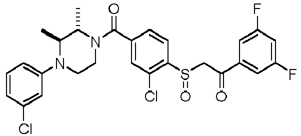

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 3,5-difluorobenzoate (219 mg, 1.175 mmol, 10 eq) according to general procedure I. This yielded the product (21 mg, 0.037 mmol, 32%). ¹H NMR (400 MHz, Chloroform-d) δ 7.94 (dd, J = 8.0, 1.2 Hz, 1H), 7.46 (dd, J = 7.5, 2.1 Hz, 2H), 7.18 (t, J = 8.2 Hz, 1H), 7.12 (dt, J = 8.3, 2.3 Hz, 2H), 7.09 (t, J = 2.3 Hz, 1H), 6.85-6.79 (m, 2H), 6.73 (d, J = 9.2 Hz, 1H), 4.60 (dd, J = 14.1, 2.8 Hz, 1H), 4.30 (dd, J = 14.1, 2.8 Hz, 1H), 3.82 - 2.53 (br, 6H), 1.50 (d, J = 6.5 Hz, 3H), 1.09 (d, J = 34.8 Hz, 3H). HRMS: calculated for [C₂₇H₂₄Cl₂F₂N₂O₃S+H]⁺ = 565.09255, found: 565.09263.

### Example 90: (±) 2-((2-Chloro-4-(4-(3-chlorophenyl)-trans-2,3-dimethylpiperazine-1-carbonyl)phenyl)sulfinyl)-1-(6-(trifluoromethyl)pyridin-3-yl)ethan-1-one

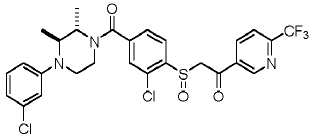

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl 6-(trifluoromethyl)nicotinate (258 mg, 1.18 mmol, 10 eq) according to general procedure I. This yielded the product (4.7 mg, 0.00785 mmol, 7%). 1H NMR (600 MHz, Chloroform-d) δ 9.20 (s, 1H), 8.45 (d, J = 8.1 Hz, 1H), 7.86 (t, J = 8.3 Hz, 2H), 7.53 (d, J = 7.6 Hz, 2H), 7.20 (t, J = 8.3 Hz, 1H), 6.85 (s, 2H), 6.77 (d, J = 8.2 Hz, 1H), 4.74 (dd, J = 13.7, 4.0 Hz, 1H), 4.36 (dd, J = 13.7, 4.6 Hz, 1H), 3.83-3.16 (br, 6H), 1.52 (d, J = 6.7 Hz, 3H), 1.09 (br, 3H). HRMS: calculated for [C₂₇H₂₄Cl₂F₃N₃O₃S +H]⁺= 598.09403, found: 598.09417.

### Example 91: (±) 1-(4-Chloro-3-(trifluoromethyl)phenyl)-2-((2-chloro-4-(4-(3-chlorophenyl)-trans-2,3-dimethyl-piperazine-1-carbonyl)-phenyl)sulfinyl)ethan-1-one

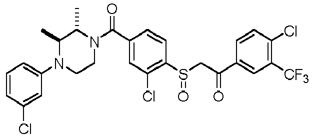

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 4-chloro-3-(trifluoromethyl)benzoate (297 mg, 1.175 mmol, 10 eq) according to general procedure I. This yielded the product (5 mg, 0.008 mmol, 7%). 1H NMR (400 MHz, Chloroform-d) δ 8.25 (s, 1H), 8.07 (dd, J = 8.4, 1.9 Hz, 1H), 7.90 (d, J = 8.4 Hz, 1H), 7.67 (d, J = 8.4 Hz, 1H), 7.52 (d, J = 8.2 Hz, 2H), 7.18 (t, J = 8.1 Hz, 1H), 6.83 (d, J = 8.6 Hz, 2H), 6.73 (d, J = 8.9 Hz, 1H), 4.66 (dd, J = 13.9, 3.2 Hz, 1H), 4.32 (dd, J = 14.0, 3.5 Hz, 1H), 3.92 - 2.96 (br, 6H), 1.50 (d, J = 6.6 Hz, 3H), 1.05 (br, 3H). HRMS: calculated for [C₂₇H₂₄Cl₂F₃N₃O₃S + H]⁺= 631.05981, found: 631.05979.

### Example 92: (±) 2-((2-Chloro-4-(4-(3-chlorophenyl)-trans-2,3-dimethylpiperazine-1-carbonyl)phenyl)sulfinyl)-1-(4-fluoro-3-(trifluoromethyl)phenyl)ethan-1-one

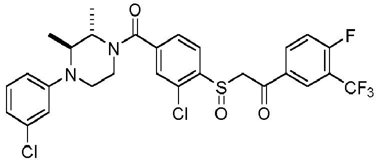

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl 4-fluoro-3-(trifluoromethyl)benzoate (278 mg, 1.175 mmol, 10 eq) according to general procedure I. ¹H NMR (400 MHz, Chloroform-d) δ 8.22 (d, J = 6.9 Hz, 1H), 8.19 (d, J = 4.9 Hz, 1H), 7.91 (d, J = 8.2 Hz, 1H), 7.52 (d, J = 9.0 Hz, 2H), 7.36 (t, J = 9.1 Hz, 1H), 7.19 (t, J = 8.1 Hz, 1H), 6.85 (d, J = 9.2 Hz, 2H), 6.75 (d, J = 7.4 Hz, 1H), 4.66 (dd, J = 14.0, 2.5 Hz, 1H), 4.32 (dd, J = 14.0, 2.3 Hz, 1H), 3.24 (br m, 6H), 1.51 (dd, J = 6.8, 2.3 Hz, 3H), 1.08 (br s, 3H). HRMS: calculated for [C₂₈H₂₄Cl₂F₄N₂O₃S+ H]+= 615.08936, found: 615.08925.

### Example 93: (±) 2-((2-Chloro-4-(4-(3-chlorophenyl)-trans-2,3-dimethylpiperazine-1-carbonyl)phenyl)sulfinyl)-1-(2,3-difluorophenyl)ethan-1-one

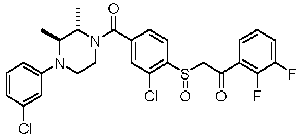

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 2,3-difluorobenzoate (278 mg, 1.175 mmol, 10 eq) according to general procedure I. This yielded the product (26 mg, 0.046 mmol, 39%). 1H NMR (400 MHz, Chloroform-d) δ 7.99 (dd, J = 8.0, 2.2 Hz, 1H), 7.72 - 7.66 (t, 1H), 7.54 (dd, J = 8.0, 1.4 Hz, 1H), 7.50 (s, 1H), 7.44 (d, J = 8.0 Hz, 1H), 7.26 - 7.22 (dd, 1H), 7.19 (t, J = 8.3 Hz, 1H), 6.84 (d, J = 6.6 Hz, 2H), 6.74 (d, J = 9.2 Hz, 1H), 4.70 (dt, J = 15.3, 2.7 Hz, 1H), 4.39 (dt, J = 15.3, 2.7 Hz, 1H), 4.06 - 2.73 (br, 6H), 1.51 (d, J = 6.7 Hz, 3H), 1.08 (br, 3H). HRMS: calculated for [C₂₇H₂₄Cl₂F₂N₂O₃S+ H]⁺= 565.09255, found: 565.09257.

### Example 94: (±) 2-((2-Chloro-4-(4-(3-chlorophenyl)-trans-2,3-dimethylpiperazine-1-carbonyl)phenyl)sulfinyl)-1-(3,4-difluorophenyl)ethan-1-one

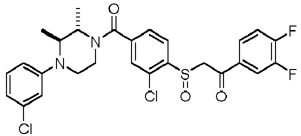

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 3,4-difluorobenzoate (278 mg, 1.175 mmol, 10 eq) according to general procedure I. This yielded the product (18.0 mg, 0.032 mmol, 27%). 1H NMR (400 MHz, Chloroform-d) δ 7.95 (d, J = 7.9 Hz, 1H), 7.81 (t, J = 8.0 Hz, 1H), 7.76 (d, J = 6.3 Hz, 1H), 7.54 (d, J = 11.3 Hz, 2H), 7.36 - 7.30 (br, 1H), 7.21 (t, J = 8.1 Hz, 1H), 6.86 (d, J = 8.7 Hz, 2H), 6.76 (d, J = 8.8 Hz, 1H), 4.63 (dd, J = 14.2, 2.5 Hz, 1H), 4.34 (dd, J = 14.2, 2.2 Hz, 1H), 4.00 - 2.99 (br, 6H), 1.54 (d, J = 6.7 Hz, 3H), 1.10 (br, 3H). HRMS: calculated for [C₂₇H₂₄Cl₂F₂N₂O₃S + H]⁺= 565.09255, found: 565.09250.

### Example 95: (±) 2-((2-Chloro-4-(4-(3-chlorophenyl)-trans-2,3-dimethylpiperazine-1-carbonyl)phenyl)sulfinyl)-1-(2-fluorophenyl)ethan-1-one

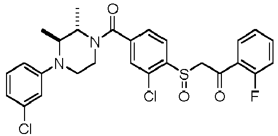

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl 2-fluorobenzoate (198 mg, 1.175 mmol, 10.00 eq) according to general procedure I. This yielded the product (5.5 mg, 0.001 mmol, 9%). 1H NMR (600 MHz, Chloroform-d) δ 8.01 (d, J = 7.9 Hz, 1H), 7.96 (t, J = 8.5 Hz, 1H), 7.61 (d, J = 6.2 Hz, 1H), 7.53 (d, J = 7.9 Hz, 1H), 7.48 (d, J = 7.6 Hz, 1H), 7.30 (d, J = 7.4 Hz, 1H), 7.21 - 7.11 (m, 2H), 6.98 (d, J = 10.9 Hz, 1H), 6.84 (d, J = 7.4 Hz, 1H), 6.75 (d, J = 8.3 Hz, 1H), 4.68 (d, J = 22.3 Hz, 1H), 4.37 (dt, J = 14.7, 2.8 Hz, 1H), 3.91 - 3.17 (br, 6H), 1.51 (d, J = 6.8 Hz, 3H), 1.08 (br, 3H). HRMS: calculated for [C₂₇H₂₅Cl₂FN₂O₃S + H]⁺= 547.10197, found: 547.10239.

### Example 96: (±) 2-((2-Chloro-4-(4-(3-chlorophenyl)-trans-2,3-dimethylpiperazine-1-carbonyl)phenyl)sulfinyl)-1-(pyridazin-4-yl)ethan-1-one

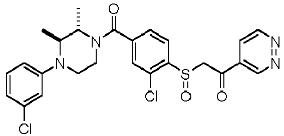

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and methyl pyridazine-4-carboxylate (162 mg, 1.175 mmol, 10 eq) according to general procedure I. This yielded the product (42 mg, 0.079 mmol, 67%). 1H NMR (400 MHz, Chloroform-d) δ 9.53 (d, J = 4.2 Hz, 2H), 7.97 (dt, J = 5.2, 2.3 Hz, 1H), 7.78 (d, J = 7.9 Hz, 1H), 7.54 - 7.47 (m, 2H), 7.19 (t, J = 8.3 Hz, 1H), 6.84 (d, J = 7.4 Hz, 2H), 6.75 (d, J = 9.2 Hz, 1H), 4.74 (dd, J = 13.5, 2.3 Hz, 1H), 4.42 (dd, J = 13.5, 3.4 Hz, 1H), 3.97 - 3.00 (br, 6H), 1.51 (d, J = 6.7 Hz, 3H), 1.08 (br, 3H). HRMS: calculated for [C₂₅H₂₄Cl₂N₄O₃S + H]⁺= 531.10189, found: 531.10185.

### Example 97: (±) 2-((2-Chloro-4-(4-(3-chlorophenyl)-trans-2,3-dimethylpiperazine-1-carbonyl)phenyl)sulfinyl)-1-(2,4-difluorophenyl)ethan-1-one

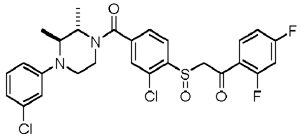

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl 2,4-difluorobenzoate (162 mg, 1.175 mmol, 10 eq) according to general procedure I. This yielded the product (29.7 mg, 0.053 mmol, 45%). 1HNMR (400 MHz, Chloroform-d) δ 8.07 - 8.03 (m, 1H), 8.04 - 8.00 (m, 1H), 7.57 (dd, J = 8.0, 1.5 Hz, 1H), 7.52 (d, J = 1.4 Hz, 1H), 7.21 (t, J = 8.1 Hz, 1H), 7.08 - 7.02 (ddd, 1H), 6.92 (ddd, J = 11.1, 8.5, 2.3 Hz, 1H), 6.86 (d, J = 8.0 Hz, 2H), 6.76 (d, J = 9.1 Hz, 1H), 4.69 (dt, J = 15.5, 2.7 Hz, 1H), 4.38 (dt, J = 15.5, 2.5 Hz, 1H), 4.17 - 2.55 (br, 6H), 1.54 (d, J = 6.7 Hz, 3H), 1.11 (br, 3H). HRMS: calculated for [C₂₇H₂₄Cl₂F₂N₂O₃S+ H]⁺= 565.09255, found: 565.09231.

### Example 98: (±) 2-((2-Chloro-4-(4-(3-chlorophenyl)-trans-2,3-dimethylpiperazine-1-carbonyl)phenyl)sulfinyl)-1-(pyridin-3-yl)ethan-1-one

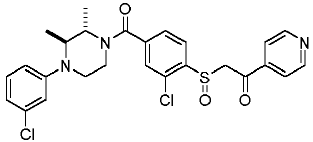

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and methyl pyridine-4-carboxylate (160 mg, 1.175 mmol, 10 eq) according to general procedure I. This yielded the product (1.5mg, 0.003, 3%). LC/MS: calculated for [C26H25ClN3O3S + H] = 530.11, found = 530.33.

### Example 99: (±) 4-(2-((2-Chloro-4-(4-(3-chlorophenyl)-trans-2,3-dimethyl-piperazine-1-carbonyl)phenyl)sulfinyl)acetyl)benzonitrile

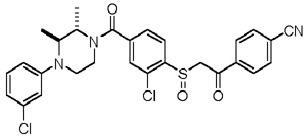

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl 4-cyanobenzoate (206 mg, 1.175 mmol, 10 eq) according to general procedure I. This yielded the product (12 mg, 0.022 mmol, 18%). 1H NMR (500 MHz, Chloroform-d) δ 8.05 (d, J = 8.0 Hz, 2H), 7.92 (d, J = 8.1 Hz, 1H), 7.82 (d, J = 7.6 Hz, 2H), 7.53 (d, J = 8.8 Hz, 2H), 7.20 (t, J = 8.1 Hz, 1H), 6.85 (d, J = 8.3 Hz, 2H), 6.76 (d, J = 8.5 Hz, 1H), 4.69 (dd, J = 14.0, 3.4 Hz, 1H), 4.36 (dd, J = 14.1, 2.9 Hz, 1H), 3.83-3.17 (br, 6H), 1.53 (d, J = 6.7 Hz, 3H), 1.09 (br, 3H). HRMS: calculated for [C₂₇H₂₄Cl₂F₂N₂O₃S+ H]⁺ = 554.10664, found: 554.10655.

### Example 100: (±) 2-((2-Chloro-4-(4-(3-chlorophenyl)-trans-2,3-dimethylpiperazine-1-carbonyl)phenyl)sulfinyl)-1-(pyrimidin-4-yl)ethan-1-one

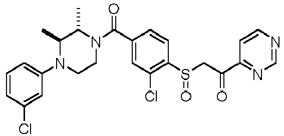

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl pyrimidine-4-carboxylate (179 mg, 1.175 mmol, 10 eq) according to general procedure I. This yielded the product (3 mg, 0.0056 mmol, 5%). 1H NMR (500 MHz, Chloroform-d) δ 9.36 (s, 1H), 9.04 (d, J = 6.8 Hz, 1H), 7.99 - 7.93 (m, 2H), 7.54 - 7.48 (m, 2H), 7.22 (t, J = 8.4 Hz, 1H), 6.88 (d, J = 7.0 Hz, 2H), 6.80 (d, J = 7.9 Hz, 1H), 4.91 (dd, J = 13.8, 3.5 Hz, 1H), 4.77 (d, J = 13.8 Hz, 1H), 3.85-3.15 (br, 6H), 1.53 (dd, J = 6.8, 3.3 Hz, 3H), 1.10 (br, 3H). HRMS: calculated for [C₂₅H₂₄Cl₂N₄O₃S⁺ H]⁺= 531.10189, found: 531.10178.

### Example 101: (±) 2-((2-Chloro-4-(4-(3-chlorophenyl)-trans-2,3-dimethylpiperazine-1-carbonyl)phenyl)sulfinyl)-1-(pyrazin-2-yl)ethan-1-one

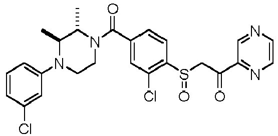

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl pyrazine-2-carboxylate (179 mg, 1.175 mmol, 10.00 eq) according to general procedure I. This yielded the product (27 mg, 0.051 mmol, 43%). 1H NMR (500 MHz, Chloroform-d) δ 9.25 (s, 1H), 8.81 (s, 1H), 8.66 (s, 1H), 7.96 (d, J = 7.5 Hz, 1H), 7.50 (d, J = 9.7 Hz, 2H), 7.19 (t, J = 8.1 Hz, 1H), 6.87 - 6.81 (m, 2H), 6.74 (d, J = 8.2 Hz, 1H), 4.92 (d, J = 14.0 Hz, 1H), 4.77 (d, J = 14.0 Hz, 1H), 3.94 - 3.05 (br, 6H), 1.52 (d, J = 6.7 Hz, 3H), 1.08 (br, 3H). HRMS: calculated for [C₂₅H₂₄Cl₂N₄O₃S+H]⁺ = 531.10189, found: 531.10188.

### Example 102: (±) 2-((2-Chloro-4-(4-(3-chlorophenyl)-trans-2,3-dimethylpiperazine-1-carbonyl)phenyl)sulfinyl)-1-(pyrimidin-2-yl)ethan-1-one

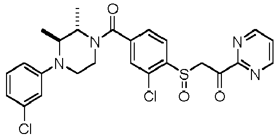

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl pyrimidine-2-carboxylate (179 mg, 1.175 mmol, 10 eq) according to general procedure I. This yielded the product (30 mg, 0.056 mmol, 47 %). 1H NMR (500 MHz, Chloroform-d) δ 8.97 (d, J = 4.9 Hz, 2H), 7.96 (d, J = 7.9 Hz, 1H), 7.55 (t, J = 4.9 Hz, 1H), 7.52 - 7.45 (t, 2H), 7.18 (t, J = 8.1 Hz, 1H), 6.83 (d, J = 12.3 Hz, 2H), 6.73 (d, J = 10.1 Hz, 1H), 5.01 (d, J = 13.9 Hz, 1H), 4.79 (d, J = 13.9 Hz, 1H), 4.03-3.07 (br, 6H), 1.51 (d, J = 6.6 Hz, 3H), 1.06 (br, 3H). HRMS: calculated for [C₂₅H₂₄Cl₂N₄O₃S+H]⁺= 531.10189, found: 531.10170.

### Example 103: (±) 2-((2-Chloro-4-(4-(3-chlorophenyl)-trans-2,3-dimethylpiperazine-1-carbonyl)phenyl)sulfinyl)-1-(4-(trifluoromethyl)phenyl)ethan-1-one

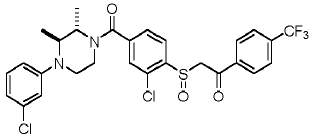

The title compound was synthesized using (±) (3-chloro-4-(methylsulfinyl)phenyl)(trans-4-(3-chlorophenyl)-2,3-dimethylpiperazin-1-yl)methanone (50 mg, 0.12 mmol, 1 eq.) and ethyl 4-(trifluoromethyl)benzoate (256 mg, 1.175 mmol, 10.00 eq) according to general procedure I. This yielded the product (16.2 mg, 0.027 mmol, 23%). 1H NMR (500 MHz, Chloroform-d) δ 8.05 (d, J = 8.6 Hz, 2H), 7.96 (d, J = 8.0 Hz, 1H), 7.77 (d, J = 8.6 Hz, 2H), 7.53 (dd, J = 8.0, 1.4 Hz, 1H), 7.51 (d, J = 1.3 Hz, 1H), 7.18 (t, J = 8.1 Hz, 1H), 6.84 (d, J = 7.8 Hz, 1H), 6.81 (t, J = 2.0 Hz, 1H), 6.73 (d, J = 8.4 Hz, 1H), 4.70 (dd, J = 14.4, 3.8 Hz, 1H), 4.40 (dd, J = 14.4, 3.3 Hz, 1H), 3.89-3.15 (br, 6H), 1.52 (d, J = 6.6 Hz, 3H), 1.05 (br, 3H). HRMS: calculated for [C₂₅H₂₄Cl₂N₄O₃S+H]⁺= 597.09878, found: 597.09841.

### Example 104: (±) 2-((2-Chloro-4-(4-(3-chlorophenyl)-trans-2,3-dimethylpiperazine-1-carbonyl)phenyl)sulfinyl)-1-phenylethan-1-one

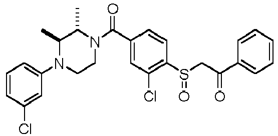

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and ethyl benzoate (177 mg, 1.175 mmol, 10 eq) according to general procedure I. This yielded the product (38.5 mg, 0.073 mmol, 62%). 1H NMR (500 MHz, Chloroform-d) δ 8.00 (d, J = 8.0 Hz, 1H), 7.93 (d, J = 7.2 Hz, 2H), 7.64 (t, J = 7.4 Hz, 1H), 7.52 (d, J = 10.6 Hz, 2H), 7.49 (d, J = 7.5 Hz, 2H), 7.19 (t, J = 8.1 Hz, 1H), 6.83 (d, J = 7.8 Hz, 1H), 6.82 (d, J = 2.1 Hz, 1H), 6.73 (d, J = 10.1 Hz, 1H), 4.66 (dd, J = 14.7, 3.6 Hz, 1H), 4.42 (dd, J = 14.7, 2.7 Hz, 1H), 3.96 - 3.07 (br, 6H), 1.51 (d, J = 6.5 Hz, 3H), 1.06 (br, 3H). HRMS: calculated for [C₂₇H₂₆Cl₂N₂O₃S+H]⁺= 529.11140, found: 529.11116.

### Example 105: (±) 6-(2-((2-Chloro-4-(4-(3-chlorophenyl)-trans-2,3-dimethylpiperazine-1-carbonyl)phenyl)sulfinyl)acetyl)nicotinonitrile

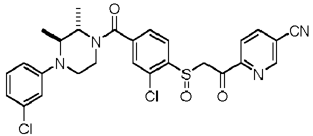

The title compound was synthesized using (±) (3-chloro-4-(methylsulfinyl)phenyl)(trans-4-(3-chlorophenyl)-2,3-dimethylpiperazin-1-yl)methanone ( 50 mg, 0.12 mmol, 1 eq.) and methyl 5-cyanopicolinate (191 mg, 1.175 mmol, 10 eq) according to general procedure I. This yielded the product (31.4 mg, 0.057 mmol, 48%). 1HNMR (600 MHz, Chloroform-d) δ 8.91 (s, 1H), 8.17 (s, 2H), 7.96 (d, J = 8.0 Hz, 1H), 7.51 (d, J = 7.2 Hz, 1H), 7.46 (d, J = 21.8 Hz, 1H), 7.18 (t, J = 8.0 Hz, 1H), 6.82 (d, J = 8.2 Hz, 2H), 6.72 (s, 1H), 4.93 (s, 1H), 4.73 (s, 1H), 3.92 - 3.08 (br, 6H), 1.50 (d, J = 11.6 Hz, 3H), 1.08 (br, 3H). HRMS: calculated for [C₂₇H₂₄Cl₂N₄O₃S+H]⁺= 555.10189, found: 555.10218.

### Example 106: Biological Data

The MAGL activity assay is based on the production of glycerol from 2-arachidonoylglycerol (2-AG) hydrolysis by MAGL-overexpressing membrane preparations from transiently transfected HEK293T cells, as previously reported in van der Wel T et al., "A natural substrate-based fluorescence assay for inhibitor screening on diacylglycerol lipase", J Lipid Res., 2015, 56(4), 927-35. The produced glycerol is coupled to the oxidation of commercially available Amplifu™Red (Sigma-Aldrich Chemie N.V., Zwijndrecht, Netherlands) via a multi-enzyme cascade, resulting in a fluorescent signal from the dye resorufin.

Standard assays were performed in HEMNB buffer (50 mM HEPES pH 7.4, 1 mM EDTA, 5 mM MgCl2, 100 mM NaCl, 0.5% (w/v) BSA) in black, flat bottom 96-wells plates. Final protein concentration of membrane preparations from overexpressing hMAGL HEK293T cells was 1.5 µg/mL (0.3 µg per well). Inhibitors were added from 40x concentrated DMSO stocks. After 20 min. incubation, 100 µL assay mix containing glycerol kinase (GK), glycerol-3-phosphate oxidase (GPO), horse radish peroxidase (HRP), adenosine triphosphate (ATP), Amplifu™Red and 2-arachidonoylglycerol (2-AG) was added and fluorescence was measured in 5 min. intervals for 60 min. on a plate reader. Final assay concentrations: 0.2 U/mL GK, GPO and HRP, 0.125 mM ATP, 10 µM Amplifu™Red, 25 µM 2-AG, 5% DMSO, 0.5% ACN in a total volume of 200 µL. All measurements were performed in N = 2, n = 2 or N = 2, n = 4 for controls, with Z' ≥ 0.6.

For IC₅₀ determination, the MAGL-overexpressing membranes were incubated with different inhibitor concentrations. Slopes of corrected fluorescence in time were determined in the linear interval of t = 10 to t = 35 min and then scaled to the corrected positive control of hMAGL-overexpressing membranes treated with vehicle (DMSO) as a 100% activity reference point. The data was exported to GraphPad Prism 5.0 and analysed in a non-linear dose-response analysis with variable slope. The plC₅₀ data is provided in Table 1 below.

**Table 1**

| **Example** | **pIC₅₀** | **Standard Deviation** |
|---|---|---|
| 2 | 6.57 | 0.13 |
| 3 | 6.71 | 0.05 |
| 4 | 5.65 | 0.05 |
| 5 | <5 | |
| 6 | <5 | |
| 7 | 5.25 | 0.05 |
| 8 | 5.91 | 0.11 |
| 9 | 6.20 | 0.18 |
| 10 | 6.33 | 0.08 |
| 11 | 6.00 | 0.08 |
| 12 | 5.37 | 0.07 |
| 13 | 6.44 | 0.06 |
| 14 | 5.95 | 0.08 |
| 15 | 6.42 | 0.09 |
| 16 | 6.23 | 0.13 |
| 17 | 5.18 | 0.07 |
| 18 | 5.31 | 0.04 |
| 19 | 5.59 | 0.06 |
| 20 | 5.87 | 0.08 |
| 21 | 6.31 | 0.04 |
| 22 | 5.59 | 0.11 |
| 23 | 5.71 | 0.13 |
| 24 | 5.74 | 0.13 |
| 25 | 6.81 | 0.03 |
| 26 | 7.40 | 0.11 |
| 27 | 7.36 | 0.08 |
| 28 | 7.06 | 0.07 |
| 29 | 7.09 | 0.06 |
| 30 | 6.94 | 0.04 |
| 31 | 6.70 | 0.08 |
| 32 | 7.11 | 0.11 |
| 33 | <5 | |
| 34 | 6.96 | 0.06 |
| 35 | 7.13 | 0.09 |
| 36 | 7.54 | 0.09 |
| 37 | 7.58 | 0.09 |
| 38 | 7.41 | 0.10 |
| 39 | 6.22 | 0.12 |
| 40 | 6.60 | 0.14 |
| 41 | 7.46 | 0.11 |
| 42 | 6.68 | 0.07 |
| 43 | 7.56 | 0.04 |
| 44 | 7.56 | 0.10 |
| 45 | 7.29 | 0.07 |
| 46 | 8.13 | 0.07 |
| 47 | 7.33 | 0.07 |
| 48 | 7.24 | 0.07 |
| 49 | 8.50 | 0.10 |
| 50 | 8.24 | 0.17 |
| 51 | 6.18 | 0.06 |
| 52 | <5 | |
| 53 | 6.47 | 0.07 |
| 54 | 8.21 | 0.11 |
| 55 | 5.94 | 0.08 |
| 56 | 8.57 | 0.12 |
| 57 | 8.41 | 0.08 |
| 58 | 8.38 | 0.09 |
| 59 | 8.41 | 0.08 |
| 60 | 8.53 | 0.08 |
| 61 | 8.18 | 0.09 |
| 62 | 7.86 | 0.12 |
| 63 | 8.35 | 0.08 |
| 64 | 8.21 | 0.11 |
| 65 | 8.04 | 0.10 |
| 66 | 7.33 | 0.10 |
| 67 | 7.89 | 0.17 |
| 68 | 7.68 | 0.14 |
| 69 | 7.06 | 0.13 |
| 70 | 8.04 | 0.11 |
| 71 | 8.06 | 0.12 |
| 72 | 7.68 | 0.13 |
| 73 | 8.13 | 0.08 |
| 74 | 6.69 | 0.15 |
| 75 | 6.63 | 0.10 |
| 76 | 6.42 | 0.07 |
| 77 | 8.63 | 0.06 |
| 78 | 8.83 | 0.05 |
| 79 | 9.30 | 0.04 |
| 80 | 7.06 | 0.21 |
| 81 | 9.07 | 0.07 |
| 82 | 9.91 | 0.06 |
| 83 | 8.47 | 0.10 |
| 84 | 8.55 | 0.09 |
| 85 | 5.01 | 0.05 |
| 86 | 5.72 | 0.04 |
| 87 | <5 | |
| 88 | 6.60 | 0.07 |
| 89 | 5.68 | 0.23 |
| 90 | 6.09 | 0.20 |
| 91 | <5 | |
| 92 | <5 | |
| 93 | 6.24 | 0.20 |
| 94 | 6.12 | 0.21 |
| 95 | 5.79 | 0.22 |
| 96 | 6.15 | 0.05 |
| 97 | 6.63 | 0.07 |
| 98 | 6.73 | 0.08 |
| 99 | 7.04 | 0.06 |
| 100 | 7.06 | 0.16 |
| 101 | 5.96 | 0.12 |
| 102 | 7.55 | 0.14 |
| 103 | 5.53 | 0.10 |
| 104 | 5.64 | 0.14 |
| 105 | 8.00 | 0.10 |

## Claims

1. A compound of formula (I), or pharmaceutically acceptable salts or solvates thereof: 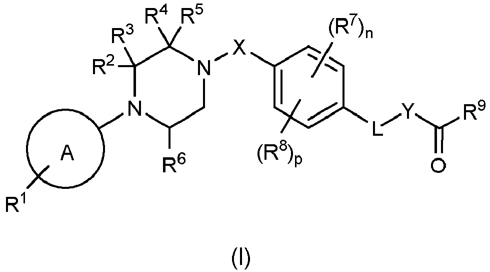 wherein
A is a 6-membered aromatic ring, or a 5- or 6-membered heteroaromatic ring optionally comprising at least one N atom;
X is selected from -C(O)- and -C(R^{a}R^{b})- ;
L is selected from S(=O)-, -SO₂-, -S-, -O-, -C(O)-, -CH₂-, -C(R^{c}R^{d})-;
Y is -CH₂-, -CF₂-, -C(R^{c}R^{d})-;
R¹ is selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl;
each R² and R⁴ is independently selected from H and C₁₋₄ alkyl;
each R³, R⁵ and R⁶ is independently selected from H and C₁₋₄ alkyl; or R³ and R⁵ together form a 5- or 6-membered ring and R⁶ is selected from H and C₁₋₄ alkyl; or R³ and R⁶ together form an alkyl or heteroalkyl bridge and R⁵ is selected from H and C₁₋₄ alkyl;
each R⁷ and R⁸ is independently selected from H, halo, C₁₋₄ alkyl, C₁₋₄ haloalkyl, C₁₋₄ alkoxy, CN and NO₂;
R⁹ is selected from H, C₁₋₆ haloalkyl, OR¹⁰, C₁₋₆ alkyl, C₂₋₈ alkyl interrupted by 1, 2 or 3 ether linkages, C₃₋₈ cycloalkyl, C₆ aryl, -(C₂₋₄ haloalkyl)-OR¹¹, -(C₁₋₆ haloalkyl)-R¹¹, ,or heterocyclyl;
R¹⁰ is selected from H, C₁₋₆ alkyl, C₃₋₈ cycloalkyl, -(C₁₋₄ alkyl)_{d}-aryl, -(C₁₋₄ alkyl)ₑ-heterocyclyl;
R¹¹ is selected from C₁₋₆ alkyl, C₂₋₈ alkyl interrupted by 1, 2 or 3 ether linkages, C₃₋₈ cycloalkyl, C₆ aryl, or heterocyclyl;
wherein each R⁹, R¹⁰ and R¹¹ is optionally substituted where chemically possible with one, two or three groups independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl;
R^{a} and R^{b} are independently selected from H, and C₁₋₄ alkyl; or wherein R^{a} and R^{b} together form a 3- to 6-membered cycloalkyl or heterocycloalkyl ring system;
R^{c} and R^{d} are independently selected at each occurrence from H, F, C₁₋₄ alkyl, and C₁₋₄ haloalkyl; or R^{c} and R^{d} together form a 3- to 6-membered cycloalkyl or heterocycloalkyl ring system;
a, b, d and e are independently selected from 0, 1 and 2;
n is selected from 0, 1, or 2; and
p is selected from 0, 1, or 2.

2. The compound of claim 1, wherein the compound is a compound of formula (II) or a pharmaceutically acceptable salt or solvate thereof: 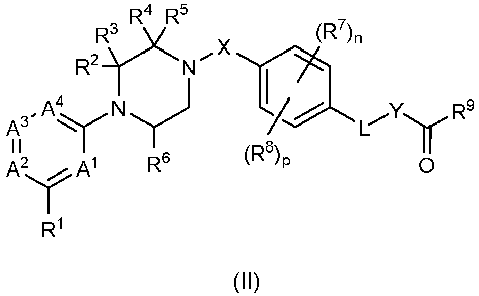
wherein A¹, A³ and A⁴ are each independently selected from CH, CR¹² or N, and A² is selected from CH or CR¹²; and
R¹² is selected from H, halo, CN, OCF₃, C₁₋₄ alkyl, C₁₋₄ haloalkyl, and C₁₋₄ alkoxy.

3. The compound of claim 2, wherein any one, two or three of A¹, A³ and A⁴ are N; and/or
wherein one of A¹ and A⁴ is N, one of A¹ and A⁴ is CH or CR¹², and A³ is CH or CR¹²

4. The compound of claim 2, wherein each of A¹, A³ and A⁴ is independently selected from CH or CR¹².

5. The compound of any preceding claim, wherein the compound is a compound of any of formulae (III), (IVa), (IVb), (IVc), or (V), or pharmaceutically acceptable salt or solvate thereof: 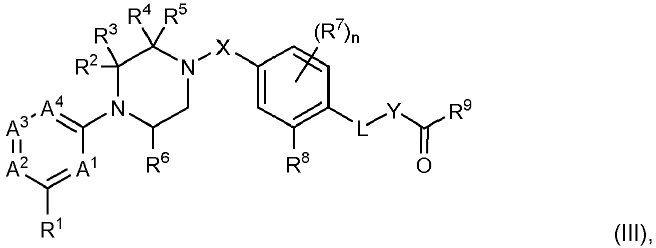 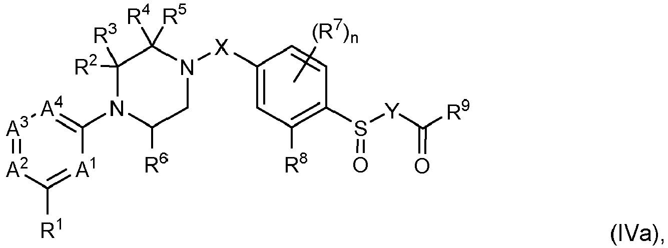 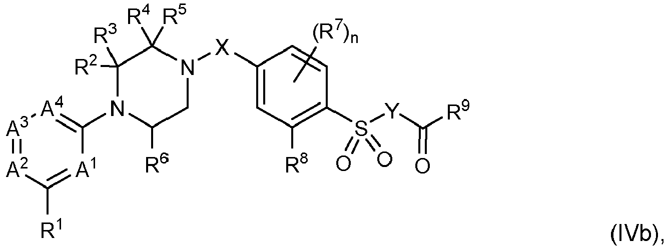 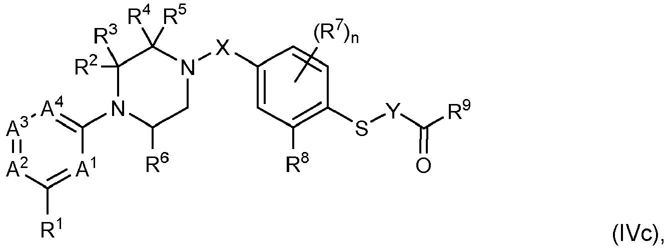 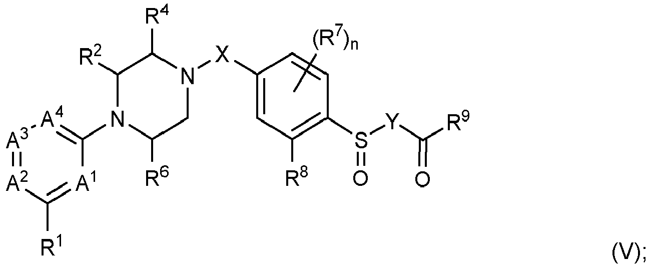 optionally wherein R² and R⁴ are arranged *trans* to one another.

6. The compound any preceding claim, wherein X is -C(O)- or -CH₂-;
optionally wherein X is -C(O)-.

7. The compound of any preceding claim, wherein Y is -CH₂-, -CH(CH₃)-, 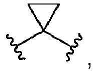 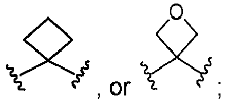 optionally wherein Y is -CH₂-.

8. The compound of any preceding clause, wherein R⁹ is selected from C₁₋₄ haloalkyl, C₂₋₆ alkyl interrupted by 1, 2 or 3 ether linkages, OR¹⁰, -(C₂₋₄ haloalkyl)-OR¹¹, (C₁₋₆ haloalkyl)-R¹¹, substituted or unsubstituted 6-membered aryl, and substituted or unsubstituted 5- or 6-membered heterocyclyl,
wherein R¹⁰ is selected from C₁₋₄ alkyl, C₃₋₈ cycloalkyl or -(C₁₋₄ alkyl)-aryl,
wherein R¹¹ is selected from C₁₋₄ alkyl, C₂₋₆ alkyl interrupted by 1, 2 or 3 ether linkages, C₃₋₆ cycloalkyl, or C₃₋₆ heterocycloalkyl,
wherein each R⁹, R¹⁰ and R¹¹ is optionally substituted where chemically possible with one, two or three groups each independently selected from H, OH, =O, CN, halo, C₁₋₄ haloalkyl, C₁₋₄ alkoxy, OCF₃, and phenyl.

9. The compound of claim 8, wherein R⁹ is selected from -CF₂CH₃, -CF₂CH₂CH₃,-CF₃, -CHF₂, -CF₂-phenyl, -OCH₂CH₃, -OCH(CH₃)₂, -OCH(CH₃)CH₂CH₃, ,-OCH₂CH(OH)CH₂OH, -OCH₂CH₂OCH₃, -OCH(CH₃)CH₂OCH₃, -OCH₂CH₂CH₂CH₃,-OCH₂CH₂CH₂OH, -OCH₂CH₂OCH₂CH₂OCH₂CH₃, -OCH₂CF₃, -OCH₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, 4-cyanophenyl,. 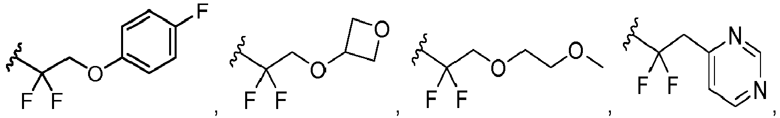 and 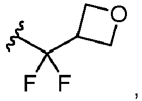 optionally wherein R⁹ is selected from -CF₂CH₃, -CF₂CH₂CH₃, -CF₃, -CHF₂ ,-CF₂-phenyl, -OCH₂CH₃, -OCH(CH₃)₂, -OCH(CH₃)CH₂CH₃, , -OCH₂CH(OH)CH₂OH,-OCH₂CH₂OCH₃, -OCH(CH₃)CH₂OCH₃, -OCH₂CH₂CH₂CH₃, -OCH₂CH₂CH₂OH,-OCH₂CH₂OCH₂CH₂OCH₂CH₃, -OCH₂CF₃, -OCH₃, -O-cyclobutane, -O-cyclopentane, -O-cyclohexane, O-tetrahydropyran, -OCH₂-3,4-dioxoymethenylphenyl, 3-cyanopyridiny-6-yl, pyrimidin-2-yl, pyrimidin-4-yl, and 4-cyanophenyl.

10. A pharmaceutical composition comprising a compound of any preceding claim;
optionally further comprising a pharmaceutically acceptable excipient.

11. The compound of any of claims 1 to 9 or the pharmaceutical composition of claim 10 for use as a medicament.

12. The compound of any of claims 1 to 9 or the pharmaceutical composition of claim 10 for use in the treatment of a condition which is modulated by monoacylglycerol lipase (MAGL).

13. The compound of any of claims 1 to 9 or the pharmaceutical composition of claim 10 for use in the treatment of a condition selected from acute pain, inflammatory pain, cancer pain, pain caused by peripheral neuropathy, central pain, fibromyalgia, migraine, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

14. The compound for use according to claim 13, wherein the condition is cancer;
optionally wherein the condition is breast cancer, ovarian cancer, melanoma, hepatocellular carcinoma, colorectal cancer, prostate cancer, or nasopharyngeal carcinoma.

15. Use of the compound of claims 1 to 9 or the pharmaceutical composition of claim 10 for the inhibition of monoacylglycerol lipase (MAGL).
